# EUROPEAN PATENT APPLICATION

(11) **EP 4 810 494 A1**
(43) Date of publication of application: **23.09.2026**
(21) Application number: 24890841.0
(22) Date of filing: 15.11.2024
(51) Int. Cl.: C07D 471/04, C07D 487/04, A61K 31/4545, A61P 35/00

(54) **HPK1 DEGRADATION AGENT AND USE THEREOF IN FIELD OF MEDICINE**

(30) Priority: 16.11.2023 CN 202311529920; 29.12.2023 CN 202311848294; 11.01.2024 CN 202410041276; 21.03.2024 CN 202410328229; 25.04.2024 CN 202410506524; 29.05.2024 CN 202410679198; 22.08.2024 CN 202411156605
(71) Applicant: Haisco Pharmaceutical Group Co., Ltd., Shannan, Xizang 856099 (CN)
(72) Inventor: ZHANG, Chen, Chengdu, Sichuan 611130 (CN); WANG, Jianmin, Chengdu, Sichuan 611130 (CN); HUANG, Zhenggang, Chengdu, Sichuan 611130 (CN); HUANG, Longbin, Chengdu, Sichuan 611130 (CN); SHI, Ronghua, Chengdu, Sichuan 611130 (CN); WANG, Ting, Chengdu, Sichuan 611130 (CN); WANG, Hongbin, Chengdu, Sichuan 611130 (CN); TANG, Pingming, Chengdu, Sichuan 611130 (CN); HUANG, Anbang, Chengdu, Sichuan 611130 (CN); LI, Yao, Chengdu, Sichuan 611130 (CN); YAN, Pangke, Chengdu, Sichuan 611130 (CN)
(74) Representative: AWA Denmark A/S
(86) International application number: PCT/CN2024/132466
(87) International publication number: WO 2025/103489

(57) **Abstract**

An HPK1 degradation agent and use thereof in the field of medicine. The invention specifically relates to a compound as shown in a general formula (I) or a stereoisomer, a racemate, a tautomer, a deuterated substance, a solvate, a prodrug, a metabolite, a pharmaceutically acceptable salt or eutectic thereof, and an intermediate thereof, and the use thereof in inhibiting or degrading HPK1-related diseases such as cancer. B-L-K (I)

## Description

### Technical Field

The present invention relates to a compound of general formula (I) or a stereoisomer, a racemate, a tautomer, a deuterated substance, a solvate, a prodrug, a metabolite, a pharmaceutically acceptable salt or a cocrystal thereof, and an intermediate thereof and a preparation method therefor, as well as the use thereof in HPK1-related diseases such as tumours.

### Background Art

Kinases catalyse the phosphorylation of proteins, lipids, sugars, nucleosides and other cellular metabolites, and play key roles in various aspects of physiology in eukaryotic cells. In particular, protein kinases and lipid kinases are involved in controlling the activated signalling events that enable cells to grow, differentiate, and survive in response to extracellular mediators or stimuli such as growth factors, cytokines or chemokines. In general, protein kinases are divided into two classes, one that preferentially phosphorylates tyrosine residues and the other that preferentially phosphorylates serine and/or threonine residues.

Hematopoietic progenitor kinase (HPK) 1 (also known as mitogen-activated protein kinase kinase kinase kinase 1, MAP4K1) is a serine/threonine protein kinase that belongs to the MAP4K family and functions as a negative signalling regulator of the T cell receptor (TCR). TCR activation recruits and activates HPK1, which phosphorylates the Ser376 amino acid residue of the SLP76 protein, thereby destabilising the TCR signalling complex and ultimately inhibiting T cell activation and proliferation. Compared with wild-type mice, HPK1 kinase-deficient mice exhibit superior T cell proliferation activity and anti-tumour immunity under TCR stimulation. Moreover, the HPK1 kinase-deficient mice do not show a lethal inflammatory response. Therefore, HPK1 has become an important therapeutic target and has attracted widespread research and development interest.

PROTAC (proteolysis targeting chimera) molecules are a class of bifunctional compounds that can simultaneously bind targeting proteins and E3 ubiquitin ligases. Such compounds can be recognised by proteasomes of cells, causing the degradation of targeting proteins, and can effectively reduce the content of targeting proteins in the cells. By introducing a ligand capable of binding to various targeting proteins into the PROTAC molecules, it is possible to apply the PROTAC technology to the treatment of various diseases, and this technology has attracted extensive attention in recent years. Compared with inhibitors, PROTAC can not only inhibit the kinase activity of the target, but also regulate its backbone function.

Therefore, it is necessary to develop novel HPK1-targeting PROTAC drugs for the treatment of HPK1-related tumour diseases.

### Summary of the Invention

An objective of the present invention is to provide a compound with a novel structure, good efficacy, high bioavailability and higher safety that can inhibit or degrade HPK1, for use in the treatment of HPK1-related diseases such as cancer.

The compound of the present invention has a good inhibitory effect on the phosphorylation of SLP76 in Jurkat cells, good degradation and inhibitory activity against HPK1 kinase, favourable pharmacokinetic properties (good oral absorption, long half-life, high Cmax, and low clearance in animal pharmacokinetic tests), no obvious inhibitory effect on the hERG potassium ion channel, and a good activating effect on IL-2.

The present invention provides a compound or a stereoisomer, a racemate, a tautomer, a deuterated substance, a solvate, a prodrug, a metabolite, a pharmaceutically acceptable salt or a cocrystal thereof, wherein the compound is a compound as shown in general formula (I),

B-L-K (I);

in some embodiments, the compound as shown in general formula (I) is general formula (Ia), 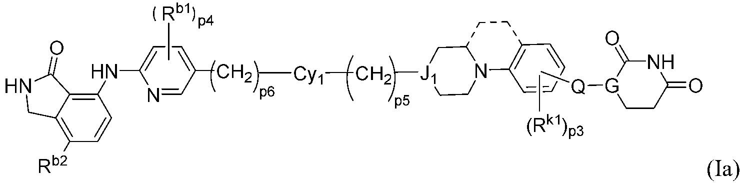
in some embodiments, 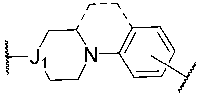 is selected from 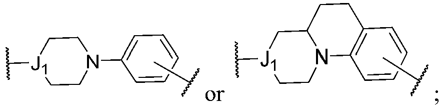
in some embodiments, 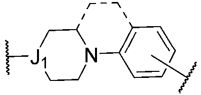 is selected from 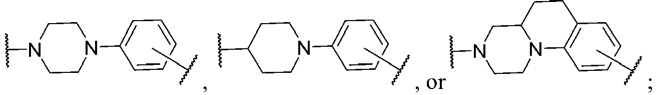
in some embodiments, J₁ is selected from N or CH;
in some embodiments, 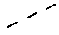 is a single bond 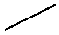 or is absent;
in some embodiments, L is selected from a bond or -C₁₋₅₀ hydrocarbyl-, wherein the hydrocarbyl has 1 to 20 methylene units optionally replaced by -Ak- or -Cy-;
in some embodiments, L is selected from a bond or -C₁₋₂₀ hydrocarbyl-, wherein the hydrocarbyl has 1 to 20 methylene units optionally replaced by -Ak- or -Cy-;
in some embodiments, each -Ak- is independently selected from -(CH₂)_{q}-, - (CH₂)_{q}-O-, -O-(CH₂)_{q}-, -(CH₂)_{q}-S-, -S-(CH₂)_{q}-, -(CH₂)_{q}-NR^{L}-, -NR^{L}-(CH₂)_{q}-, - (CH₂)_{q}-NR^{L}C(=O)-, -NR^{L}(CH₂)_{q}C(=O)-, -(CH₂)_{q}-C(=O)NR^{L}-, -C(=O)-, -C(=O)-(CH₂)_{q}-NR^{L}-, -(C≡C)_{q}-, -CH=CH-, -Si(R^{L})₂-, -Si(OH)(R^{L})-, -Si(OH)₂-, - P(=O)(OR^{L})-, -P(=O)(R^{L})-, -S-, -S(=O)-, -S(=O)₂- or a bond, wherein the CH and - CH₂- are optionally substituted with 1 to 2 R^{z};
in some embodiments, each q is independently selected from 0, 1, 2, 3, 4, 5 or 6;
in some embodiments, R^{L} is selected from H, C₁₋₄ alkyl, C₃₋₇ carbocyclyl, or 4- to 10-membered heterocyclyl, wherein the alkyl, carbocyclyl or heterocyclyl is optionally substituted with 1 to 4 R^{z};
in some embodiments, each -Cy- is independently selected from a bond or one of the following groups optionally substituted with 1 to 4 R^{L2}: 4- to 8-membered mono-heterocyclyl, 4- to 12-membered fused-heterocyclyl, 5- to 13-membered spiro-heterocyclyl, 7- to 12-membered bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl;
in some embodiments, Ak is selected from Ak1, Ak2, Ak3, Ak4, Ak5, Ak6, Ak7, Ak8 or Ak9;
in some embodiments, Ak is selected from Ak1, Ak2, Ak3, Ak4 or Ak5;
in some embodiments, -Cy- is selected from Cy1, Cy2, Cy3, Cy4 or Cy5;
in some embodiments, -Cy- is selected from Cy1, Cy2, Cy3 or Cy4;
in some embodiments, L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, - Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, -Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, - Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, -Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, - Ak1-Ak2-Ak3-Ak4-Cy1-Cy2-Cy3-Ak5-Cy4-, -Akl-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, or -Ak1-Ak2-Ak3-Ak4-Ak5-Ak6-Ak7-Ak8-Ak9-;
in certain embodiments, L is selected from a bond, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Cy1-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, -Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, - Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4- or - Ak1-Cy2-Ak2-Ak3-;
in certain embodiments, L is -Ak1-Cy1-Ak2-Cy2-Ak3-Cy3-Ak4-Cy4-Ak5-;
in certain embodiments, L is selected from a bond, -Akl-, -NHCH₂-, -Cy1-, - Cy1-CH₂-, -Cy1-C≡C-, -Cy1-Cy2-, -Cy1-CH₂-Cy2-, -CH₂-Cy2-, -Cy1-Cy2-Cy3-, - Cy1-CH₂-Cy2-Cy3-, -Cy1-Cy2-CH₂-Cy3-, -NH-Cy1-, -NH-Cy1-Cy2-, -NH-Cy1-CH₂-Cy2, -Cy1-Ak2-, -Ak1-Cy1-, -Ak1-Cy1-Ak2-, -Ak1-Cy2-Ak2-Cy3-, or -Ak1-Cy2-Cy3-;
in certain embodiments, L is selected from a bond, -Akl-, -NHCH₂-, -Cy1-, - Cy1-CH₂-, -Cy1-C≡C-, -Cy1-Cy2-, -Cy1-CH₂-Cy2-, -CH₂-Cy2-, -Cy1-Cy2-Cy3-, - Cy1-CH₂-Cy2-Cy3-, -Cy1-Cy2-CH₂-Cy3-, -NH-Cy1-, -NH-Cy1-Cy2-, -NH-Cy1-CH₂-Cy2, -Cy1-CH₂-, -CH₂-Cy1-, - CH₂-Cyl-CH₂-, -CH₂-Cy2-CH₂-Cy3-, or - CH₂-Cy2-Cy3-;
in certain embodiments, Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-S-, -S-(CH₂)_{q}-, -(CH₂)_{q}-NR^{L}-, -NR^{L}-(CH₂)_{q}-, -(CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the - CH₂- is optionally substituted with 1 to 2 R^{z};
in certain embodiments, Ak1, Ak2, Ak3, Ak4, Ak5, Ak6, Ak7, Ak8 and Ak9 are each independently selected from a bond, -O-, -S-, -OCH₂-, -CH₂O-, - OCH₂CH₂-, -CH₂CH₂O-, -C≡C-, -C(CH₃)₂-, -CH₂-, -CH₂CH₂-, -CH₂CH₂CH₂-, - N(CH₃)-, -NH-, -CH₂N(CH₃)-, -CH₂NH-, -NHCH₂-, -CH₂CH₂N(CH₃)-, - CH₂CH₂NH-, -NHCH₂CH₂-, -C(=O)-, -C(=O)CH₂NH-, -CH₂C(=O)NH-, - C(=O)NH- or -NHC(=O)-;
in certain embodiments, R^{L} is selected from H or C₁₋₄ alkyl;
in certain embodiments, R^{L} is selected from H, methyl or ethyl;
in certain embodiments, each Cy1, Cy2, Cy3, Cy4 or Cy5 is independently selected from a bond or one of the following groups optionally substituted with 1 to 4 R^{L2}: 4- to 7-membered nitrogen-containing mono-heterocyclyl, 4- to 12-membered nitrogen-containing fused-heterocyclyl, 5- to 13-membered nitrogen-containing spiro-heterocyclyl, 7- to 12-membered nitrogen-containing bridged-heterocyclyl, C₃₋₇ monocycloalkyl, C₄₋₇ monocycloalkenyl, C₄₋₁₂ fused cycloalkyl, C₅₋₁₃ spirocycloalkyl, C₇₋₁₂ bridged cycloalkyl, 5- to 10-membered heteroaryl or C₆₋₁₀ aryl;
in certain embodiments, Cy5 is defined the same as Cy1;
in certain embodiments, Cy1, Cy2, Cy3 and Cy4 are each independently selected from a bond or one of the following groups optionally substituted with 1 to 4 R^{L2}: phenyl, pyridyl, pyrimidyl, pyrazinyl, pyridazinyl, pyrazolyl, thiazolyl, oxazolyl, triazolyl, 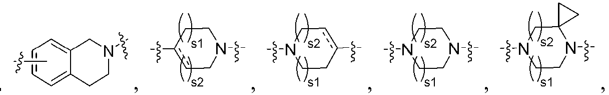 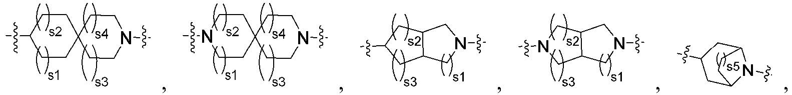 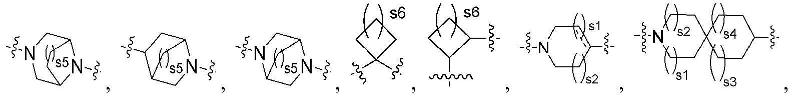 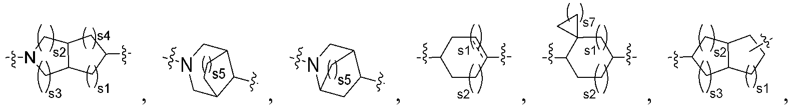 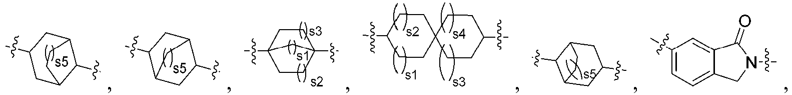 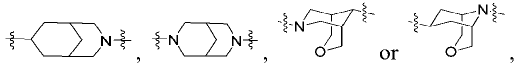 wherein s1, s3 and s5 are each independently selected from 0, 1 or 2; s2 and s4 are each independently selected from 0 or 1; s6 is selected from 0, 1, 2 or 3; and s7 is selected from 1, 2 or 3;
in certain embodiments, Cy1, Cy2, Cy3, Cy4 and Cy5 are each independently selected from a bond or one of the following optionally substituted groups: 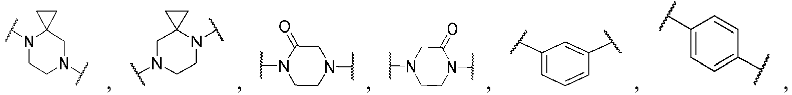 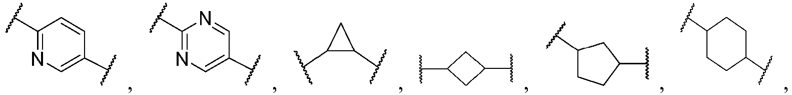 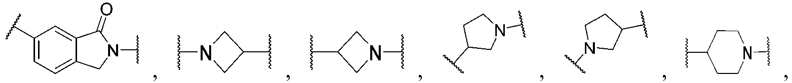 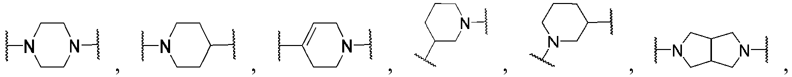 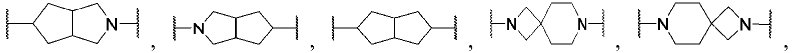 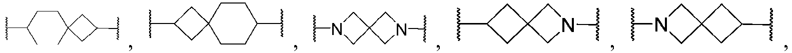 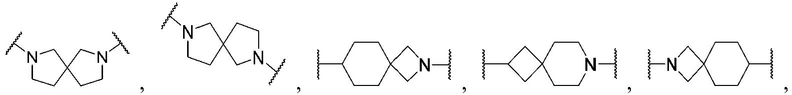 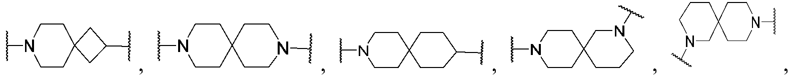 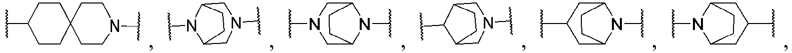 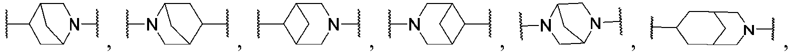 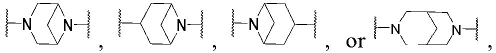 which, when substituted, is substituted with 1 to 4 substituents selected from deuterium, F, CF₃, OH, methyl, =O, hydroxymethyl, methoxy, COOH, CN or NH₂;
in certain embodiments, Cy1, Cy2 and Cy3 are each independently selected from one of the following optionally substituted groups: 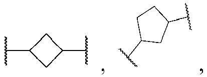 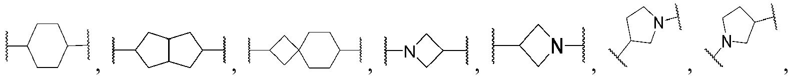 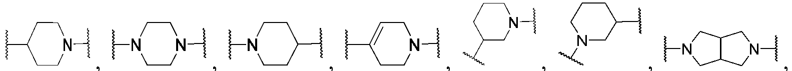 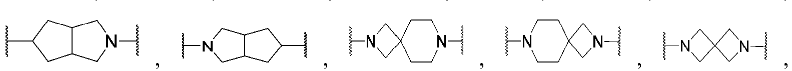 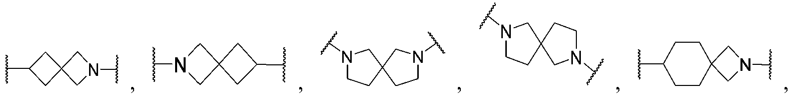 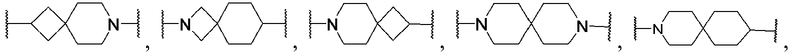 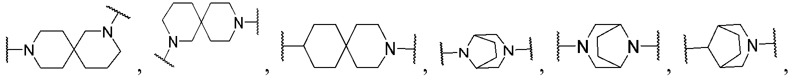 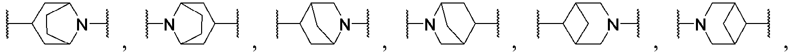 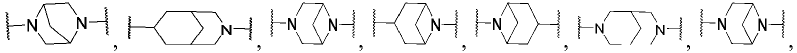 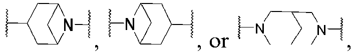 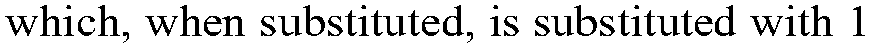 to 4 substituents selected from deuterium, F, CF₃, OH, =O, COOH, CN, NH₂, hydroxymethyl, methyl, methoxy, or cyclopropyl;
in certain embodiments, Cy1 is selected from one of the following optionally substituted groups: 4- to 7-membered nitrogen-containing mono-heterocyclyl, 4- to 12-membered nitrogen-containing fused-heterocyclyl, 5- to 13-membered nitrogen-containing spiro-heterocyclyl, 7- to 12-membered nitrogen-containing bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl, preferably one of the following optionally substituted groups: 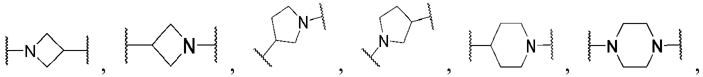 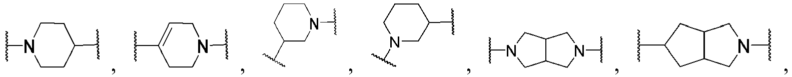 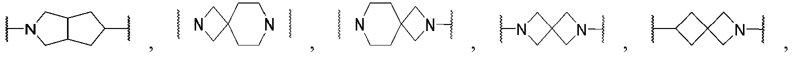 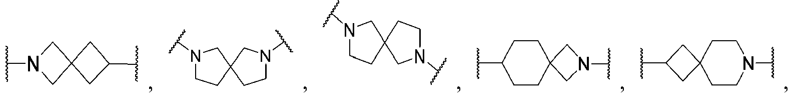 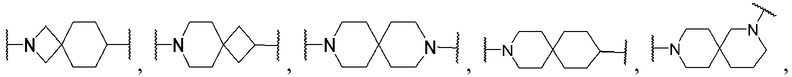 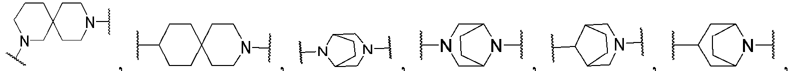 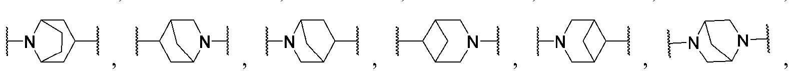 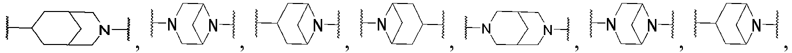 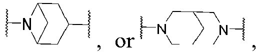 which, when substituted, is substituted with 1 to 4 substituents selected from deuterium, F, CF₃, OH, =O, COOH, CN, NH₂, hydroxymethyl, methyl, methoxy, or cyclopropyl;
in certain embodiments, L is selected from a group shown in Table L-1, wherein the left side of the group is linked to B;
in certain embodiments, L is selected from a bond or a group shown in Table L-1 or Table L-2, wherein the left side of the group is linked to B; 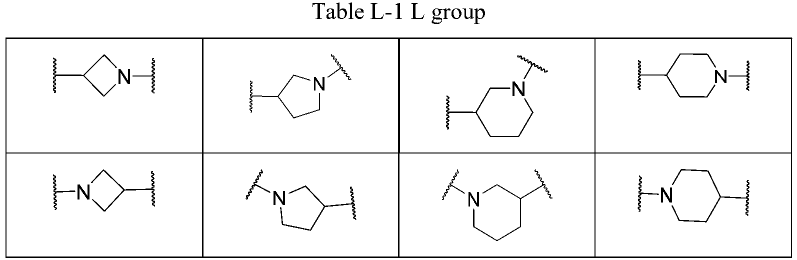 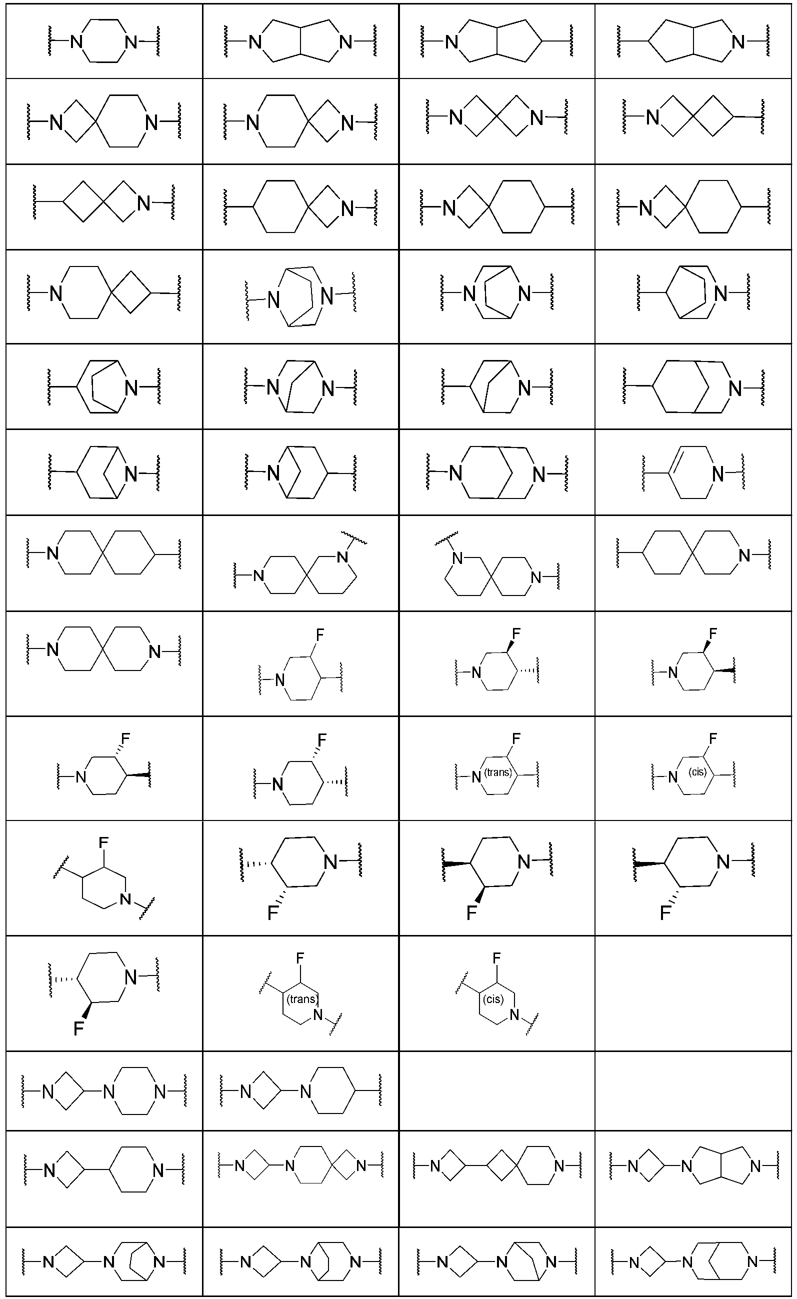 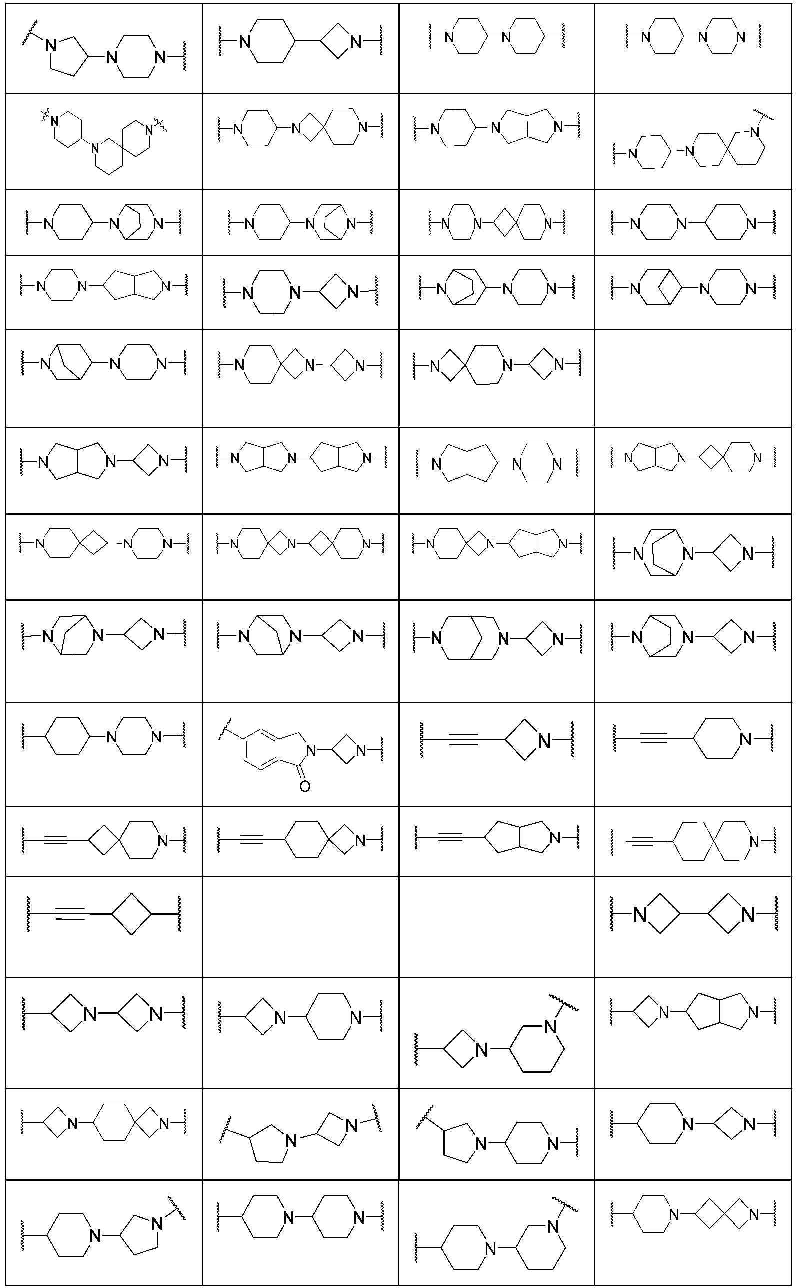 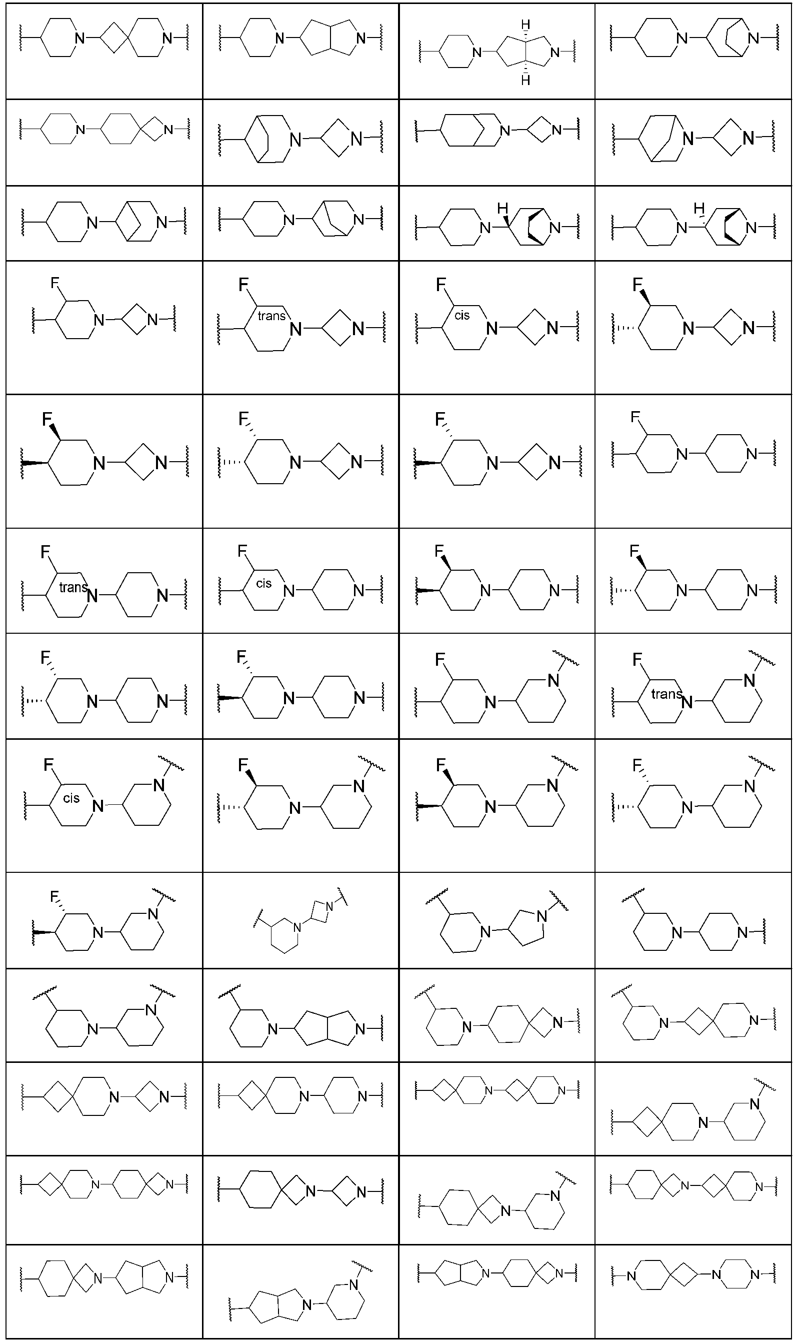 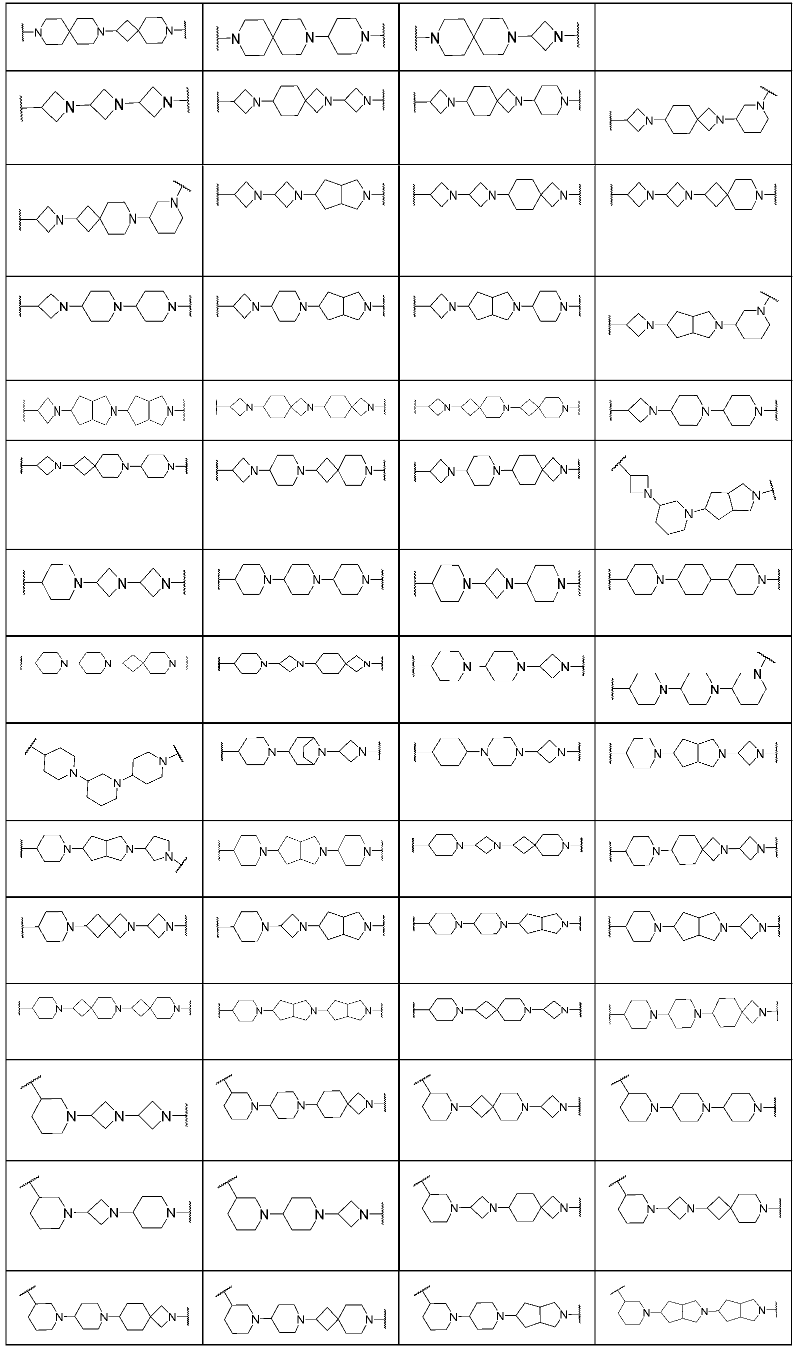 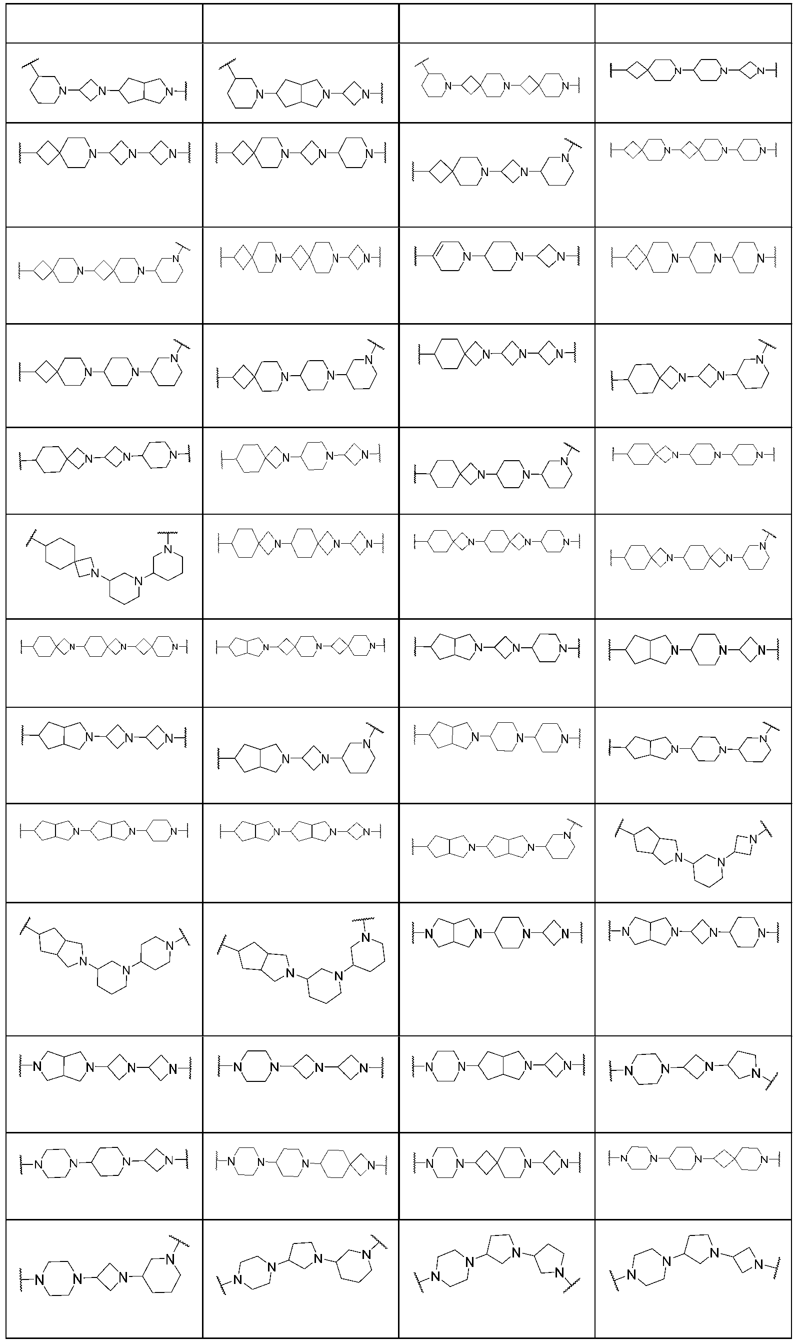 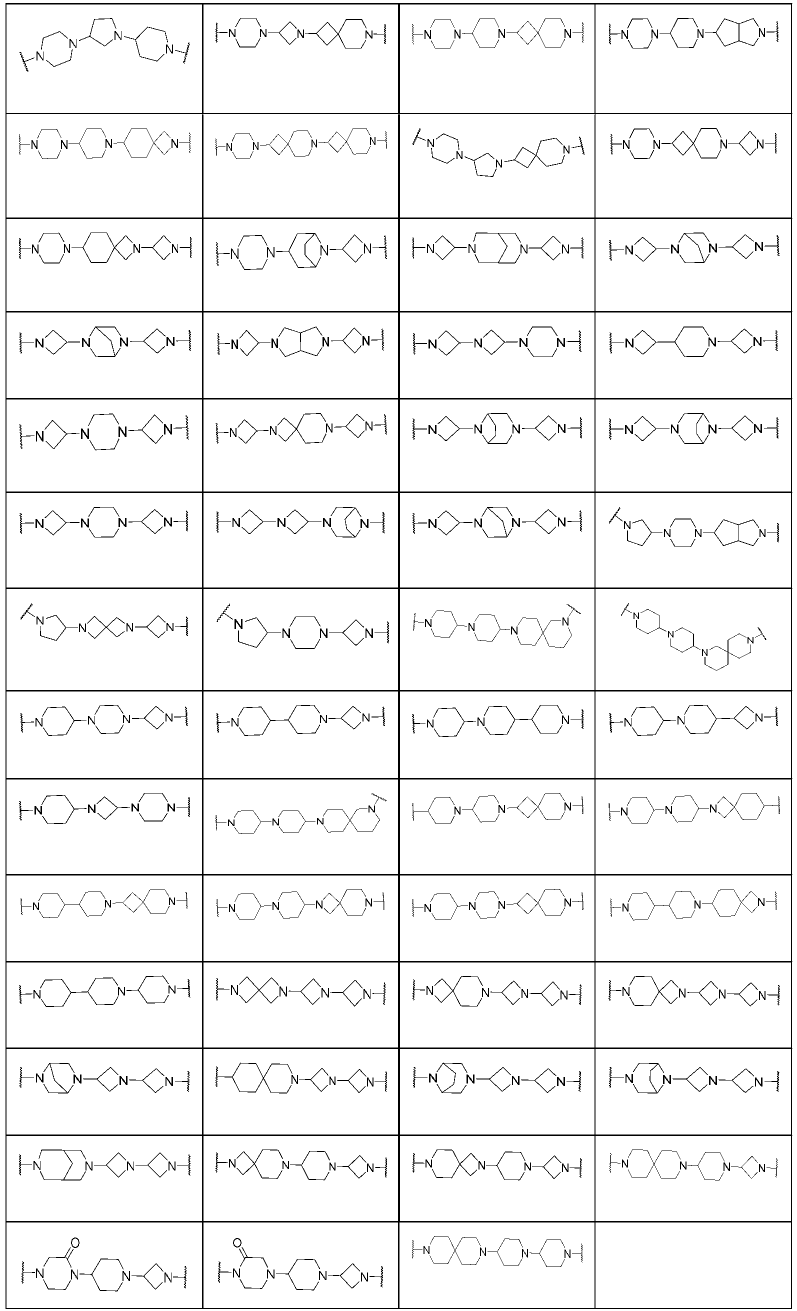 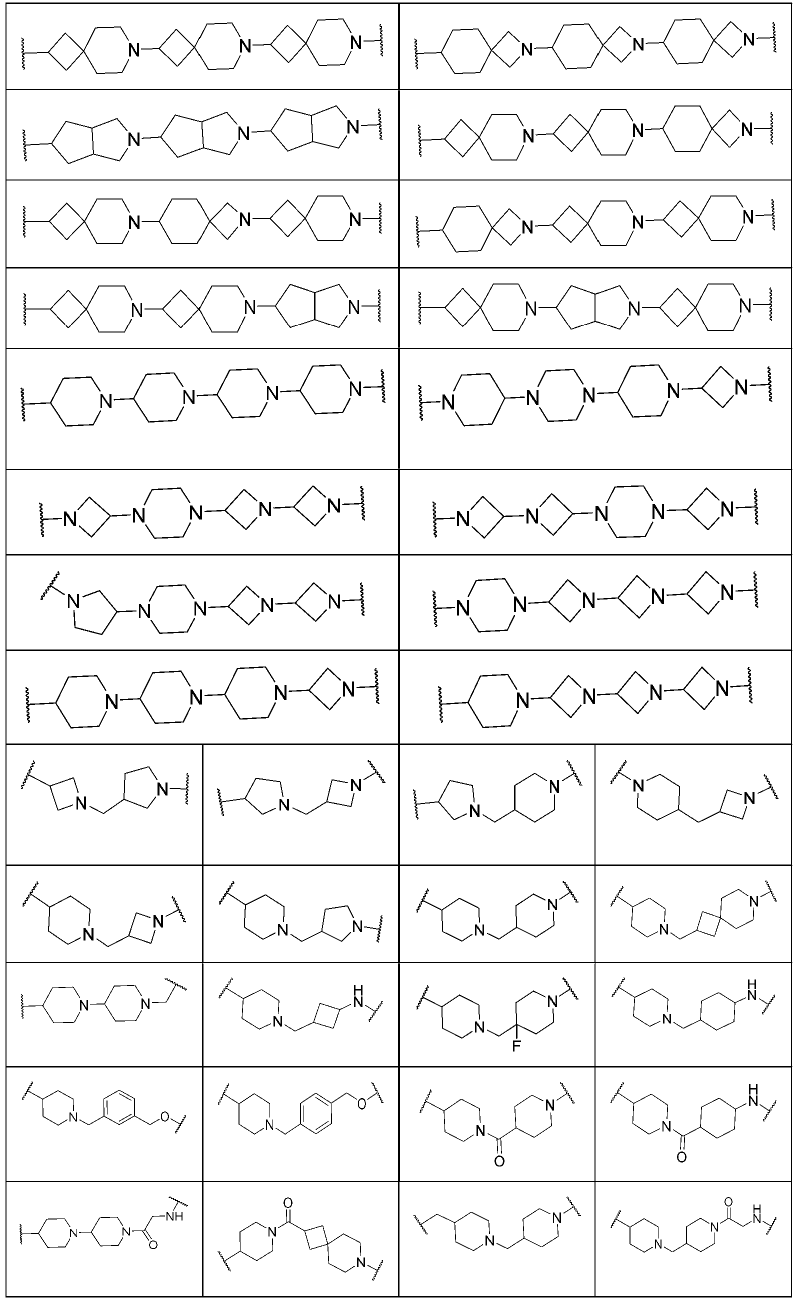 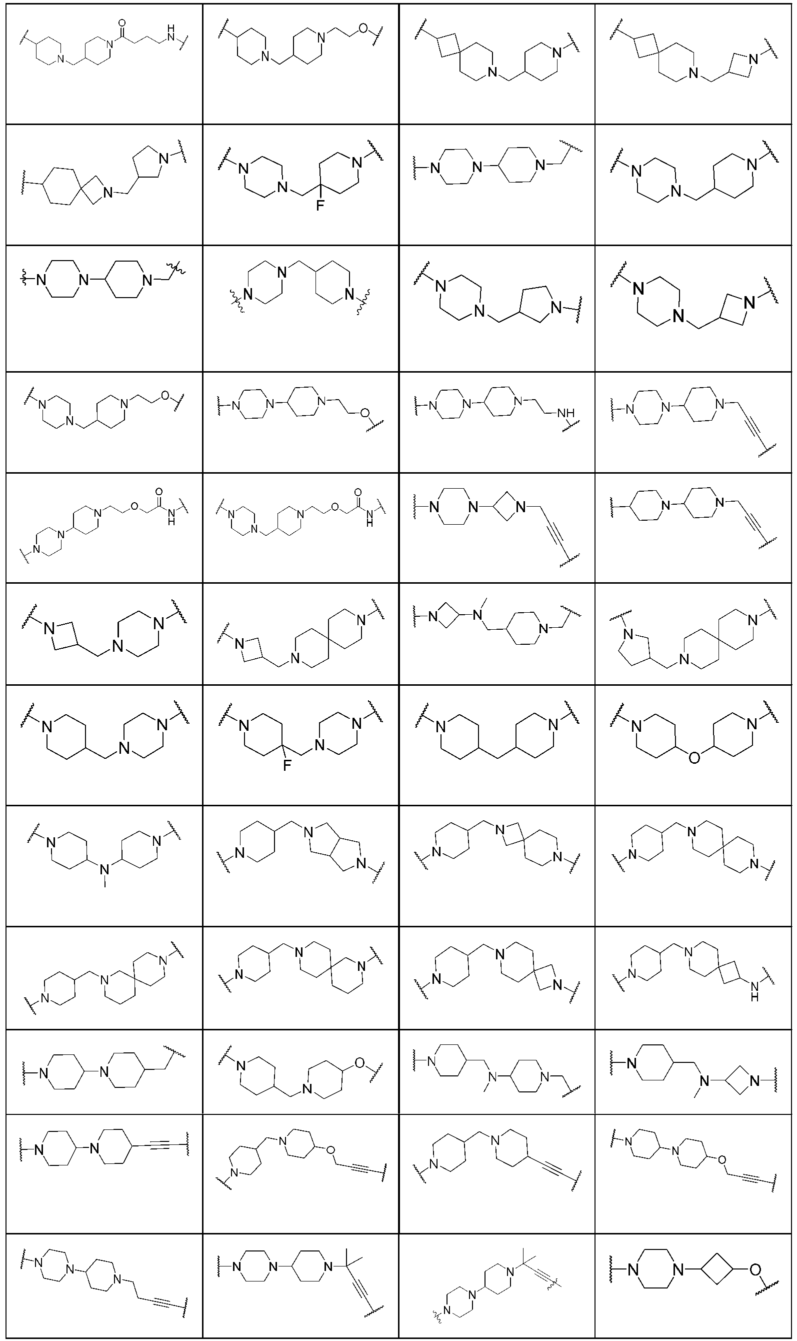 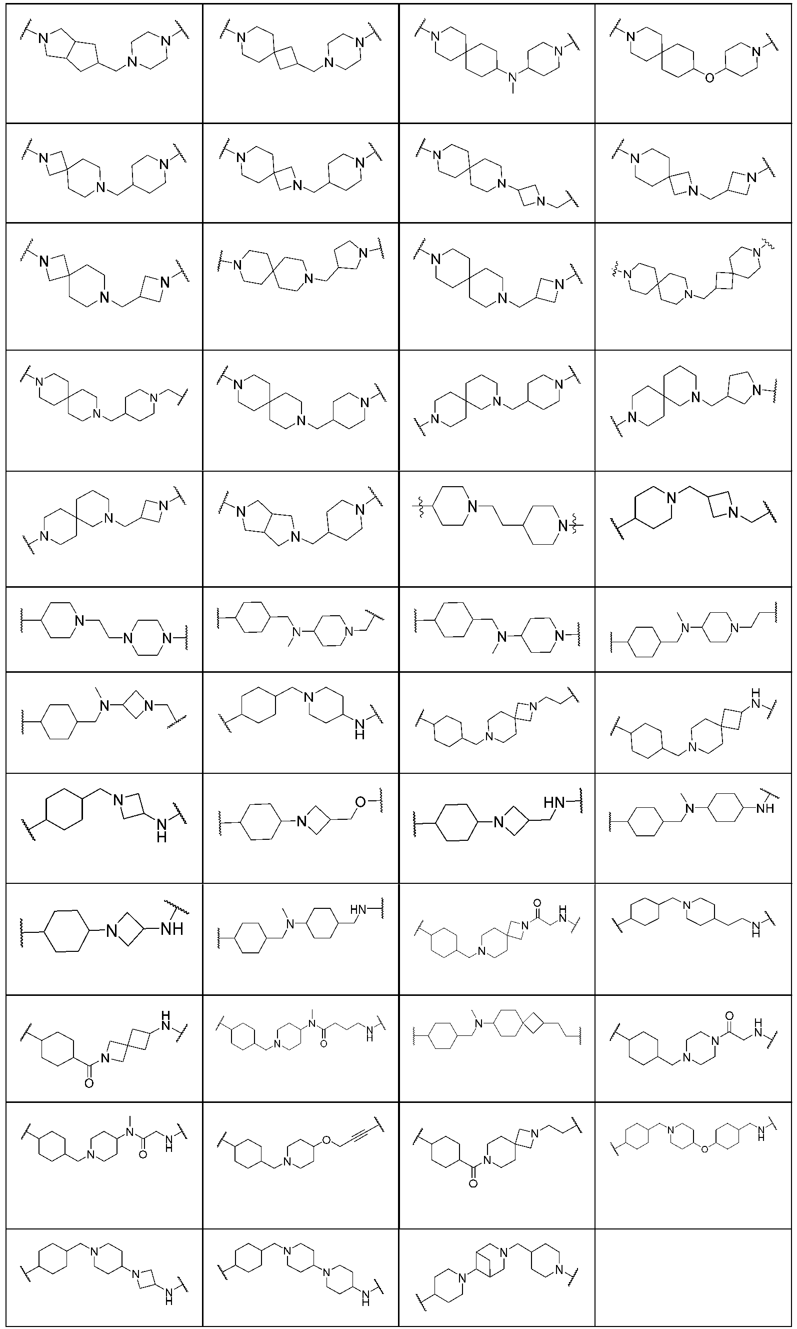 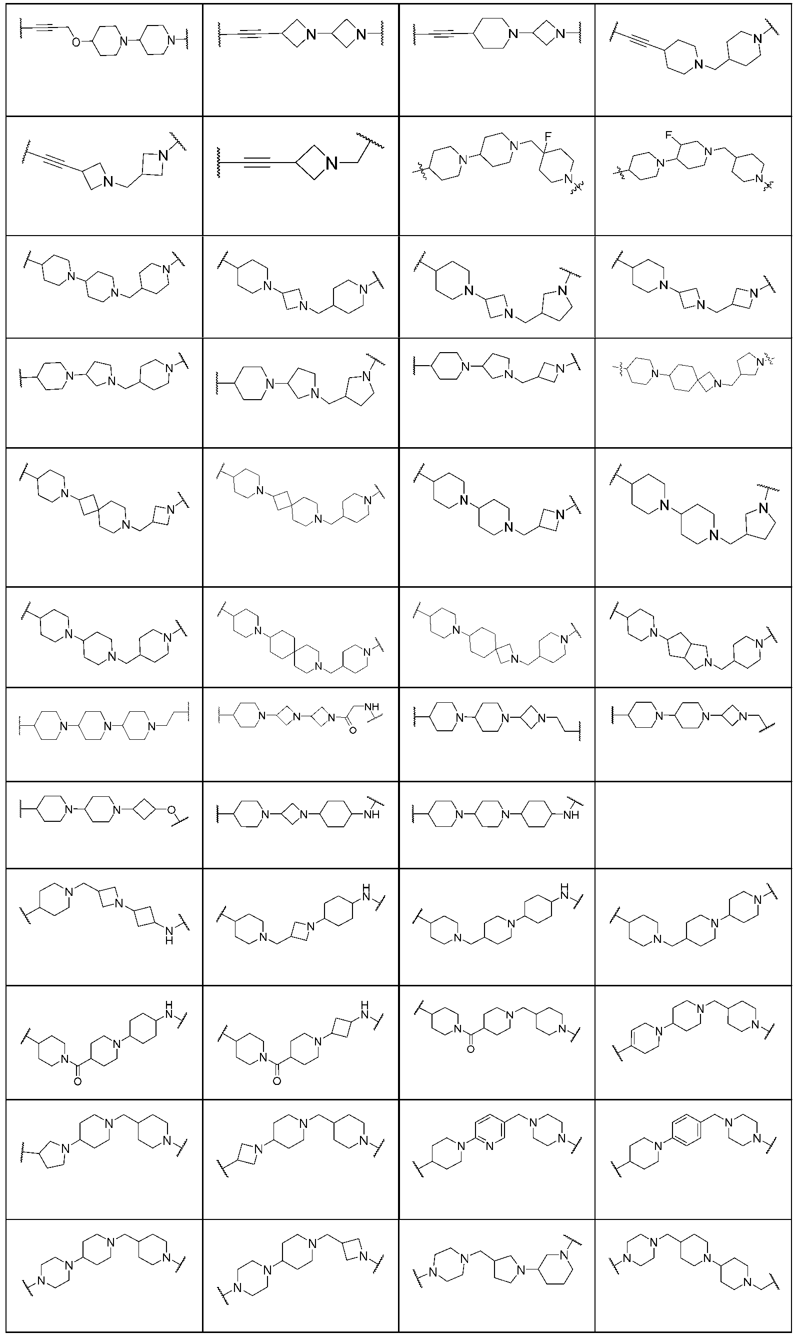 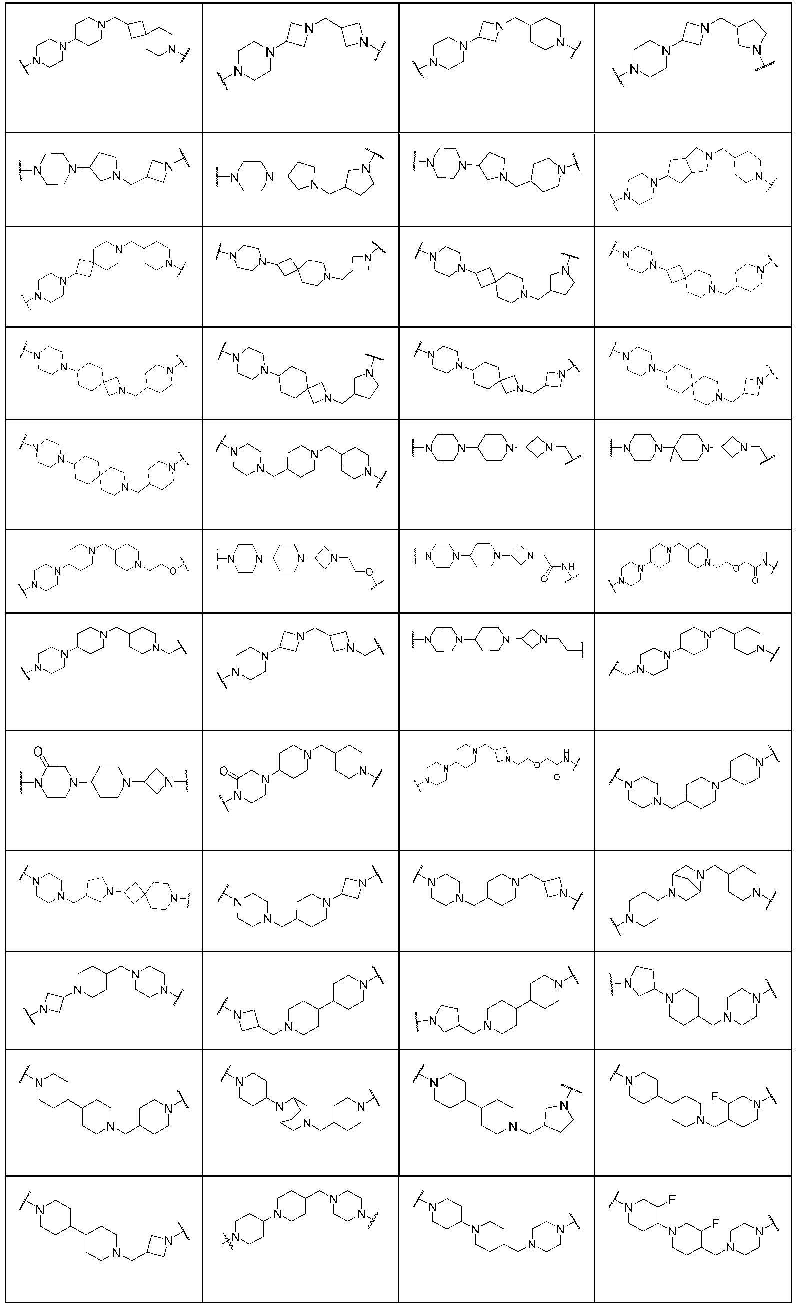 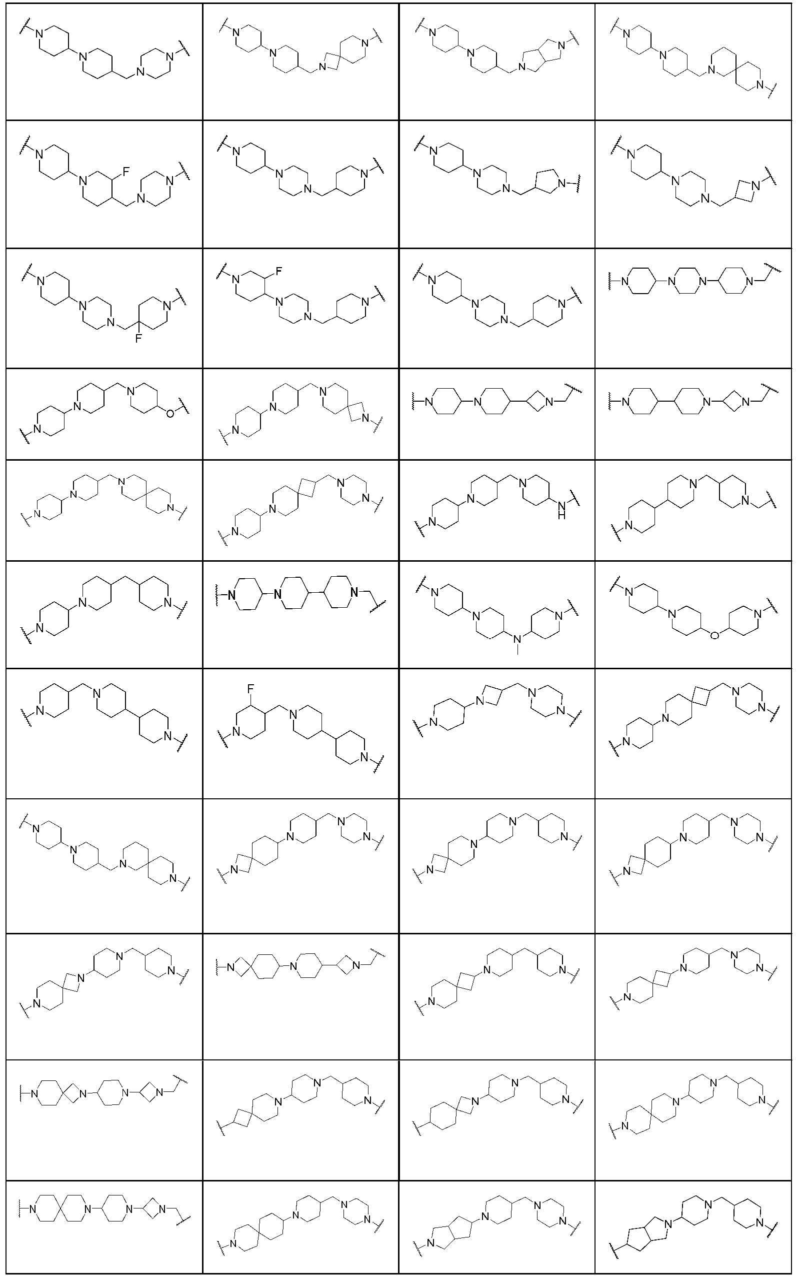 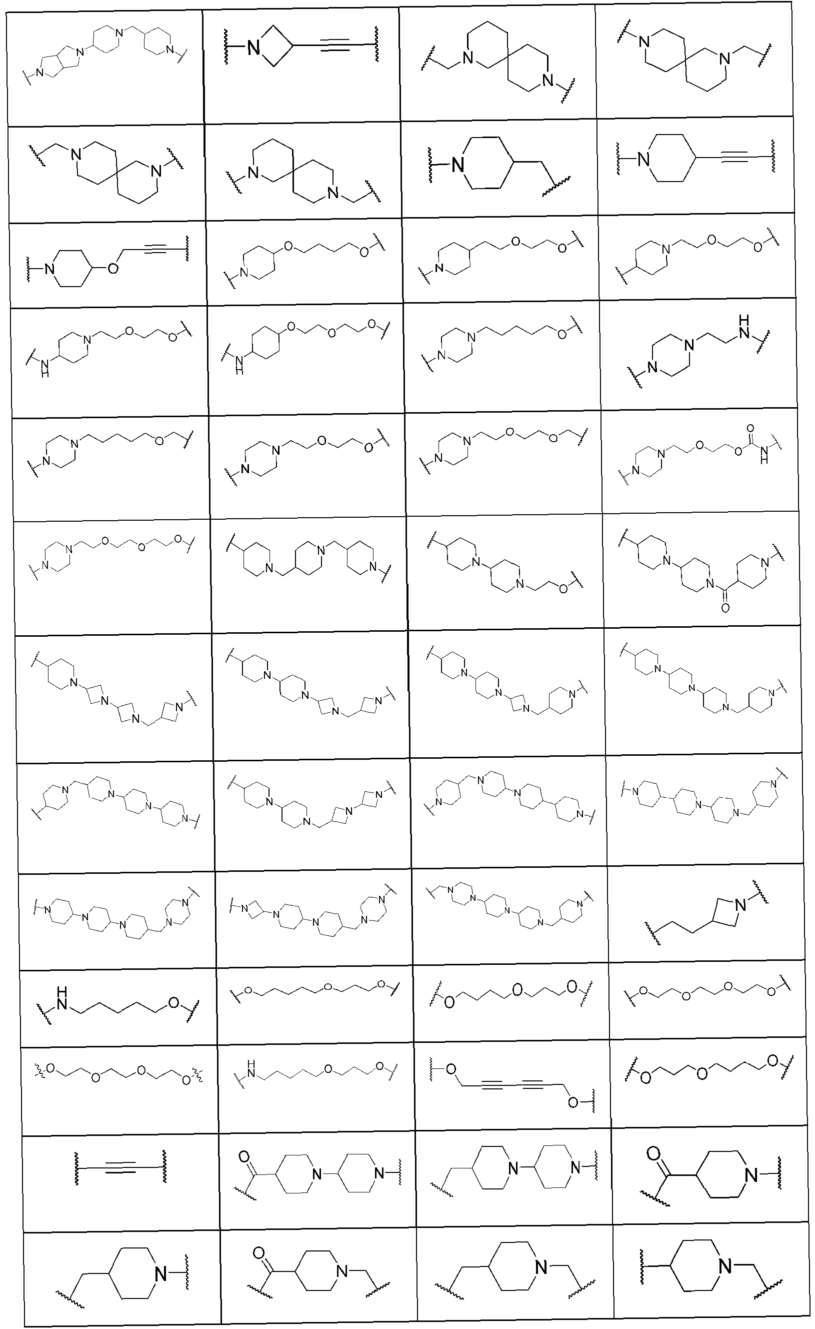 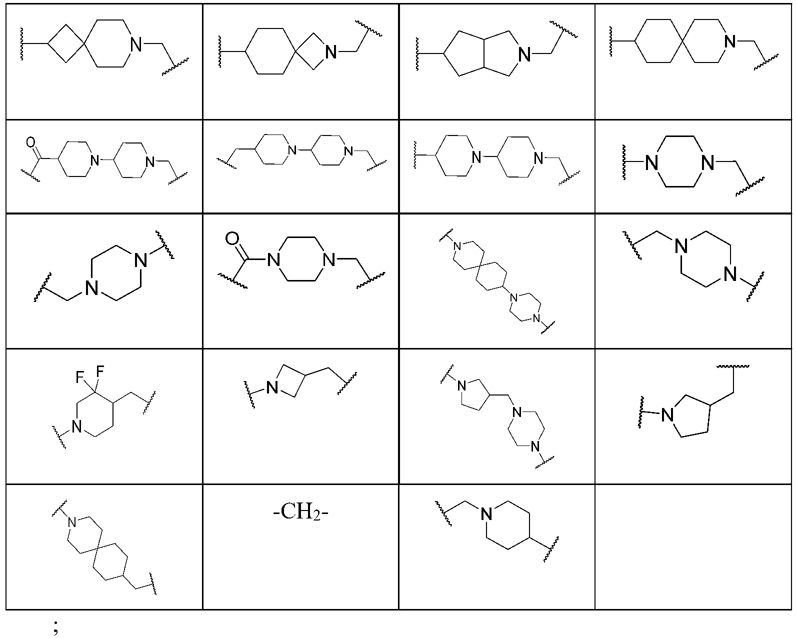 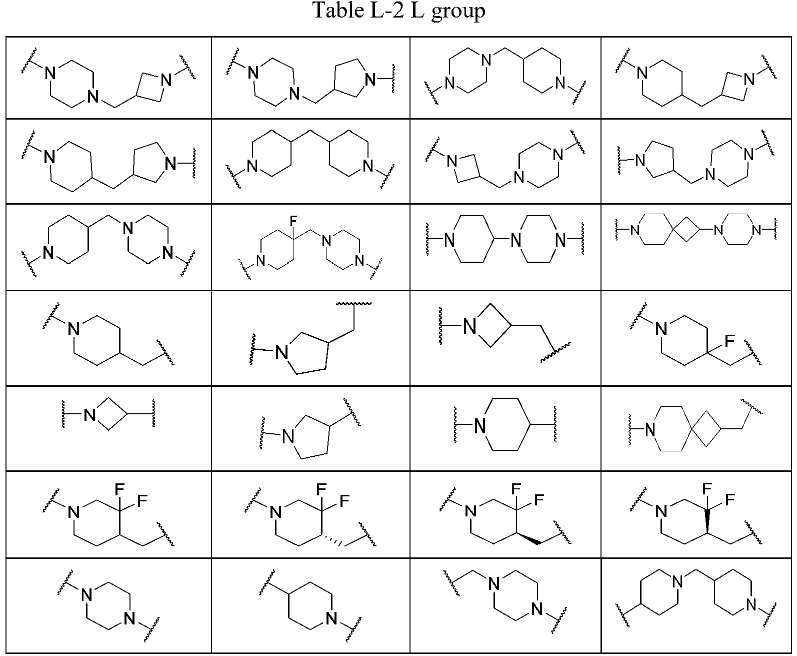 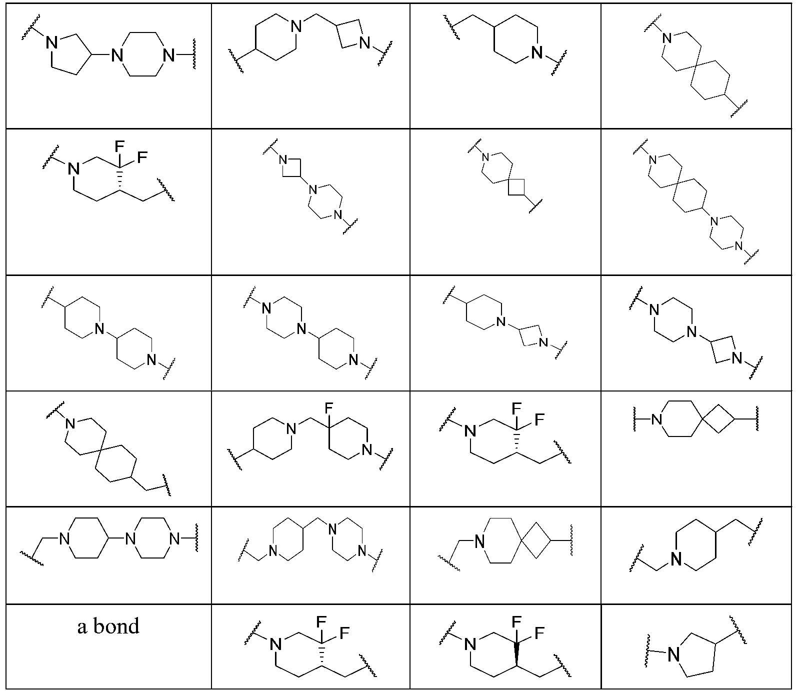
in certain embodiments, B is 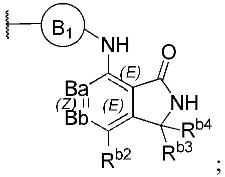
in certain embodiments, B is 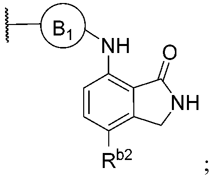
in certain embodiments, B is 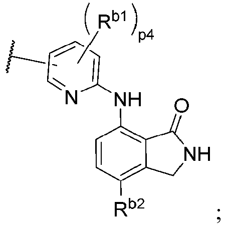
in certain embodiments, B is selected from 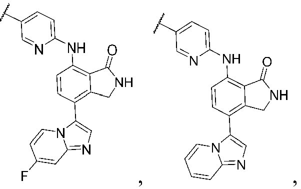 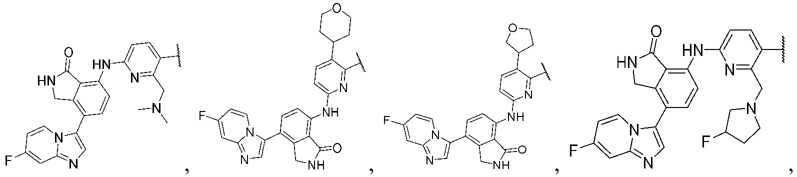 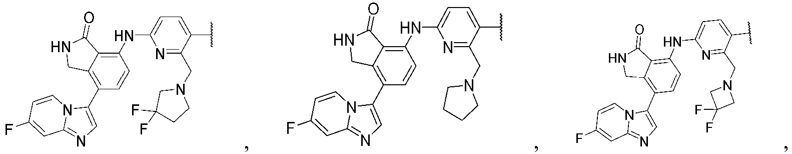 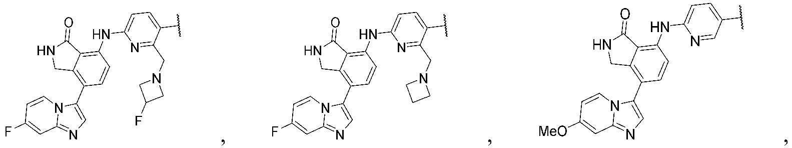 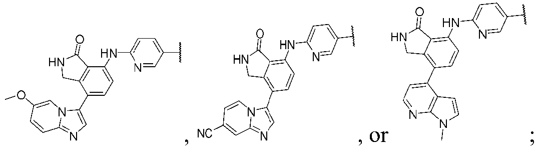
in certain embodiments, Ba or Bb is selected from N or CR^{b};
in certain embodiments, Ba or Bb is selected from N or CH;
in certain embodiments, R^{b} is selected from H, deuterium, halogen, OH, CN, NH₂, NHC₁₋₄ alkyl, N(C₁₋₄ alkyl)₂, C₁₋₄ alkyl, C₂₋₄ alkenyl, C₂₋₄ alkynyl, C₁₋₄ alkoxy, or -C₀₋₂ alkylene-C₃₋₆ cycloalkyl, wherein the alkyl, alkylene, alkenyl, alkynyl, alkoxy or cycloalkyl is optionally substituted with 1 to 4 R^{z};
in certain embodiments, B₁ is selected from C₆₋₁₀ carbocyclyl or 5- to 10-membered heterocyclyl, wherein the B₁ is optionally substituted with 1 to 4 R^{b1};
in certain embodiments, B₁ is selected from phenyl, benzo C₄₋₆ carbocyclyl, benzo 4- to 6-membered heterocyclyl, 5- to 6-membered heteroaryl, or 8- to 10-membered heteroaryl, wherein the B₁ is optionally substituted with 1 to 4 R^{b1};
in certain embodiments, B₁ is selected from phenyl, thiazolyl, furyl, thienyl, oxazolyl, pyrazolyl, pyridyl, pyrimidyl, pyrazinyl, or pyridazinyl, wherein the B₁ is optionally substituted with 1 to 4 R^{b1};
in certain embodiments, R^{b2} is selected from C₆₋₁₀ carbocyclyl or 5- to 10-membered heterocyclyl, wherein the R^{b2} is optionally substituted with 1 to 4 R^{b2a};
in certain embodiments, R^{b2} is selected from phenyl, benzo C₄₋₆ carbocyclyl, benzo 4- to 6-membered heterocyclyl, 5- to 6-membered heteroaryl, or 8- to 10-membered heteroaryl, wherein the R^{b2} is optionally substituted with 1 to 4 R^{b2a};
in certain embodiments, R^{b2} is selected from 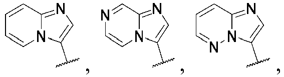 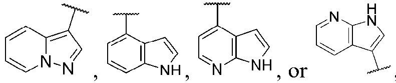 wherein the R^{b2} is optionally substituted with 1 to 4 R^{b2a}; in certain embodiments, R^{b2} is selected from 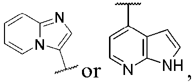 wherein the R^{b2} is optionally substituted with 1 to 4 R^{b2a};
in certain embodiments, R^{b2} is selected from 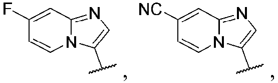 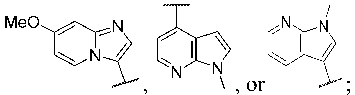
in certain embodiments, R^{b3} and R^{b4} are each independently selected from H, deuterium, halogen, or C₁₋₄ alkyl, wherein the alkyl is optionally substituted with 1 to 4 halogen;
in certain embodiments, R^{b1} and R^{b2a} are each independently selected from deuterium, halogen, OH, CN, NH₂, C₁₋₄ alkyl, C₂₋₄ alkenyl, C₂₋₄ alkynyl, C₁₋₄ alkoxy, -C₀₋₂ alkylene-C₃₋₆ cycloalkyl, -C₀₋₂ alkylene-O-C₃₋₆ cycloalkyl, -C₀₋₂ alkylene-4- to 7-membered heterocycloalkyl, -C₀₋₂ alkylene-O-4- to 7-membered heterocycloalkyl, -C₀₋₂ alkylene-NHC₁₋₄ alkyl, or -C₀₋₂ alkylene-N(C₁₋₄ alkyl)₂, wherein the alkyl, alkylene, alkenyl, alkynyl, alkoxy, cycloalkyl or heterocycloalkyl is optionally substituted with 1 to 4 R^{z};
in certain embodiments, R^{b1} and R^{b2a} are each independently selected from deuterium, F, Cl, Br, I, OH, CN, NH₂, NHCH₃, N(CH₃)₂ or one of the following groups optionally substituted with 1 to 4 R^{z}: methyl, ethyl, propyl, isopropyl, ethynyl, -CH₂-ethynyl, methoxy, ethoxy, cyclopropyl, cyclobutyl, -O-cyclopropyl, -O-cyclobutyl, -CH₂-cyclopropyl, -CH₂-cyclobutyl, oxetanyl, azetidinyl, tetrahydrofuryl, pyrrolidyl, -CH₂NHCH₃, -CH₂N(CH₃)₂, tetrahydropyranyl, piperidyl, -CH₂-azetidinyl, -CH₂-pyrrolidyl, or -CH₂-piperidyl;
in certain embodiments, each R^{b2a} is independently selected from deuterium, F, Cl, Br, OH, CN, NH₂, NHCH₃, N(CH₃)₂, methyl, ethyl, methoxy, or CF₃;
in certain embodiments, each R^{b1} is independently selected from deuterium, F, Cl, Br, I, OH, CN, NH₂, NHCH₃, N(CH₃)₂, methyl, ethyl, -CH₂NHCH₃, - CH₂N(CH₃)₂, tetrahydrofuryl, tetrahydropyranyl, piperidyl, -CH₂-azetidinyl, -CH₂-pyrrolidyl, or -CH₂-piperidyl, wherein the methyl, ethyl, tetrahydrofuryl, tetrahydropyranyl, piperidyl, azetidinyl, pyrrolidyl, or piperidyl is optionally substituted with 1 to 4 substituents selected from deuterium, F, CF₃, OH, =O, COOH, CN, or NH₂;
in certain embodiments, each R^{b1} is independently selected from deuterium, N(CH₃)₂, -CH₂N(CH₃)₂, 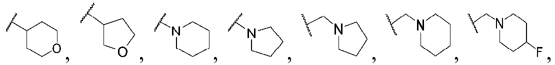 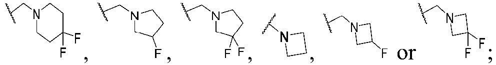
in certain embodiments, K is 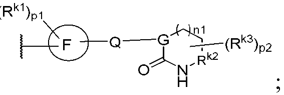
in certain embodiments, 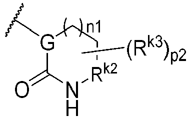 is selected from 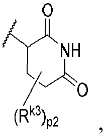 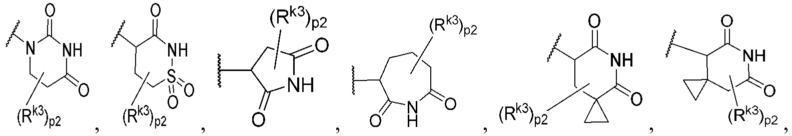 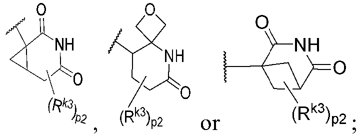
in certain embodiments, G is selected from N or CH;
in certain embodiments, each Q is independently selected from a bond, -O-, - S-, -CH₂-, -NR^{q}-, -C(=O)-, -NR^{q}C(=O)-, or -C(=O)NR^{q}-;
in certain embodiments, Q is selected from a bond, CH₂, NH, N(CH₃), O, S, C(=O), NHC(=O), C(=O)NH, N(CH₃)C(=O), or C(=O)N(CH₃);
in certain embodiments, Q is selected from a bond or C(=O)NH;
in certain embodiments, a nitrogen-nitrogen bond, a nitrogen-oxygen bond, or a nitrogen-sulphur bond cannot be directly formed between Q and G;
in certain embodiments, R^{q} is selected from H or C₁₋₄ alkyl;
in certain embodiments, F is selected from C₃₋₂₀ carbocyclyl, C₆₋₂₀ aryl, 3- to 20-membered heterocyclyl or 5- to 20-membered heteroaryl;
in certain embodiments, F is selected from a C₃₋₇ monocyclic group, a C₄₋₁₀ fused ring group, a C₅₋₁₂ spiro ring group, a C₅₋₁₀ bridged ring group, 4- to 7-membered mono-heterocyclyl, 4- to 10-membered bicyclic fused-heterocyclyl, 8-to 15-membered tricyclic fused-heterocyclyl, 12- to 19-membered tetracyclic fused-heterocyclyl, 5- to 17-membered spiro-heterocyclyl, 5- to 10-membered bridged-heterocyclyl, C₆₋₁₄ aryl, or 5- to 10-membered heteroaryl;
in certain embodiments, F is selected from cyclobutyl, cyclopentyl, cyclohexyl, bicyclo[1.1.1]pentanyl, 6,7-dihydro-5H-cyclopenta[c]pyridyl, 2,3-dihydro-1H-indenyl, phenyl, naphthyl, anthracenyl, phenanthryl, azetidinyl, pyrrolidyl, piperidyl, morpholinyl, pyridyl, pyrimidyl, pyridazinyl, pyrazinyl, triazinyl, pyrrolyl, pyrazolyl, imidazolyl, triazolyl, oxazolyl, furyl, thienyl, thiazolyl, 2-pyridonyl, 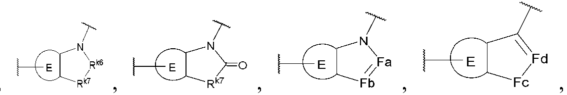 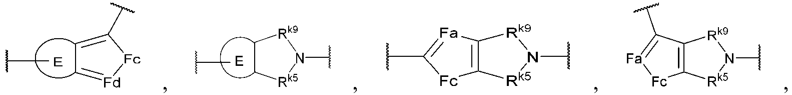 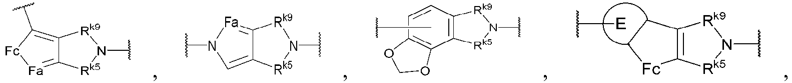 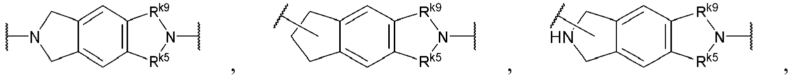 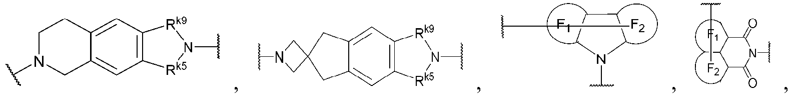 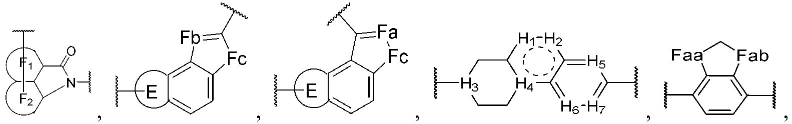 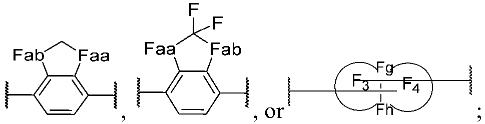 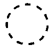 represents that the ring is an aromatic ring or a non-aromatic ring;
in certain embodiments, Fa is selected from N, CH or CR^{k1}; in certain embodiments, Fb is selected from N, CH or CR^{k1};
in certain embodiments, Fc is selected from O, S, NH, N(CH₃) or NR^{k7a}; in certain embodiments, Fd is selected from N, CH or CR^{k1};
in certain embodiments, Fg is selected from N or C; in certain embodiments, Fh is selected from N or C; in certain embodiments, Faa is selected from a bond, O, or CH₂; in certain embodiments, Fab is selected from O or CH₂;
in certain embodiments, H₁ is selected from N, NH, CH, CH₂, CHR^{k1}, NR^{k1}, CR^{k1}, C(=O), or C(R^{k1})₂;
in certain embodiments, H₂ is selected from a bond, O, N, NH, CH, CH₂, CHR^{k1}, NR^{k1}, CR^{k1} or C(R^{k1})₂;
in certain embodiments, H₃ is selected from N or CH; in certain embodiments, H₄ is selected from C, N or CH;
in certain embodiments, H₅, H₆ and H₇ are each independently selected from N, C, CH or CR^{k1}, and H₅, H₆ and H₇ contain at most 2 N;
in certain embodiments, ring E is selected from phenyl or 5- to 6-membered heteroaryl, wherein the ring E is optionally substituted with 1 to 3 R^{k1};
in certain embodiments, each ring E is independently selected from phenyl, pyridyl, pyridazinyl, pyrazinyl, pyrimidyl, pyrrolyl, pyrazolyl, imidazolyl, thiazolyl, furyl, thienyl or oxazolyl, wherein the ring E is optionally substituted with 1 to 3 R^{k1};
in certain embodiments, ring F₁, ring F₂, ring F₃ and ring F₄ are each independently selected from phenyl or 5- to 6-membered heteroaryl, wherein the ring F₁, ring F₂, ring F₃ and ring F₄ are optionally substituted with 1 to 2 R^{k1};
in certain embodiments, ring F₁ and ring F₂ are each independently selected from phenyl, pyridyl, thiazolyl, furyl, thienyl or oxazolyl, wherein the ring F₁ and ring F₂ are optionally substituted with 1 to 2 R^{k1};
in certain embodiments, ring F₃ and ring F₄ are each independently selected from phenyl, pyridyl, pyridazinyl, pyrazinyl, pyrimidyl, triazinyl, pyrrolyl, pyrazolyl, imidazolyl, thiazolyl, furyl, thienyl or oxazolyl, wherein the ring F₃ and ring F₄ are optionally substituted with 1 to 2 R^{k1};
in certain embodiments, each R^{k1} is independently selected from H, deuterium, halogen, OH, =O, NH₂, CN, COOH, CONH₂, C₁₋₄ alkyl, C₂₋₄ alkenyl, C₂₋₄ alkynyl, C₁₋₄ alkoxy, C₃₋₆ cycloalkyl, or 3- to 6-membered heterocycloalkyl, wherein the alkyl, alkenyl, alkynyl, alkoxy, cycloalkyl or heterocycloalkyl is optionally substituted with 1 to 4 R^{z};
in certain embodiments, each R^{k2} is independently selected from a bond, - C(=O)-, -S(=O)₂-, -S(=O)- or -C(R^{k3})₂-;
in certain embodiments, each R^{k3} is independently selected from H, deuterium, halogen, OH, =O, NH₂, CN, COOH, CONH₂, C₁₋₄ alkyl, C₁₋₄ alkoxy, C₃₋₈ cycloalkyl, or 3- to 8-membered heterocyclyl, wherein the alkyl, alkoxy, cycloalkyl or heterocyclyl is optionally substituted with 1 to 4 R^{z};
in certain embodiments, R^{k1} and R^{k3} are each independently selected from H, deuterium, F, Cl, Br, I, OH, =O, NH₂, CF₃, CN, COOH, CONH2, methyl, ethyl, isopropyl, methoxy, ethoxy, isopropoxy, or cyclopropyl, wherein the methyl, ethyl, isopropyl, methoxy, ethoxy, isopropoxy or cyclopropyl is optionally substituted with 1 to 4 substituents selected from deuterium, F, Cl, Br, I, OH, or NH₂;
alternatively, two R^{k3} are directly connected to form C₃₋₈ carbocyclyl or 4- to 8-membered heterocyclyl, wherein the carbocyclyl or heterocyclyl is optionally substituted with 1 to 4 R^{z};
in certain embodiments, each R^{k1} is independently selected from H, deuterium, F, Cl, Br, NH₂, CF₃, CN, methyl, or ethyl;
in certain embodiments, R^{L2} and R^{z} are each independently selected from deuterium, halogen, OH, =O, CF₃, SF₅, CN, NH₂, NO₂, COOH, CONH₂, NHC₁₋₄ alkyl, N(C₁₋₄ alkyl)₂, COOH, CONH₂, C₁₋₄ alkyl, C₂₋₄ alkenyl, C₂₋₄ alkynyl, C₁₋₄ alkoxy, -S-C₁₋₄ alkyl, or -C₀₋₄ alkylene-C₃₋₆ cycloalkyl, wherein the alkyl, alkylene, alkoxy, alkenyl, alkynyl, or cycloalkyl is optionally substituted with 1 to 4 substituents selected from deuterium, F, Cl, Br, I, OH, CN, C₁₋₄ alkyl, or C₁₋₄ alkoxy;
in certain embodiments, R^{L2} and R^{z} are each independently selected from deuterium, F, Cl, Br, I, OH, =O, CF₃, SF₅, CN, NH₂, NO₂, COOH, CONH₂, N(CH₃)₂, NHCH₃, methyl, ethyl, vinyl, ethynyl, methoxy, ethoxy, methylthio, cyclopropyl, cyclobutyl, cyclopentyl, cyclohexyl, -CH₂-cyclopropyl, -CH₂-cyclobutyl, -CH₂-cyclopentyl, or -CH₂-cyclohexyl, wherein the methyl, ethyl, vinyl, ethynyl, methoxy, ethoxy, methylthio, cyclopropyl, cyclobutyl, cyclopentyl, or cyclohexyl is optionally substituted with 1 to 4 substituents selected from deuterium, F, Cl, Br, I, OH, CN, C₁₋₄ alkyl, or C₁₋₄ alkoxy;
in certain embodiments, each R^{k5} is independently selected from 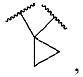 C(CH₃)₂, C(=O), CH₂, CH₂CH₂, S(=O)₂, 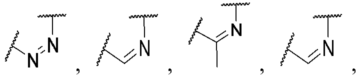 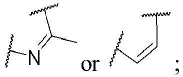
in certain embodiments, each R^{k6} is independently selected from C(=O), CH, S(=O), S(=O)₂, CH₂ or N;
in certain embodiments, each R^{k7} is independently selected from 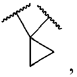 C(CH₃)₂, CH₂, O or NR^{k7a};
in certain embodiments, R^{k7a} is selected from H, methyl, ethyl, propyl, isopropyl, vinyl, propenyl, allyl, ethynyl, propynyl, propargyl, cyclopropyl, cyclobutyl, cyclopentyl, cyclohexyl, azetidinyl, pyrrolidyl, piperidyl, oxetanyl, tetrahydrofuryl, or tetrahydropyranyl, wherein the methyl, ethyl, propyl, isopropyl, cyclopropyl, cyclobutyl, cyclopentyl, cyclohexyl, azetidinyl, pyrrolidyl, piperidyl, oxetanyl, tetrahydrofuryl or tetrahydropyranyl is optionally substituted with 1 to 4 substituents selected from deuterium, F, Cl, Br, I, OH, CN, CF₃, C₁₋₄ alkyl, C₁₋₄ alkoxy, C₂₋₄ alkenyl, C₂₋₄ alkynyl, or C₃₋₆ cycloalkyl;
in certain embodiments, each R^{k9} is independently selected from a bond, 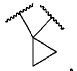 C(CH₃)₂, C(=O), CH₂, CH₂CH₂ or S(=O)₂;
in certain embodiments, n1 is selected from 0, 1, 2 or 3;
in certain embodiments, each p1 or p2 is independently selected from 0, 1, 2, 3, 4 or 5;
in certain embodiments, each p2 is independently selected from 0, 1, 2 or 3; in certain embodiments, p3 is selected from 0, 1, 2 or 3;
in certain embodiments, p4 is selected from 0, 1, 2 or 3; in certain embodiments, p5 is selected from 0 or 1; in certain embodiments, p6 is selected from 0 or 1;
in certain embodiments, K is selected from one of the structural fragments shown in Table K-1; in certain embodiments, K is selected from one of the structural fragments shown in Table K-2;
in certain embodiments, K is selected from 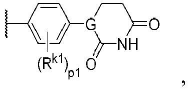 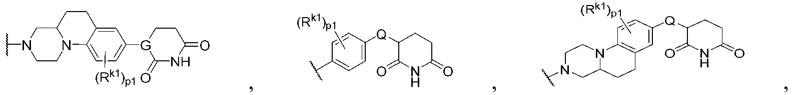 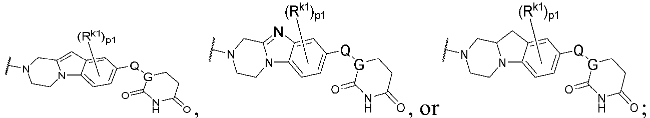 in certain embodiments, K is selected from 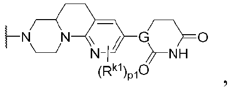 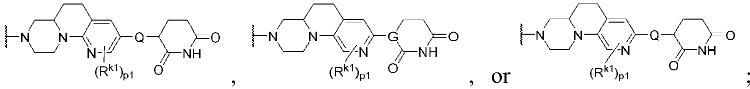 in certain embodiments, K is selected from

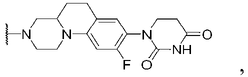

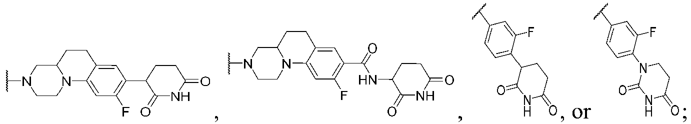

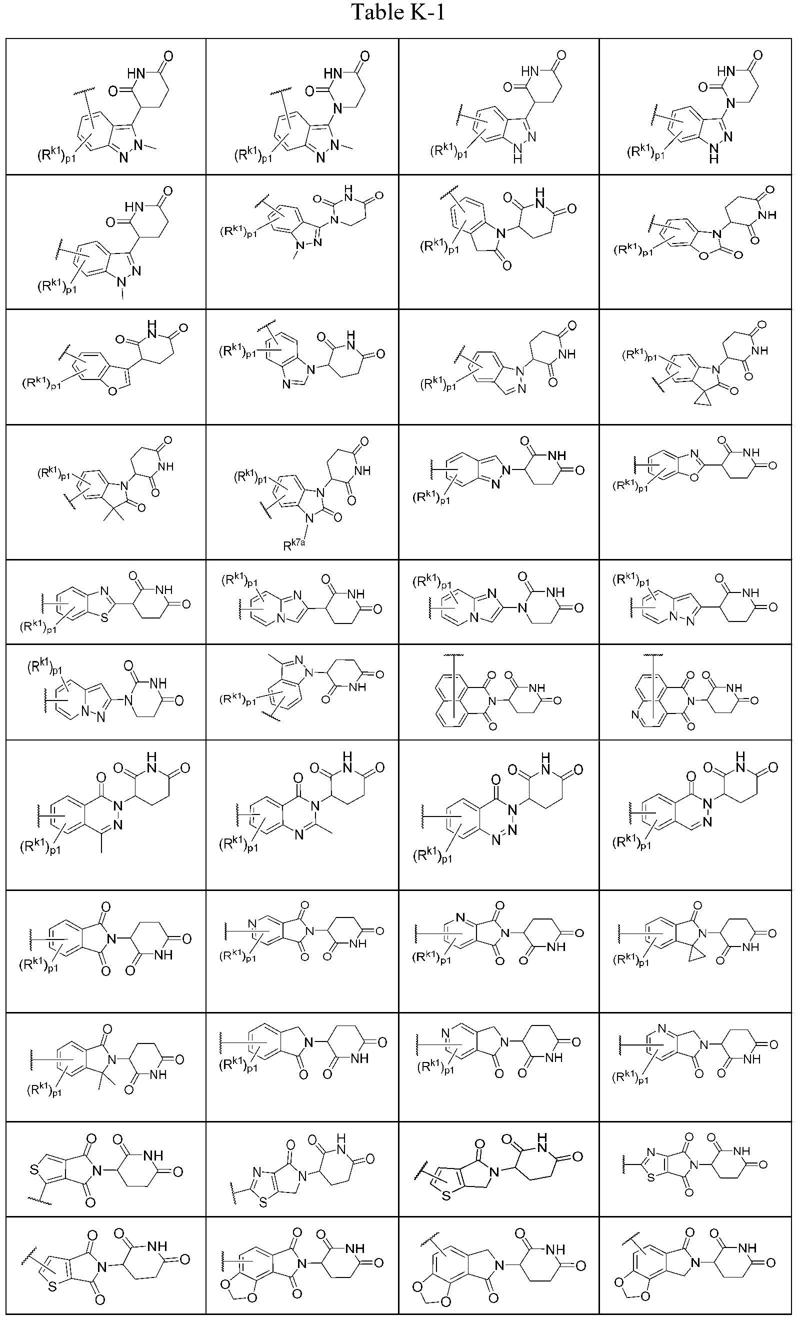

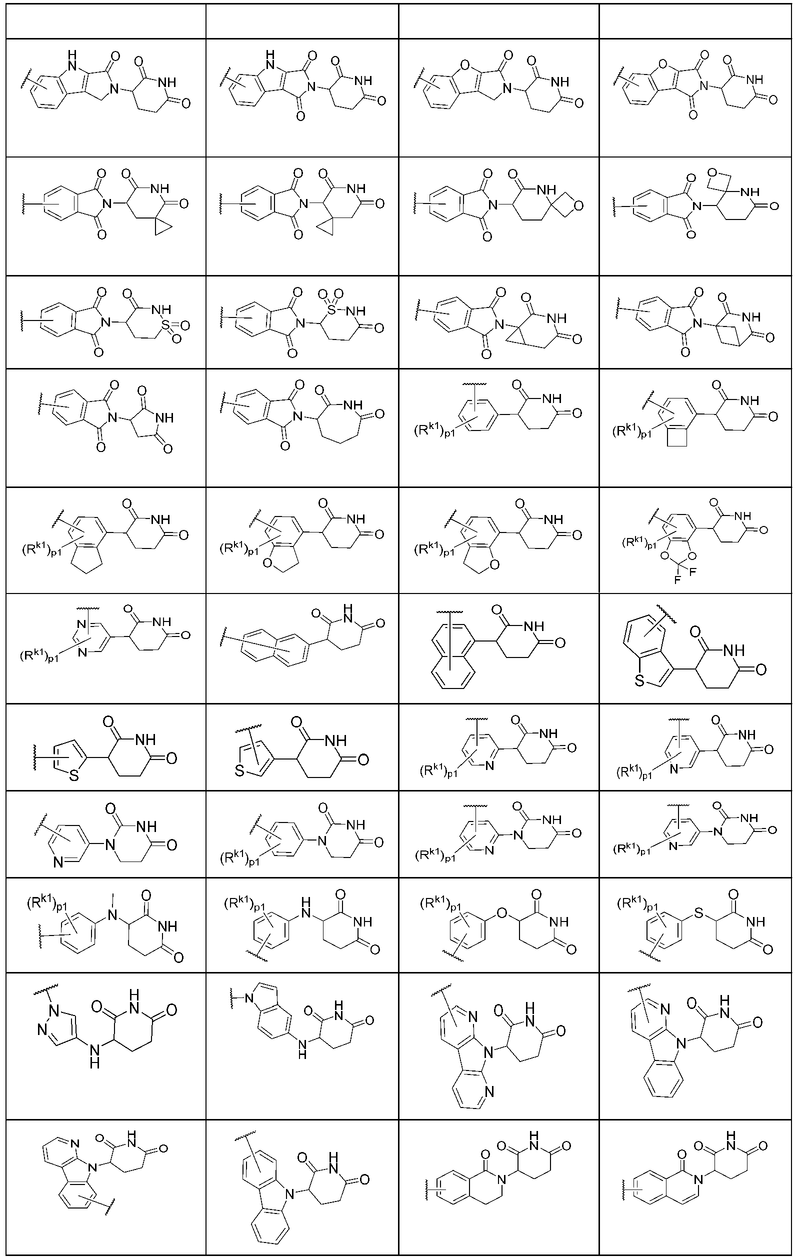

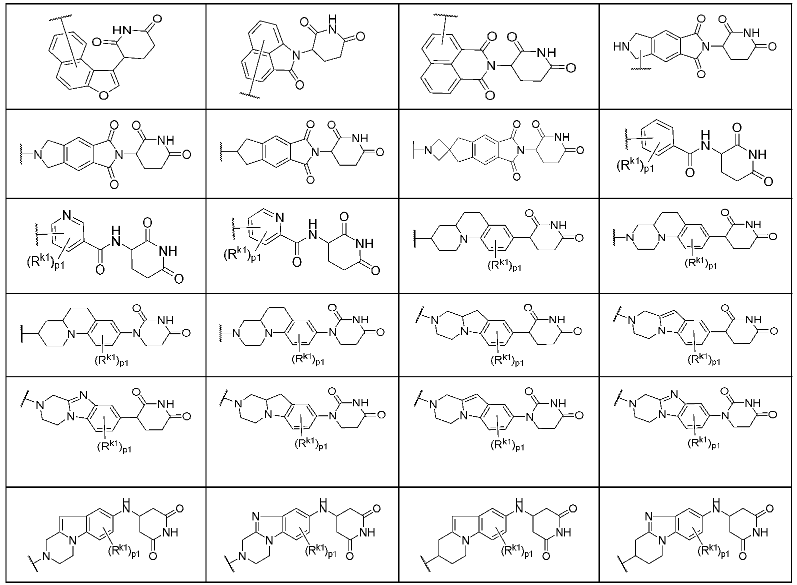

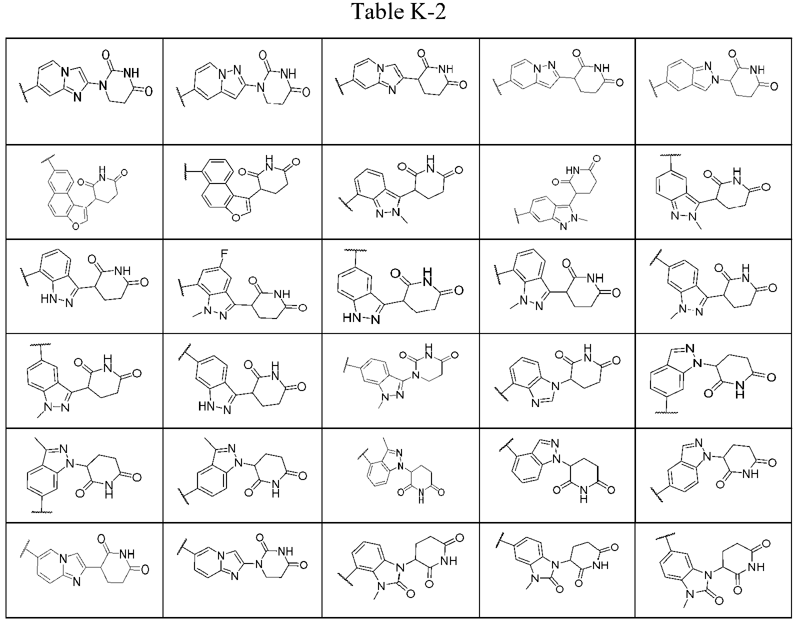

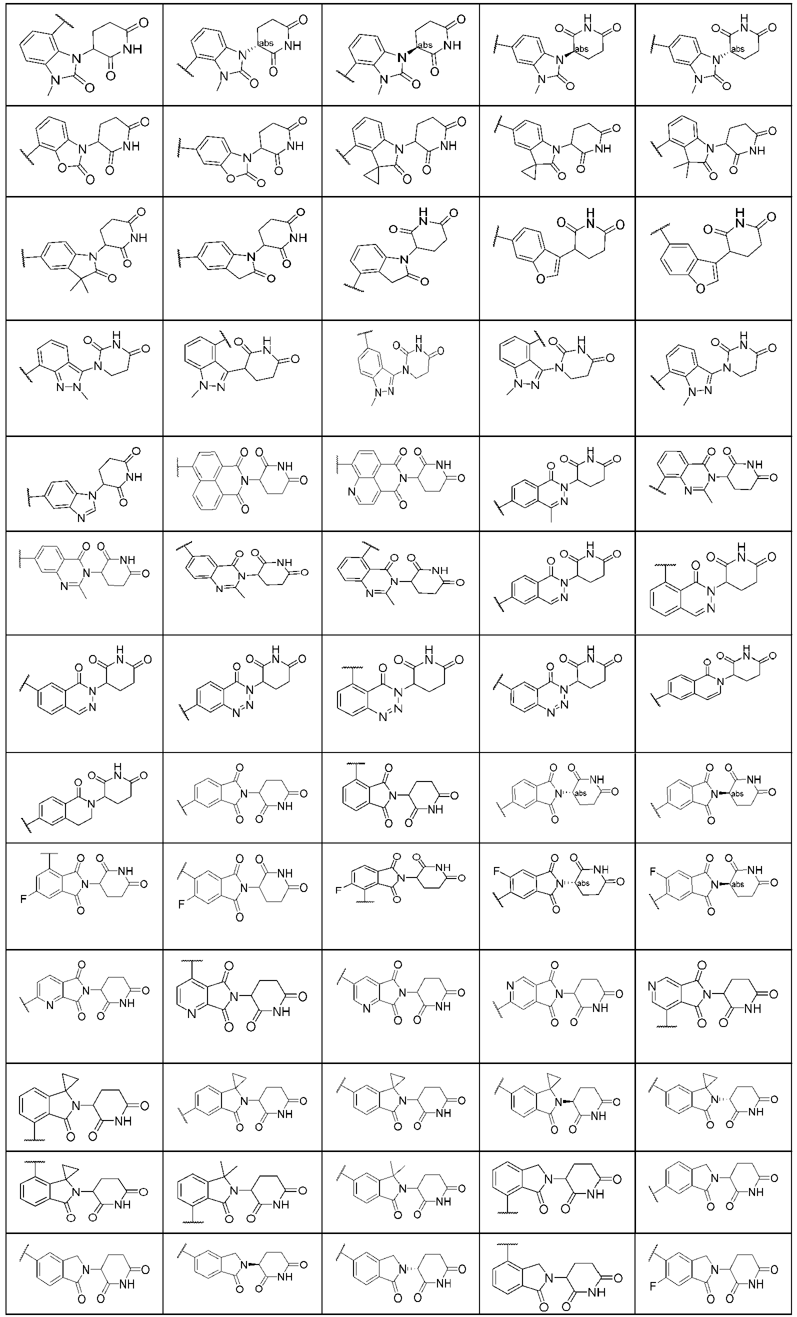

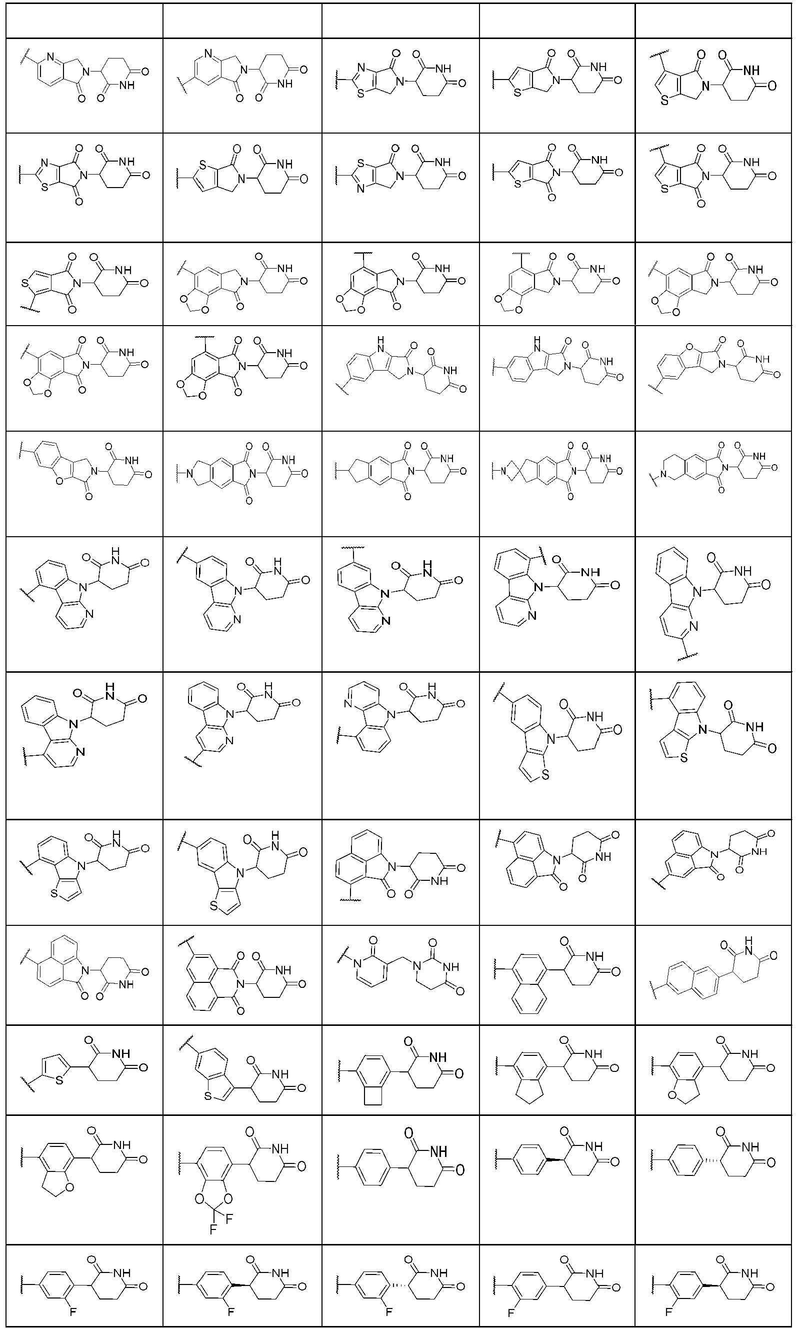

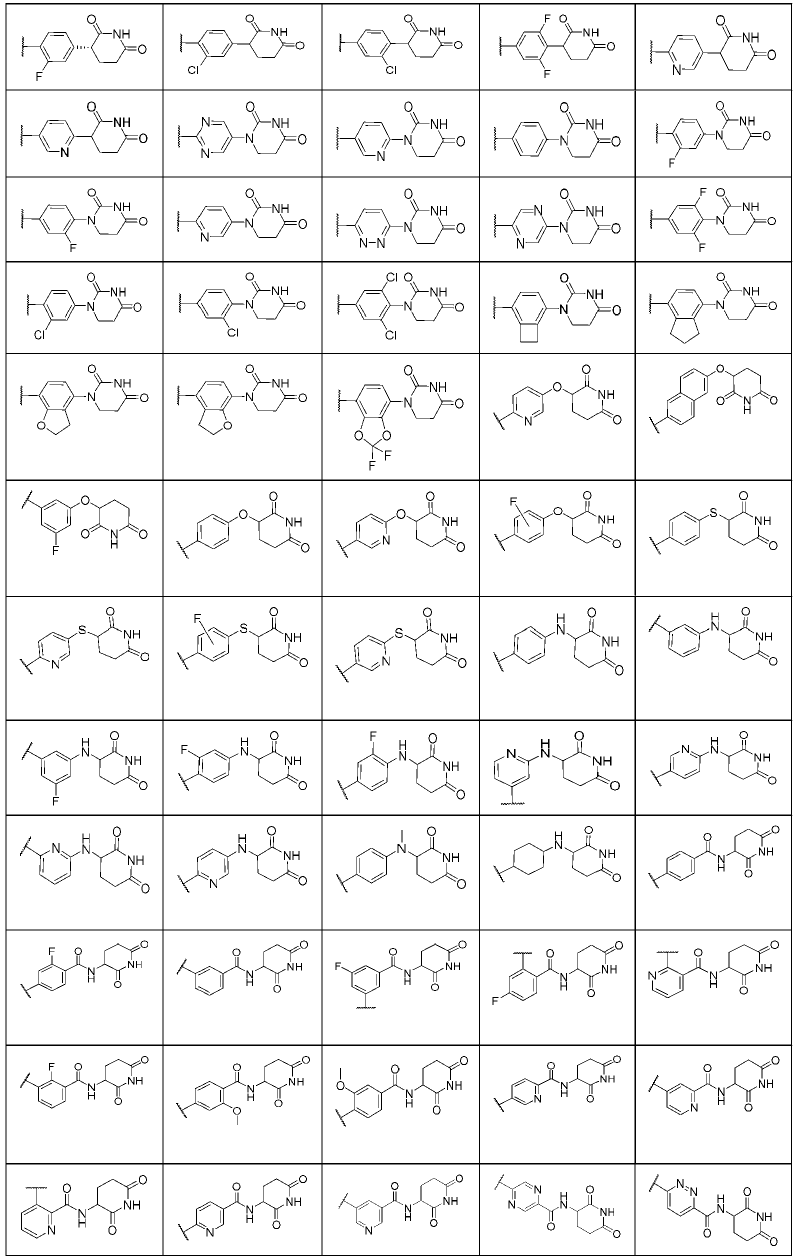

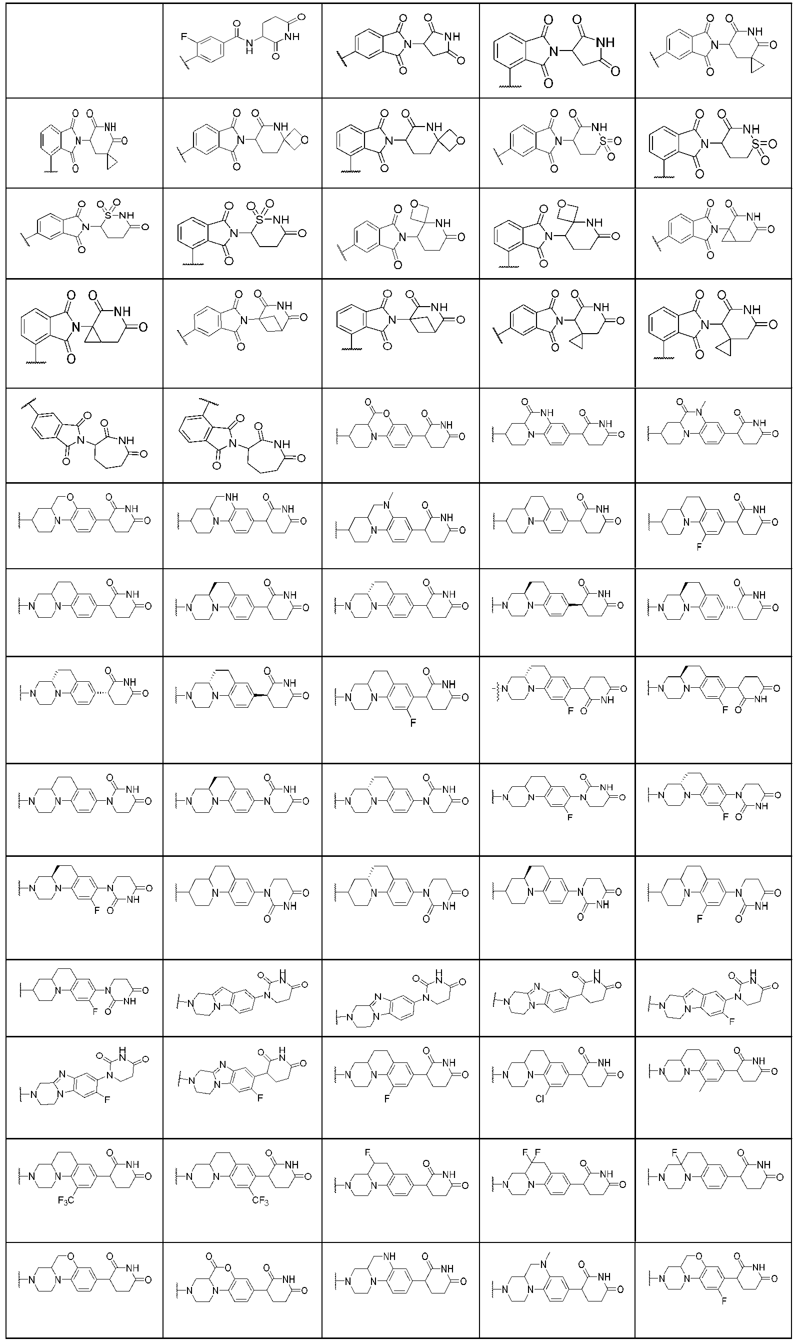

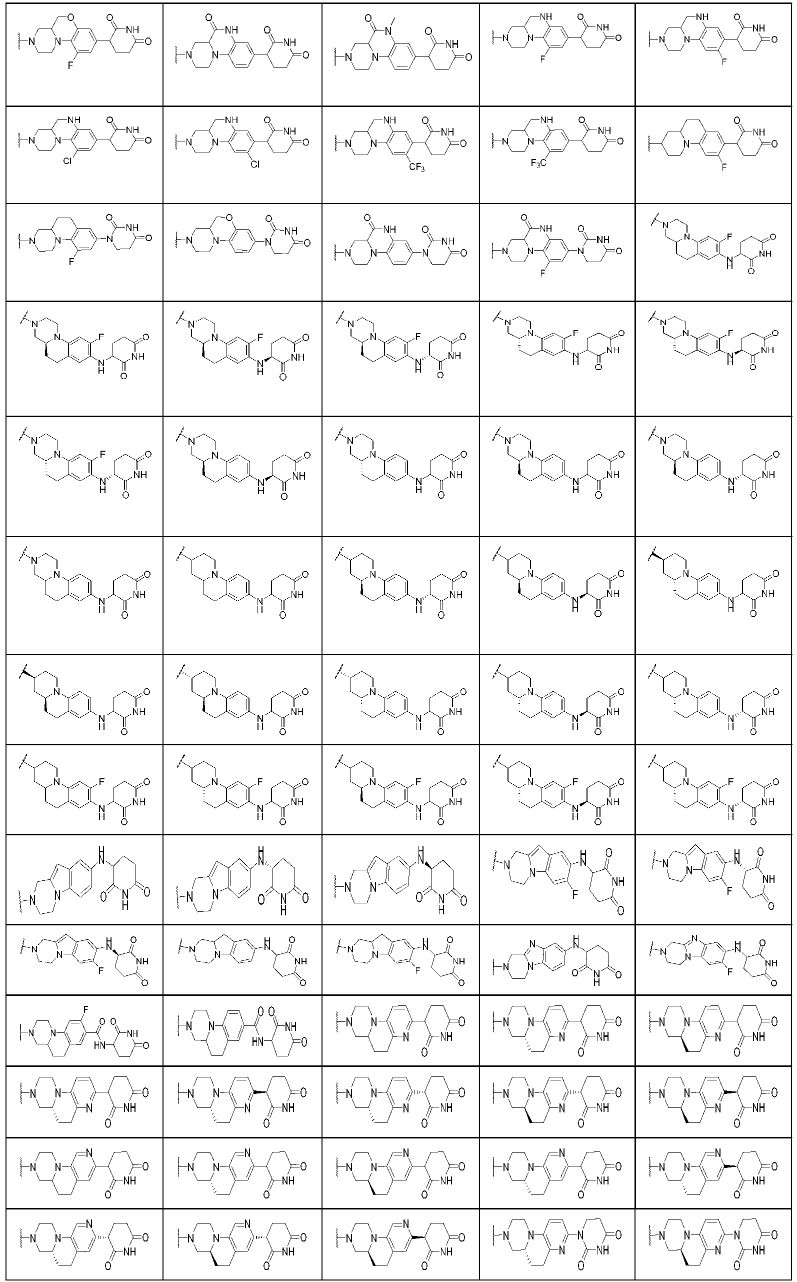

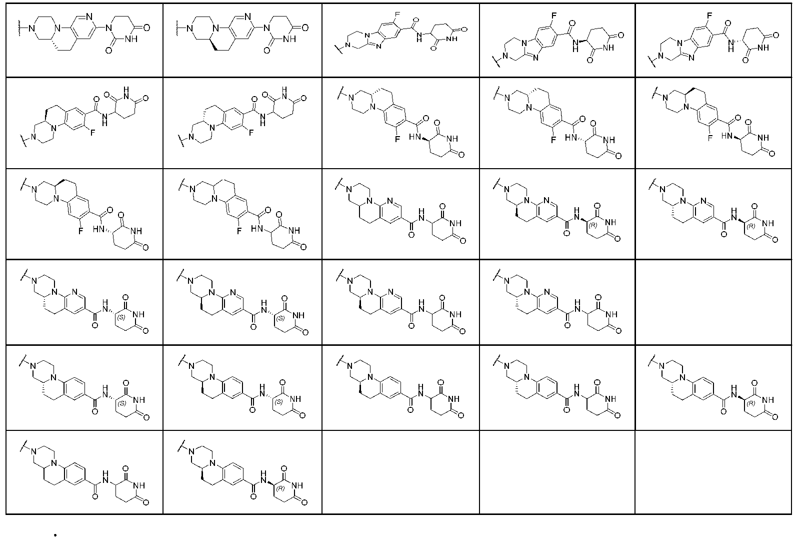

As a first embodiment of the present invention, provided is the compound as shown in the above general formula (I) or the stereoisomer, the racemate, the tautomer, the deuterated substance, the solvate, the prodrug, the metabolite, the pharmaceutically acceptable salt or the cocrystal thereof, wherein
L is selected from a bond or -C₁₋₅₀ hydrocarbyl-, wherein the hydrocarbyl has 1 to 20 methylene units optionally replaced by -Ak- or -Cy-;
each -Ak- is independently selected from -(CH₂)_{q}-, -(CH₂)_{q}-O-, -O-(CH₂)_{q}-, - (CH₂)_{q}-S-, -S-(CH₂)_{q}-, -(CH₂)_{q}-NR^{L}-, -NR^{L}-(CH₂)_{q}-, -(CH₂)_{q}-NR^{L}C(=O)-, - NR^{L}(CH₂)_{q}C(=O)-, -(CH₂)_{q}-C(=O)NR^{L}-, -C(=O)-, -C(=O)-(CH₂)_{q}-NR^{L}-, -(C≡C)_{q}-, -CH=CH-, -Si(R^{L})₂-, -Si(OH)(R^{L})-, -Si(OH)₂-, -P(=O)(OR^{L})-, -P(=O)(R^{L})-, -S-, - S(=O)-, -S(=O)₂- or a bond, wherein the CH and -CH₂- are optionally substituted with 1 to 2 R^{z};
each q is independently selected from 0, 1, 2, 3, 4, 5 or 6;
R^{L} is selected from H, C₁₋₄ alkyl, C₃₋₇ carbocyclyl, or 4- to 10-membered heterocyclyl, wherein the alkyl, carbocyclyl or heterocyclyl is optionally substituted with 1 to 4 R^{z};
each -Cy- is independently selected from a bond or one of the following groups optionally substituted with 1 to 4 R^{L2}: 4- to 8-membered mono-heterocyclyl, 4- to 12-membered fused-heterocyclyl, 5- to 13-membered spiro-heterocyclyl, 7- to 12-membered bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl;
B is 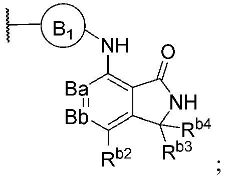
Ba or Bb is selected from N or CR^{b};
R^{b} is selected from H, deuterium, halogen, OH, CN, NH₂, NHC₁₋₄ alkyl, N(C₁₋₄ alkyl)₂, C₁₋₄ alkyl, C₂₋₄ alkenyl, C₂₋₄ alkynyl, C₁₋₄ alkoxy, or -C₀₋₂ alkylene-C₃₋₆ cycloalkyl, wherein the alkyl, alkylene, alkenyl, alkynyl, alkoxy or cycloalkyl is optionally substituted with 1 to 4 R^{z};
B₁ is selected from C₆₋₁₀ carbocyclyl or 5- to 10-membered heterocyclyl, wherein the B₁ is optionally substituted with 1 to 4 R^{b1};
R^{b2} is selected from C₆₋₁₀ carbocyclyl or 5- to 10-membered heterocyclyl, wherein the R^{b2} is optionally substituted with 1 to 4 R^{b2a};
R^{b3} and R^{b4} are each independently selected from H, deuterium, halogen, or C₁₋₄ alkyl, wherein the alkyl is optionally substituted with 1 to 4 halogen;
R^{b1} and R^{b2a} are each independently selected from deuterium, halogen, OH, CN, NH₂, C₁₋₄ alkyl, C₂₋₄ alkenyl, C₂₋₄ alkynyl, C₁₋₄ alkoxy, -C₀₋₂ alkylene-C₃₋₆ cycloalkyl, -C₀₋₂ alkylene-O-C₃₋₆ cycloalkyl, -C₀₋₂ alkylene-4- to 7-membered heterocycloalkyl, -C₀₋₂ alkylene-O-4- to 7-membered heterocycloalkyl, -C₀₋₂ alkylene-NHC₁₋₄ alkyl, or -C₀₋₂ alkylene-N(C₁₋₄ alkyl)₂, wherein the alkyl, alkylene, alkenyl, alkynyl, alkoxy, cycloalkyl or heterocycloalkyl is optionally substituted with 1 to 4 R^{z};
K is 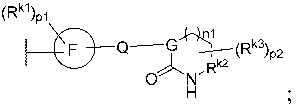
G is selected from N or CH;
each Q is independently selected from a bond, -O-, -S-, -CH₂-, -NR^{q}-, - C(=O)-, -NR^{q}C(=O)-, or -C(=O)NR^{q}-;
a nitrogen-nitrogen bond, a nitrogen-oxygen bond, or a nitrogen-sulphur bond cannot be directly formed between Q and G;
R^{q} is selected from H or C₁₋₄ alkyl;
F is selected from C₃₋₂₀ carbocyclyl, C₆₋₂₀ aryl, 3- to 20-membered heterocyclyl or 5- to 20-membered heteroaryl;
each R^{k1} is independently selected from H, deuterium, halogen, OH, =O, NH₂, CN, COOH, CONH₂, C₁₋₄ alkyl, C₂₋₄ alkenyl, C₂₋₄ alkynyl, C₁₋₄ alkoxy, C₃₋₆ cycloalkyl, or 3- to 6-membered heterocycloalkyl, wherein the alkyl, alkenyl, alkynyl, alkoxy, cycloalkyl or heterocycloalkyl is optionally substituted with 1 to 4 R^{z};
each R^{k2} is independently selected from a bond, -C(=O)-, -S(=O)₂-, -S(=O)- or -C(R^{k3})₂-;
each R^{k3} is independently selected from H, deuterium, halogen, OH, =O, NH₂, CN, COOH, CONH₂, C₁₋₄ alkyl, C₁₋₄ alkoxy, C₃₋₈ cycloalkyl, or 3- to 8-membered heterocyclyl, wherein the alkyl, alkoxy, cycloalkyl or heterocyclyl is optionally substituted with 1 to 4 R^{z};
alternatively, two R^{k3} are directly connected to form C₃₋₈ carbocyclyl or 4- to 8-membered heterocyclyl, wherein the carbocyclyl or heterocyclyl is optionally substituted with 1 to 4 R^{z};
R^{L2} and R^{z} are each independently selected from deuterium, halogen, OH, =O, CF₃, SF₅, CN, NH₂, NO₂, COOH, CONH₂, NHC₁₋₄ alkyl, N(C₁₋₄ alkyl)₂, COOH, CONH₂, C₁₋₄ alkyl, C₂₋₄ alkenyl, C₂₋₄ alkynyl, C₁₋₄ alkoxy, -S-C₁₋₄ alkyl, or -C₀₋₄ alkylene-C₃₋₆ cycloalkyl, wherein the alkyl, alkylene, alkoxy, alkenyl, alkynyl, or cycloalkyl is optionally substituted with 1 to 4 substituents selected from deuterium, F, Cl, Br, I, OH, CN, C₁₋₄ alkyl, or C₁₋₄ alkoxy;
n1 is selected from 0, 1, 2 or 3;
each p1 or p2 is independently selected from 0, 1, 2, 3, 4 or 5.

As a second embodiment of the present invention, provided is the compound as shown in the above general formula (I) or the stereoisomer, the racemate, the tautomer, the deuterated substance, the solvate, the prodrug, the metabolite, the pharmaceutically acceptable salt or the cocrystal thereof, wherein
L is -Ak1-Cy1-Ak2-Cy2-Ak3-Cy3-Ak4-Cy4-Ak5-;
Ak1, Ak2, Ak3, Ak4 and Ak5 are each independently selected from -(CH₂)_{q}-, -(CH₂)_{q}-O-, -O-(CH₂)_{q}-, -(CH₂)_{q}-S-, -S-(CH₂)_{q}-, -(CH₂)_{q}-NR^{L}-, -NR^{L}-(CH₂)_{q}-, - (CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 R^{z};
each R^{L} is independently selected from H or C₁₋₄ alkyl;
each Cy1, Cy2, Cy3 or Cy4 is independently selected from a bond or one of the following groups optionally substituted with 1 to 4 R^{L2}: 4- to 7-membered nitrogen-containing mono-heterocyclyl, 4- to 12-membered nitrogen-containing fused-heterocyclyl, 5- to 13-membered nitrogen-containing spiro-heterocyclyl, 7-to 12-membered nitrogen-containing bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl;
F is selected from a C₃₋₇ monocyclic group, a C₄₋₁₀ fused ring group, a C₅₋₁₂ spiro ring group, a C₅₋₁₀ bridged ring group, 4- to 7-membered mono-heterocyclyl, 4- to 10-membered bicyclic fused-heterocyclyl, 8- to 15-membered tricyclic fused-heterocyclyl, 12- to 19-membered tetracyclic fused-heterocyclyl, 5- to 17-membered spiro-heterocyclyl, 5- to 10-membered bridged-heterocyclyl, C₆₋₁₄ aryl, or 5- to 10-membered heteroaryl;
B₁ is selected from phenyl, benzo C₄₋₆ carbocyclyl, benzo 4- to 6-membered heterocyclyl, 5- to 6-membered heteroaryl, or 8- to 10-membered heteroaryl, wherein the B₁ is optionally substituted with 1 to 4 R^{b1};
R^{b2} is selected from phenyl, benzo C₄₋₆ carbocyclyl, benzo 4- to 6-membered heterocyclyl, 5- to 6-membered heteroaryl, or 8- to 10-membered heteroaryl, wherein the R^{b2} is optionally substituted with 1 to 4 R^{b2a};
the remaining definitions are the same as those in the first embodiment of the present invention.

As a third embodiment of the present invention, provided is the compound as shown in the above general formula (I) or the stereoisomer, the racemate, the tautomer, the deuterated substance, the solvate, the prodrug, the metabolite, the pharmaceutically acceptable salt or the cocrystal thereof, wherein
R^{L} is selected from H, methyl or ethyl;
Cy1, Cy2, Cy3 and Cy4 are each independently selected from a bond or one of the following groups optionally substituted with 1 to 4 R^{L2}: phenyl, pyridyl, pyrimidyl, pyrazinyl, pyridazinyl, pyrazolyl, thiazolyl, oxazolyl, triazolyl, 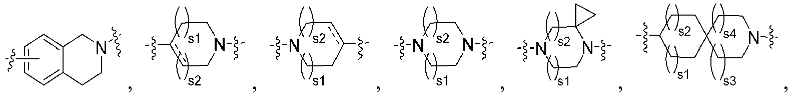 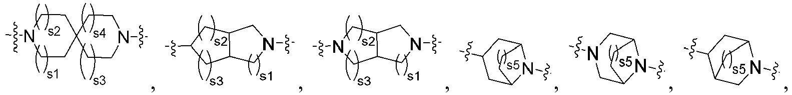 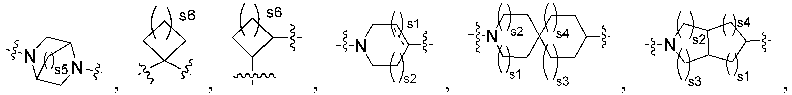 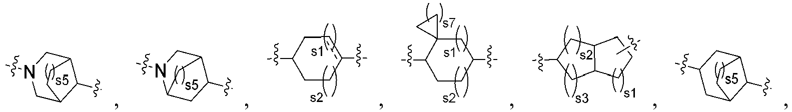 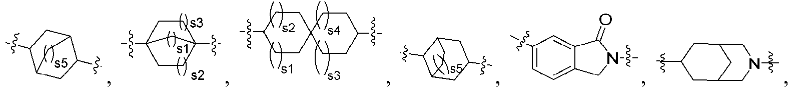 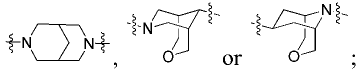
s1, s3 and s5 are each independently selected from 0, 1 or 2;
s2 and s4 are each independently selected from 0 or 1;
s6 is selected from 0, 1, 2 or 3;
s7 is selected from 1, 2 or 3; 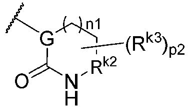 is selected from 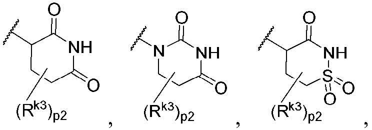 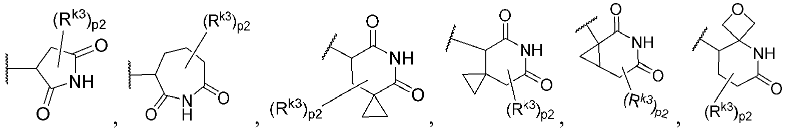 or 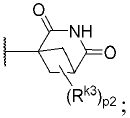
F is selected from cyclobutyl, cyclopentyl, cyclohexyl, bicyclo[1.1.1]pentanyl, 6,7-dihydro-5H-cyclopenta[c] pyridyl, 2,3-dihydro-1H-indenyl, phenyl, naphthyl, anthracenyl, phenanthryl, azetidinyl, pyrrolidyl, piperidyl, morpholinyl, pyridyl, pyrimidyl, pyridazinyl, pyrazinyl, triazinyl, pyrrolyl, pyrazolyl, imidazolyl, triazolyl, oxazolyl, furyl, thienyl, thiazolyl, 2-pyridonyl, 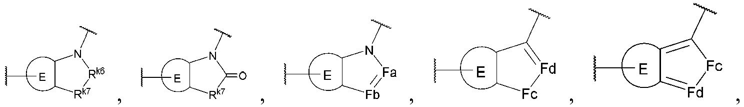 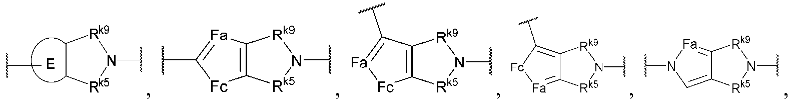 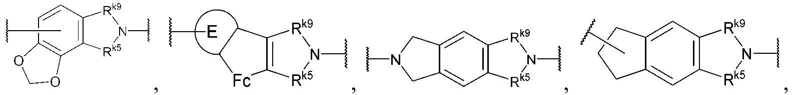 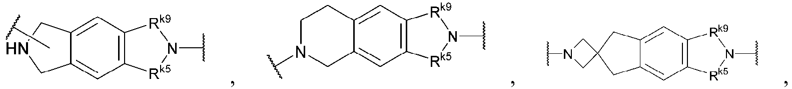 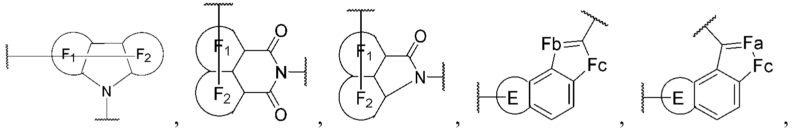 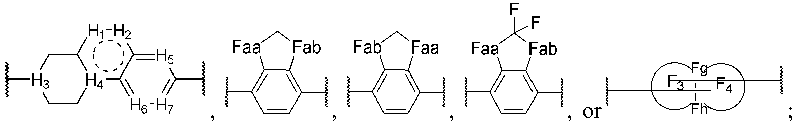 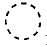 represents that the ring is an aromatic ring or a non-aromatic ring;
Fa is selected from N, CH or CR^{k1};
Fb is selected from N, CH or CR^{k1};
Fc is selected from O, S, NH, N(CH₃) or NR^{k7a};
Fd is selected from N, CH or CR^{k1};
Fg is selected from N or C;
Fh is selected from N or C;
Faa is selected from a bond, O, or CH₂;
Fab is selected from O or CH₂;
H₁ is selected from N, NH, CH, CH₂, CHR^{k1}, NR^{k1}, CR^{k1}, C(=O), or C(R^{k1})₂;
H₂ is selected from a bond, O, N, NH, CH, CH₂, CHR^{k1}, NR^{k1}, CR^{k1} or C(R^{k1})₂;
H₃ is selected from N or CH;
H₄ is selected from C, N or CH;
H₅, H₆ and H₇ are each independently selected from N, C, CH or CR^{k1}, and H₅, H₆ and H₇ contain at most 2 N;
ring E is selected from phenyl or 5- to 6-membered heteroaryl, wherein the ring E is optionally substituted with 1 to 3 R^{k1};
ring F₁, ring F₂, ring F₃ and ring F₄ are each independently selected from phenyl or 5- to 6-membered heteroaryl, wherein the ring F₁, ring F₂, ring F₃ and ring F₄ are optionally substituted with 1 to 2 R^{k1};
Q is selected from a bond, CH₂, NH, N(CH₃), O, S, C(=O), NHC(=O), C(=O)NH, N(CH₃)C(=O), or C(=O)N(CH₃);
R^{k1} and R^{k3} are each independently selected from H, deuterium, F, Cl, Br, I, OH, =O, NH₂, CF₃, CN, COOH, CONH2, methyl, ethyl, isopropyl, methoxy, ethoxy, isopropoxy, or cyclopropyl, wherein the methyl, ethyl, isopropyl, methoxy, ethoxy, isopropoxy or cyclopropyl is optionally substituted with 1 to 4 substituents selected from deuterium, F, Cl, Br, I, OH, or NH₂;
each R^{k5} is independently selected from 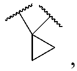 C(CH₃)₂, C(=O), CH₂, CH₂CH₂, S(=O)₂, 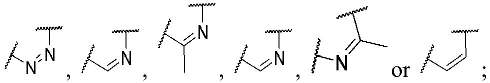
each R^{k6} is independently selected from C(=O), CH, S(=O), S(=O)₂, CH₂ or N;
each R^{k7} is independently selected from 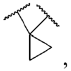 C(CH₃)₂, CH₂, O or NR^{k7a};
R^{k7a} is selected from H, methyl, ethyl, propyl, isopropyl, vinyl, propenyl, allyl, ethynyl, propynyl, propargyl, cyclopropyl, cyclobutyl, cyclopentyl, cyclohexyl, azetidinyl, pyrrolidyl, piperidyl, oxetanyl, tetrahydrofuryl, or tetrahydropyranyl, wherein the methyl, ethyl, propyl, isopropyl, cyclopropyl, cyclobutyl, cyclopentyl, cyclohexyl, azetidinyl, pyrrolidyl, piperidyl, oxetanyl, tetrahydrofuryl or tetrahydropyranyl is optionally substituted with 1 to 4 substituents selected from deuterium, F, Cl, Br, I, OH, CN, CF₃, C₁₋₄ alkyl, C₁₋₄ alkoxy, C₂₋₄ alkenyl, C₂₋₄ alkynyl, or C₃₋₆ cycloalkyl;
each R^{k9} is independently selected from a bond, 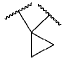 C(CH₃)₂, C(=O), CH₂, CH₂CH₂ or S(=O)₂;
R^{L2} and R^{z} are each independently selected from deuterium, F, Cl, Br, I, OH, =O, CF₃, SF₅, CN, NH₂, NO₂, COOH, CONH₂, N(CH₃)₂, NHCH₃, methyl, ethyl, vinyl, ethynyl, methoxy, ethoxy, methylthio, cyclopropyl, cyclobutyl, cyclopentyl, cyclohexyl, -CH₂-cyclopropyl, -CH₂-cyclobutyl, -CH₂-cyclopentyl, or -CH₂-cyclohexyl, wherein the methyl, ethyl, vinyl, ethynyl, methoxy, ethoxy, methylthio, cyclopropyl, cyclobutyl, cyclopentyl, or cyclohexyl is optionally substituted with 1 to 4 substituents selected from deuterium, F, Cl, Br, I, OH, CN, C₁₋₄ alkyl, or C₁₋₄ alkoxy;
each p2 is independently selected from 0, 1, 2 or 3;
the remaining definitions are the same as those in the first or second embodiment of the present invention.

As a fourth embodiment of the present invention, provided is the compound as shown in the above general formula (I) or the stereoisomer, the racemate, the tautomer, the deuterated substance, the solvate, the prodrug, the metabolite, the pharmaceutically acceptable salt or the cocrystal thereof, wherein
each ring E is independently selected from phenyl, pyridyl, pyridazinyl, pyrazinyl, pyrimidyl, pyrrolyl, pyrazolyl, imidazolyl, thiazolyl, furyl, thienyl or oxazolyl, wherein the ring E is optionally substituted with 1 to 3 R^{k1};
ring F₁ and ring F₂ are each independently selected from phenyl, pyridyl, thiazolyl, furyl, thienyl or oxazolyl, wherein the ring F₁ and ring F₂ are optionally substituted with 1 to 2 R^{k1};
ring F₃ and ring F₄ are each independently selected from phenyl, pyridyl, pyridazinyl, pyrazinyl, pyrimidyl, triazinyl, pyrrolyl, pyrazolyl, imidazolyl, thiazolyl, furyl, thienyl or oxazolyl, wherein the ring F₃ and ring F₄ are optionally substituted with 1 to 2 R^{k1};
B₁ is selected from phenyl, thiazolyl, furyl, thienyl, oxazolyl, pyrazolyl, pyridyl, pyrimidyl, pyrazinyl, or pyridazinyl, wherein the B₁ is optionally substituted with 1 to 4 R^{b1};
R^{b2} is selected from 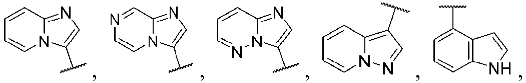 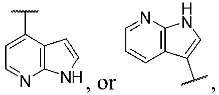 wherein the R^{b2} is optionally substituted with 1 to 4 R^{b2a};
R^{b1} and R^{b2a} are each independently selected from deuterium, F, Cl, Br, I, OH, CN, NH₂, NHCH₃, N(CH₃)₂ or one of the following groups optionally substituted with 1 to 4 R^{z}: methyl, ethyl, propyl, isopropyl, ethynyl, -CH₂-ethynyl, methoxy, ethoxy, cyclopropyl, cyclobutyl, -O-cyclopropyl, -O-cyclobutyl, -CH₂-cyclopropyl, -CH₂-cyclobutyl, oxetanyl, azetidinyl, tetrahydrofuryl, pyrrolidyl, -CH₂NHCH₃, - CH₂N(CH₃)₂, tetrahydropyranyl, piperidyl, -CH₂-azetidinyl, -CH₂-pyrrolidyl, or - CH₂-piperidyl;
Ak1, Ak2, Ak3, Ak4 and Ak5 are each independently selected from a bond, - O-, -S-, -OCH₂-, -CH₂O-, -OCH₂CH₂-, -CH₂CH₂O-, -C=C-, -C(CH₃)₂-, -CH₂-, - C(CH₃)₂-, -CH₂CH₂-, -CH₂CH₂CH₂-, -N(CH₃)-, -NH-, -CH₂N(CH₃)-, -CH₂NH-, - NHCH₂-, -CH₂CH₂N(CH₃)-, -CH₂CH₂NH-, -NHCH₂CH₂-, -C(=O)-, - C(=O)CH₂NH-, -CH₂C(=O)NH-, -C(=O)NH- or -NHC(=O)-;
Cy1, Cy2, Cy3 and Cy4 are each independently selected from a bond or one of the following optionally substituted groups: 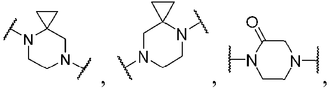 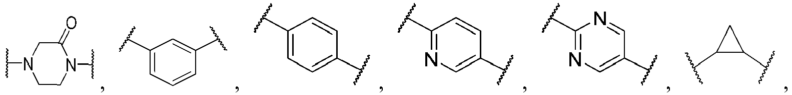 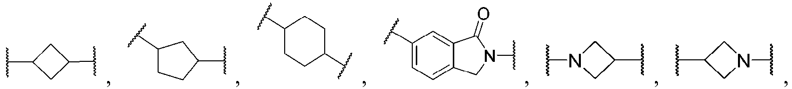 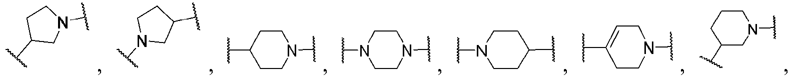 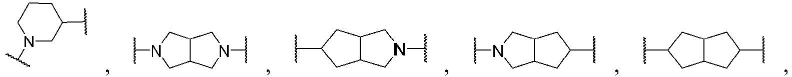 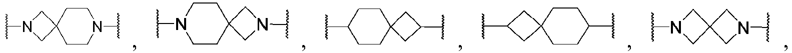 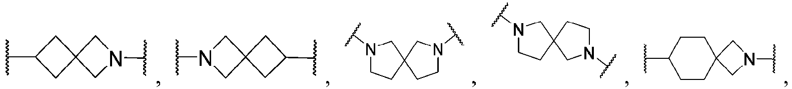 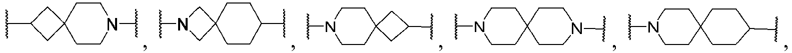 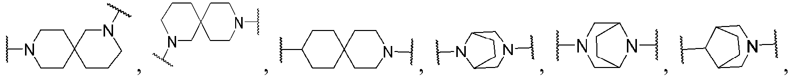 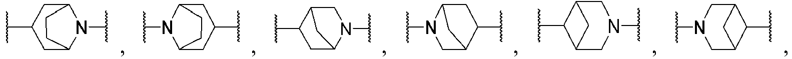 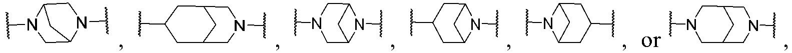 which, when substituted, is substituted with 1 to 4 substituents selected from deuterium, F, CF₃, OH, =O, COOH, CN, NH₂, hydroxymethyl, methyl, methoxy, or cyclopropyl;
the remaining definitions are the same as those in the first, second, or third embodiment of the present invention.

As a fifth embodiment of the present invention, provided is the compound as shown in the above general formula (I) or the stereoisomer, the racemate, the tautomer, the deuterated substance, the solvate, the prodrug, the metabolite, the pharmaceutically acceptable salt or the cocrystal thereof, wherein
B is selected from 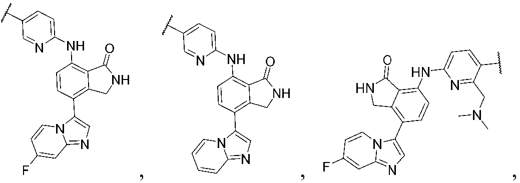 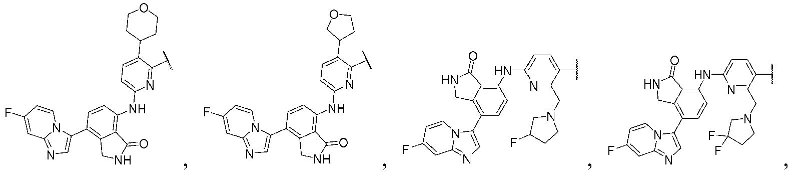 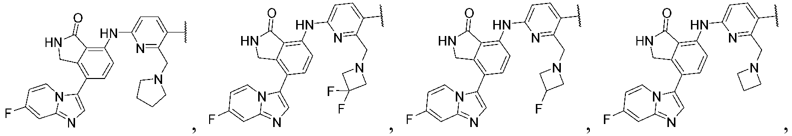 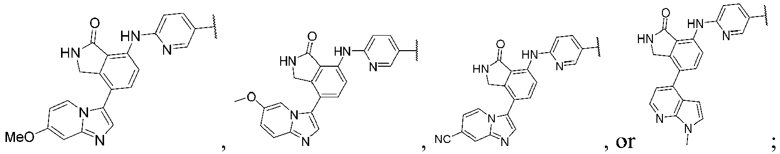
L is selected from a bond, -Ak1-, -NHCH₂-, -Cy1-, -Cy1-CH₂-, -Cy1-C≡C-, - Cy1-Cy2-, -Cy1-CH₂-Cy2-, -CH₂-Cy2-, -Cy1-Cy2-Cy3-, -Cy1-CH₂-Cy2-Cy3-, - Cy1-Cy2-CH₂-Cy3-, -NH-Cy1-, -NH-Cy1-Cy2-, -NH-Cy1-CH₂-Cy2, -Cy1-Ak2-, - Ak1-Cy1-, -Ak1-Cy1-Ak2-, -Ak1-Cy2-Ak2-Cy3-, or -Ak1-Cy2-Cy3-;
Cy1, Cy2 and Cy3 are each independently selected from one of the following optionally substituted groups: 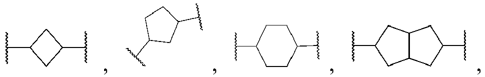 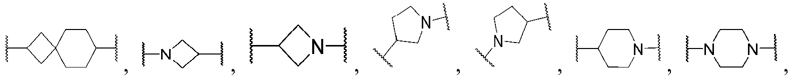 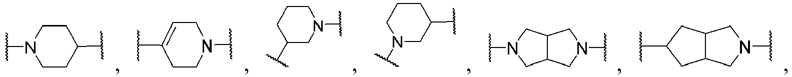 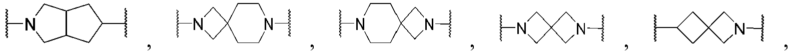 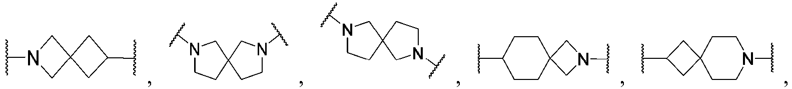 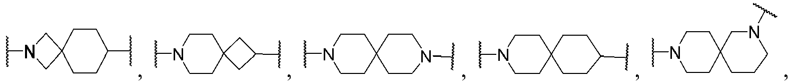 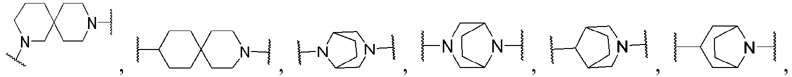 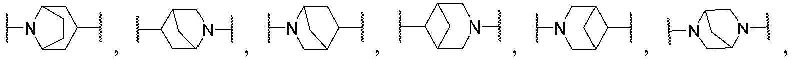 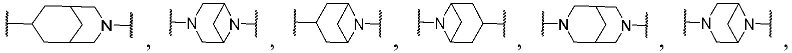 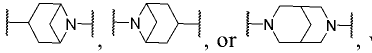 which, when substituted, is substituted with 1 to 4 substituents selected from deuterium, F, CF₃, OH, =O, COOH, CN, NH₂, hydroxymethyl, methyl, methoxy, or cyclopropyl;
the remaining definitions are the same as those in the first, second, third, or fourth embodiment of the present invention.

As a sixth embodiment of the present invention, provided is the compound as shown in the above general formula (I) or the stereoisomer, the racemate, the tautomer, the deuterated substance, the solvate, the prodrug, the metabolite, the pharmaceutically acceptable salt or the cocrystal thereof, wherein
L is selected from one of the structures shown in Table L-1 or Table L-2;
K is selected from one of the structural fragments shown in Table K-1 or Table K-2;
the remaining definitions are the same as those in the first, second, third, fourth, or fifth embodiment of the present invention.

As a seventh embodiment of the present invention, provided is the compound as shown in the above general formula (I) or the stereoisomer, the racemate, the tautomer, the deuterated substance, the solvate, the prodrug, the metabolite, the pharmaceutically acceptable salt or the cocrystal thereof, wherein the compound as shown in general formula (I) is general formula (Ia), 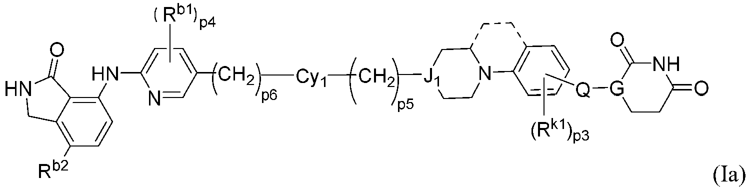
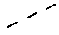 is a single bond 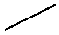 or is absent;
J₁ is selected from N or CH;
Cy1 is selected from one of the following optionally substituted groups: 4- to 7-membered nitrogen-containing mono-heterocyclyl, 4- to 12-membered nitrogen-containing fused-heterocyclyl, 5- to 13-membered nitrogen-containing spiro-heterocyclyl, 7- to 12-membered nitrogen-containing bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl, preferably one of the following optionally substituted groups: 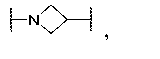 , 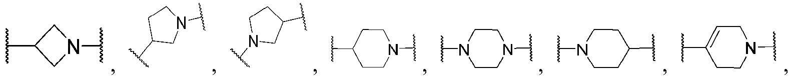 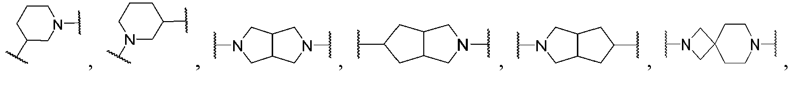 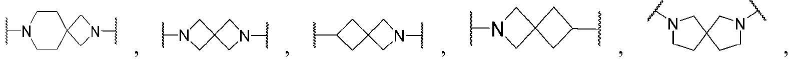 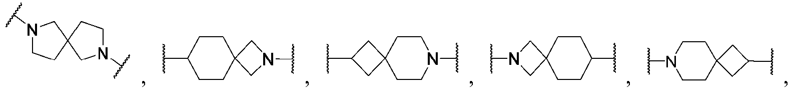 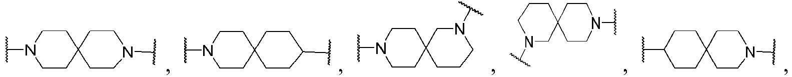 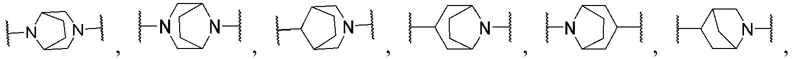 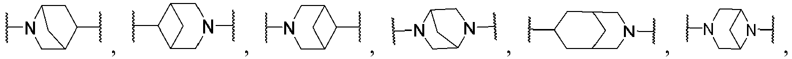 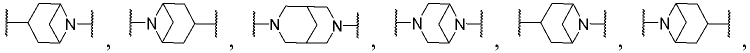 or 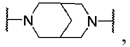 which, when substituted, is substituted with 1 to 4 substituents selected from deuterium, F, CF₃, OH, =O, COOH, CN, NH₂, hydroxymethyl, methyl, methoxy, or cyclopropyl;
G is selected from N or CH;
Q is selected from C(=O)NH or a bond;
R^{b2} is selected from 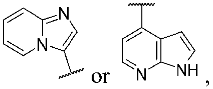 wherein the R^{b2} is optionally substituted with 1 to 4 R^{b2a};
each R^{b2a} is independently selected from deuterium, F, Cl, Br, OH, CN, NH₂, NHCH₃, N(CH₃)₂, methyl, ethyl, methoxy, or CF₃;
each R^{b1} is independently selected from deuterium, F, Cl, Br, I, OH, CN, NH₂, NHCH₃, N(CH₃)₂, methyl, ethyl, -CH₂NHCH₃, -CH₂N(CH₃)₂, tetrahydrofuryl, tetrahydropyranyl, piperidyl, -CH₂-azetidinyl, -CH₂-pyrrolidyl, or -CH₂-piperidyl, wherein the methyl, ethyl, tetrahydrofuryl, tetrahydropyranyl, piperidyl, azetidinyl, pyrrolidyl, or piperidyl is optionally substituted with 1 to 4 substituents selected from deuterium, F, CF₃, OH, =O, COOH, CN, or NH₂;
each R^{k1} is independently selected from H, deuterium, F, Cl, Br, NH₂, CF₃, CN, methyl, or ethyl;
p3 is selected from 0, 1, 2 or 3;
p4 is selected from 0, 1, 2 or 3;
p5 is selected from 0 or 1;
p6 is selected from 0 or 1.

The present invention relates to a compound as described below or a stereoisomer, a racemate, a tautomer, a deuterated substance, a solvate, a prodrug, a metabolite, a pharmaceutically acceptable salt or a cocrystal thereof, wherein the compound has a structure selected from one of the structures shown in Table E below:

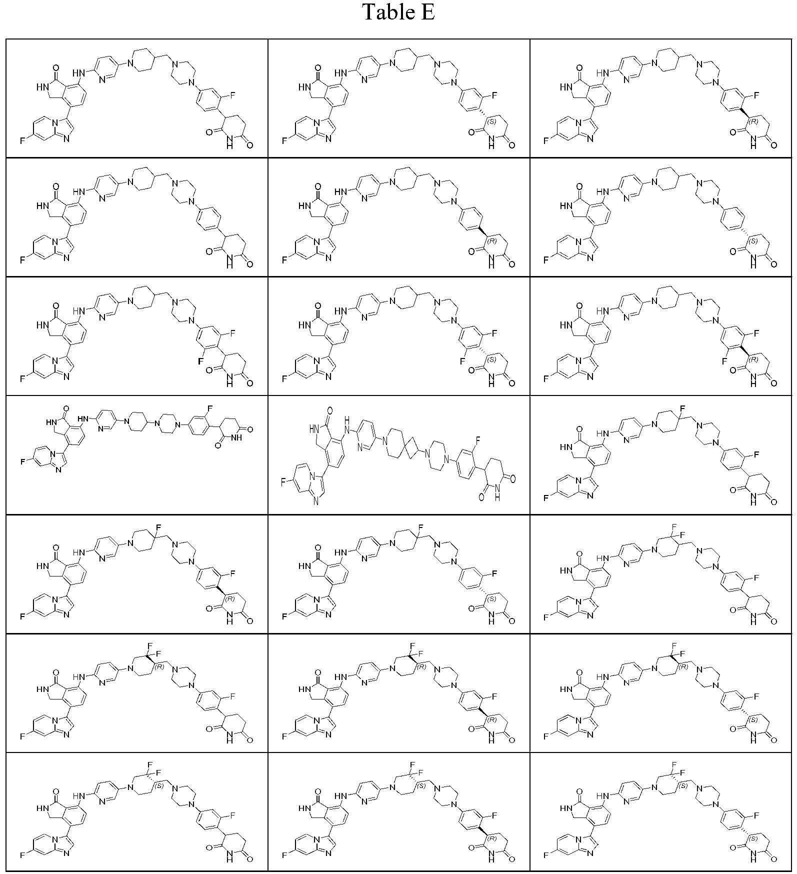

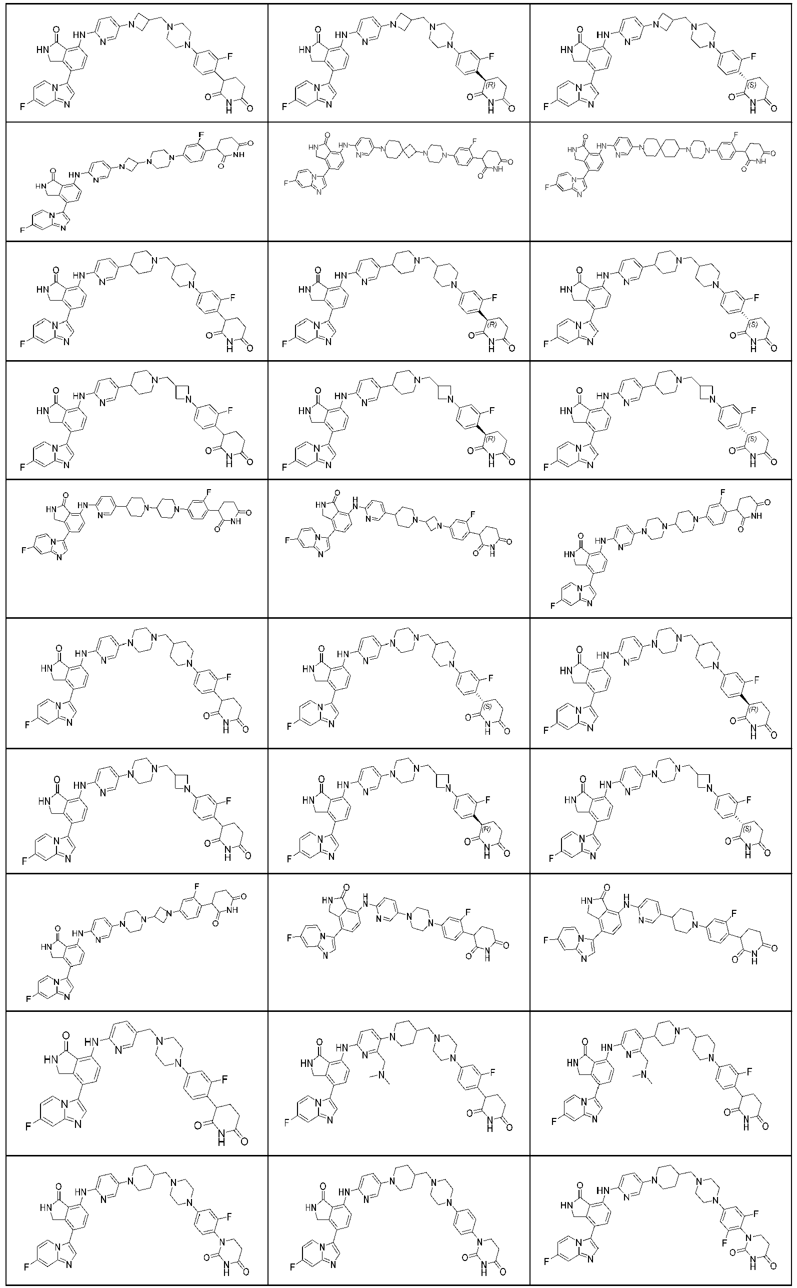

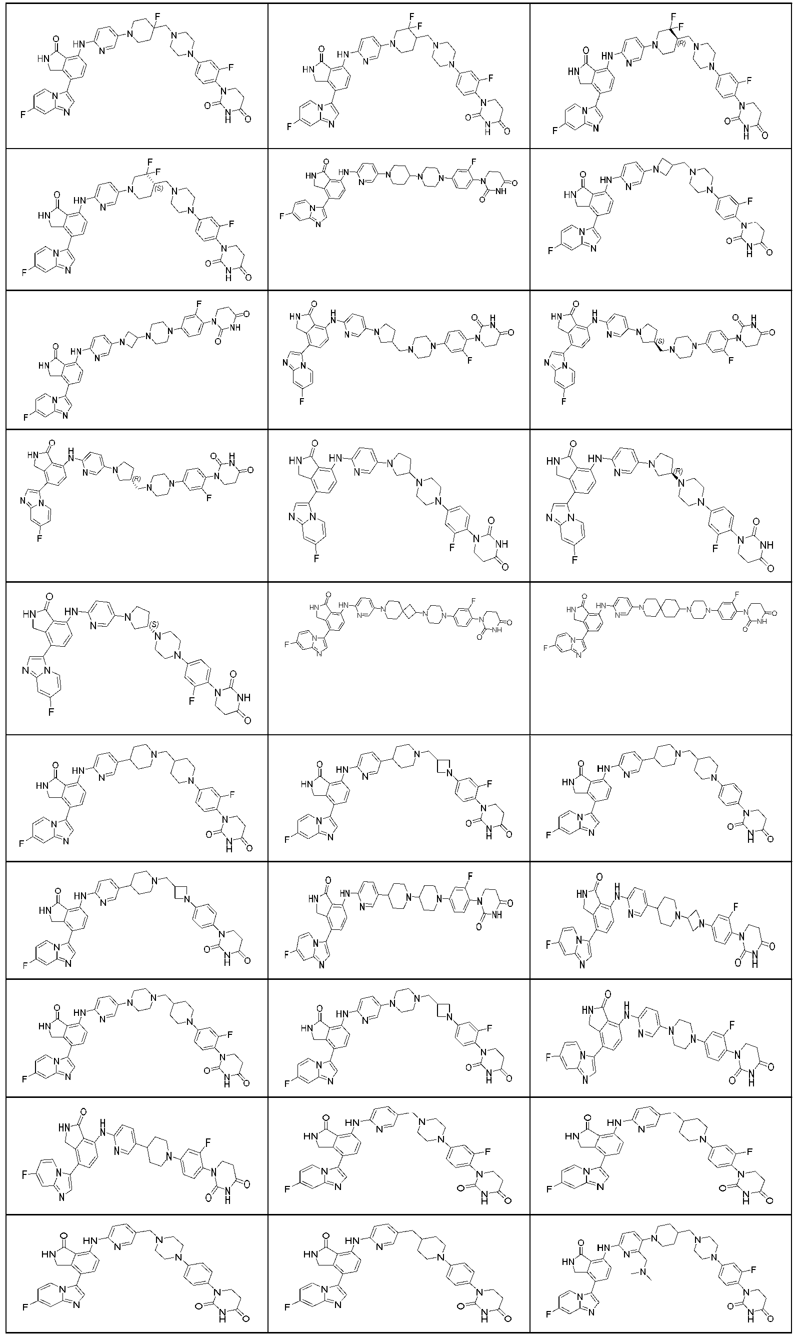

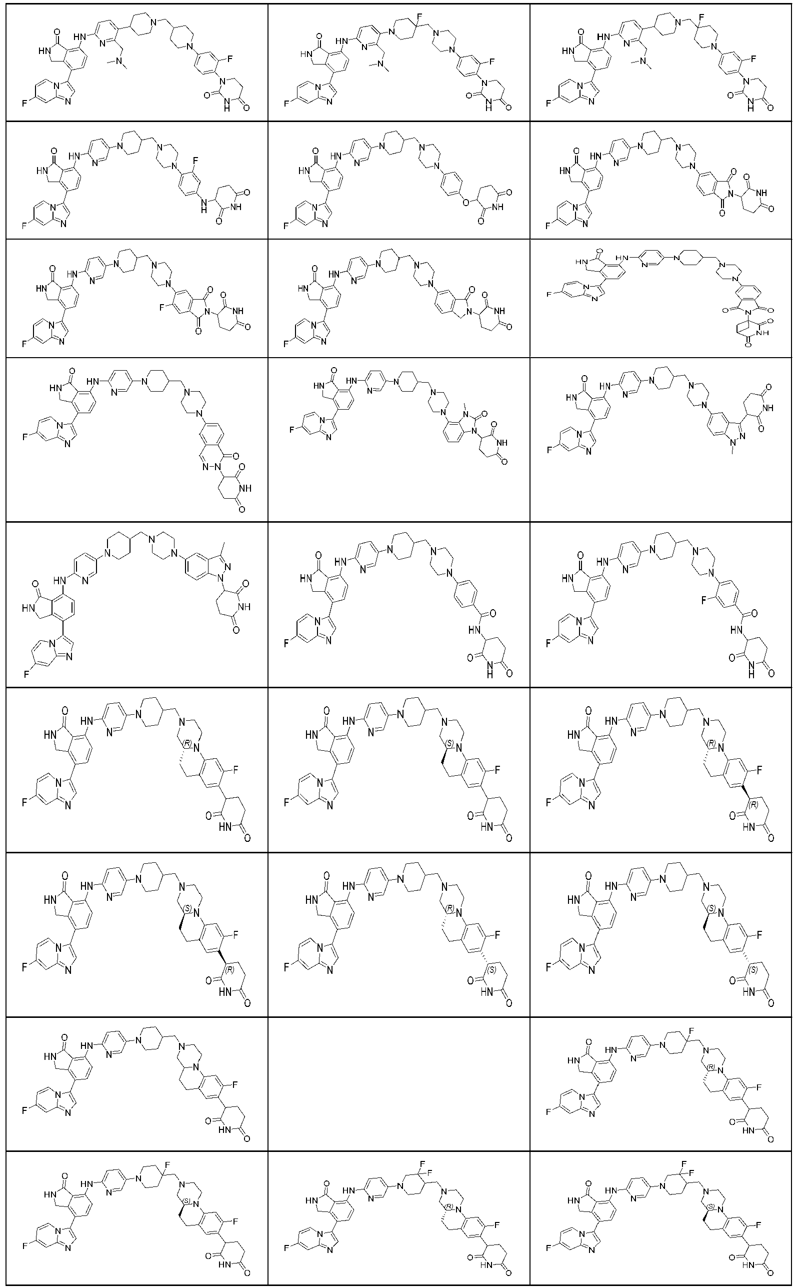

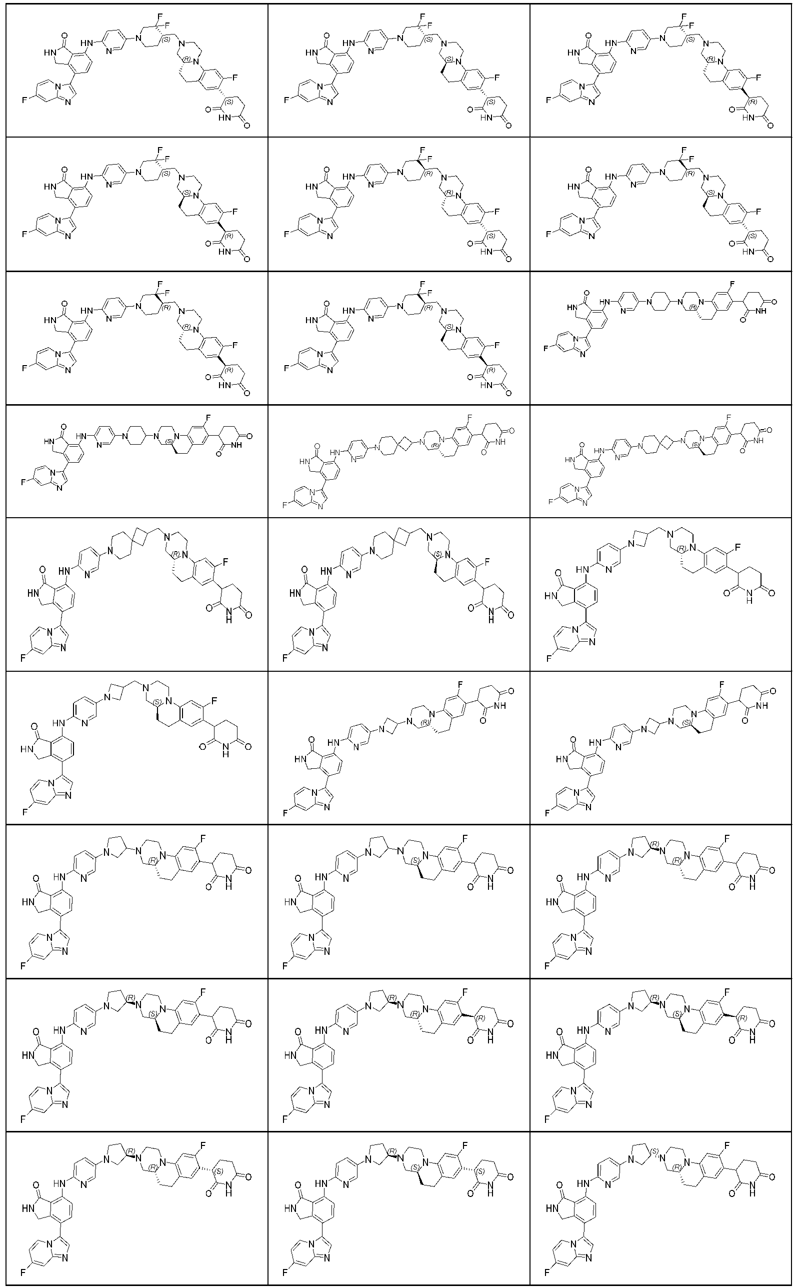

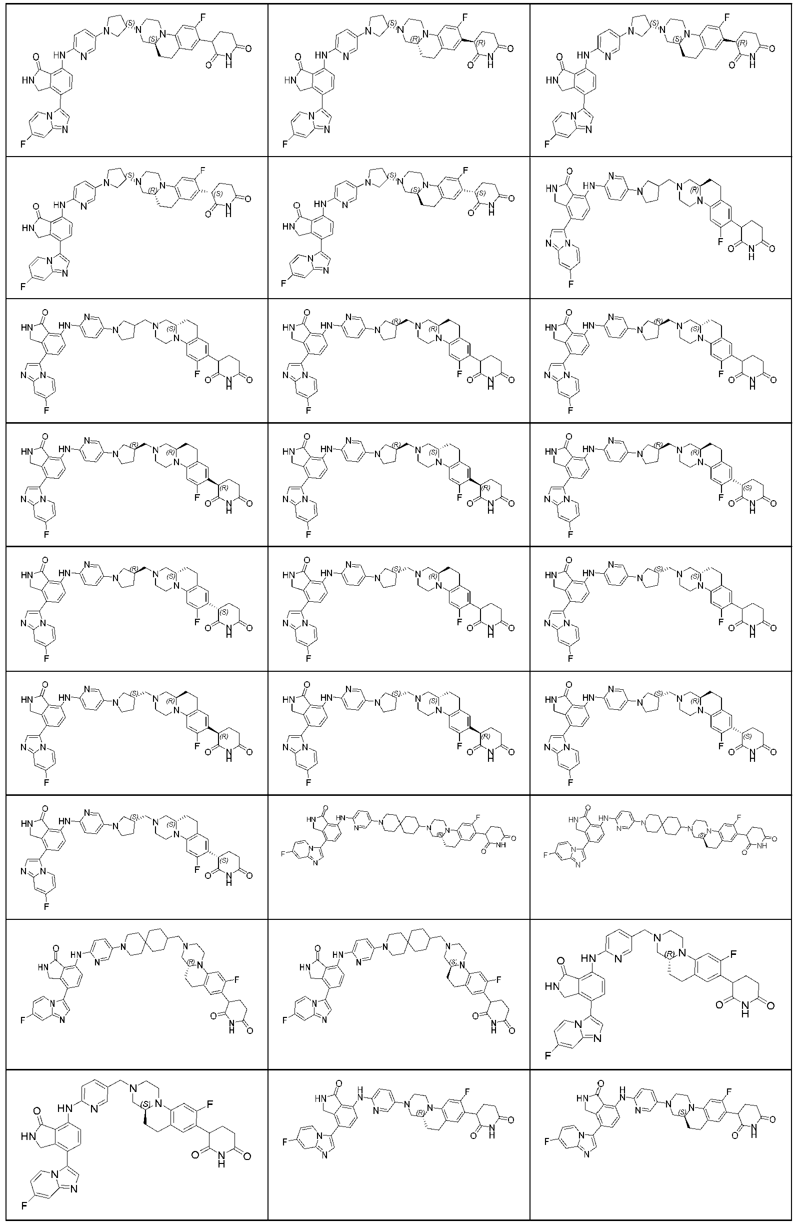

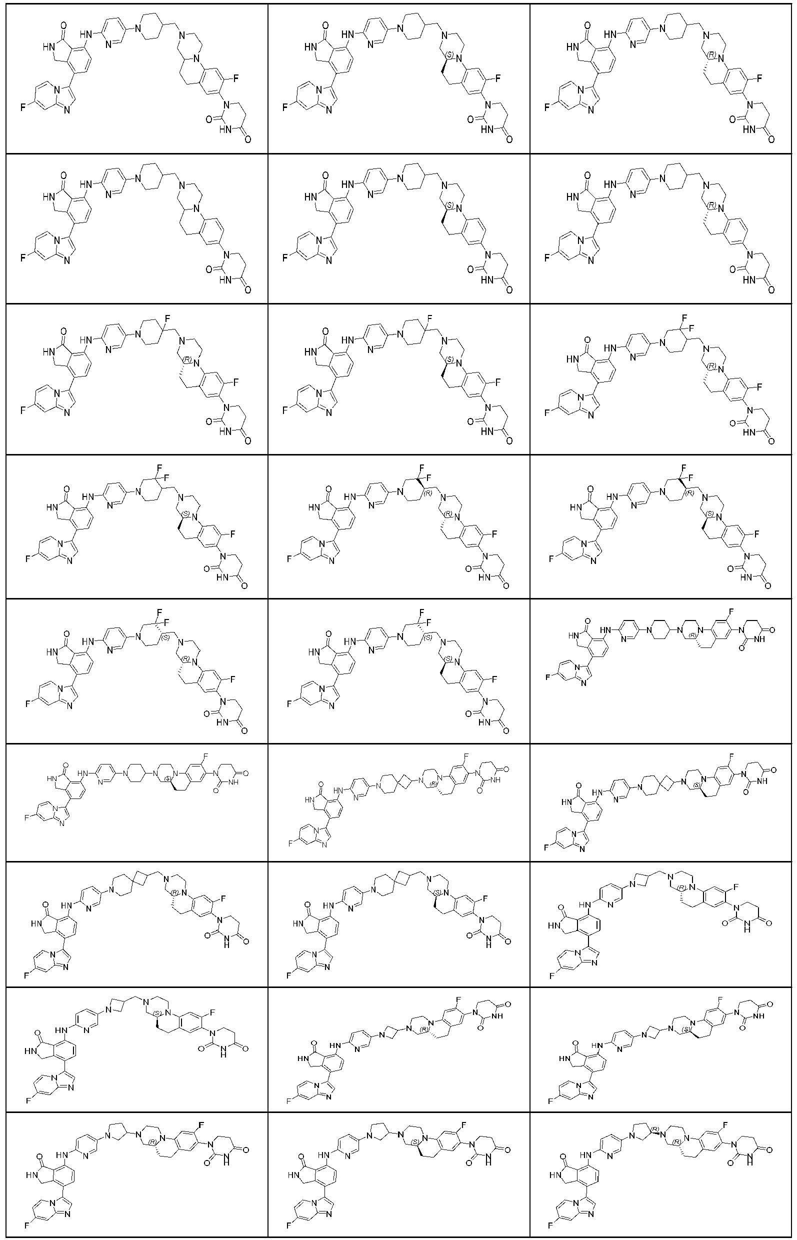

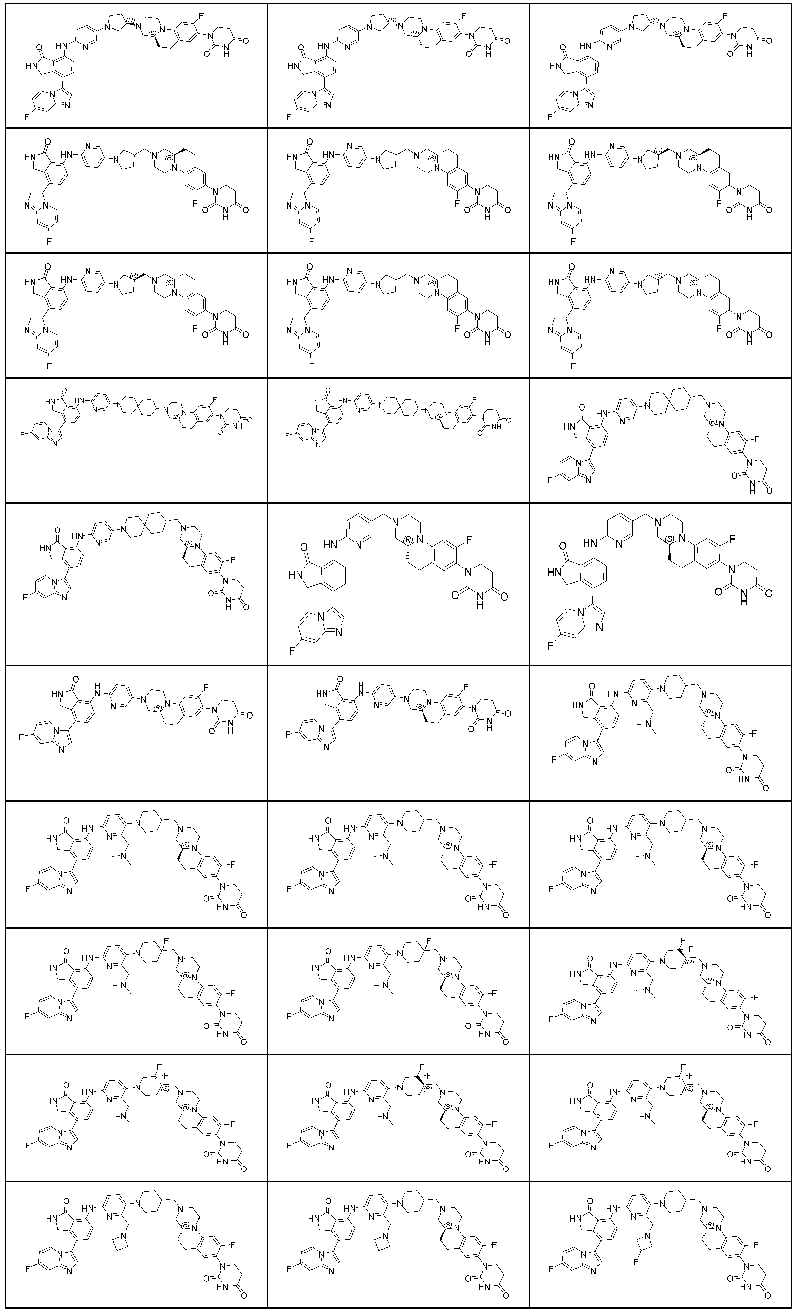

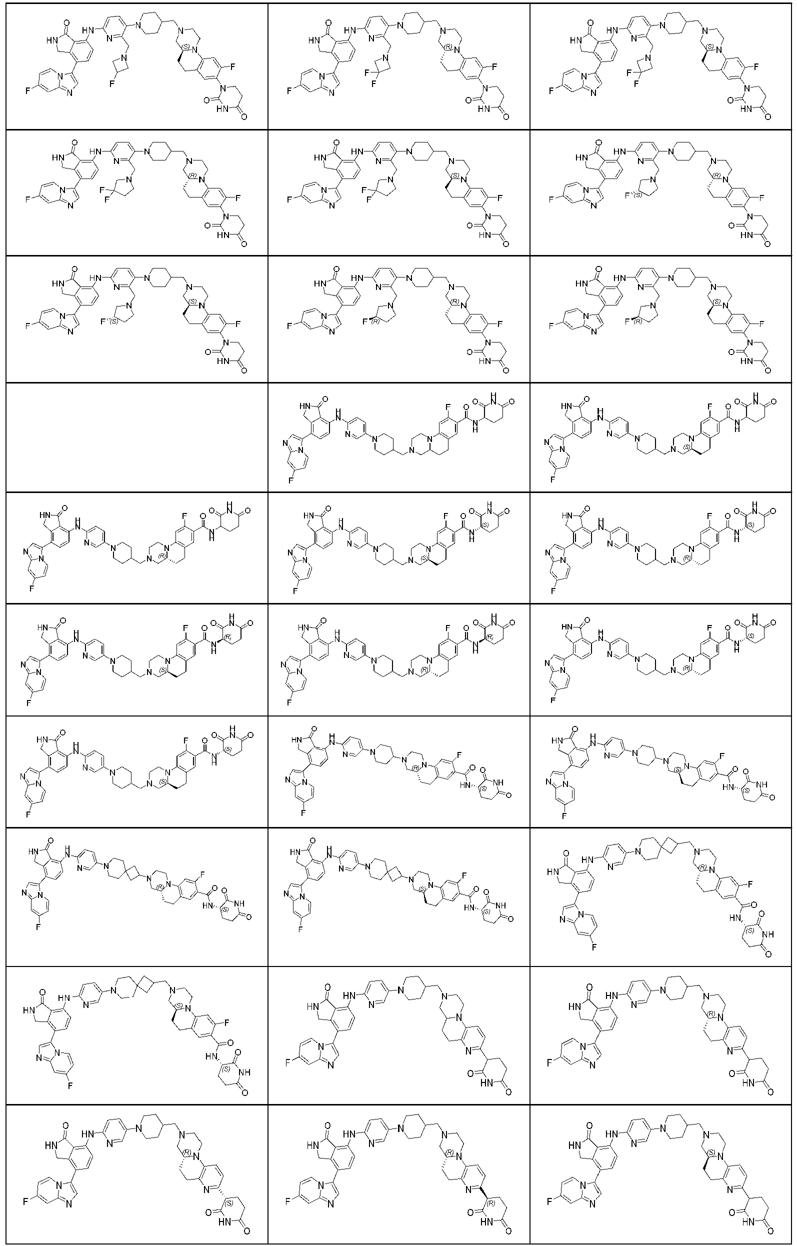

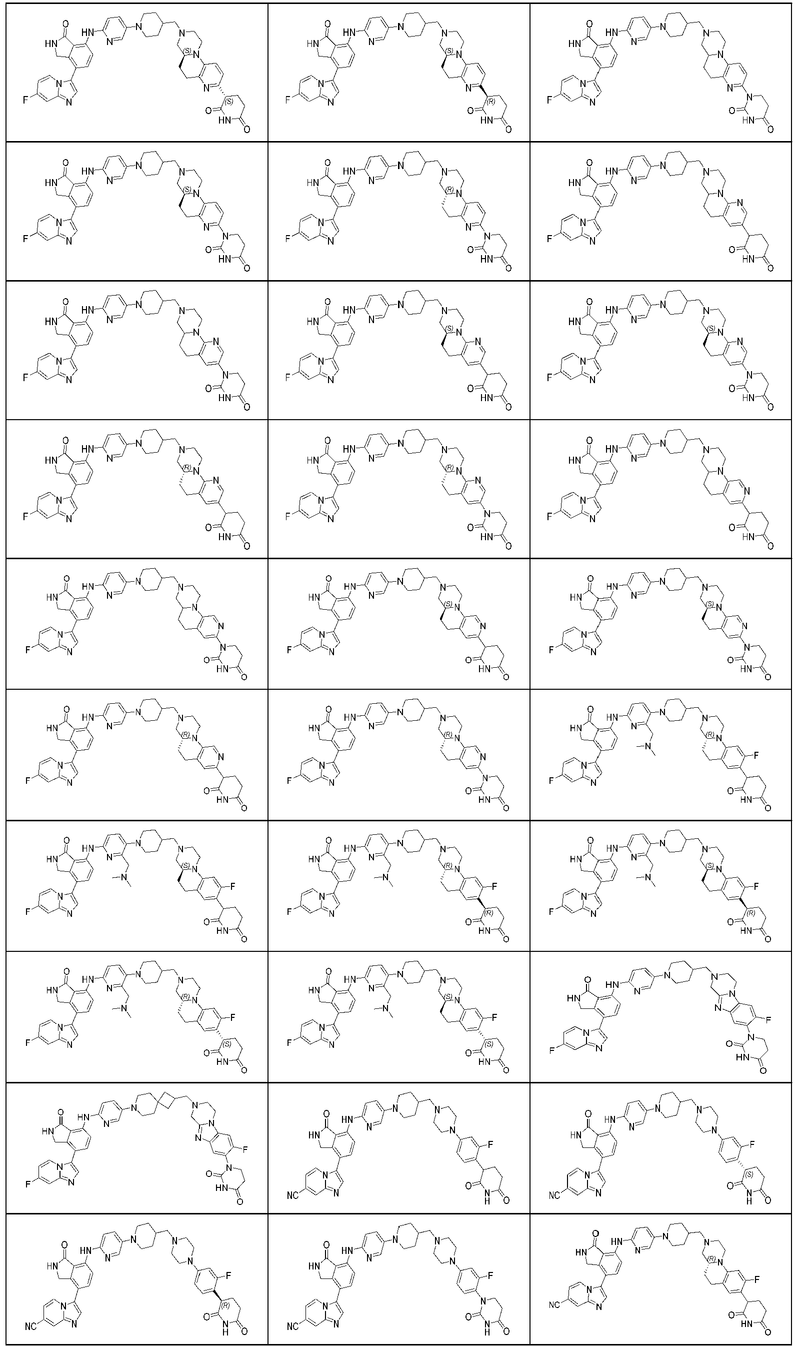

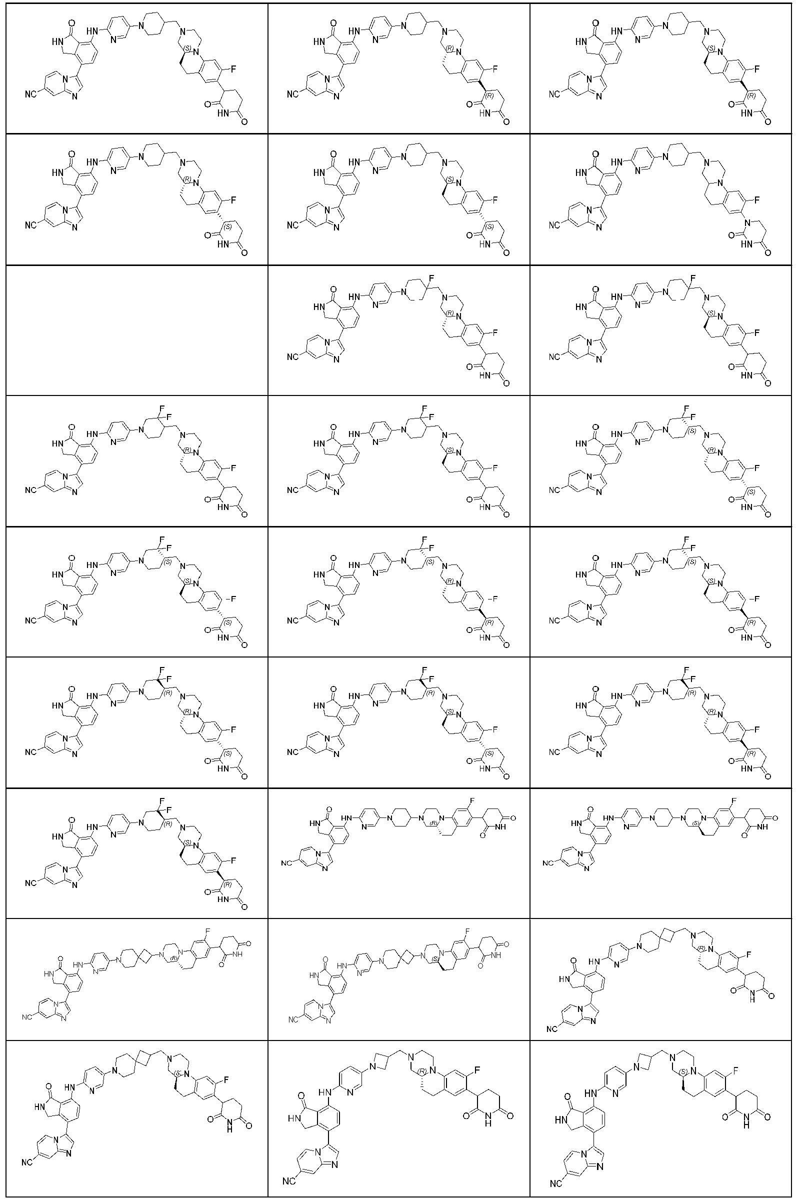

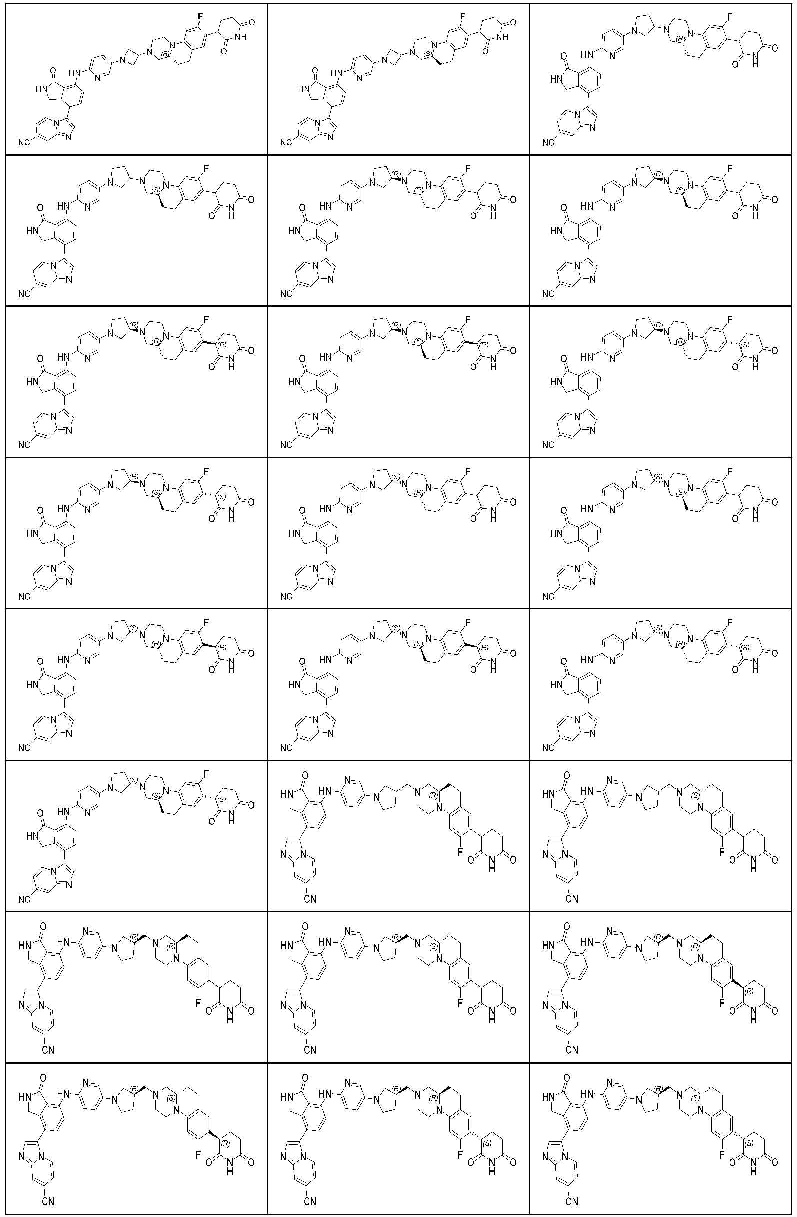

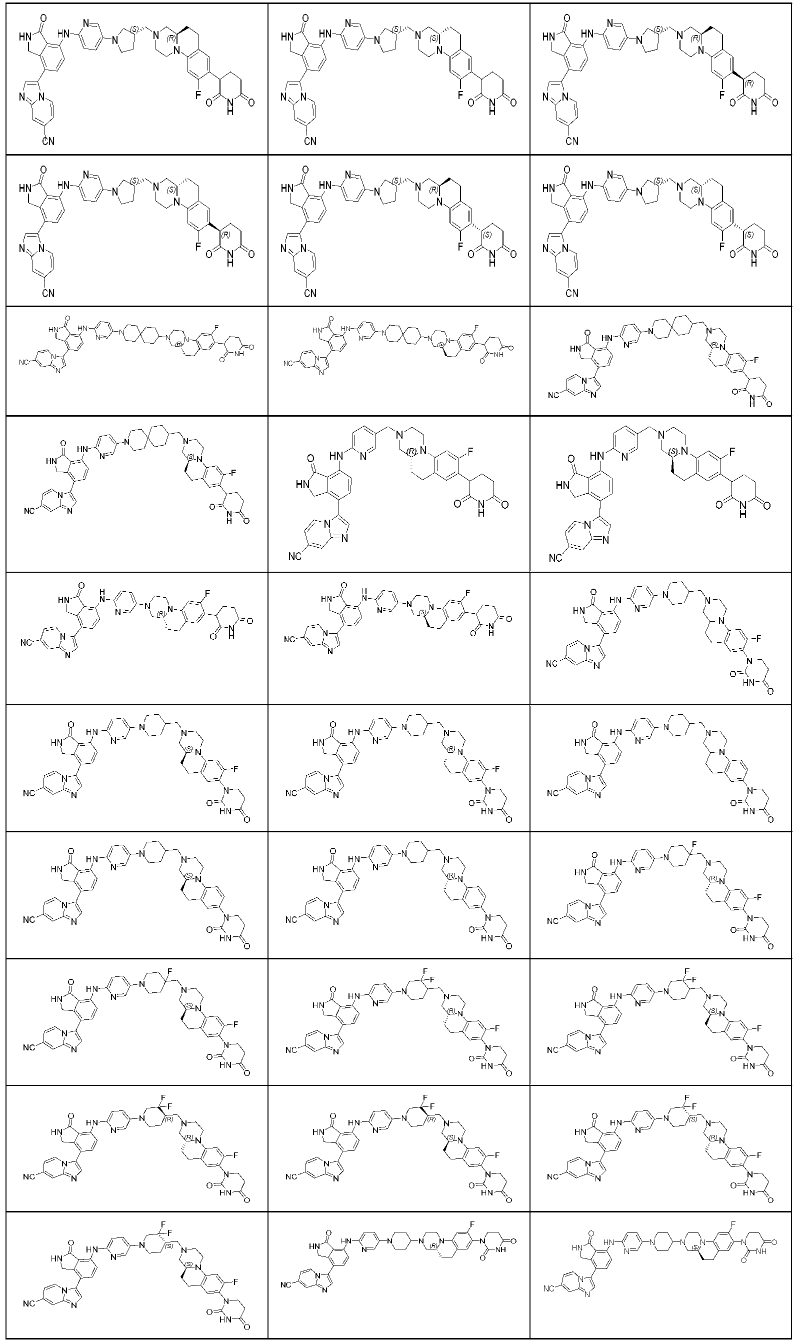

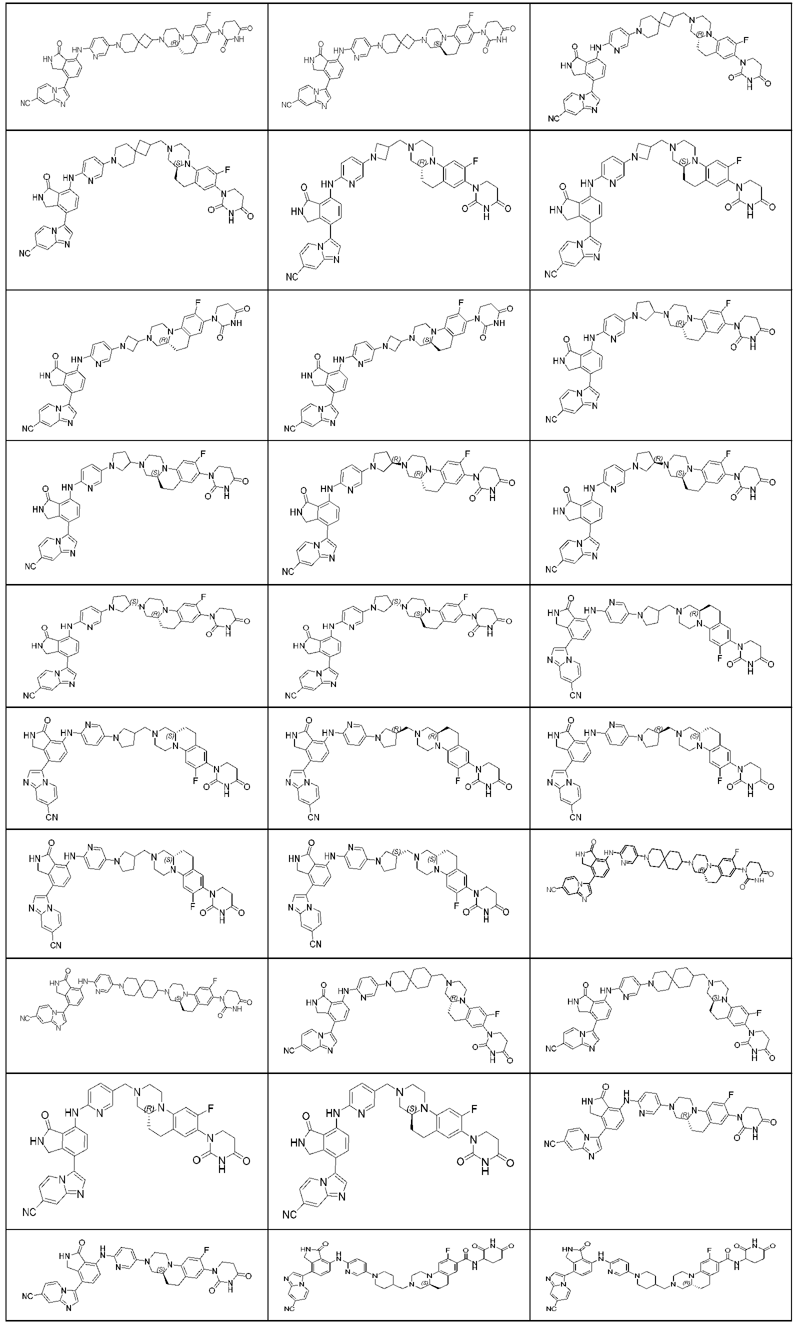

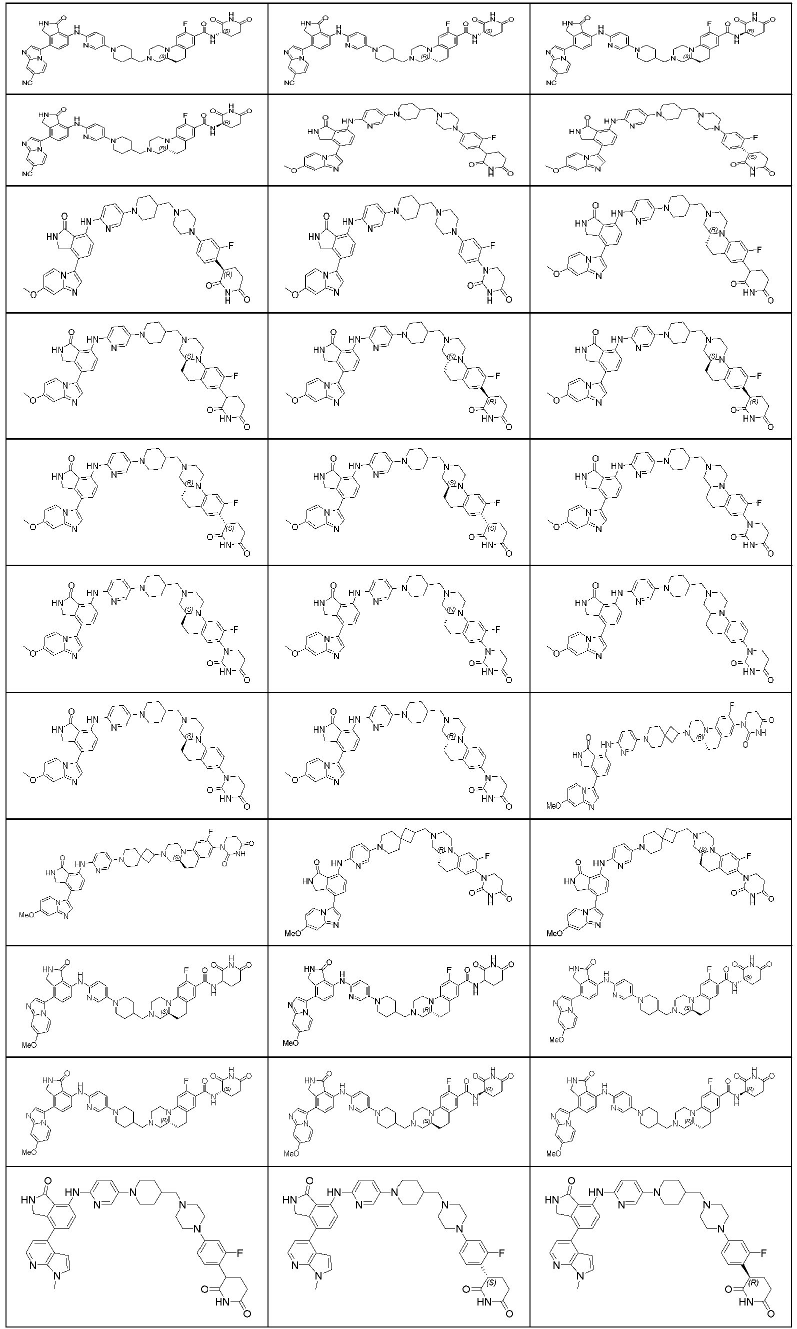

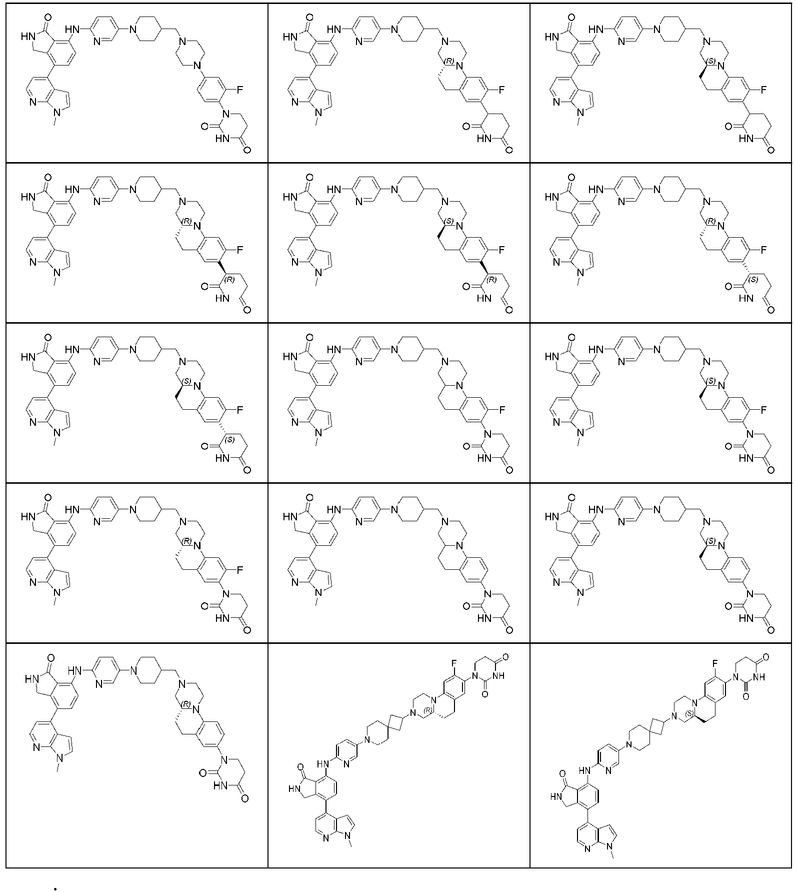

The present invention relates to a pharmaceutical composition, comprising the compound or the stereoisomer, the racemate, the tautomer, the deuterated substance, the solvate, the prodrug, the metabolite, the pharmaceutically acceptable salt or the cocrystal thereof as described above in the present invention, and a pharmaceutically acceptable carrier.

The present invention relates to the use of the compound or the stereoisomer, the racemate, the tautomer, the deuterated substance, the solvate, the prodrug, the metabolite, the pharmaceutically acceptable salt or the cocrystal thereof as described above in the present invention in the preparation of a medicament for the treatment of a disease related to HPK1 activity or expression.

The present invention relates to the use of the compound or the stereoisomer, the racemate, the tautomer, the deuterated substance, the solvate, the prodrug, the metabolite, the pharmaceutically acceptable salt or the cocrystal thereof as described above in the present invention in the preparation of a medicament for the treatment of a disease related to the inhibition or degradation of HPK1.

In some embodiments, the disease related to the inhibition or degradation of HPK1 is cancer, preferably a solid tumour.

The present invention relates to a pharmaceutical composition or a pharmaceutical preparation, comprising a therapeutically effective amount of the compound or the stereoisomer, the racemate, the tautomer, the deuterated substance, the solvate, the prodrug, the metabolite, the pharmaceutically acceptable salt or the cocrystal thereof according to the present invention, and a pharmaceutical excipient. The pharmaceutical composition can be in a unit preparation form (the amount of the drug substance in the unit preparation is also referred to as the "preparation strength").

The present invention further provides a method for treating a disease in a mammal, comprising administering to the mammal a therapeutically effective amount of the compound or the stereoisomer, the racemate, the tautomer, the deuterated substance, the solvate, the prodrug, the metabolite, the pharmaceutically acceptable salt or the cocrystal thereof, or the pharmaceutical composition according to the present invention. In some embodiments, the mammal mentioned in the present invention includes human.

The term "effective amount" or "therapeutically effective amount" according to the present application refers to a sufficient amount of the compound disclosed in the present application that is administered to ameliorate, to some extent, one or more symptoms of a disease or condition (such as cancer) being treated. In some embodiments, the outcome is the reduction and/or remission of signs, symptoms or causes of the disease, or any other desired change in the biological system. For example, an "effective amount" in terms of the therapeutic use is an amount of the included compound disclosed in the present application that is required to provide clinically significant reduction of the symptoms of the disease. Examples of the therapeutically effective amount include, but are limited to 1-1500 mg, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1500 mg, 80-1000 mg, and 80-800 mg;
in some embodiments, the pharmaceutical composition comprises the compound or the stereoisomer, the racemate, the tautomer, the deuterated substance, the solvate, the prodrug, the metabolite, the pharmaceutically acceptable salt or the cocrystal thereof according to the present invention in an amount including but not limited to 1-1500 mg, 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, 840 mg, and 1000 mg.

The present invention provides a method for treating a disease in a mammal, comprising administering to a subject a therapeutically effective amount of the compound or the stereoisomer, the racemate, the tautomer, the deuterated substance, the solvate, the prodrug, the metabolite, the pharmaceutically acceptable salt or the cocrystal thereof according to the present invention, wherein the therapeutically effective amount is preferably 1-1500 mg, and the disease is preferably cancer, more preferably a solid tumour.

The present invention provides a method for treating a disease in a mammal, comprising administering to a subject a medicament, i.e., the compound or the stereoisomer, the racemate, the tautomer, the deuterated substance, the solvate, the prodrug, the metabolite, the pharmaceutically acceptable salt or the cocrystal thereof according to the present invention in a daily dose of 1-1500 mg/day, wherein the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, 1000 mg/day, and 1500 mg/day.

The present invention relates to a kit, wherein the kit may comprise a composition in the form of a single dose or multiple doses and comprises the compound or the stereoisomer, the racemate, the tautomer, the deuterated substance, the solvate, the prodrug, the metabolite, the pharmaceutically acceptable salt or the cocrystal thereof according to the present invention, and the amount of the compound or the stereoisomer, the racemate, the tautomer, the deuterated substance, the solvate, the prodrug, the metabolite, the pharmaceutically acceptable salt or the cocrystal thereof according to the present invention is identical to the amount of same in the above-mentioned pharmaceutical composition.

In the present invention, the amount of the compound or the stereoisomer, the racemate, the tautomer, the deuterated substance, the solvate, the prodrug, the metabolite, the pharmaceutically acceptable salt or the cocrystal thereof according to the present invention is calculated in the form of free base in each case.

Unless otherwise stated, the terms used in the description and claims have the following meanings.

The carbon, hydrogen, oxygen, sulphur, nitrogen, F, Cl, Br and I involved in the groups and compounds of the present invention all comprise their isotopes, and the carbon, hydrogen, oxygen, sulphur or nitrogen involved in the groups and compounds of the present invention is optionally further substituted with one or more of their corresponding isotopes, wherein the isotopes of carbon comprise ¹²C, ¹³C and ¹⁴C, the isotopes of hydrogen comprise protium (H), deuterium (D, also known as heavy hydrogen) and tritium (T, also known as superheavy hydrogen), the isotopes of oxygen comprise ¹⁶O, ¹⁷O and ¹⁸O, the isotopes of sulphur comprise ³²S, ³³S, ³⁴S and ³⁶S, the isotopes of nitrogen comprise ¹⁴N and ¹⁵N, the isotopes of fluorine comprise ¹⁷F and ¹⁹F, the isotopes of chlorine comprise ³⁵Cl and ³⁷Cl, and the isotopes of bromine comprise ⁷⁹Br and ⁸¹Br.

"CN" refers to cyano.

"Halogen" refers to F, Cl, Br or I.

"Halogen-substituted" refers to substitution with F, Cl, Br, or I, including but not limited to substitution with 1 to 10 substituents selected from F, Cl, Br, or I, or substitution with 1 to 6 substituents selected from F, Cl, Br, or I, or substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The alkyl can be monovalent, divalent, trivalent or tetravalent.

"Alkylene" refers to a substituted or unsubstituted linear or branched divalent saturated hydrocarbon group, including -(CH₂)ᵥ- (v is an integer from 1 to 10), and examples of alkylene include, but are not limited to, methylene, ethylene, propylene, butylene, etc.

"Cycloalkyl" refers to a substituted or unsubstituted saturated carbocyclic hydrocarbon group, typically having 3 to 12 carbon atoms, and the cycloalkyl can be monocyclic, fused, bridged or spirocyclic. Non-limiting examples include cyclopropyl, cyclobutyl, cyclopentyl, cyclohexyl, cycloheptyl, cyclobutyl fused cyclobutyl, cyclobutyl spiro cyclobutyl, adamantane, etc. The cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 12 atoms, 3 to 8 atoms, or 1 to 3 heteroatoms selected from N, O or S. The C, N and S in the ring of the heterocycloalkyl can be oxidised to various oxidation states. The heterocycloalkyl can be monocyclic, fused, bridged or spirocyclic. The heterocycloalkyl can be connected to a heteroatom or a carbon atom, and non-limiting examples include oxiranyl, aziridinyl, oxetanyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl, piperazinyl, 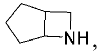 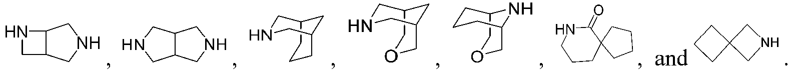 The heterocycloalkyl can be monovalent, divalent, trivalent or tetravalent.

"Alkenyl" refers to a substituted or unsubstituted linear or branched unsaturated hydrocarbon group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The alkenyl can be monovalent, divalent, trivalent or tetravalent.

"Alkynyl" refers to a substituted or unsubstituted linear or branched unsaturated hydrocarbon group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. The alkynyl can be monovalent, divalent, trivalent or tetravalent.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system. The carbocyclyl can be connected to an aromatic ring or non-aromatic ring, and the ring is optionally monocyclic, fused, bridged or spirocyclic. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring, 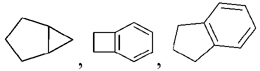 or 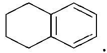 The "carbocyclyl" or "carbocyclic ring" can be monovalent, divalent, trivalent or tetravalent.

"Heterocyclyl" or "heterocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system, and contains 1 or more (including but not limited to 2, 3, 4 or 5) heteroatoms selected from N, O, S or Se, and the selectively substituted C, N and S in the ring of the heterocyclyl can be oxidised to various oxidation states. The heterocyclyl can be connected to a heteroatom or a carbon atom, can be connected to an aromatic ring or a non-aromatic ring, and is optionally monocyclic, bridged, fused or spirocyclic. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl, 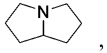 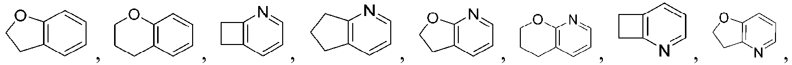 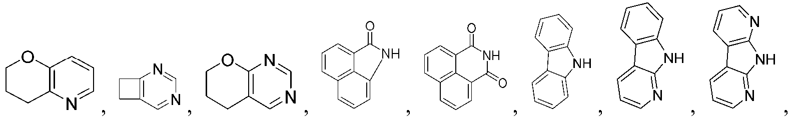 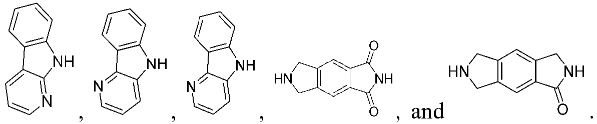 The "heterocyclyl" or "heterocyclic ring" can be monovalent, divalent, trivalent or tetravalent.

"Spiro ring" or "spiro ring group" refers to a substituted or unsubstituted polycyclic group that shares one atom (called a spiro atom) between monocyclic rings. The number of ring atoms in the spiro ring system includes, but is not limited to 5 to 20, 6 to 14, 6 to 12, or 6 to 10, wherein one or more rings can contain 0 or more (including but not limited to 1, 2, 3 or 4) double bonds, and can optionally contain 0 to 5 heteroatoms selected from N, O, S(=O)ₙ or Se(=O)ₙ (n is 0, 1 or 2).

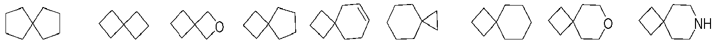

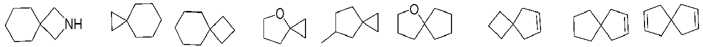

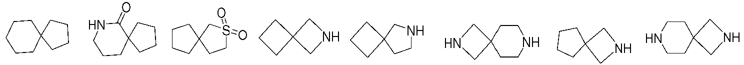

The "spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings can contain 0 or more (including but not limited to 1, 2, 3 or 4) double bonds, and can be substituted or unsubstituted, and each ring in the fused ring system can contain 0 to 5 heteroatoms or groups containing heteroatoms (including but not limited to N, S(=O)ₙ, Se(=O)ₙ or O, wherein n is 0, 1 or 2). The number of ring atoms in the fused ring system includes, but is not limited to 5 to 20, 5 to 14, 5 to 12, or 5 to 10. Non-limiting examples include: 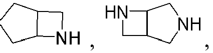 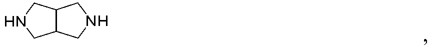 and 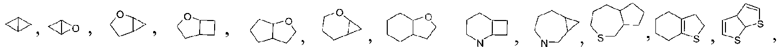 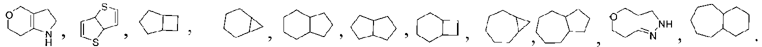 The "fused ring" or "fused ring group" can be monovalent, divalent, trivalent or tetravalent.

"Bridged ring" or "bridged ring group" refers to a substituted or unsubstituted polycyclic group containing any two atoms that are not directly connected, and can contain 0 or more double bonds. Any ring in the bridge ring system can contain 0 to 5 heteroatoms or groups containing heteroatoms (including but not limited to N, S(=O)n, Se(=O)ₙ or O, wherein n is 0, 1 or 2). The number of ring atoms includes, but is not limited to 5 to 20, 5 to 14, 5 to 12, or 5 to 10. Non-limiting examples include cubane, adamantane, and 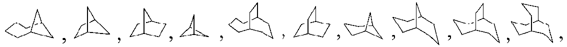 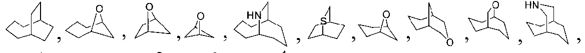 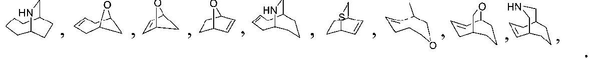 The "bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused-heterocyclic ring", "fused-heterocyclyl", "fused ring heterocyclyl" or "fused-heterocyclic ring group" refers to a "fused ring" containing a heteroatom.

"Spiro-heterocyclic ring", "spiro-heterocyclyl", "spiro ring heterocyclyl" or "spiro-heterocyclic ring group" refers to a "spiro ring" containing a heteroatom.

"Bridged-heterocyclic ring", "bridged-heterocyclyl", "bridged ring heterocyclyl" or "bridged-heterocyclic ring group" refers to a "bridged ring" containing a heteroatom.

"Aryl" or "aromatic ring" refers to a substituted or unsubstituted aromatic hydrocarbon group with a monocyclic ring or a fused ring, wherein the number of ring atoms in the aromatic ring includes, but is not limited to 6 to 18, 6 to 12, or 6 to 10 carbon atoms. The aryl ring can be fused to a saturated or unsaturated carbocyclic ring, wherein the ring connected to the parent structure is an aryl ring. Non-limiting examples include a benzene ring, a naphthalene ring, and 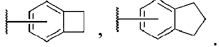 The "aryl" or "aromatic ring" can be monovalent, divalent, trivalent or tetravalent. When divalent, trivalent or tetravalent, the points of connection are on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O, S(=O)n or Se(=O)ₙ, wherein n is 0, 1 or 2), wherein the number of ring atoms in the heteroaromatic ring includes, but is not limited to 5-15, 5-10, or 5-6. The atoms C, N, S, and Se in the ring are optionally oxidised (i.e., C(=O), NO, S(=O)n, and Se(=O)n, wherein n is 1 or 2). Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazolyl, benzimidazolyl, benzopyridyl, pyrrolopyridyl, pyridonyl, etc. The heteroaryl ring can be fused to a saturated or unsaturated carbocyclic ring or heterocyclic ring, wherein the ring connected to the parent structure is an aryl ring. Non-limiting examples include 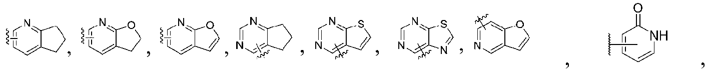 and 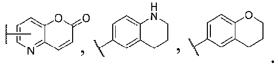 The definition of the heteroaryl herein is consistent with this definition. The heteroaryl can be monovalent, divalent, trivalent or tetravalent. When divalent, trivalent or tetravalent, the points of connection are on the aromatic ring.

"Substitution" or "substituted" refers to substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, a bridged ring group, a spiro ring group, a fused ring group, hydroxyalkyl, =O, carbonyl, aldehyde, carboxylic acid, carboxylate, -(CH₂)ₘ-C(=O)-R^{a}, -O-(CH₂)ₘ-C(=O)-R^{a}, -(CH₂)ₘ-C(=O)-NR^{b}R^{c}, -(CH₂)ₘS(=O)ₙR^{a}, -(CH₂)ₘ-alkenyl-R^{a}, OR^{d}, -(CH₂)ₘ-alkynyl-R^{a} (wherein m and n are 0, 1 or 2), arylthio, thiocarbonyl, silyl, -NR^{b}R^{c}, etc., wherein R^{b} and R^{c} are independently selected from H, hydroxyl, amino, carbonyl, alkyl, alkoxy, cycloalkyl, heterocyclyl, aryl, heteroaryl, sulphonyl, or trifluoromethylsulphonyl; alternatively, R^{b} and R^{c} may form five- or six-membered cycloalkyl or heterocyclyl; R^{a} and R^{d} are each independently selected from aryl, heteroaryl, alkyl, alkoxy, cycloalkyl, heterocyclyl, carbonyl, an ester group, a bridged ring group, a spiro ring group or a fused ring group.

"Substituted with 1 to X substituents selected from ..." refers to substitution with 1, 2, 3 ... X substituents selected from ..., wherein X is any integer from 1 to 10. For example, "substituted with 1 to 4 R^{k}" refers to substitution with 1, 2, 3 or 4 R^{k}. For example, "substituted with 1 to 5 substituents selected from ..." refers to substitution with 1, 2, 3, 4 or 5 substituents selected from ... For example, "bridged-heterocyclic ring is optionally substituted with 1 to 4 substituents selected from H or F" means that the bridged-heterocyclic ring is optionally substituted with 1, 2, 3 or 4 substituents selected from H or F.

An X- to Y-membered ring (X and Y are integers, 3≤ X < Y, and X < Y ≤ 20 (i.e., any integer from 4 to 20)) includes X-, X+1-, X+2-, X+3-, X+4-,...or Y-membered rings. Rings include a heterocyclic ring, a carbocyclic ring, an aromatic ring, aryl, heteroaryl, cycloalkyl, a mono-heterocyclic ring, a fused-heterocyclic ring, a spiro-heterocyclic ring, or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

A C_{x-y} carbocyclic ring (including aryl, cycloalkyl, a mono-carbocyclic ring, a spiro-carbocyclic ring, a fused-carbocyclic ring, or a bridged-carbocyclic ring) includes Cₓ-, Cₓ₊₁-, Cₓ₊₂-, Cₓ₊₃-, Cₓ₊₄-,...or C_{y}-membered rings (x is an integer, 3 ≤ x < y, and y is any integer from 4 to 20). For example, "C₃₋₆ cycloalkyl" refers to C₃, C₄, C₅ or C₆ cycloalkyl.

When a group has one or more connectable sites, any one or more sites of the group can be connected to other groups via chemical bonds. When the connection mode of the chemical bond is indeterminate and there are hydrogen atoms at the connectable sites, the number of H atoms at the site will decrease correspondingly with the number of connected chemical bonds when the chemical bond is connected, forming a group with the corresponding valence. For example, 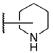 indicates that any connectable site on the piperidyl group can be connected to other groups via one chemical bond, including at least 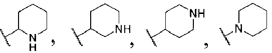 these 4 connection modes. Even if an H atom is shown on the N atom, 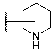 also includes 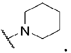 For example, 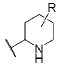 indicates that the R group on the piperidyl group can be located on C or N, including at least

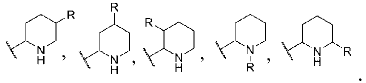

When the connecting groups listed do not specify their connection direction, their connection directions include both the left-to-right and right-to-left reading orders. For example, for A-L-B, wherein L is -M-W-, it includes both A-M-W-B and A-W-M-B.

"Optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds or the stereoisomers, the racemates, the tautomers, the deuterated substances, the solvates, the prodrugs, the metabolites, the pharmaceutically acceptable salts or the cocrystals thereof according to the present invention and other chemical components, wherein the "other chemical components" refer to pharmaceutically acceptable carriers, excipients and/or one or more other therapeutic agents.

Unless otherwise stated, the wedge-shaped solid bond ( 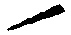 ) and the wedge-shaped dotted bond ( 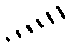 ) represent the absolute configuration of a stereoscopic centre.

"Preparation strength" refers to the weight of the drug substance contained in each vial, tablet or other unit preparation.

"Carrier" refers to a material that does not cause significant irritation to an organism and does not eliminate the biological activity and characteristics of a compound administered.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or removed in vivo to obtain a parent compound. When the prodrug of the present invention is administered to a mammalian individual, the prodrug is split to form a free amino or carboxyl group.

"Cocrystal" refers to a crystal formed by the combination of active pharmaceutical ingredient (API) and cocrystal former (CCF) under the action of hydrogen bonds or other non-covalent bonds. The pure states of API and CCF are both solid at room temperature, and there is a fixed stoichiometric ratio among various components. The cocrystal is a multi-component crystal, which includes both a binary cocrystal formed between two neutral solids and a multi-element cocrystal formed between a neutral solid and a salt or solvate.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerisation and amide-imino alcohol isomerisation.

"IC₅₀" refers to the concentration of a medicament or inhibitor required to inhibit half of a given biological process (or a component of the process such as an enzyme, a receptor and a cell).

### Synthesis method I:

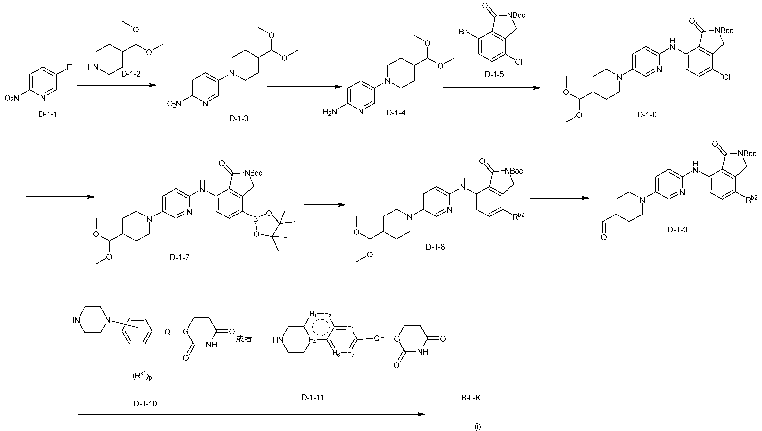

K is selected from 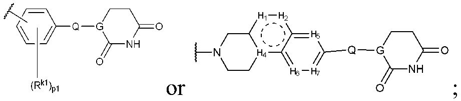
the definitions of the remaining groups are consistent with those in the description;
a compound of general formula (D-1-1) or a compound of general formula (D-1-2) is subjected to a substitution reaction to obtain a compound of general formula (D-1-3);
the compound of general formula (D-1-3) is subjected to a reduction reaction to obtain a compound of general formula (D-1-4);
the compound of general formula (D-1-4) and a compound of general formula (D-1-5) are subjected to a coupling reaction to obtain a compound of general formula (D-1-6);
the compound of general formula (D-1-6) is subjected to a coupling reaction to obtain a compound of general formula (D-1-7);
the compound of general formula (D-1-7) is subjected to a coupling reaction to obtain a compound of general formula (D-1-8);
the compound of general formula (D-1-8) is subjected to a deprotection reaction to obtain a compound of general formula (D-1-9);
the compound of general formula (D-1-9) and a compound of general formula (D-1-10) or a compound of general formula (D-1-11) are subjected to a reductive amination or substitution reaction to obtain a compound of general formula (I).

### Detailed Description of Embodiments

The technical solutions of the present invention will be described in detail by the following examples, but the scope of protection of the present invention includes but is not limited thereto.

The compounds used in the reactions described herein are prepared according to organic synthesis techniques known to those skilled in the art, and starting from commercially available chemicals and(or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The structures of the compounds are determined by nuclear magnetic resonance (NMR) or (and) mass spectrometry (MS). The NMR shift (δ) is given in the unit of 10⁻⁶ (ppm). NMR is determined with (Bruker Avance III 400 and Bruker Avance 300) nuclear magnetic resonance instrument; the solvents for determination are deuterated dimethyl sulphoxide (DMSO-*d₆*), deuterated chloroform (CDCl₃) and deuterated methanol (CD₃OD); and the internal standard is tetramethylsilane (TMS).

MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM).

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.

For the column chromatography, Yantai Huanghai silica gel of 200-300 mesh silica gel is generally used as a carrier;
EDCI: 1-(3-Dimethylaminopropyl)-3-ethylcarbodiimide hydrochloride (CAS: 7084-11-9);
XANT PHOS/Xant-Phos: 4,5-Bis(diphenylphosphino)-9,9-dimethylxanthene (CAS: 161265-03-8);
HOBT: 1-Hydroxybenzotriazole (CAS: 2592-95-2); DIPEA: *N,N-*Diisopropylethylamine (CAS: 7087-68-5);
Boc: Tert-butyloxycarbonyl; XPhos Pd G4: CAS: 1599466-81-5; Pd₂(dba)₃: CAS: 60748-47-2;
TFA: Trifluoroacetic acid; XPHOS-Pd-G2: CAS: 1310584-14-5; X-PHOS: CAS: 564483-18-7;
TBAF: Tetrabutylammonium fluoride, CAS: 429-41-4.

**Acidic prep-HPLC** of final product: Instrument: waters 2767 preparative liquid chromatographic instrument; Chromatographic column: XBridge@ Prep C18 (30 mm×150 mm); Composition of mobile phase: acetonitrile, water (containing 0.1% trifluoroacetic acid)), obtained by lyophilising the prepared solution.

### Example 1: Preparation of compound 1

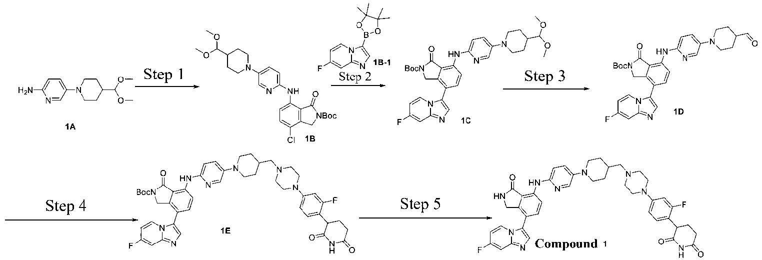

### Step 1: Preparation of 1B

1A (1.2 g, 4.77 mmol, CAS: 3011833-57-8, synthesised with reference to patent WO 2023220640) and tert-butyl 7-bromo-4-chloro-1-oxoisoindoline-2-carboxylate (CAS: 2628351-94-8, 1.65 g, 4.77 mmol) were dissolved in 1,4-dioxane solution (70 mL), and tris(dibenzylideneacetone)dipalladium (0.44 g, 0.48 mmol), Xant-phos (0.55 g, 0.95 mmol) and caesium carbonate (3.89 g, 11.92 mmol) were added. After the addition was completed, the mixture was reacted at 90°C under nitrogen for 3 h. The reaction solution was cooled to room temperature, diluted with ethyl acetate (100 mL), and subjected to suction filtration through celite, the filter cake was washed 3 times with ethyl acetate, and the filtrates were combined, and then washed once with water and once with saturated sodium chloride. The organic phase was dried over anhydrous sodium sulphate, and concentrated under reduced pressure, and the residue was subjected to flash column chromatography (mobile phase: dichloromethane/methanol (v/v)=20/1) to afford 1B (1.8 g, yield: 73%).

LCMS m/z =517.4[M+H]⁺
¹H NMR (400 MHz, CDCl₃) δ 9.46 (s, 1H), 8.60 (d, 1H), 8.00 (s, 1H), 7.41 (d, 1H), 7.35 - 7.26 (m, 1H), 6.77 (d, 1H), 4.67 (s, 2H), 4.09 (d, 1H), 3.64 - 3.51 (m, 2H), 3.38 (s, 6H), 2.77 - 2.57 (m, 2H), 1.99 - 1.83 (m, 2H), 1.80 - 1.70 (m, 1H), 1.62 (s, 9H), 1.57 - 1.47 (m, 2H).

### Step 2: Preparation of 1C

1B (1.8 g, 3.48 mmol), XPHOS-Pd-G2 (CAS: 1310584-14-5) (0.27 g, 0.35 mmol), 1B-1 (1.37 g, 5.22 mmol), X-PHOS (0.33 g, 0.7 mmol) and potassium phosphate (2.22 g, 10.44 mmol) were added to a mixed solvent of dioxane (60 mL) and water (20 mL), and the mixture was reacted at 100°C under nitrogen atmosphere for 2 h. The reaction system was cooled to room temperature, and then water (30 mL) was added. The mixture was extracted with ethyl acetate (30 mL×3), the organic phases were combined, dried over anhydrous sodium sulphate, filtered and then concentrated under reduced pressure, and the concentrated residue was purified by silica gel column chromatography (petroleum ether/ethyl acetate (v/v)=2/3) to afford 1C (2.05 g, yield: 95 %).
LCMS m/z =617.3[M+H]⁺

### Step 3: Preparation of 1D

1C (2.05 g, 3.32 mmol) was dissolved in tetrahydrofuran (20 mL), hydrochloric acid (2*N*) (120 mL) was added dropwise at room temperature, and the mixture was stirred at room temperature for 2 h. The resulting mixture was quenched with saturated sodium bicarbonate aqueous solution, and extracted with ethyl acetate (110 mL×3). The organic phases were combined, dried over anhydrous sodium sulphate, filtered and then concentrated under reduced pressure, and the concentrated residue was purified by silica gel column chromatography (dichloromethane/methanol v/v)=100/3) to afford 1D (1.9 g).
LCMS m/z =571.2[M+H]⁺

### Step 4: Preparation of 1E

1D (0.15 g, 0.26 mmol) and 3-(2-fluoro-4-(piperazin-1-yl)phenyl)piperidine-2,6-dione (CAS: 2861233-27-2, 0.076 g, 0.26 mmol) were dissolved in dichloromethane (10 mL), and glacial acetic acid (0.031 g, 0.52 mmol) was added dropwise at room temperature. Sodium sulphate (0.088 g, 0.62 mmol) and sodium triacetoxyborohydride (0.11 g, 0.52 mmol) were added, and the mixture was stirred at room temperature for 16 h. The resulting mixture was adjusted to be basic with 1*N* sodium hydroxide aqueous solution, and extracted 3 times with dichloromethane, the organic phases were combined, dried over anhydrous sodium sulphate, and concentrated under reduced pressure, and the residue was purified by silica gel column chromatography (dichloromethane/methanol (V/V)=15/1) to afford 1E (0.1 g, yield: 45%).
LCMS m/z =846.5[M+H]⁺

### Step 5: Preparation of compound 1

1E (0.1 g, 0.12 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure to remove trifluoroacetic acid, and the crude product was subjected to acidic prep-HPLC to afford the trifluoroacetic acid salt of compound 1 (0.035 g).

LCMS m/z =746.2[M+H]⁺
¹H NMR (400 MHz, DMSO-*d₆*) δ 10.82 (s, 1H), 9.90 (s, 1H), 8.80 (s, 1H), 8.67 - 8.51 (m, 2H), 8.22 (s, 1H), 8.05 (d, 1H), 7.94 - 7.84 (m, 1H), 7.72 (d, 1H), 7.50 (dd, 1H), 7.41 - 7.30 (m, 1H), 7.17 (t, 1H), 7.01 (d, 1H), 6.93 - 6.74 (m, 2H), 4.37 (s, 2H), 3.96 - 3.84 (m, 3H), 3.72 - 3.57 (m, 4H), 3.26 - 3.03 (m, 6H), 2.80 - 2.65 (m, 3H), 2.58 - 2.52 (m, 1H), 2.23 - 2.10 (m, 1H), 2.07 - 1.92 (m, 2H), 1.92 - 1.83 (m, 2H), 1.46 - 1.31 (m, 2H).

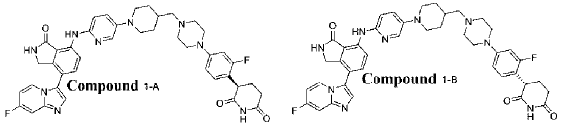

The trifluoroacetic acid salt of compound 1 (280 mg) was subjected to chiral separation and purification.

Preparation conditions: Instrument: SHIMADZU LC-20AP; Column: Chiral WHEIK column; Mobile phase: A for n-Hexane; B for 0.1%IPAm in ethanol and acetonitrile;Gradient: B for 100%; Flow rate: 80 mL/min; Column temperature: room temperature; Wavelength: 220 nm; Cycle time: 20.0min; Sample preparation: Compound concentration was 10 mg/ml, dissolved in acetonitrile.; Injection: 3.0 ml per injection.

Analysis conditions: Instrument: SHIMADZU LC-20AD; Column: Chiral WHEIK column; Mobile phase: A for n-Hexane; B for 0.1%IPAm in ethanol and acetonitrile; Gradient: B for 80%; Flow rate: 1 mL/min; Column temperature: 35°C; Wavelength: 220 nm

After preparative separation, the components with the same retention time were combined and concentrated under reduced pressure to afford compound 1-1 (87 mg) and compound 1-2 (92 mg).

Among compound 1-1 and compound 1-2, one is 1-A and the other is 1-B.
Retention time of compound 1-1 under analysis conditions: 2.855 min, LCMS m/z =746.3[M+H]⁺
Retention time of compound 1-2 under analysis conditions: 4.270 min, LCMS m/z =746.3[M+H]⁺

### Example 2: Preparation of compound 2

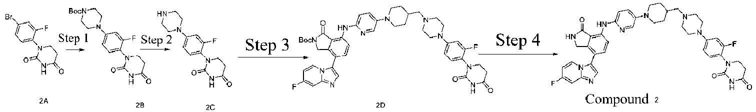

### Step 1: Preparation of 2B

2A (2.87 g, 10 mmol) and N-BOC-piperazine (1.86 g, 10 mmol) were dissolved in toluene (50 mL), 2-dicyclohexylphosphino-2',6'-diisopropoxy-1,1'-biphenyl (0.47 g, 1 mmol), RuPhos Pd G3 (CAS 1445085-77-7) (0.84 g, 1 mmol) and lithium bis(trimethylsilyl)amide (30 mL, 60 mmol) were added, and the mixture was reacted at 80°C under nitrogen for 0.5 h. The reaction solution was quenched with water and extracted 3 times with dichloromethane, the organic phase was dried over anhydrous sodium sulphate and concentrated under reduced pressure, and then the residue was subjected to flash column chromatography (mobile phase: petroleum ether/ethyl acetate =1/1) to afford compound 2B (1.6 g, yield: 41%)

### Step 2: Preparation of 2C

2B (1.6 g, 4.08 mmol) was dissolved in dichloromethane solution (15 mL), trifluoroacetic acid (5 mL) was added dropwise at room temperature, and the mixture was stirred at room temperature for 1 h. The reaction solution was directly concentrated under reduced pressure to remove trifluoroacetic acid, and the residue was redissolved in 100 mL of dichloromethane/methanol (V/V=10/1). The resulting mixture was adjusted to be basic with sodium hydroxide aqueous solution, and subjected to liquid separation and extraction, and the organic phase was dried over anhydrous sodium sulphate and concentrated under reduced pressure to afford 2C (0.8 g).

### Step 3: Preparation of 2D

1D (0.15 g, 0.26 mmol) and 2C (76 mg,0.14 mmol) were dissolved in dichloromethane solution (10 mL), and glacial acetic acid (0.031 g, 0.52 mmol) was added dropwise at room temperature. Then, sodium sulphate (0.088 g, 0.62 mmol) and sodium triacetoxyborohydride (0.11 g, 0.52 mmol) were added, and the mixture was stirred at room temperature for 16 h. The resulting mixture was adjusted to be basic with 1*N* sodium hydroxide aqueous solution, and extracted 3 times with dichloromethane, the organic phases were combined, dried over anhydrous sodium sulphate, and concentrated under reduced pressure, and the residue was purified by silica gel column chromatography (dichloromethane/methanol (V/V)=15/1) to afford 2D (0.1 g, yield: 45%).

### Step 4: Preparation of compound 2

2D (0.1 g, 0.12 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure to remove trifluoroacetic acid, and the crude product was subjected to acidic prep-HPLC to afford the trifluoroacetic acid salt of compound 2 (0.03 g).

LCMS m/z =747.6[M+H]⁺
¹H NMR (400 MHz, DMSO-*d₆*) δ 10.39 (s, 1H), 9.91 (s, 1H), 8.80 (s, 1H), 8.70 - 8.61 (m, 1H), 8.59 - 8.51 (m, 1H), 8.26 (s, 1H), 8.08 - 7.99 (m, 1H), 7.96 - 7.85 (m, 1H), 7.73 (d, 1H), 7.56 - 7.45 (m, 1H), 7.43 - 7.34 (m, 1H), 7.29 (t, 1H), 7.06 - 6.93 (m, 2H), 6.87 (dd, 1H), 4.37 (s, 2H), 3.98 - 3.83 (m, 2H), 3.70 - 3.54 (m, 6H), 3.24 - 3.03 (m, 6H), 2.81 - 2.65 (m, 4H), 2.12 - 1.95 (m, 1H), 1.93 - 1.83 (m, 2H), 1.49 - 1.32 (m, 2H).

### Example 3: Preparation of compound 3

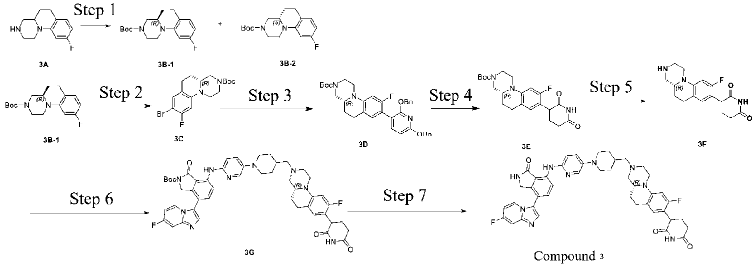

### Step 1: Preparation of compounds 3B-1 and 3B-2

Compound 3A (2.8 g, 13.58 mmol) (see Bioorganic & Medicinal Chemistry Letters, 2016, 26, 5877-5882 for the synthesis method) was dissolved in dichloromethane (50 mL), di-tert-butyl dicarbonate (5.93 g, 27.17 mmol) and DMAP (3.32 g, 27.18 mmol) were added, and the mixture was reacted at room temperature for 16 h. The reaction system was washed with 0.5 mol/L hydrochloric acid (50 mL), dried over anhydrous sodium sulphate, and concentrated under reduced pressure. The crude was separated and purified by silica gel column chromatography (ethyl acetate:petroleum ether (v/v) = 0:1-1:9) to afford the racemate of compound 3B (3.4 g, yield: 82%).

The racemate of 3B was subjected to chiral resolution, and the chiral resolution method was as follows:
1. Instrument: SFC Prep 150 AP; Chromatographic column: Daicel IC-H (19 mm × 250 mm).
2. The sample was dissolved in methanol, and the mixture was filtered through a 0.45 µm filter to prepare a sample solution.
3. Preparative chromatography conditions: a. the mobile phase consists of systems A and B: mobile phase A: CO₂; mobile phase B: methanol/isopropanol (v/v) = 1:1; b. isocratic elution, mobile phase B content: 20%; c. flow rate: 40 mL/min.

Retention time: chiral isomer 1 (compound 3B-1): 5.7 min, chiral isomer 2 (compound 3B-2): 6.47 min.

According to the MicroED structure determination, compound 3B-1 is in the R configuration, and compound 3B-2 is in the S configuration.
LCMS m/z = 307.3 [M+1]⁺

### Step 2: Preparation of 3C

3B-1 (0.45 g, 1.47 mmol) and NBS (0.31 g, 1.74 mmol) were added to acetonitrile (10 mL), and the mixture was reacted at room temperature for 1 h. The reaction system was directly concentrated under reduced pressure, and the residue was purified by silica gel column chromatography (mobile phase: petroleum ether/ethyl acetate (V/V)=0-50%) to afford 3C (0.50 g, yield: 88%).
LCMS m/z = 329.1 [M-55]⁺

### Step 3: Preparation of 3D

3C (0.5 g, 1.30 mmol), 2,6-bis(benzyloxy)-3-(4,4,5,5-tetramethyl-1,3,2-dioxaborolan-2-yl)pyridine (1.36 g, 3.26 mmol), Pd(dppf)Cl₂·DCM (0.11 g, 0.13 mmol) and caesium carbonate (1.27 g, 3.90 mmol) were added to dioxane (15 mL) and water (3 mL), and the mixture was reacted at 100°C under nitrogen atmosphere overnight. Water (30 mL) was added, the resulting mixture was extracted with ethyl acetate (30 mL×3), and the organic phases were combined, dried over anhydrous sodium sulphate, filtered, and concentrated under reduced pressure. The concentrate was purified by silica gel column chromatography (mobile phase: petroleum ether/ethyl acetate (V/V)=0-60%) to afford 3D (0.34 g, yield: 44%).

### Step 4: Preparation of 3E

3D (0.34 g, 0.57 mmol) and palladium on carbon (10%, 0.49 g) were added to THF (10 mL), and the mixture was reacted at 35°C under hydrogen atmosphere overnight. The reaction system was subjected to suction filtration through celite, the filtrate was concentrated under reduced pressure, and the concentrate was purified by silica gel column chromatography (mobile phase: methanol/dichloromethane (V/V)=0-10%) to afford 3E (0.22 g, yield: 92%).
LCMS m/z = 418.2 [M+1]⁺

### Step 5: Preparation of 3F

3E (0.22 g, 0.53 mmol) was dissolved in 3 mL of dichloromethane, 3 mL of trifluoroacetic acid was added, and the mixture was stirred at room temperature for 30 min. The reaction solution was concentrated under reduced pressure, a mixed solvent of dichloromethane/methanol (10:1) (30 mL×5) and saturated sodium bicarbonate solution (30 mL) were added, and the mixture was stirred for layer separation. The organic phase was collected, dried over anhydrous sodium sulphate, and filtered, and the filtrate was concentrated to afford compound 3F (160 mg).

### Step 6: Preparation of 3G

Compound 1D (0.15 g, 0.26 mmol) and 3F (83 mg,0.26 mmol) were dissolved in dichloromethane solution (10 mL), glacial acetic acid (0.031 g, 0.52 mmol) was added dropwise at room temperature, and then sodium sulphate (0.088 g, 0.62 mmol) and sodium triacetoxyborohydride (0.11 g, 0.52 mmol) were added. After the addition was completed, the mixture was stirred at room temperature for 16 h. The resulting mixture was adjusted to be basic with 1*N* sodium hydroxide aqueous solution, and extracted 3 times with dichloromethane, the organic phases were combined, dried over anhydrous sodium sulphate, and concentrated under reduced pressure, and the residue was purified by silica gel column chromatography (dichloromethane/methanol (V/V)=15/1) to afford 3G (0.1 g, yield: 44%).

### Step 7: Preparation of compound 3

3G (0.1 g, 0.121 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure to remove trifluoroacetic acid, and the crude product was subjected to acidic prep-HPLC to afford the trifluoroacetic acid salt of compound 3 (0.03 g).

LCMS m/z =772.6[M+H]⁺
¹H NMR (400 MHz, DMSO-*d₆*) δ 10.78 (s, 1H), 9.90 (s, 1H), 8.79 (s, 1H), 8.67-8.53 (m, 2H), 8.22 (s, 1H), 8.05 (d, 1H), 7.89 (d, 1H), 7.72 (d, 1H), 7.50 (dd, 1H), 7.36 (s, 1H), 7.01 (d, 1H), 6.88 (d, 1H), 6.80 (d, 1H), 4.37 (s, 2H), 4.13 - 4.06 (m, 1H), 3.89 - 3.82 (m, 1H), 3.73 - 3.57 (m, 4H), 3.43 - 3.31 (m, 1H), 3.21 - 3.00 (m, 4H), 2.94 - 2.82 (m, 1H), 2.80 - 2.51 (m, 6H), 2.20 - 2.08 (m, 1H), 2.07 - 1.83 (m, 5H), 1.70 - 1.54 (m, 1H), 1.46 - 1.32 (m, 2H).

### Example 4: Preparation of compound 4

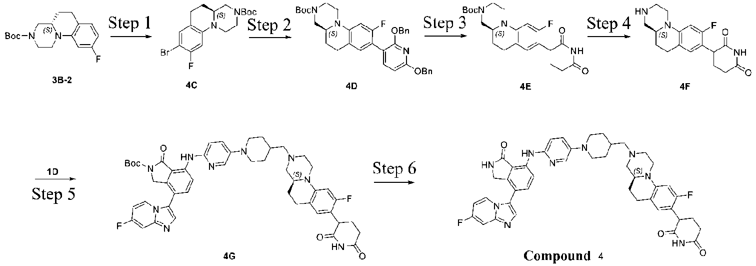

### Step 1: Preparation of 4C

3B-2 (1.145 g, 3.74 mmol) and NBS (0.73 g, 4.11 mmol) were added to acetonitrile (30 mL), and the mixture was reacted at room temperature for 1 h. The reaction solution was diluted with 200 mL of ethyl acetate, and then washed 3 times with water and once with saturated sodium bicarbonate aqueous solution, the organic phase was collected, dried over anhydrous sodium sulphate, and concentrated under reduced pressure, and the residue was purified by flash column chromatography (mobile phase: ethyl acetate/petroleum ether (V/V)=0-50%) to afford 4C (1.30 g, yield: 90%).
LCMS m/z = 329.0 [M-55]⁺

### Step 2: Preparation of 4D

**4C** (1.30 g, 3.37 mmol), 2,6-bis(benzyloxy)-3-(4,4,5,5-tetramethyl-1,3,2-dioxaborolan-2-yl)pyridine (2.11 g, 5.05 mmol), Pd(dppf)Cl₂·DCM (0.28 g, 0.34 mmol) and caesium carbonate (3.29 g, 10.12 mmol) were added to dioxane (50 mL) and water (5 mL), and the mixture was reacted at 80°C under nitrogen atmosphere overnight. Water (50 mL) was added, the resulting mixture was extracted with ethyl acetate (50 mL×3), and the organic phases were combined, dried over anhydrous sodium sulphate, filtered, and concentrated under reduced pressure. The concentrate was purified by silica gel column chromatography (mobile phase: ethyl acetate/petroleum ether (V/V)=0-60%) to afford **4D** (1.75 g, yield: 87%).

### Step 3: Preparation of 4E

**4D** (1.75 g, 2.94 mmol) and palladium on carbon (10%, 1.75 g) were added to THF (20 mL), and the mixture was reacted at 25°C under hydrogen atmosphere overnight. The reaction system was filtered through celite to remove palladium on carbon, and the filter cake was washed with 200 mL of dichloromethane, and concentrated under reduced pressure to afford **4E** (1.20 g).
LCMS m/z = 362.1 [M-55]⁺

### Step 4: Preparation of 4F

**4E** (0.50 g, 1.20 mmol) was dissolved in 15 mL of dichloromethane, 15 mL of trifluoroacetic acid was added, and the mixture was stirred at room temperature for 30 min. The reaction solution was concentrated under reduced pressure, a mixed solvent of dichloromethane/methanol (10:1) (30 mL×5) and saturated sodium bicarbonate aqueous solution (30 mL) were added, and the mixture was stirred for layer separation. The organic phase was collected, dried over anhydrous sodium sulphate, and filtered, and the filtrate was concentrated to afford compound **4F** (0.38 g).
LCMS m/z = 318.2 [M+H]⁺

### Step 5: Preparation of 4G

To a reaction flask were added **4F** (0.11 g, 0.35 mmol), **1D** (0.20 g, 0.35 mmol), 1,2-dichloroethane (10 mL), sodium triacetoxyborohydride (0.15 g, 0.70 mmol) and glacial acetic acid (0.042 g, 0.70 mmol), and the mixture was stirred and reacted at room temperature overnight. Dichloromethane and saturated sodium bicarbonate aqueous solution were added, and then the resulting mixture was stirred for layer separation. The organic layer was dried over anhydrous sodium sulphate, filtered and then concentrated under reduced pressure, and the concentrated residue was purified by silica gel column chromatography (eluents: DCM-CH₃OH=100-0 to 90-10) to afford **4G** (0.17 g, yield: 51.50%).

### Step 6: Preparation of compound 4

To a reaction flask were added **4G** (0.11 g, 0.13 mmol) and dichloromethane (5 mL), and trifluoroacetic acid (2 mL) was added under stirring. The mixture was stirred and reacted at room temperature for 2 hours, and concentrated under reduced pressure to dryness, the crude product was subjected to **acidic prep-HPLC,** and the prepared solution was lyophilised to afford the trifluoroacetic acid salt of **compound 4** (41 mg).

LCMS m/z =772.4[M+H]⁺
¹H NMR (400 MHz, DMSO-*d₆*) δ 10.91 - 10.56 (m, 1H), 9.82 (s, 1H), 8.73 (s, 1H), 8.53 (d, 1H), 8.46 - 8.33 (m, 1H), 7.99 (d, 1H), 7.79 (s, 1H), 7.67 (d, 1H), 7.50 (dd, 1H), 7.43 (dd, 1H), 7.00 - 6.90 (m, 2H), 6.82 - 6.72 (m, 1H), 6.62 (d, 1H), 4.36 (s, 2H), 3.86 - 3.77 (m, 1H), 3.76 - 3.67 (m, 1H), 3.65 - 3.52 (m, 3H), 3.02 - 2.89 (m, 2H), 2.78 - 2.56 (m, 7H), 2.55-2.51 (m, 1H), 2.21 (d, 2H), 2.16 - 2.00 (m, 2H), 2.00-1.91 (dd, 1H), 1.88 - 1.72 (m, 4H), 1.63 - 1.55 (m, 1H), 1.33 - 1.26 (m, 2H).

### Example 5: Preparation of compound 5

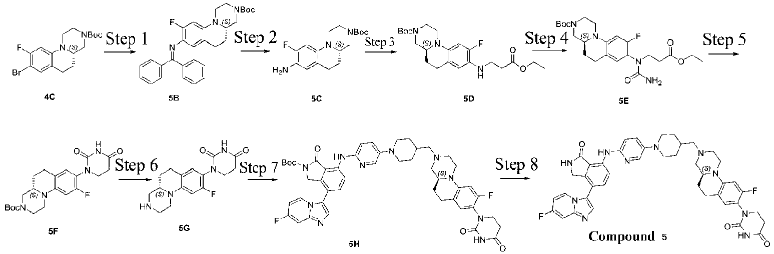

### Step 1: Preparation of 5B

**4C** (6.29 g, 16.32 mmol), benzophenone imine (4.14 g, 22.85 mmol), caesium carbonate (10.63 g, 32.64 mmol), palladium acetate (0.73 g, 3.26 mmol), and XANT PHOS (0.94 g, 1.63 mmol) were added to dioxane solution (100 mL), and the mixture was reacted at 105°C under nitrogen atmosphere for 16 h. The reaction solution was cooled to room temperature, and filtered through celite to remove the solid, and the filter cake was washed with dichloromethane. The organic phase was collected and concentrated under reduced pressure, and the residue was purified by silica gel column chromatography (mobile phase: ethyl acetate/petroleum ether (v/v)=1/5) to afford **5B** (6.94 g, yield: 87%).
LCMS m/z = 486.2 [M+H]⁺

### Step 2: Preparation of 5C

**5B** (6.94 g, 14.29 mmol) was added to methanol (200 mL), palladium on carbon (6.92 g , wt%=10%) and ammonium acetate (6.79 g, 88.03 mmol) were added, and the mixture was reacted at room temperature under hydrogen atmosphere (balloon pressure) for 12 h. The reaction solution was filtered through celite, the filtrate was concentrated under reduced pressure, and the residue was purified by silica gel column chromatography (mobile phase: ethyl acetate/petroleum ether (v/v)=1/1) to afford **5C** (4.15 g, yield: 90%).
LCMS m/z = 322.2[M+H]⁺

### Step 3: Preparation of 5D

**5C** (4.15 g, 12.91 mmol), ethyl acrylate (3.88 g, 38.73 mmol) and *N,N-*diisopropylethylamine (5.01 g, 38.73 mmol) were successively added to ethanol (60 mL), and the mixture was reacted at 100°C for 72 h. The reaction solution was cooled to room temperature and then concentrated under reduced pressure, and the residue was separated and purified by column chromatography (mobile phase: methanol/dichloromethane (v/v)=1/20) to afford **5D** (3.91 g, yield: 71%).
LCMS m/z =422.3[M+H]⁺

### Step 4: Preparation of 5E

**5D** (0.50 g, 1.19 mmol) and N,N-diisopropylethylamine (0.46 g, 3.56 mmol) were added to tetrahydrofuran (20 mL), and then triphosgene (0.39 g, 1.31 mmol) was slowly added, and the mixture was reacted at room temperature for 1 h. Aqueous ammonia (5 mL) was added, and the resulting mixture was heated to 50°C and reacted for another 2 h. The reaction solution was cooled to room temperature and then diluted with 100 mL of ethyl acetate, the organic phase was washed 3 times with water and once with saturated sodium chloride, dried over anhydrous sodium sulphate, and concentrated under reduced pressure, and the residue was separated and purified by column chromatography (mobile phase: methanol/dichloromethane (v/v)=1/15) to afford **5E** (0.46 g, yield: 83%).
LCMS m/z = 465.3 [M+H]⁺

### Step 5: Preparation of 5F

**5E** (0.46 g, 1.00 mmol) was added to acetonitrile (10 mL), and then benzyltrimethylammonium hydroxide (40% solution in methanol, 1.2 mL) was added, and the mixture was heated to 60°C and reacted for 2 h. The reaction system was cooled to room temperature, and then an appropriate amount of silica gel was added. The resulting mixture was concentrated under reduced pressure, and residue was separated and purified by column chromatography (mobile phase: methanol/dichloromethane (v/v)=1/15) to afford **5F** (0.21 g, yield: 50%).

### Step 6: Preparation of 5G

**5F** (0.21 g, 0.50 mmol) was dissolved in dichloromethane (5 mL), trifluoroacetic acid (5 mL) was added, and the mixture was stirred at room temperature for 1 h. The reaction solution was concentrated under reduced pressure to remove trifluoroacetic acid, and then the residue was dissolved in 10 mL of dichloromethane and 1 mL of isopropanol. The resulting mixture was adjusted to be basic with sodium bicarbonate aqueous solution and extracted 3 times with dichloromethane/isopropanol (v/v)=10/1, and the organic phases were combined, dried over anhydrous sodium sulphate, and concentrated under reduced pressure to afford **5G** (0.158 g).
LCMS m/z =319.2[M+H]⁺

### Step 7: Preparation of 5H

To a reaction flask were added **5G** (0.02 g, 0.28 mmol), **1D** (0.16 g, 0.28 mmol), 1,2-dichloroethane (10 mL), sodium triacetoxyborohydride (0.12 g, 0.56 mmol) and glacial acetic acid (0.034 g, 0.56 mmol), and the mixture was stirred and reacted at room temperature overnight. Dichloromethane and saturated sodium bicarbonate aqueous solution were added, and the resulting mixture was stirred for layer separation. The organic layer was dried over anhydrous sodium sulphate, filtered and then concentrated under reduced pressure, and the concentrated residue was purified by column chromatography (eluents: DCM-CH₃OH=100-0 to 90-10) to afford **5H** (0.12 g, yield: 49%).

### Step 8: Preparation of compound 5

To a reaction flask were added **5H** (0.12 g, 0.14 mmol) and dichloromethane (5 mL), and trifluoroacetic acid (2 mL) was added under stirring. The mixture was reacted at room temperature for 2 h, and concentrated under reduced pressure, and then the residue was subjected to **acidic prep-HPLC** to afford **compound 5** (37 mg, yield: 34%).

LCMS m/z =773.3[M+H]⁺
¹H NMR (400 MHz, DMSO-*d₆*) δ 10.45 - 10.15 (m, 1H), 9.82 (s, 1H), 8.73 (s, 1H), 8.54 (d, 1H), 8.46 - 8.35 (m, 1H), 8.00 (d, 1H), 7.79 (s, 1H), 7.67 (d, 1H), 7.51 (dd, 1H), 7.43 (dd, 1H), 7.03 - 6.89 (m, 3H), 6.75 - 6.68 (m, 1H), 4.36 (s, 2H), 3.77 - 3.70 (m, 1H), 3.66 - 3.53 (m, 4H), 3.05 - 2.96 (m, 1H), 2.95 - 2.85 (m, 2H), 2.77 - 2.58 (m, 7H), 2.21 (d, 2H), 2.11 - 1.98 (m, 1H), 1.95-1.75 (m, 4H), 1.73 - 1.55 (m, 2H), 1.31 - 1.25 (m, 2H).

### Example 6: Preparation of compound 6

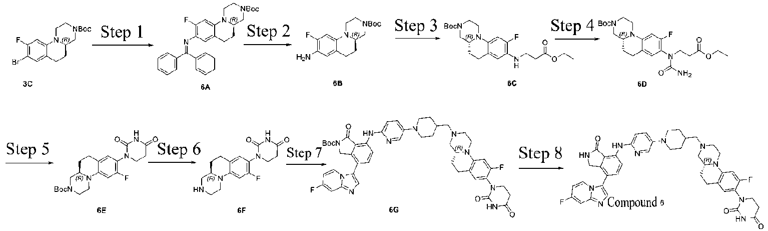

### Step 1: Preparation of 6A

**3C** (5.0 g, 12.98 mmol), benzophenone imine (3.05 mL, 3.29 g, 18.17 mmol), caesium carbonate (9.30 g, 28.56 mmol), palladium acetate (583 mg, 2.6 mmol), and XANT PHOS (751 mg, 1.3 mmol) were added to dioxane solution (100 mL), and the mixture was reacted at 100°C under nitrogen atmosphere for 16 h. The reaction solution was cooled to room temperature, and filtered through celite to remove the solid, and the filter cake was washed with dichloromethane. The organic phase was collected and concentrated under reduced pressure, and the residue was purified by silica gel column chromatography (mobile phase: ethyl acetate/petroleum ether (v/v)=1/5) to afford **6A** (5.6 g, yield: 89%).
LCMS m/z = 486.3 [M+H]⁺

### Step 2: Preparation of 6B

**6A** (5.6 g, 11.53 mmol) was added to methanol (200 mL), palladium on carbon (3.0 g, wt%=10%) and ammonium acetate (5.6 g, 72.6 mmol) were added, and the mixture was reacted at room temperature under hydrogen atmosphere (balloon pressure) for 16 h. The reaction system was filtered through celite, the filtrate was concentrated under reduced pressure, and the residue was purified by silica gel column chromatography (mobile phase: ethyl acetate/petroleum ether (v/v)=1/1) to afford **6B** (3.6 g, yield: 97%).
LCMS m/z = 322.3[M+H]⁺

### Step 3: Preparation of 6C

**6B** (3.6 g, 11.2 mmol), ethyl acrylate (3.36 g, 33.60 mmol) and N,N-diisopropylethylamine (4.34 g, 33.60 mmol) were successively added to ethanol (100 mL), and the mixture was heated to 100°C and reacted for 72 h. The reaction system was concentrated under reduced pressure, and the residue was separated and purified by column chromatography (mobile phase: methanol/dichloromethane (v/v)=1/20) to afford **6C** (3.0 g, yield: 64%).
LCMS m/z =422.3[M+H]⁺

### Step 4: Preparation of 6D

**6C** (1.0 g, 2.37 mmol) and *N*,*N*-diisopropylethylamine (0.92 g, 7.11 mmol) were added to tetrahydrofuran (30 mL), and then triphosgene (0.77 g, 2.61 mmol) was slowly added, and the mixture was reacted at room temperature for 1 h. Aqueous ammonia (9 mL) was added, and the resulting mixture was heated to 50°C and reacted for another 2 h. The reaction solution was diluted with 100 mL of ethyl acetate, the organic phase was washed 3 times with water and once with saturated sodium chloride, dried over anhydrous sodium sulphate, and concentrated under reduced pressure, and the residue was separated and purified by column chromatography (mobile phase: methanol/dichloromethane (v/v)=1/15) to afford **6D** (1.0 g, yield: 91%).
LCMS m/z = 465.3 [M+H]⁺

### Step 5: Preparation of 6E

**6D** (1.0 g, 2.15 mmol) was added to acetonitrile (20 mL), and then benzyltrimethylammonium hydroxide (40% solution in methanol, 2.5 mL, 6.45 mmol) was added, and the mixture was heated to 60°C and reacted for 2 h. Silica gel was added, the resulting mixture was concentrated under reduced pressure, and the residue was separated and purified by column chromatography (mobile phase: methanol/dichloromethane (v/v)=1/15) to afford **6E** (0.69 g, yield: 77%).
LCMS m/z = 363.2 [M-55]⁺

### Step 6: Preparation of 6F

**6E** (0.5 g, 1.19 mmol) was dissolved in dichloromethane (5 mL), trifluoroacetic acid (5 mL) was added, and the mixture was stirred at room temperature for 1 h. The reaction solution was concentrated under reduced pressure to remove trifluoroacetic acid, and then the residue was dissolved in 10 mL of dichloromethane and 1 mL of isopropanol. The resulting mixture was adjusted to be basic with sodium carbonate aqueous solution and extracted 3 times with dichloromethane/isopropanol (v/v)=10/1, and the organic phases were combined, dried over anhydrous sodium sulphate, and concentrated under reduced pressure to afford **6F** (0.37 g).

### Step 7: Preparation of compound 6G

Compound **1D** (0.15 g, 0.26 mmol) and **6F** (83 mg,0.26 mmol) were dissolved in dichloromethane solution (10 mL), glacial acetic acid (0.031 g, 0.52 mmol) was added dropwise at room temperature, and then sodium sulphate (0.088 g, 0.62 mmol) and sodium triacetoxyborohydride (0.11 g, 0.52 mmol) were added. After the addition was completed, the mixture was stirred at room temperature for 16 h. The resulting mixture was adjusted to be basic with 1N sodium hydroxide aqueous solution, and extracted 3 times with dichloromethane, the organic phases were combined, dried over anhydrous sodium sulphate, and concentrated under reduced pressure, and the residue was purified by silica gel column chromatography (dichloromethane/methanol (V/V)=15/1) to afford compound **6G** (0.1 g, yield: 44%).

### Step 8: Preparation of compound 6

**6G** (0.1 g, 0.11 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure to remove trifluoroacetic acid, and the crude product was subjected to **acidic prep-HPLC** to afford the trifluoroacetic acid salt of **compound 6** (0.025 g).

LCMS m/z =387.3[(M+2H)/2]⁺
¹H NMR (400 MHz, D₂O) δ 8.58 (dd, 1H), 8.27 - 8.17 (m, 1H), 8.16 - 8.00 (m, 3H), 7.94 - 7.78 (m, 2H), 7.54 (d, 1H), 7.49 - 7.40 (m, 1H), 7.17 (d, 1H), 6.92 (d, 1H), 4.49 (s, 2H), 4.23 - 4.09 (m, 1H), 3.94 - 3.79 (m, 5H), 3.79 - 3.69 (m, 1H), 3.62 - 3.49 (m, 1H), 3.42 - 3.21 (m, 6H), 3.16 - 3.02 (m, 1H), 2.96 (t, 2H), 2.92-2.76 (m, 2H), 2.49 - 2.32 (m, 1H), 2.26 - 2.09 (m, 3H), 1.93 - 1.65 (m, 3H).

### Example 7: Preparation of compound 7

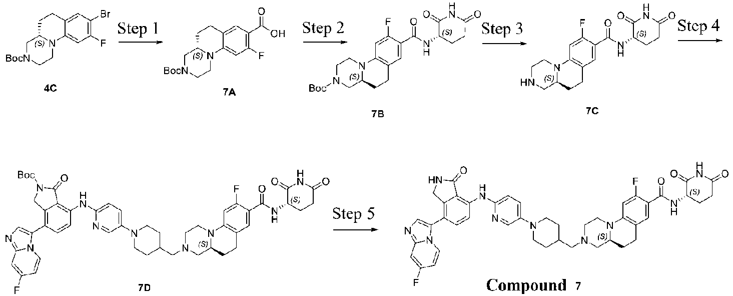

### Step 1: Preparation of 7A

Under nitrogen atmosphere, **4C** (7.00 g, 18.17 mmol) and 70 mL of tetrahydrofuran were respectively added to a reaction flask, the mixture was cooled to -78°C, and 2.5 M n-butyllithium n-hexane solution (14.50 mL, 36.34 mmol) was slowly added dropwise. The system was stirred at -78°C for 1.5 h, and then purged three times with carbon dioxide. The system temperature was controlled below - 40°C, and the system was reacted under carbon dioxide balloon atmosphere for 0.5 h. The system was returned to room temperature, 20 mL of ethyl acetate was added, and the mixture was adjusted to pH 2 with 1 M dilute hydrochloric acid, and extracted with ethyl acetate (30 mL×3). The organic phases were combined, dried over anhydrous sodium sulphate, and concentrated under reduced pressure, and the crude was separated and purified by a silica gel chromatographic column (petroleum ether/ethyl acetate (v/v) = 10:1 to 2:1) to afford **7A** (2.4 g, reaction yield: 37%).

### Step 2: Preparation of 7B

To a reaction flask were added **7A** (0.15 g, 0.43 mmol), (S)-3-aminopiperidine-2,6-dione hydrochloride (0.071 g, 0.43 mmol), EDCI (0.16 g, 0.86 mmol), HOBT (0.087 g, 0.65 mmol) and DMF (5 mL), N-methylmorpholine (0.13 g, 1.29 mmol) was added under stirring, and the mixture was reacted at room temperature for 3 h. Ethyl acetate and saturated sodium bicarbonate aqueous solution were added, and the resulting mixture was stirred, and left to stand for layer separation. The organic layer was washed once with saturated sodium bicarbonate aqueous solution and once with saturated sodium chloride aqueous solution, dried over anhydrous sodium sulphate, filtered, and concentrated under reduced pressure to dryness, to afford **7B** (0.18 g).

### Step 3: Preparation of 7C

To a reaction flask were added **7B** (0.18 g, 0.39 mmol) and dichloromethane (5 mL), trifluoroacetic acid (2 mL) was added under stirring, and the mixture was reacted at room temperature for 2 h. The reaction system was concentrated under reduced pressure to dryness, and a mixed solution of dichloromethane/isopropanol (2/1) and saturated sodium bicarbonate aqueous solution were added. The resulting mixture was stirred for layer separation, the aqueous layer was extracted once with a mixed solution of dichloromethane/isopropanol (2/1), and the organic layers were combined, dried over anhydrous sodium sulphate, filtered, and concentrated under reduced pressure to dryness, to afford **7C** (0.13 g).

### Step 4: Preparation of 7D

To a reaction flask were added **7C** (0.13 g, 0.36 mmol), **1D** (0.21 g, 0.36 mmol), 1,2-dichloroethane (10 mL), sodium triacetoxyborohydride (0.15 g, 0.72 mmol) and glacial acetic acid (0.043 g, 0.72 mmol), and the mixture was stirred and reacted at room temperature overnight. Dichloromethane and saturated sodium bicarbonate aqueous solution were added, and the resulting mixture was stirred for layer separation. The organic layer was dried over anhydrous sodium sulphate, filtered and then concentrated under reduced pressure to dryness, and the residue was purified by column chromatography (eluents: DCM-CH3OH=100-0 to 90-10) to afford **7D** (0.17 g, yield: 52%).

### Step 5: Preparation of compound 7

To a reaction flask were added **7D** (0.17 g, 0.19 mmol) and dichloromethane (5 mL), and trifluoroacetic acid (2 mL) was added under stirring. The mixture was reacted at room temperature for 2 h, and concentrated under reduced pressure to dryness, and the crude product was subjected to **acidic prep-HPLC to afford** the trifluoroacetic acid salt of **compound 7** (3 mg).

LCMS m/z =408.6[(M+2H)/2]⁺
¹H NMR (400 MHz, CD₃OD) δ 8.62 (dd, 1H), 8.47 (d, 1H), 8.18 (s, 1H), 8.14 - 8.05 (m, 1H), 7.83 (dd, 1H), 7.78 - 7.63 (m, 2H), 7.54 (d, 1H), 7.48 - 7.37 (m, 1H), 7.22 - 7.10 (m, 1H), 6.75 (d, 1H), 4.79 (dd, 1H), 4.42 (s, 2H), 4.26 - 4.14 (m, 1H), 3.84 - 3.66 (m, 4H), 3.61 - 3.48 (m, 1H), 3.30 - 3.12 (m, 4H), 3.08 - 2.92 (m, 3H), 2.90 - 2.66 (m, 4H), 2.38 - 2.26 (m, 1H), 2.25 - 2.09 (m, 3H), 2.08 - 1.94 (m, 2H), 1.86 - 1.72 (m, 1H), 1.70 - 1.51 (m, 2H).

### Example 8: Preparation of compound 8

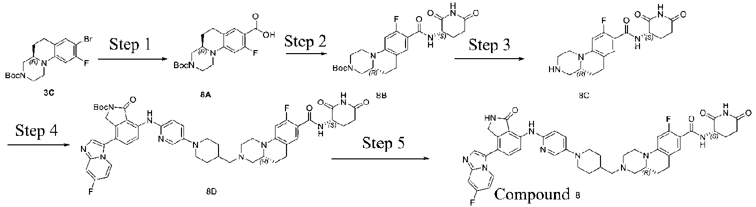

With reference to the synthetic method of compound 7 and by **acidic prep-HPLC,** the trifluoroacetic acid salt of **compound 8** (10 mg) was obtained.
LCMS m/z =408.2[(M+2H)/2]⁺

### Example 9: Preparation of compound 9

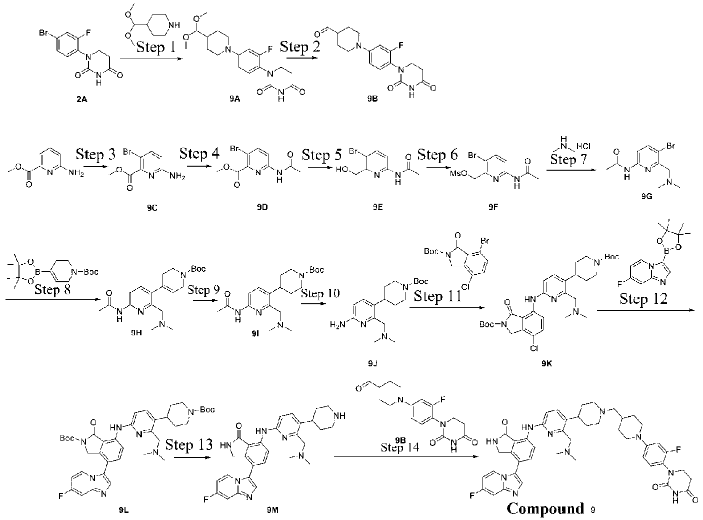

### Step 1: Preparation of 9A

Compound **2A** (0.50 g, 1.74 mmol) and 4-(dimethoxymethyl)-piperidine (0.55 g, 3.48 mmol) were dissolved in toluene (10 mL), 2-dicyclohexylphosphino-2',6'-diisopropoxy-1,1'-biphenyl (0.16 g, 0.35 mmol), RuPhos Pd G3 (0.29 g, 0.35 mmol) and lithium bis(trimethylsilyl)amide (10.44 mL, 10.44 mmol, 1 M) were added, and the mixture was stirred at 80°C under nitrogen for 0.5 h. The reaction solution was cooled to room temperature, and directly concentrated under reduced pressure, and the residue was subjected to flash column chromatography (mobile phase: dichloromethane/methanol (v/v)=10/1) to afford **9A** (0.27 g, yield: 42%).
LCMS m/z =366.2[M+H]⁺

### Step 2: Preparation of 9B

**9A** (0.27 g, 0.75 mmol) was dissolved in tetrahydrofuran (4 mL), hydrochloric acid (3N) (4 mL) was added dropwise at room temperature, and the mixture was stirred at 60°C for 2 h. The resulting mixture was quenched with saturated sodium carbonate aqueous solution, and extracted with dichloromethane/methanol (v/v)=10/1 (50 mL×4), and the organic phases were combined, dried over anhydrous sodium sulphate, filtered and then concentrated under reduced pressure to afford **9B** (0.23 g).

### Step 3: Preparation of 9C

Methyl 6-aminopicolinate (10.00 g, 65.72 mmol) was dissolved in acetonitrile (300 mL), and NBS (12.28 g, 69.01 mmol) was added. After the addition was completed, the mixture was stirred at room temperature for 1 h. The reaction solution was diluted with 500 mL of ethyl acetate, and then washed 3 times with water and once with saturated sodium bicarbonate aqueous solution, the organic phase was collected, dried over anhydrous sodium sulphate, and concentrated under reduced pressure, and the residue was slurried and purified with petroleum ether/ethyl acetate (20 mL/80 mL) to afford **9C** (11.00 g, yield: 72%).
LCMS m/z =231.1[M+H]⁺

### Step 4: Preparation of 9D

**9C** (5.00 g, 65.72 mmol) was dissolved in acetic anhydride (20 mL), and the mixture was stirred at 70°C for 1 h. The reaction solution was cooled to room temperature, and water was added to precipitate a yellow solid. The mixture was subjected to suction filtration, the filter cake was washed 3 times with water and redissolved in 200 mL of dichloromethane, and the resulting mixture was dried over anhydrous sodium sulphate and concentrated under reduced pressure to afford **9D** (5.90 g).
LCMS m/z =273.0[M+H]⁺

### Step 5: Preparation of 9E

**9D** (5.90 g, 21.61 mmol) was dissolved in methanol (300 mL), and the mixture was cooled to 0°C. Sodium borohydride (4.09 g, 108.05 mmol) was slowly added, and the resulting mixture was stirred at 80°C for 16 h. The reaction solution was cooled to room temperature, and quenched with saturated ammonium chloride aqueous solution, and then 500 mL of ethyl acetate was added, and the mixture was stirred for layer separation. The organic phase was collected, dried over anhydrous sodium sulphate, and concentrated under reduced pressure, and the residue was purified by flash column chromatography (mobile phase: petroleum ether/ethyl acetate (v/v)=1/1) to afford compound **9E** (1.00 g, yield: 19%).
LCMS m/z =245.0[M+H]⁺

### Step 6: Preparation of 9F

**9E** (1.00 g, 4.08 mmol) was dissolved in DCM (30 mL), triethylamine (1.24 g, 12.24 mmol) was added, and the mixture was cooled to 0°C. Methanesulphonic anhydride (1.07 g, 6.12 mmol) was slowly added, and the resulting mixture was reacted at room temperature for 0.5 h. The reaction solution was washed 3 times with water, once with saturated ammonium chloride aqueous solution, and once with saturated sodium chloride aqueous solution, and then dried over anhydrous sodium sulphate, and concentrated under reduced pressure to afford compound **9F** (1.11 g).
LCMS m/z =323.0[M+H]⁺

### Step 7: Preparation of 9G

**9F** (1.11 g, 3.43 mmol) was dissolved in acetonitrile (30 mL), DIPEA (1.77 g, 13.72 mmol) and dimethylamine hydrochloride (0.56 g, 6.86 mmol) were added, and the mixture was reacted at 80°C for 1 h. The reaction solution was cooled to room temperature, and directly concentrated under reduced pressure, and the residue was purified by flash column chromatography (mobile phase: dichloromethane/methanol (v/v)=10/1) to afford compound **9G** (0.90 g, yield: 96%).
LCMS m/z =272.0[M+H]⁺

### Step 8: Preparation of 9H

**9G** (0.90 g, 3.31 mmol) and N-Boc-1,2,5,6-tetrahydropyridine-4-boronic acid pinacol ester (CAS: 286961-14-6, 1.54 g, 4.96 mmol) were dissolved in 1,4-dioxane (40 mL), XPhos Pd G4 (0.28 g, 0.33 mmol), X-PHOS (0.32 g, 0.66 mmol) and a solution of potassium phosphate (2.11 g, 9.93 mmol) in water (10 mL) were added, and the mixture was reacted at 105°C under nitrogen for 16 h. The reaction solution was cooled to room temperature, and then diluted with 300 mL of dichloromethane for layer separation, the organic phase was dried over anhydrous sodium sulphate, and concentrated under reduced pressure, and the residue was purified by flash column chromatography (mobile phase: dichloromethane/methanol (v/v)=10/1) to afford compound **9H** (0.72 g, yield: 58%).
LCMS m/z =375.3[M+H]⁺

### Step 9: Preparation of 9I

**9H** (0.72 g, 1.93 mmol) was added to a mixed solvent of methanol (15 mL) and ethanol (15 mL), palladium on carbon (0.72 g , wt%=10%) was added, and the mixture was reacted at 40°C under hydrogen atmosphere (balloon pressure) for 16 h. The reaction solution was cooled to room temperature and filtered through celite, the filter cake was washed with dichloromethane/methanol (v/v)=10/1, and the organic phases were combined, and concentrated under reduced pressure to afford **9I** (0.72 g).
LCMS m/z = 377.2 [M+H]⁺

### Step 10: Preparation of 9J

**9I** (0.74 g, 1.98 mmol) was dissolved in ethanol (15 mL), water (15 mL) and sodium hydroxide (0.63 g, 15.84 mmol) were added, and the mixture was reacted at 80°C for 16 h. The reaction solution was cooled to room temperature and extracted with dichloromethane, the organic phase was collected and dried over anhydrous sodium sulphate, and the residue was concentrated under reduced pressure to afford **9J** (0.62 g).
LCMS m/z =335.2[M+H]⁺

### Step 11: Preparation of 9K

**9J** (0.52 g, 1.55 mmol) and tert-butyl 7-bromo-4-chloro-1-oxoisoindoline-2-carboxylate (CAS: 2628351-94-8, 0.54 g, 1.55 mmol) were dissolved in 1,4-dioxane solution (10 mL), and tris(dibenzylideneacetone)dipalladium (0.14 g, 0.16 mmol), Xant-phos (0.18 g, 0.31 mmol) and potassium carbonate (0.64 g, 4.65 mmol) were added. The mixture was reacted at 100°C under nitrogen for 4 h. The reaction solution was cooled to room temperature, and concentrated under reduced pressure, and the residue was subjected to flash column chromatography (mobile phase: dichloromethane/methanol (v/v)=15/1) to afford **9K** (0.38 g, yield: 40%).
LCMS m/z =600.2[M+H]⁺

### Step 12: Preparation of 9L

**9K** (0.45 g, 0.75 mmol), 1B-1 (0.29 g, 1.13 mmol), X-PHOS (0.07 g, 0.15 mmol), XPHOS-Pd-G2 (0.06 g, 0.075 mmol) and potassium phosphate (0.48 g, 2.25 mmol) were added to a mixed solvent of dioxane (20 mL) and water (5 mL), and the mixture was purged three times with nitrogen and reacted at 100°C for 3 h. The reaction solution was diluted with 200 mL of dichloromethane for layer separation, the organic phase was dried over anhydrous sodium sulphate, and concentrated under reduced pressure, and the residue was purified by flash column chromatography (mobile phase: dichloromethane/methanol (v/v)=15/1) to afford **9L** (0.34 g, yield: 65 %).
LCMS m/z =700.5[M+H]⁺

### Step 13: Preparation of 9M

Compound **9L** (0.34 g, 0.49 mmol) was dissolved in DCM (10 mL), trifluoroacetic acid (10 mL) was added, and the mixture was reacted at room temperature for 1 h. The reaction solution was concentrated under reduced pressure, the residue was redissolved in dichloromethane/methanol=15/1 (200 mL), and the resulting mixture was adjusted to be basic with sodium carbonate aqueous solution. After layer separation, the aqueous phase was extracted 3 times with DCM, the organic phases were combined and dried over anhydrous sodium sulphate, and the residue was concentrated under reduced pressure to afford **9M** (0.23 g).
LCMS m/z =500.0[M+H]⁺

### Step 14: Preparation of compound 9

**9M** (0.12 g, 0.24 mmol) and **9B** (0.08 g, 0.24 mmol) were dissolved in trichloromethane (10 mL), glacial acetic acid (0.03 g, 0.48 mmol) was added at room temperature, and anhydrous sodium sulphate (0.07 g, 0.48 mmol) was added. The mixture was reacted at 60°C for 16 h, sodium triacetoxyborohydride (0.51 g, 2.40 mmol) was slowly added, and the resulting mixture was reacted at 60°C for 3 h. The reaction system was adjusted to be basic with saturated sodium bicarbonate aqueous solution, and extracted 3 times with dichloromethane, the organic phases were combined, dried over anhydrous sodium sulphate, and concentrated under reduced pressure, and the residue was subjected to **acidic prep-HPLC** to afford the trifluoroacetic acid salt of **compound 9** (38 mg).

LCMS m/z =803.0[M+H]⁺
¹H NMR (400 MHz, CD₃OD) δ 8.64 - 8.54 (m, 1H), 8.38 (d, 1H), 8.16 (s, 1H), 7.85 - 7.74 (m, 3H), 7.45 - 7.35 (m, 1H), 7.29 (d, 1H), 7.22 (t, 1H), 6.86 - 6.76 (m, 2H), 4.66 (s, 2H), 4.43 (s, 2H), 3.88 - 3.76 (m, 4H), 3.73 (t, 2H), 3.27 - 3.11 (m, 5H), 3.09 (s, 6H), 2.92 - 2.82 (m, 2H), 2.80 (t, 2H), 2.27 - 2.00 (m, 5H), 1.99 - 1.88 (m, 2H), 1.57 - 1.41 (m, 2H).

### Example 10: Preparation of compound 10

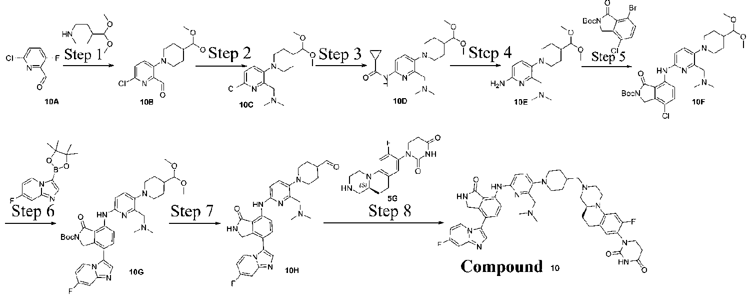

With reference to the synthesis of the aforementioned compound and by **acidic prep-HPLC,** the trifluoroacetic acid salt of **compound 10** (10 mg) was obtained.

LCMS m/z =830.3[M+H]⁺
¹H NMR (400 MHz, DMSO-*d₆*) δ 10.37 (s, 1H), 10.07 (s, 1H), 8.88 (s, 1H), 8.51 (dd, 1H), 8.41 (d, 1H), 8.10 (s, 1H), 7.87 - 7.67 (m, 3H), 7.35 - 7.17 (m, 2H), 7.03 (d, 1H), 6.91 (d, 1H), 4.54 - 4.45 (m, 2H), 4.40 (s, 2H), 4.16-4.10 (m, 1H), 3.73 - 3.56 (m, 4H), 3.49 - 3.33 (m, 1H), 3.22 - 3.04 (m, 4H), 3.01 - 2.83 (m, 9H), 2.82 - 2.59 (m, 6H), 2.11 - 1.97 (m, 2H), 1.96 - 1.82 (m, 2H), 1.74 - 1.59 (m, 1H), 1.57 - 1.36 (m, 2H).

### Example 11: Preparation of compound 11

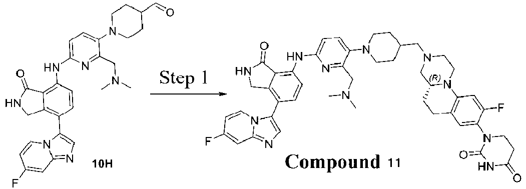

With reference to the synthesis of the aforementioned compound and by **acidic prep-HPLC,** the trifluoroacetic acid salt of **compound 11** (10 mg) was obtained.

LCMS m/z =830.4[M+H]⁺
¹H NMR (400 MHz, DMSO-*d₆*) δ 10.36 (s, 1H), 10.06 (s, 1H), 8.86 (s, 1H), 8.54 - 8.34 (m, 2H), 8.04 (s, 1H), 7.83 - 7.68 (m, 3H), 7.32 - 7.19 (m, 2H), 7.03 (d, 1H), 6.91 (d, 1H), 4.53 - 4.46 (m, 2H), 4.40 (s, 2H), 4.17 - 4.09 (m, 1H), 3.68 - 3.59 (m, 4H), 3.44 - 3.35 (m, 1H), 3.21 - 3.04 (m, 4H), 3.02 - 2.84 (m, 9H), 2.83 - 2.61 (m, 6H), 2.10 - 1.97 (m, 2H), 1.97 - 1.81 (m, 2H), 1.73 - 1.59 (m, 1H), 1.55 - 1.38 (m, 2H).

### Example 12: Preparation of compound 12

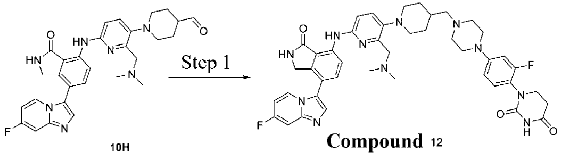

With reference to the synthetic method of step 4 of example 1 and by **acidic prep-HPLC,** the trifluoroacetic acid salt of **compound 12** (12 mg) was obtained.
LCMS m/z =804.2[M+H]⁺

### Example 13: Preparation of compound 13

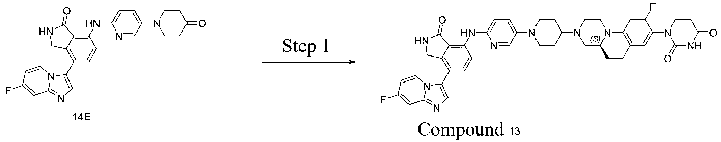

With reference to the synthetic method of step 4 of example 1 and by **acidic prep-HPLC,** the trifluoroacetic acid salt of **compound 13** (18 mg) was obtained.

LCMS m/z =759.5[M+H]⁺
¹H NMR (400 MHz, CD₃OD) δ 8.65 - 8.51 (m, 2H), 8.15 (s, 1H), 8.07 (d, 1H), 7.80 (dd, 1H), 7.71 (d, 1H), 7.54 (dd, 1H), 7.46 - 7.38 (m, 1H), 7.12 - 7.00 (m, 2H), 6.81 (d, 1H), 4.40 (s, 2H), 4.21 - 4.11 (m, 1H), 3.89 - 3.80 (m, 2H), 3.79 - 3.65 (m, 4H), 3.50 - 3.32 (m, 4H), 3.20 - 3.08 (m, 1H), 3.08 - 2.99 (m, 1H), 2.90 - 2.77 (m, 5H), 2.38 - 2.26 (m, 2H), 2.17 - 2.07 (m, 1H), 2.03 - 1.89 (m, 2H), 1.88 - 1.75 (m, 1H).

### Example 14: Preparation of compound 14

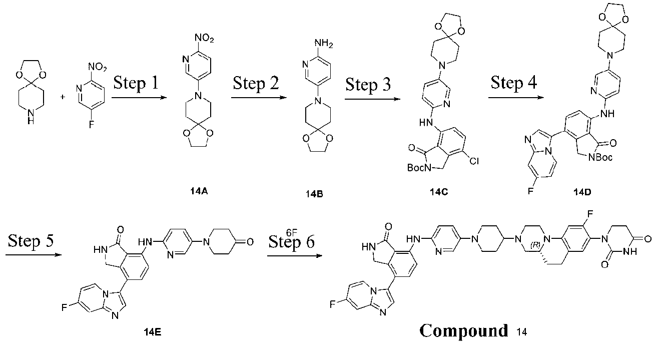

### Step 1: Preparation of 14A

To a reaction flask were added 5-fluoro-2-nitropyridine (2.8 g, 19.71 mmol), 4-piperidone ethylene acetal (2.82 g, 19.71 mmol), sodium bicarbonate (1.66 g, 19.71 mmol) and DMF(30 mL), and the mixture was reacted at 90°C for 4 h. The reaction system was cooled to room temperature, water (100 mL) was added, and the resulting mixture was stirred and crystallised for 1 h and filtered. The filter cake was washed with water and concentrated under reduced pressure to dryness, to afford **14A** (4.82 g).

### Step 2: Preparation of 14B

To a reaction flask were added **14A** (2 g, 7.54 mmol), tetrahydrofuran (30 mL) and water (7 mL), zinc powder (2.47 g, 37.7 mmol) and ammonium chloride (2.02 g, 37.7 mmol) were added under stirring, and the mixture was reacted at room temperature for 1 h. Dichloromethane and saturated sodium bicarbonate aqueous solution were added, the resulting mixture was stirred and then filtered, and the filtrate was subjected to layer separation. An appropriate amount of silica gel was added to the organic layer, and then the mixture was concentrated under reduced pressure, and the residue was purified by silica gel column chromatography (eluents: DCM-CH₃OH=100-0 to 90-10) to afford **14B** (1.2 g, yield: 68%).

With reference to the synthetic method of steps 1 and 2 of example 1, **14D** (0.5 g, yield: 52%) was obtained.

### Step 5: Preparation of 14E

To a reaction flask were added **14D** (0.6 g, 1.0 mmol), tetrahydrofuran (6 mL) and 3M hydrochloric acid (6 mL), and the mixture was reacted at 60°C for 2 h. The reaction system was cooled to room temperature, ethyl acetate was added, and the mixture was adjusted to pH 8-9 with saturated sodium carbonate aqueous solution. After layer separation, an appropriate amount of silica gel was added to the organic layer, the mixture was concentrated under reduced pressure, and the residue was purified by column chromatography (eluents: DCM-CH₃OH=100-0 to 90-10) to afford **14E** (0.28 g, yield: 61%).
LCMS m/z =457.3[M+H]⁺

### Step 6: Preparation of compound 14

With reference to the synthetic method of step 4 of example 1 and by **acidic prep-HPLC,** the trifluoroacetic acid salt of **compound 14** (27 mg) was obtained.

LCMS m/z =759.5[M+H]⁺
¹H NMR (400 MHz, DMSO-*d₆*) δ 10.36 (s, 1H), 9.92 (s, 1H), 8.80 (s, 1H), 8.67 - 8.61 (m, 1H), 8.58 (d, 1H), 8.23 (s, 1H), 8.07 (d, 1H), 7.91 (dd, 1H), 7.73 (d, 1H), 7.52 (dd, 1H), 7.41 - 7.32 (m, 1H), 7.02 (dd, 2H), 6.91 (d, 1H), 4.38 (s, 2H), 4.20 - 4.13 (m, 1H), 3.87 - 3.78 (m, 2H), 3.73 - 3.64 (m, 2H), 3.60 (d, 2H), 3.48 - 3.37 (m, 1H), 3.35 - 3.26 (m, 1H), 3.21 - 3.00 (m, 2H), 2.98 - 2.87 (m, 1H), 2.82 - 2.60 (m, 6H), 2.27 - 2.15 (m, 2H), 2.09 - 1.97 (m, 1H), 1.88 - 1.74 (m, 2H), 1.73 - 1.60 (m, 1H).

### Example 15: Preparation of compound 15

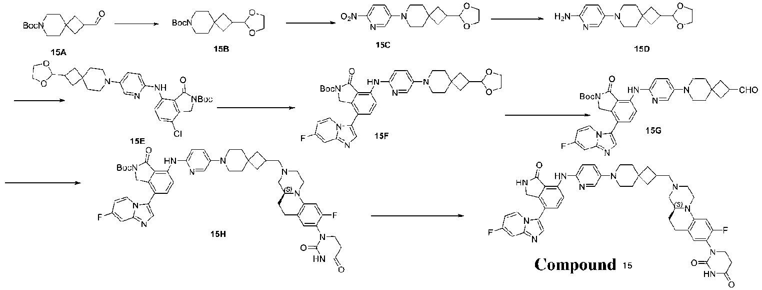

### Step 1: Preparation of 15B

**15A** (4.0 g, 15.79 mmol), ethylene glycol (8 mL, 157.9 mmol) and pyridinium 4-toluenesulphonate (0.4 g, 1.58 mmol) were added to toluene (20 mL), and the mixture was reacted at 140°C for 5 h. The reaction system was concentrated under reduced pressure, and the residue was purified by silica gel column chromatography (mobile phase: ethyl acetate/petroleum ether (V/V)=1/5) to afford **15B** (3.86 g, yield: 82%).

### Step 2: Preparation of 15C

**15B** (1.5 g, 5.04 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure to remove trifluoroacetic acid, 5-fluoro-2-nitropyridine (1.43 g, 10.08 mmol), triethylamine (2.55 g, 25.2 mmol) and DMF (30 mL) were added to the crude product, and the resulting mixture was reacted at 90°C for 4 h, and cooled to room temperature. Water (100 mL) was added, the mixture was stirred and crystallised for 1 h, and filtered, and the filter cake was washed with water and concentrated under reduced pressure to dryness, to afford **15C** (1.3 g).

### Step 3: Preparation of compound 15D

Palladium on carbon (1.3 g) was added to a solution of compound **15C** (1.3 g, 4.08 mmol) in methanol (30 mL), the mixture was stirred at room temperature under hydrogen atmosphere overnight, filtered through celite, and eluted with methanol, and the eluate was concentrated under reduced pressure to afford **15D** (1 g).
LCMS m/z =290.2[M+H]⁺

The trifluoroacetic acid salt of **compound 15** (0.2 g) was obtained by the synthetic method of step 4 to step 8 referring to example 1 and acidic prep-HPLC.

LCMS m/z =813.0[M+H]⁺
¹H NMR (400 MHz, CD₃OD) δ 8.61 (dd, 1H), 8.55 (d, 1H), 8.21 (d, 1H), 8.17 (s, 1H), 7.82 (dd, 1H), 7.78 - 7.70 (m, 2H), 7.46 - 7.39 (m, 1H), 7.15 (d, 1H), 7.03 (d, 1H), 6.79 (d, 1H), 4.42 (s, 2H), 4.16 - 4.06 (m, 1H), 3.72 (t, 2H), 3.67 - 3.58 (m, 1H), 3.58 - 3.49 (m, 1H), 3.40 - 3.32 (m, 5H), 3.28 - 3.23 (m, 2H), 3.22 - 3.07 (m, 2H), 3.01 - 2.90 (m, 1H), 2.90 - 2.68 (m, 5H), 2.33 - 2.21 (m, 2H), 2.13 - 2.03 (m, 1H), 2.03 - 1.95 (m, 2H), 1.91 - 1.82 (m, 2H), 1.82 - 1.71 (m, 3H).

### Example 16: Preparation of compound 16

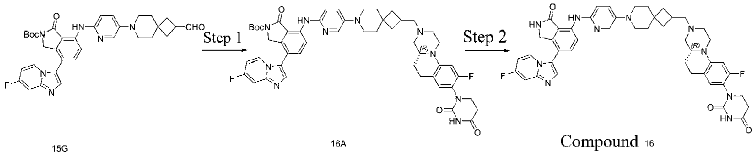

### Step 1: Preparation of 16A

To a reaction flask were added **15G** (0.2 g, 0.39 mmol), **6F** (0.12 g, 0.39 mmol), dichloromethane (10 mL), and glacial acetic acid (0.070 g, 1.17 mmol), and the mixture was stirred at room temperature for 1 h. Sodium triacetoxyborohydride (0.25 g, 1.17 mmol) was added, and the resulting mixture was reacted at room temperature for 3 h. Water and dichloromethane were added, and the mixture was stirred for layer separation. The organic layer was washed with saturated sodium bicarbonate aqueous solution, and concentrated under reduced pressure, and the residue was purified by silica gel column chromatography (eluents: DCM-CH₃OH=100-0 to 90-10) to afford **16A** (0.22 g, yield: 69 %).

### Step 2: Preparation of compound 16

To a reaction flask were added **16A** (0.22 g, 0.24 mmol) and dichloromethane (5 mL), trifluoroacetic acid (3.07 g, 26.93 mmol) was added dropwise under stirring, and the mixture was reacted at room temperature for 2 h. The reaction system was concentrated under reduced pressure to dryness, dichloromethane/methanol and saturated sodium bicarbonate aqueous solution were added, and the resulting mixture was stirred for layer separation. An appropriate amount of silica gel was added to the organic phase, and then the mixture was concentrated under reduced pressure, and the residue was purified by silica gel column chromatography (eluents: DCM-CH₃OH=100-0 to 90-10) to afford **compound 16** (0.11 g, yield: 56%).

LCMS m/z =812.9[M+H]⁺
¹H NMR (400 MHz, DMSO-*d₆*) δ 10.32 (s, 1H), 9.82 (s, 1H), 8.72 (s, 1H), 8.52 (d, 1H), 8.44 - 8.33 (m, 1H), 8.09 - 7.89 (m, 1H), 7.79 (s, 1H), 7.67 (d, 1H), 7.56 - 7.30 (m, 2H), 7.04 - 6.83 (m, 3H), 6.70 (d, 1H), 4.36 (s, 2H), 3.79 - 3.65 (m, 1H), 3.59 (t, 2H), 3.13 - 2.90 (m, 5H), 2.90 - 2.78 (m, 2H), 2.77-2.64 (m, 4H), 2.64 - 2.53 (m, 1H), 2.49 - 2.32 (m, 3H), 2.13 - 1.82 (m, 4H), 1.82 - 1.65 (m, 3H), 1.64 - 1.50 (m, 3H), 1.49 - 1.36 (m, 2H).

### Example 17: Preparation of compound 17

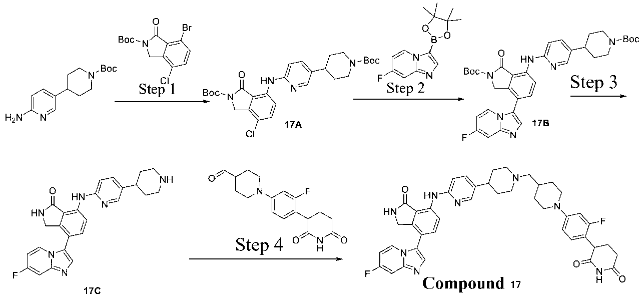

With reference to the synthetic method of step 11 to step 14 of example 9 and by acidic prep-HPLC, the trifluoroacetic acid salt of **compound 17** (114 mg) was obtained.

LCMS m/z =745.0[M+H]⁺
¹H NMR (400 MHz, CD₃OD) δ 8.72 (d, 1H), 8.63 (dd, 1H), 8.31 - 8.22 (m, 1H), 8.19 (s, 1H), 7.84 (dd, 1H), 7.77 (d, 1H), 7.71 (dd, 1H), 7.48 - 7.39 (m, 1H), 7.17 (t, 1H), 7.07 (d, 1H), 6.91 - 6.76 (m, 2H), 4.42 (s, 2H), 3.93 (dd, 1H), 3.85 - 3.68 (m, 4H), 3.24 - 3.08 (m, 4H), 3.03 - 2.85 (m, 3H), 2.81 - 2.60 (m, 2H), 2.34 - 2.02 (m, 7H), 2.02 - 1.90 (m, 2H), 1.64 - 1.45 (m, 2H).

### Example 18: Preparation of compound 18

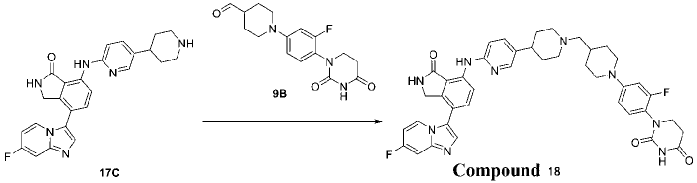

With reference to the reaction condition of step 14 of example 9 and by acidic prep-HPLC, the trifluoroacetic acid salt of **compound 18** (54 mg) was obtained.

LCMS m/z =746.0[M+H]⁺
¹H NMR (400 MHz, CD₃OD) δ 8.75 (d, 1H), 8.63 (dd, 1H), 8.31 - 8.22 (m, 1H), 8.19 (s, 1H), 7.84 (dd, 1H), 7.76 (d, 1H), 7.70 (dd, 1H), 7.48 - 7.37 (m, 1H), 7.23 (t, 1H), 7.06 (d, 1H), 6.90 - 6.74 (m, 2H), 4.42 (s, 2H), 3.88 - 3.66 (m, 6H), 3.23 - 3.08 (m, 4H), 3.02 - 2.83 (m, 3H), 2.80 (t, 2H), 2.29 - 2.01 (m, 5H), 1.99 - 1.82 (m, 2H), 1.58 - 1.38 (m, 2H).

### Example 19: Preparation of compound 19

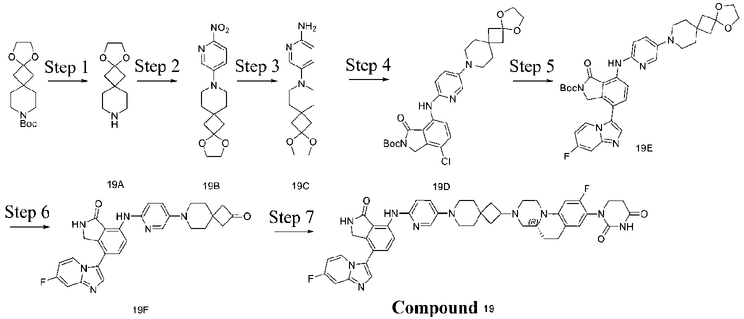

### Step 1: Preparation of 19A

To a reaction flask were added 7-N-BOC-heterospiro[3.5]nonane-2-one ethylene acetal (CAS:2761524-74-5, 4 g, 14.12 mmol) and dichloromethane (20 mL), trifluoroacetic acid (8 mL) was added under stirring, and the mixture was reacted at room temperature for 1 h. The reaction system was concentrated under reduced pressure to dryness, dichloromethane was added, and the mixture was adjusted to pH 9-10 with saturated sodium carbonate aqueous solution, and stirred for layer separation. The aqueous layer was extracted twice with dichloromethane, and the organic layers were combined, and then dried over anhydrous sodium sulphate, filtered, and concentrated under reduced pressure to afford **19A** (2 g).

### Step 2: Preparation of 19B

To a reaction flask were added 5-fluoro-2-nitropyridine (1.1 g, 7.74 mmol), **19A** (1.42 g, 7.74 mmol), sodium bicarbonate (1.30 g, 15.48 mmol) and DMF(15 mL), and the mixture was reacted at 90°C for 4 h. The reaction system was cooled to room temperature, water (50 mL) was added, and the resulting mixture was stirred for 1 h to precipitate a solid, and filtered. The filter cake was washed with water, and concentrated under reduced pressure to afford **19B** (2.1 g).

### Step 3: Preparation of 19C

To a reaction flask were added **19B** (2.1 g, 6.88 mmol), palladium on carbon (0.4 g) and methanol (20 mL), the mixture was purged three times with hydrogen, and the hydrogenation was carried out under a hydrogen balloon. The resulting mixture was stirred and reacted at room temperature overnight, and filtered through an appropriate amount of celite, the filter cake washed with methanol, and the filtrate was concentrated under reduced pressure to dryness, to afford **19C** (1.2 g).

### Step 4: Preparation of 19D

To a reaction flask were added **19C** (0.34 g, 1.23 mmol), tert-butyl 7-bromo-4-chloro-1-oxoisoindoline-2-carboxylate (CAS: 2628351-94-8, 0.47 g, 1.35 mmol), Pd₂(dba)₃ (0.11 g, 0.12 mmol), Xant-Phos (0.14 g, 0.25 mmol), potassium carbonate (0.51 g, 3.69 mmol) and 1,4-dioxane (10 mL), and the mixture was purged three times with nitrogen, and reacted at 80°C under nitrogen overnight. The reaction system was cooled to room temperature, and an appropriate amount of silica gel was added. Then, the mixture was concentrated under reduced pressure, and the residue was purified by silica gel column chromatography (eluents: DCM-CH₃OH=100-0 to 90-10) to afford **19D** (0.6 g, yield: 90%).
LCMS m/z =541.3[M+H]⁺

### Step 5: Preparation of compound 19E

To a reaction flask were added **19D** (0.5 g, 0.92 mmol), 1B-1 (0.36 g, 1.38 mmol), XPHOS-Pd-G2 (0.072 g, 0.092 mmol), X-PHOS (0.088 g, 0.18 mmol), potassium phosphate (0.59 g, 2.76 mmol), 1,4-dioxane (10 mL) and water (3 mL), and the mixture was purged three times with nitrogen and reacted at 95°C under nitrogen for 5 h. The reaction system was cooled to room temperature, ethyl acetate and water were added, and the mixture was stirred for layer separation. An appropriate amount of silica gel was added to the organic layer, the resulting mixture was concentrated under reduced pressure, and the residue was purified by silica gel column chromatography (eluents: DCM-CH₃OH=100-0 to 90-10) to afford **19E** (0.32 g, yield: 54%).
LCMS m/z =641.4[M+H]⁺

### Step 6: Preparation of 19F

To a reaction flask were added **19E**(0.32 g, 0.50 mmol), tetrahydrofuran (3 mL) and 3M hydrochloric acid (3 mL), and the mixture was reacted at 60°C for 2 h. The reaction system was cooled to room temperature, ethyl acetate was added, and the mixture was adjusted to pH 8-9 with saturated sodium carbonate aqueous solution. After layer separation, an appropriate amount of silica gel was added to the organic layer, the mixture was concentrated under reduced pressure, and the residue was purified by column chromatography (eluents: DCM-CH₃OH=100-0 to 90-10) to afford **19F** (0.15 g, yield: 60%).

### Step 7: Preparation of compound 19

To a reaction flask were added **19F** (0.15 g, 0.30 mmol), **6F** (0.11 g, 0.36 mmol), acetic acid (0.054 g, 0.90 mmol), anhydrous sodium sulphate (0.43 g, 3.0 mmol) and chloroform (10 mL), and mixture was heated to 50°C and reacted overnight. Sodium triacetoxyborohydride (0.19 g, 0.90 mmol) was added, and the resulting mixture was reacted for 3 h. Then, sodium triacetoxyborohydride (0.19 g, 0.90 mmol) was added, and the mixture was reacted for another 2 h. The reaction system was cooled to room temperature, water and dichloromethane were added, and the mixture was stirred for layer separation. The organic layer was washed with saturated sodium bicarbonate aqueous solution, and an appropriate amount of silica gel was added. The resulting mixture was concentrated under reduced pressure, the residue was purified by column chromatography (eluents: DCM-CH₃OH=100-0 to 90-10), and the obtained product was subjected to **acidic prep-HPLC** to afford the trifluoroacetic acid salt of **compound 19** (100 mg).

LCMS m/z =799.0[M+H]⁺
¹H NMR (400 MHz, D₂O) δ 8.57 (dd, 1H), 8.35-8.28 (m, 1H), 8.23- 8.15 (m, 1H), 8.12 (s, 1H), 8.10 - 8.00 (m, 1H), 7.91 - 7.77 (m, 2H), 7.52 - 7.41 (m, 2H), 7.16 (d, 1H), 6.92 (d, 1H), 4.48 (s, 2H), 4.26 - 4.12 (m, 1H), 3.98 - 3.82 (m, 3H), 3.78 - 3.63 (m, 2H), 3.64 -3.42 (m, 5H), 3.32 - 3.17 (m, 1H), 3.16 - 3.03 (m, 1H), 2.96 (t, 2H), 2.93 - 2.73 (m, 3H), 2.68 - 2.54 (m, 2H), 2.35 - 2.21 (m, 2H), 2.21 - 2.03 (m, 5H), 1.92 - 1.76 (m, 1H).

### Example 20: Preparation of compound 20

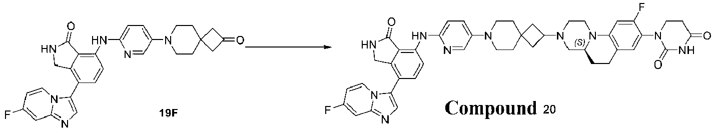

With reference to the synthetic method of example 13 and by **acidic prep-HPLC,** the trifluoroacetic acid salt of **compound 20** (150 mg) was obtained.

LCMS m/z =799.2 [M+H]⁺
¹H NMR (400 MHz, D₂O) δ 8.57 (dd, 1H), 8.33 - 8.27 (m, 1H), 8.23- 8.15 (m, 1H), 8.11 (s, 1H), 8.10 - 8.00 (m, 1H), 7.90 - 7.79 (m, 2H), 7.51 - 7.39 (m, 2H), 7.16 (d, 1H), 6.92 (d, 1H), 4.48 (s, 2H), 4.24 - 4.13 (m, 1H), 3.96 - 3.81 (m, 3H), 3.79 - 3.63 (m, 2H), 3.62 - 3.41 (m, 5H), 3.32 - 3.18 (m, 1H), 3.16 - 3.03 (m, 1H), 2.96 (t, 2H), 2.93 - 2.75 (m, 3H), 2.67 - 2.53 (m, 2H), 2.33 - 2.20 (m, 2H), 2.20 - 1.99 (m, 5H), 1.91 - 1.76 (m, 1H).

### Example 21: Preparation of compound 21

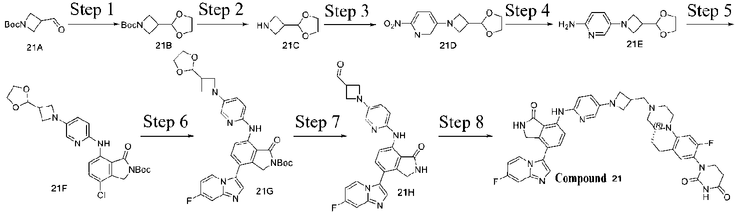

### Step 1: Preparation of 21B

**21A**(2.0 g, 10.80 mmol), ethylene glycol (5.5 mL, 108.0 mmol) and pyridinium 4-toluenesulphonate (0.27 g, 1.08 mmol) were added to toluene (20 mL), and the mixture was warmed to 140°C and reacted for 5 h. The reaction system was concentrated under reduced pressure, and the residue was purified by silica gel column chromatography (mobile phase: ethyl acetate/petroleum ether (V/V)=1/5) to afford **21B** (0.63 g, yield: 25%)

### Step 2: Preparation of 21C

**21B** (0.63 g, 2.75 mmol) was dissolved in dichloromethane (5 mL), trifluoroacetic acid (3 mL) was added dropwise at room temperature, and the mixture was stirred at room temperature for 1 h. The reaction solution was concentrated under reduced pressure to afford a crude trifluoroacetic acid product, and the product was redissolved in 10 mL of dichloromethane. 0.3 mL of N,N-diisopropylethylamine was added, and the mixture was concentrated to afford **21C.**

### Step 3: Preparation of 21D

**21C** obtained in the previous step was dissolved in acetonitrile (15 mL), 5-fluoro-2-nitropyridine (0.39 g, 2.75 mmol) and potassium carbonate (1.14 g, 8.25 mmol) were added, and the mixture was reacted at 65°C for 3 h and cooled to room temperature. The reaction solution was filtered, the filtrate was concentrated under reduced pressure, and the residue was purified by silica gel column chromatography (mobile phase: ethyl acetate/petroleum ether (V/V)=1/1) to afford **21D** (0.59 g, yield: 85%).

### Step 4: Preparation of 21E

Palladium on carbon (0.3 g) was added to a solution of **21D** (0.59 g, 2.35 mmol) in methanol (30 mL), and the mixture was stirred at room temperature under hydrogen atmosphere overnight and filtered through celite. The filter cake was washed with methanol, and the filtrate was concentrated under reduced pressure to afford **21E** (0.46 g).
LCMS m/z =222.1 [M+H]⁺

### Step 5: Preparation of 21F

**21E** (0.4 g, 1.81 mmol) and tert-butyl 7-bromo-4-chloro-1-oxoisoindoline-2-carboxylate (CAS: 2628351-94-8, 0.63 g, 1.81 mmol) were dissolved in 1,4-dioxane solution (15 mL), and tris(dibenzylideneacetone)dipalladium (0.17 g, 0.18 mmol), Xant-phos (0.21 g, 0.36 mmol) and potassium carbonate (0.63 g, 4.53 mmol) were added. The mixture was reacted at 90°C under nitrogen for 8 h. The reaction solution was cooled and subjected to suction filtration through celite, and the filter cake was washed 3 times with ethyl acetate. The filtrates were combined and then concentrated under reduced pressure, and the residue was subjected to flash column chromatography (mobile phase: dichloromethane/methanol (v/v)=20/1) to afford **21F** (0.65 g, yield: 74%).

### Step 6: Preparation of 21G

**21F** (0.65 g, 1.33 mmol), 1B-1 (0.52 g, 2.0 mmol), XPHOS-Pd-G2 (0.21 g, 0.27 mmol), X-PHOS (0.13 g, 0.27 mmol) and potassium phosphate (0.71 g, 3.33 mmol) were added to a mixed solvent of dioxane (20 mL) and water (4 mL), and the mixture was reacted at 90°C under nitrogen atmosphere overnight. Water (20 mL) was added, the resulting mixture was extracted with ethyl acetate (20 mL×3), and the organic phases were combined, dried over anhydrous sodium sulphate, filtered and then concentrated under reduced pressure. The residue was purified by silica gel column chromatography (petroleum ether/ethyl acetate (v/v)=2/3) to afford **21G** (0.7 g, yield: 54%).

### Step 7: Preparation of 21H

**21G** (0.7 g, 1.19 mmol) was dissolved in tetrahydrofuran (5 mL), sulphuric acid (3M) (10 mL) was added dropwise at room temperature, and the mixture was heated to 50°C and stirred for 2 h. The resulting mixture was quenched with saturated sodium carbonate aqueous solution and extracted with dichloromethane (20 mL×3), the organic phases were combined, dried over anhydrous sodium sulphate, filtered and then concentrated under reduced pressure, and the residue was eluted through a reverse phase C18 column (mobile phase: 0-30% acetonitrile /water (containing 0.1% trifluoroacetic acid)) to afford **21H** (0.3 g, yield: 57%).

### Step 8: Preparation of compound 21

**21H** (0.15 g, 0.34 mmol) and **6F** (108 mg, 0.34 mmol) were dissolved in trichloromethane solution (10 mL), glacial acetic acid (61 mg, 1.0 mmol) was added dropwise at room temperature, and the mixture was heated to 60°C and stirred overnight. Then, sodium triacetoxyborohydride (216 mg, 1.0 mmol) was added in small amounts and multiple portions, and after the addition was completed, the mixture was stirred for another 2 h. The resulting mixture was adjusted to be basic with 1*N* sodium hydroxide aqueous solution, and extracted 3 times with dichloromethane, the organic phases were combined, dried over anhydrous sodium sulphate, and concentrated under reduced pressure, and the residue was subjected to **acidic prep-HPLC** to afford the trifluoroacetic acid salt of **compound 21** (12 mg).

LCMS m/z =745.0 [M+H]⁺
¹H NMR (400 MHz, CD₃OD) δ 8.60 (dd, 1H), 8.42 (d, 1H), 8.14 (s, 1H), 7.79 (dd, 1H), 7.67 (d, 1H), 7.65 (d, 1H), 7.45 - 7.36 (m, 1H), 7.11 - 6.98 (m, 3H), 6.81 (d, 1H), 4.39 (s, 2H), 4.23 - 4.08 (m, 3H), 3.78 - 3.69 (m, 4H), 3.69 - 3.55 (m, 4H), 3.42 - 3.32 (m, 2H), 3.28 - 3.21 (m, 1H), 3.21 - 3.08 (m, 1H), 3.07 - 2.95 (m, 1H), 2.93 - 2.69 (m, 4H), 2.18 - 2.01 (m, 1H), 1.87 - 1.72 (m, 1H).

### Example 22: Preparation of compound 22

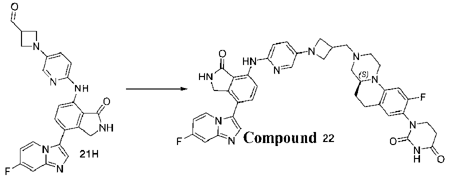

With reference to the synthetic method of example 20 and by **acidic prep-HPLC,** the trifluoroacetic acid salt of **compound 22** (10 mg) was obtained.

LCMS m/z =745.0 [M+H]⁺
¹H NMR (400 MHz, CD₃OD) δ 8.58 (dd, 1H), 8.42 (d, 1H), 8.12 (s, 1H), 7.78 (dd, 1H), 7.69 - 7.63 (m, 2H), 7.43 - 7.34 (m, 1H), 7.09 - 6.98 (m, 3H), 6.81 (d, 1H), 4.39 (s, 2H), 4.23 - 4.03 (m, 3H), 3.78 - 3.69 (m, 4H), 3.69 - 3.54 (m, 4H), 3.43 - 3.32 (m, 2H), 3.28-3.20 (m, 1H), 3.19 - 3.09 (m, 1H), 3.07 - 2.97 (m, 1H), 2.91 - 2.71 (m, 4H), 2.15 - 2.04 (m, 1H), 1.89 - 1.71 (m, 1H).

### Example 23: Preparation of compound 23

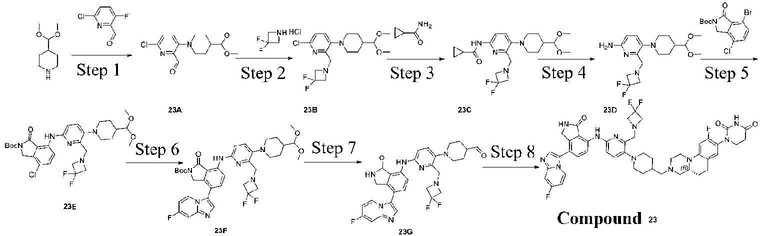

With reference to the synthesis of the **aforementioned compound** and by **acidic prep-HPLC,** the trifluoroacetic acid salt of **compound 23** (19 mg) was obtained.

LCMS m/z =878.3[M+H]⁺
¹H NMR (400 MHz, CD₃OD) δ 8.60 (dd, 1H), 8.32 (d, 1H), 8.17 (s, 1H), 7.86 - 7.75 (m, 3H), 7.46 - 7.36 (m, 1H), 7.24 (d,1H), 7.04 (d, 1H), 6.81 (d, 1H), 4.90 (t, 4H), 4.85 (s, 2H), 4.43 (s, 2H), 4.18 - 4.05 (m, 1H), 3.83 - 3.63 (m, 4H), 3.52 - 3.38 (m, 1H), 3.28 - 3.14 (m, 4H), 3.14 - 3.04 (m, 2H), 3.04 - 2.94 (m, 1H), 2.94 - 2.71 (m, 6H), 2.21 - 2.06 (m, 2H), 2.04 - 1.94 (m, 2H), 1.87 - 1.73 (m, 1H), 1.70 - 1.53 (m, 2H).

### Example 24: Preparation of compound 24

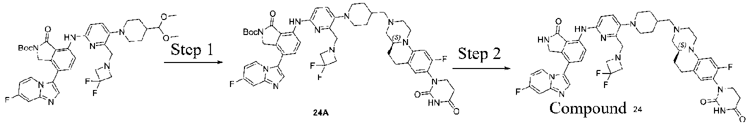

With reference to the synthesis of the **aforementioned compound** and by **acidic prep-HPLC,** the trifluoroacetic acid salt of **compound 24** (0.13 g) was obtained.

LCMS m/z =439.9[(M+2H)/2]⁺
¹H NMR (400 MHz, D₂O) δ 8.55 (dd, 1H), 8.24 (d, 1H), 8.09 (s, 1H), 7.92 - 7.76 (m, 3H), 7.50 - 7.41 (m, 1H), 7.34 (d, 1H), 7.17 (d, 1H), 6.93 (d, 1H), 4.95 (t, 4H), 4.89 (s, 2H), 4.46 (s, 2H), 4.24 - 4.09 (m, 1H), 3.94 - 3.71 (m, 4H), 3.61 - 3.48 (m, 1H), 3.41 - 3.23 (m, 4H), 3.21 - 3.12 (m, 2H), 3.07 (t, 1H), 3.02 - 2.74 (m, 6H), 2.32 - 2.09 (m, 2H), 2.09 - 1.96 (m, 2H), 1.93 - 1.78 (m, 1H), 1.73 - 1.55 (m, 2H).

### Example 25: Preparation of compound 25

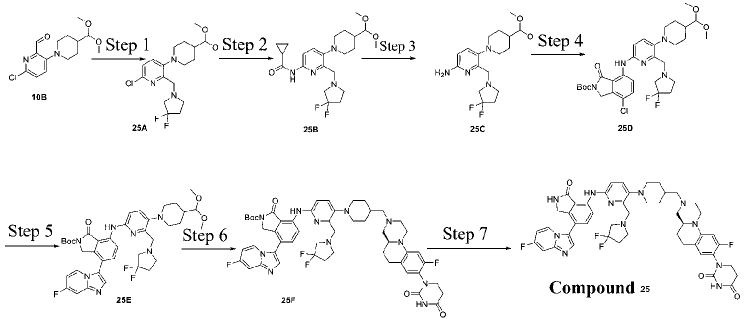

With reference to the synthesis of the **aforementioned compound** and by **acidic prep-HPLC,** the trifluoroacetic acid salt of **compound 25** (0.28 g) was obtained.

LCMS m/z =446.9[(M+2H)/2]⁺
¹H NMR (400 MHz, D₂O) δ 8.61 - 8.49 (m, 1H), 8.30 (d, 1H), 8.09 (s, 1H), 7.91 (d, 1H), 7.87 - 7.75 (m, 2H), 7.49 - 7.40 (m, 1H), 7.35 (d, 1H), 7.16 (d, 1H), 6.92 (d, 1H), 4.79 - 4.63 (m, 2H), 4.45 (s, 2H), 4.22 - 4.12 (m, 1H), 4.06 (t, 2H), 3.94 - 3.81 (m, 5H), 3.79 - 3.70 (m, 1H), 3.61 - 3.49 (m, 1H), 3.39 - 3.25 (m, 4H), 3.24 - 3.15 (m, 2H), 3.14 - 2.98 (m, 3H), 2.95 (t, 2H), 2.92 - 2.72 (m, 4H), 2.34 - 2.20 (m, 1H), 2.19-2.10 (m, 1H), 2.10 - 1.99 (m, 2H), 1.93 - 1.79 (m, 1H), 1.73 - 1.56 (m, 2H).

### Example 26: Preparation of compound 26

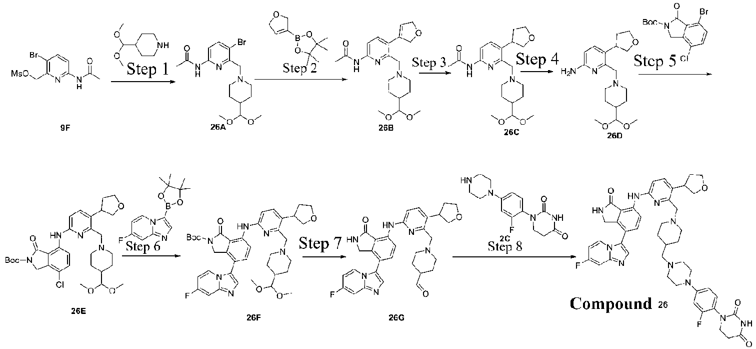

With reference to the synthesis of the **aforementioned compound** and by **acidic prep-HPLC,** the trifluoroacetic acid salt of **compound 26** (16 mg) was obtained.

LCMS m/z =831.0[M+H]⁺
¹H NMR (400 MHz, CD₃OD) δ 8.57 (dd, 1H), 8.36 (d, 1H), 8.13 (s, 1H), 7.93 - 7.71 (m, 3H), 7.44 - 7.22 (m, 3H), 6.97 - 6.78 (m, 2H), 4.65 (s, 2H), 4.42 (s, 2H), 4.17 - 4.05 (m, 2H), 4.00 - 3.80 (m, 3H), 3.80 - 3.71 (m, 3H), 3.68 - 3.32 (m, 9H), 3.30-3.13 (m, 4H), 2.80 (t, 2H), 2.55 - 2.29 (m, 2H), 2.26 - 2.12 (m, 2H), 2.08 - 1.92 (m, 1H), 1.91 - 1.66 (m, 2H).

### Example 27: Preparation of compound 27

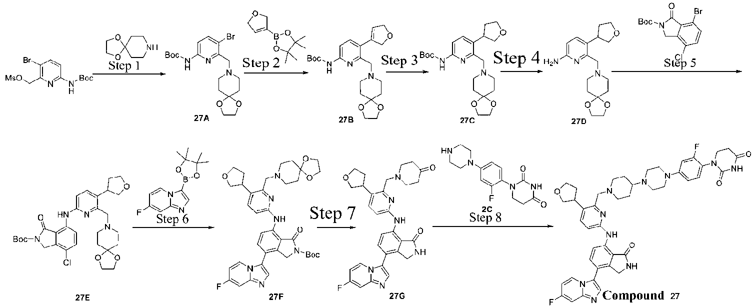

With reference to the synthesis of the **aforementioned compound** and by **acidic prep-HPLC,** the trifluoroacetic acid salt of **compound 27** (56 mg) was obtained.

LCMS m/z =817.0[M+H]⁺
¹H NMR (400 MHz, CD₃OD) δ 8.61 (dd, 1H), 8.43 (d, 1H), 8.18 (s, 1H), 7.92 - 7.76 (m, 3H), 7.49 - 7.19 (m, 3H), 6.96 - 6.78 (m, 2H), 4.69 (s, 2H), 4.43 (s, 2H), 4.15 - 4.06 (m, 2H), 4.06 - 3.88 (m, 3H), 3.80 - 3.70 (m, 3H), 3.69 - 3.32 (m, 12H), 2.80 (t, 2H), 2.63 - 2.51 (m, 2H), 2.51 - 2.40 (m, 1H), 2.40 - 2.23 (m, 2H), 2.05 - 1.91 (m, 1H).

### Example 28: Preparation of compound 28

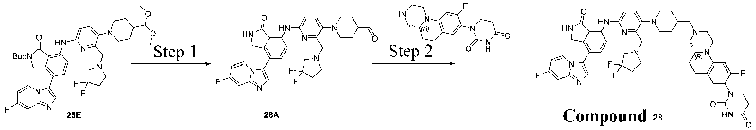

With reference to the synthesis of the **aforementioned compound** and by **acidic prep-HPLC,** the trifluoroacetic acid salt of **compound 28** (20 mg) was obtained.

LCMS m/z =892.3[M+H]⁺
¹H NMR (400 MHz, CD₃OD) δ 8.56 (dd, 1H), 8.46 (d, 1H), 8.14 (s, 1H), 7.84 - 7.71 (m, 3H), 7.42 - 7.34 (m, 1H), 7.22 (d, 1H), 7.04 (d, 1H), 6.81 (d, 1H), 4.70 (s, 2H), 4.42 (s, 2H), 4.18 - 3.94 (m, 3H), 3.81 (t, 2H), 3.78 - 3.62 (m, 4H), 3.51 - 3.35 (m, 1H), 3.28 - 3.06 (m, 6H), 3.03 - 2.63 (m, 9H), 2.20 - 2.05 (m, 2H), 2.05 - 1.93 (m, 2H), 1.86 - 1.73 (m, 1H), 1.67 - 1.52 (m, 2H).

### Example 29: Preparation of compound 29

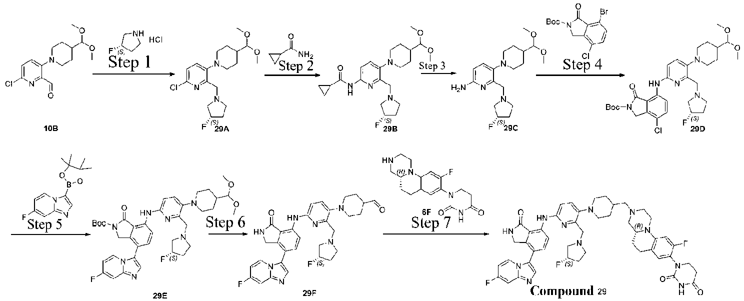

### Step 1: Preparation of 29A

**10B** (1.90 g, 6.36 mmol) was dissolved in 50 mL of trichloromethane, (3S)-3-fluoropyrrolidine hydrochloride (0.96 g, 7.63 mmol), acetic acid (0.36 mL, 6.36 mmol) and anhydrous sodium sulphate (1.81 g, 12.72 mmol) were added, and the mixture was stirred at room temperature for 1 h. Sodium triacetylborohydride (6.74 g, 31.80 mmol) was slowly added, and the resulting mixture was stirred at room temperature for 1 h. 50 mL of saturated sodium bicarbonate aqueous solution was added to the reaction solution, and the mixture was stirred for layer separation. The organic phase was washed once with saturated sodium chloride aqueous solution, dried over anhydrous sodium sulphate, and concentrated under reduced pressure, and the residue was subjected to flash column chromatography (mobile phase: petroleum ether/ethyl acetate (v/v)=4/1) to afford compound **29A** (0.70 g, yield: 30%).
LCMS m/z =372.1[M+H]⁺

### Step 2: Preparation of 29B

**29A** (0.77 g, 2.07 mmol) was dissolved in 1,4-dioxane (20 mL), cyclopropanamide (0.35 g, 4.14 mmol), caesium carbonate (2.02 g, 6.21 mmol), tris(dibenzylideneacetone)dipalladium (0.19 g, 0.21 mmol) and XantPhos (0.24 g, 0.41 mmol) were added, and the mixture was purged three times with nitrogen, heated to 100°C and reacted overnight. After the reaction was completed, the reaction solution was diluted with ethyl acetate (100 mL), and subjected to suction filtration through celite, the filter cake was washed 3 times with ethyl acetate, and the filtrates were combined, and then washed once with water and once with saturated sodium chloride aqueous solution. The organic phase was dried over anhydrous sodium sulphate, and concentrated under reduced pressure, and the residue was subjected to flash column chromatography (mobile phase: dichloromethane/methanol (v/v)=20/1) to afford **29B** (0.86 g).
LCMS m/z =421.1[M+H]⁺

### Step 3: Preparation of 29C

**29B** (0.86 g, 2.05 mmol) was dissolved in methanol (20 mL), water (5 mL) and sodium hydroxide (1.64 g, 41.00 mmol) were added, and the mixture was reacted at 80°C overnight . The reaction system was concentrated to remove methanol, 100 mL of dichloromethane and 20 mL of water were added, and the mixture was stirred and then subjected to layer separation . The aqueous layer was extracted twice with dichloromethane, and the organic phases were combined, washed once with saturated sodium chloride aqueous solution, dried over anhydrous sodium sulphate, and concentrated under reduced pressure to afford **29C** (0.67 g).
LCMS m/z =353.1[M+H]⁺

### Step 4: Preparation of 29D

**29C** (0.67 g, 1.90 mmol) and tert-butyl 7-bromo-4-chloro-1-oxoisoindoline-2-carboxylate (CAS: 2628351-94-8, 0.66 g, 1.90 mmol) were dissolved in 1,4-dioxane solution (20 mL), and tris(dibenzylideneacetone)dipalladium (0.17 g, 0.19 mmol), Xant-phos (0.22 g, 0.38 mmol) and potassium carbonate (0.79 g, 5.70 mmol) were added. The mixture was reacted at 100°C under nitrogen for 4 h. The reaction solution was diluted with ethyl acetate (100 mL), and subjected to suction filtration through celite, the filter cake was washed 3 times with ethyl acetate, and the filtrates were combined, and then washed once with water and once with saturated sodium chloride aqueous solution. The organic phase was dried over anhydrous sodium sulphate, and concentrated under reduced pressure, and the residue was subjected to flash column chromatography (mobile phase: dichloromethane/methanol (v/v)=20/1) to afford compound **29D** (0.93 g, yield: 79%).
LCMS m/z =618.3[M+H]⁺

### Step 5: Preparation of 29E

**29D** (0.93 g, 1.50 mmol), 1B-1 (0.59 g, 2.25 mmol), X-PHOS (0.14 g, 0.30 mmol), XPHOS-Pd-G2 (0.12 g, 0.15 mmol) and potassium phosphate (0.96 g, 4.50 mmol) were added to a mixed solvent of dioxane (20 mL) and water (5 mL), and the mixture was purged three times with nitrogen and reacted at 100°C for 3 h. Water (30 mL) was added, and then the resulting mixture was extracted with ethyl acetate (30 mL×3). The organic phases were combined, dried over anhydrous sodium sulphate, filtered and then concentrated under reduced pressure, and the concentrated residue was purified by silica gel column chromatography (mobile phase: dichloromethane/methanol =15/1) to afford **29E** (0.67 g, yield: 62%).
LCMS m/z =718.4[M+H]⁺

### Step 6: Preparation of 29F

**29E** (0.67 g, 0.93 mmol) was dissolved in tetrahydrofuran (6 mL), 2 M sulphuric acid aqueous solution (13.3 mL) was added, and the mixture was heated to 60°C and reacted for 2 h. The reaction solution was cooled to room temperature, adjusted to be basic with sodium carbonate aqueous solution, extracted with dichloromethane/isopropanol and subjected to liquid separation. The organic phase was dried over anhydrous sodium sulphate, and concentrated under reduced pressure to afford **29F** (0.53 g).
LCMS m/z =572.1[M+H]⁺

### Step 7: Preparation of compound 29

**29F** (0.30 g, 0.31 mmol) and **6F** (0.099 g, 0.31 mmol) were dissolved in trichloromethane (10 mL), glacial acetic acid (0.037 g, 0.62 mmol) was added at room temperature, and then anhydrous sodium sulphate (0.037 g, 0.26 mmol) was added. The mixture was heated to 60°C and reacted for 16 h, sodium triacetoxyborohydride (0.66 g, 3.10 mmol) was slowly added, and after the addition was completed, the resulting mixture was reacted at 60°C for 3 h. The reaction system was adjusted to be basic with saturated sodium bicarbonate aqueous solution, and extracted 3 times with dichloromethane, the organic phases were combined, dried over anhydrous sodium sulphate, and concentrated under reduced pressure, and the residue was subjected to acidic prep-HPLC to afford the trifluoroacetic acid salt of **compound 29** (27 mg).

LCMS m/z =874.4[M+H]⁺
¹H NMR (400 MHz, CD₃OD) δ 8.55 (dd, 1H), 8.46 (d, 1H), 8.13 (s, 1H), 7.87 - 7.70 (m, 3H), 7.43 - 7.32 (m, 1H), 7.22 (d, 1H), 7.04 (d, 1H), 6.81 (d, 1H), 5.67 - 5.31 (m, 1H), 4.77 (s, 2H), 4.42 (s, 2H), 4.18 - 4.07 (m, 1H), 4.00 - 3.37 (m, 9H), 3.28 - 3.14 (m, 4H), 3.11 - 3.02 (m, 2H), 3.01 - 2.69 (m, 7H), 2.62 - 2.31 (m, 2H), 2.23 - 1.91 (m, 4H), 1.85 - 1.71 (m, 1H), 1.69 - 1.49 (m, 2H).

### Example 30: Preparation of compound 30

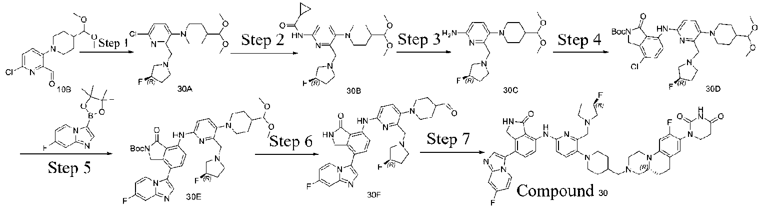

With reference to the synthesis of **compound 29** and by **acidic prep-HPLC,** the trifluoroacetic acid salt of **compound 30** (80 mg) was obtained.

LCMS m/z =874.4[M+H]⁺
¹H NMR (400 MHz, D₂O) δ 8.33 (dd, 1H), 8.10 (d, 1H), 7.87 (s, 1H), 7.72 - 7.53 (m, 3H), 7.28 - 7.18 (m, 1H), 7.12 (d, 1H), 6.94 (d, 1H), 6.70 (d, 1H), 5.55 - 5.26 (m, 1H), 4.57 (s, 2H), 4.23 (s, 2H), 3.98 - 3.90 (m, 1H), 3.85 - 3.24 (m, 9H), 3.18 - 3.01 (m, 4H), 2.98 - 2.80 (m, 3H), 2.78 - 2.54 (m, 6H), 2.44-2.20 (m, 2H), 2.09 - 1.88 (m, 2H), 1.87 - 1.73 (m, 2H), 1.72 - 1.54 (m, 1H), 1.50 - 1.30 (m, 2H).

### Example 31: Preparation of compound 31

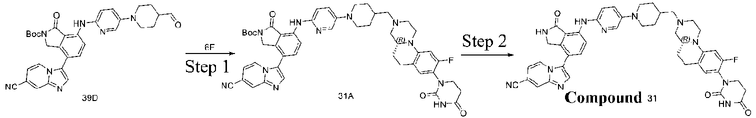

With reference to the synthetic method of example 16 and by **acidic prep-HPLC,** the trifluoroacetic acid salt of **compound 31** (44 mg) was obtained.

LCMS m/z =780.3[M+H]⁺
¹H NMR (400 MHz, DMSO-*d₆*) δ 10.36 (s, 1H), 9.93 (s, 1H), 8.79 (s, 1H), 8.58 - 8.50 (m, 2H), 8.47 (s, 1H), 8.18 (s, 1H), 8.07 (d, 1H), 7.74 (d, 1H), 7.53 (dd, 1H), 7.25 (dd, 1H), 7.03 (d, 2H), 6.90 (d, 1H), 4.40 (s, 2H), 4.19 - 4.03 (m, 1H), 3.76 - 3.55 (m, 6H), 3.45 - 3.33 (m, 1H), 3.22 - 3.04 (m, 4H), 2.97 - 2.83 (m, 1H), 2.83 - 2.62 (m, 6H), 2.13 - 1.99 (m, 2H), 1.97 - 1.82 (m, 2H), 1.72 - 1.57 (m, 1H), 1.50 - 1.32 (m, 2H).

### Example 32: Preparation of compound 32

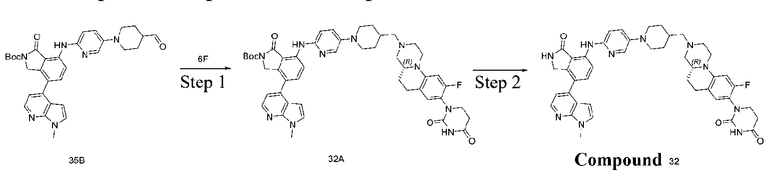

### Step 1: Preparation of 32A

To a reaction flask were added **35B** (0.17 g, 0.28 mmol), **6F** (0.106 g, 0.33 mmol) and dichloromethane (15 mL). The mixture was stirred at room temperature for 1 h, and then sodium triacetoxyborohydride (0.19 g, 0.90 mmol) and glacial acetic acid (0.054 g, 0.90 mmol) were added. The resulting mixture was reacted at room temperature for 3 h, and then saturated sodium bicarbonate aqueous solution and dichloromethane were added, and the mixture was stirred for layer separation. The organic layer was concentrated under reduced pressure to dryness, and the residue was purified by silica gel column chromatography (eluents: DCM-CH₃OH=100-0 to 90-10) to afford **32A** (0.14 g, yield: 54%).

### Step 2: Preparation of compound 32

To a reaction flask were added **32A** (0.14 g, 0.16 mmol) and dichloromethane (5 mL), and trifluoroacetic acid (2 mL) was added under stirring. The mixture was reacted at room temperature for 3 h, and concentrated under reduced pressure to dryness, and the residue was subjected to **acidic prep-HPLC to afford** the trifluoroacetic acid salt of **compound 32** (90 mg).

LCMS m/z =385.3[(M+2H)/2]⁺
¹H NMR (400 MHz, DMSO-*d₆*) δ 10.36 (s, 1H), 9.98 (s, 1H), 8.75 (s, 1H), 8.50 (d, 1H), 8.32 (d, 1H), 8.07 (d, 1H), 7.73 (d, 1H), 7.62 - 7.48 (m, 2H), 7.26 (d, 1H), 7.03 (dd, 2H), 6.91 (d, 1H), 6.47 (d, 1H), 4.45 (s, 2H), 4.21 - 4.04 (m, 1H), 3.87 (s, 3H), 3.73 - 3.56 (m, 6H), 3.45 - 3.33 (m, 1H), 3.24 - 3.01 (m, 4H), 2.97 - 2.60 (m, 7H), 2.16 - 1.98 (m, 2H), 1.98 - 1.80 (m, 2H), 1.72 - 1.57 (m, 1H), 1.52 - 1.32 (m, 2H).

### Example 33: Preparation of compound 33

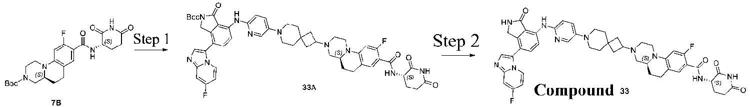

### Step 1: Preparation of compound 33A

Compound **7B** (100 mg, 0.17 mmol) was dissolved in dichloromethane (10 mL), trifluoroacetic acid (3 mL) was added, and the mixture was reacted at room temperature for 1 h. The reaction solution was concentrated under reduced pressure, triethylamine (3 mL) was added to the residue, and the resulting mixture was stirred and then concentrated under reduced pressure. Trichloromethane (15 mL), **37D** (94 mg, 0.20 mmol), glacial acetic acid (10 mg, 0.17 mmol), and anhydrous sodium sulphate (0.037 g, 0.26 mmol) were added, and the mixture was reacted at 60°C for 16 h. Sodium triacetoxyborohydride (108 mg, 0.51 mmol) was slowly added, and after the addition was completed, the resulting mixture was reacted for another 3 h. The reaction system was adjusted to be basic with saturated sodium bicarbonate aqueous solution, and extracted 3 times with dichloromethane, the organic phases were combined, dried over anhydrous sodium sulphate, and concentrated under reduced pressure, and the residue was subjected to flash column chromatography (mobile phase: dichloromethane/methanol (v/v)=20/1) to afford compound **33A** (200 mg).

### Step 2: Preparation of compound 33

**33A** (0.16 g, 0.17 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure, and then the residue was subjected to **acidic prep-HPLC** to afford the trifluoroacetic acid salt of **compound 33** (30 mg).

LCMS m/z =421.2[(M+2H)/2]⁺
¹H NMR (400 MHz, CD₃OD) δ 8.62 (dd, 1H), 8.49 (d, 1H), 8.18 (s, 1H), 8.12 (1H), 7.82 (dd, 1H), 7.74 (d, 1H), 7.68 (dd, 1H), 7.54 (d, 1H), 7.48 - 7.38 (m, 1H), 7.14 (d, 1H), 6.76 (d, 1H), 4.81 - 4.74 (m, 1H), 4.41 (s, 2H), 4.31 - 4.15 (m, 1H), 3.88 - 3.72 (m, 1H), 3.69 - 3.53 (m, 2H), 3.53 - 3.41 (m, 1H), 3.30 - 3.17 (m, 5H), 3.09 - 2.95 (m, 1H), 2.91 - 2.65 (m, 5H), 2.52 - 2.38 (m, 2H), 2.37 - 2.25 (m, 1H), 2.22 - 2.07 (m, 4H), 1.98 - 1.85 (m, 4H), 1.84 - 1.71 (m, 1H).

### Example 34: Preparation of compound 34

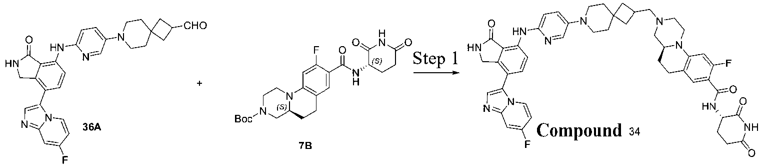

With reference to the synthetic method of step 1 of example 33 and by **acidic prep-HPLC,** the trifluoroacetic acid salt of **compound 34** (45 mg) was obtained.

LCMS m/z =428.4[(M+2H)/2]⁺
¹H NMR (400 MHz, CD₃OD) δ 8.62 (dd, 1H), 8.55 (d, 1H), 8.24 - 8.13 (m, 2H), 7.82 (dd, 1H), 7.77 - 7.67 (m, 2H), 7.54 (d, 1H), 7.46 - 7.36 (m, 1H), 7.13 (d, 1H), 6.75 (d, 1H), 4.81 - 4.74 (m, 1H), 4.41 (s, 2H), 4.28 - 4.11 (m, 1H), 3.69 - 3.32 (m, 7H), 3.27 - 3.09 (m, 4H), 3.04 - 2.61 (m, 6H), 2.39 - 2.21 (m, 3H), 2.21 - 2.05 (m, 2H), 2.05 - 1.92 (m, 2H), 1.91 - 1.69 (m, 5H).

### Example 35: Preparation of compound 35

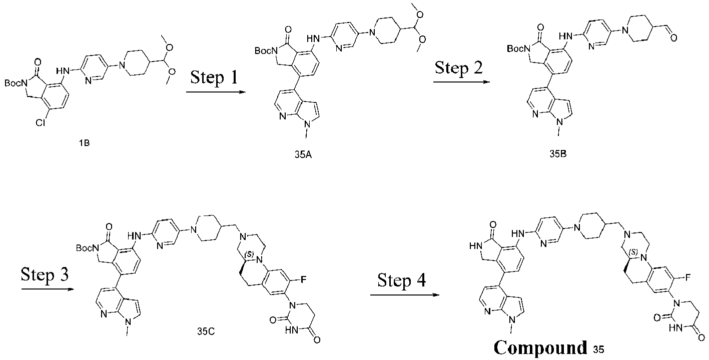

### Step 1: Preparation of 35A

Compound **1B** (0.8 g, 1.55 mmol), XPHOS-Pd-G2 (0.12 g, 0.16 mmol), 1-methyl-4-(tetramethyl-1,3,2-dioxaborolan-2-yl)-1H-pyrrolo[2,3-b]pyridine (0.48 g, 1.86 mmol), X-PHOS (0.15 g, 0.31 mmol) and potassium phosphate (0.99 g, 4.65 mmol) were added to a mixed solvent of dioxane (60 mL) and water (20 mL), and the mixture was reacted at 100°C under nitrogen atmosphere for 2 h. Water (30 mL) was added, and then the mixture was extracted with ethyl acetate (30 mL×3). The organic phases were combined, dried over anhydrous sodium sulphate, filtered and then concentrated under reduced pressure, and the concentrated residue was purified by silica gel column chromatography (petroleum ether/ethyl acetate (v/v)=2/3) to afford **35A** (0.5 g, yield: 53%).
LCMS m/z =613.3[M+H]⁺

### Step 2: Preparation of compound 35B

Compound **35A** (0.5 g, 3.32 mmol) was dissolved in tetrahydrofuran (10 mL), hydrochloric acid (2N) (60 mL) was added dropwise at room temperature, and the mixture was stirred at room temperature for 2 h. The resulting mixture was quenched with saturated sodium bicarbonate aqueous solution, and extracted with ethyl acetate (110 mL×3). The organic phases were combined, dried over anhydrous sodium sulphate, filtered and then concentrated under reduced pressure, and the concentrated residue was purified by silica gel column chromatography (dichloromethane/methanol (v/v)=100/5) to afford compound **35B** (0.5 g, yield: 71%).
LCMS m/z =567.3[M+H]⁺

### Step 3: Preparation of compound 35C

Compound **35B** (0.16 g, 0.28 mmol) and compound **5G** (0.089 g, 0.28 mmol) were dissolved in dichloromethane solution (10 mL), glacial acetic acid (0.034 g, 0.56 mmol) was added dropwise at room temperature, and then sodium sulphate (0.088 g, 0.62 mmol) and sodium triacetoxyborohydride (0.12 g, 0.56 mmol) were added. After the addition was completed, the mixture was stirred at room temperature for 16 h. The resulting mixture was adjusted to be basic with 1*N* sodium hydroxide aqueous solution, and extracted 3 times with dichloromethane, the organic phases were combined, dried over anhydrous sodium sulphate, and concentrated under reduced pressure, and the residue was purified by silica gel column chromatography (dichloromethane/methanol (V/V)=15/1) to afford compound **35C** (0.2 g, yield: 82%).
LCMS m/z =869.5[M+H]⁺

### Step 4: Preparation of compound 35

**35C**(0.2 g, 0.23 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure to remove trifluoroacetic acid, and the crude product was subjected to **acidic prep-HPLC** to afford the trifluoroacetic acid salt of **compound 35** (0.08 g).

LCMS m/z =769.2[M+H]⁺
¹H NMR (400 MHz, D₂O) δ 8.44 (d, 1H), 8.17 - 8.02 (m, 2H), 8.00 - 7.88 (m, 2H), 7.71 - 7.59 (m, 2H), 7.49 (d, 1H), 7.14 (d, 1H), 6.90 (d, 1H), 6.81 (d, 1H), 4.57 (s, 2H), 4.19 - 4.10 (m, 1H), 4.06 (s, 3H), 3.88 - 3.70 (m, 6H), 3.59 - 3.48 (m, 1H), 3.37 - 3.21 (m, 6H), 3.12 - 3.01 (m, 1H), 2.94 (t, 2H), 2.91 - 2.73 (m, 2H), 2.45 - 2.29 (m, 1H), 2.24 - 2.05 (m, 3H), 1.90 - 1.79 (m, 1H), 1.78 - 1.62 (m, 2H).

### Example 36: Preparation of compound 36

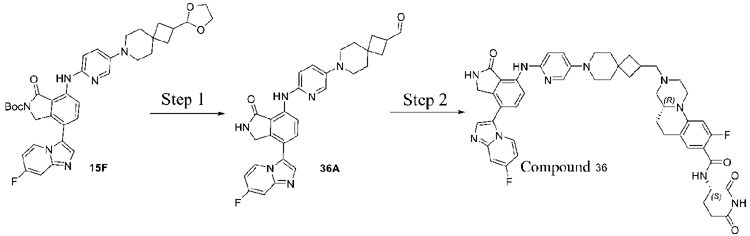

### Step 1: Preparation of compound 36A

Compound **15F**(0.27 g, 0.41 mmol) was dissolved in tetrahydrofuran (4 mL), sulphuric acid (2N) (4 mL) was added dropwise at room temperature, and the mixture was heated to 60°C and stirred for 2 h. The resulting mixture was quenched with saturated sodium carbonate aqueous solution, and extracted with dichloromethane/isopropanol (v/v)=10/1 (50 mL×4), and the organic phases were combined, dried over anhydrous sodium sulphate, filtered and then concentrated under reduced pressure to afford compound **36A** (0.2 g).

### Step 2: Preparation of compound 36

To a reaction flask were added **8B** (116 mg, 0.25 mmol) and dichloromethane (2 mL), trifluoroacetic acid (1 mL) was added under stirring, and the mixture was reacted at room temperature for 1 h. The reaction system was concentrated under reduced pressure, and then the residue was redissolved in 3 mL of dichloromethane, and 0.5 mL of triethylamine was added. The mixture was stirred and then concentrated under reduced pressure, **36A** (90 mg, 0.18 mmol), acetic acid (0.022 g, 0.36 mmol), anhydrous sodium sulphate (51 mg, 0.36 mmol) and chloroform (10 mL) were added to the residue, and the resulting mixture was stirred at 60°C overnight. Sodium triacetoxyborohydride (0.11 g, 0.54 mmol) was added in small amounts and multiple portions, and the mixture was reacted for another 5 h. The reaction system was adjusted to be basic with saturated sodium bicarbonate aqueous solution, and extracted 3 times with dichloromethane, the organic phases were combined, dried over anhydrous sodium sulphate, and concentrated under reduced pressure, and the residue was subjected to **acidic prep-HPLC to afford** the trifluoroacetic acid salt of **compound 36** (110 mg).

LCMS m/z =855.3 [M+H]⁺
¹H NMR (400 MHz, D₂O) δ 8.47 (dd, 1H), 8.32 (d, 1H), 8.21 (d, 1H), 8.01 (s, 1H), 7.94 (dd, 1H), 7.82 - 7.69 (m, 2H), 7.43 (d, 1H), 7.38 - 7.25 (m, 2H), 6.71 (d, 1H), 4.87 - 4.78 (m, 1H), 4.37 (s, 2H), 4.18 - 4.01 (m, 1H), 3.73 - 3.39 (m, 7H), 3.38 - 3.06 (m, 4H), 3.02 - 2.63 (m, 6H), 2.35 - 2.17 (m, 4H), 2.14 - 2.01 (m, 3H), 1.99 - 1.88 (m, 2H), 1.86 - 1.64 (m, 3H).

### Example 37: Preparation of compound 37

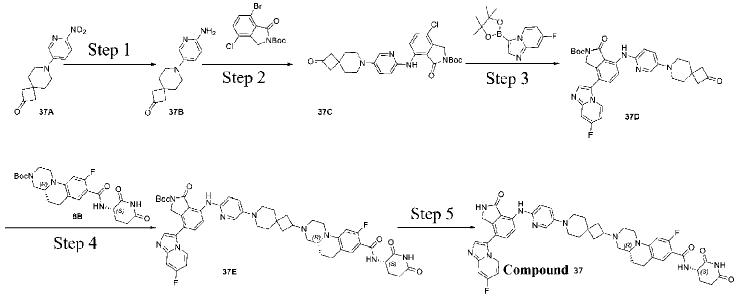

### Step 1: Preparation of 37B

**37A** (3 g, 11.48 mmol) was dissolved in 100 mL of methanol, and 2.0 g palladium on carbon catalyst was added. The mixture was purged three times with hydrogen, and the hydrogenation was carried out under a hydrogen balloon. The resulting mixture was stirred and reacted at room temperature overnight, and filtered through an appropriate amount of celite, the filter cake washed with methanol, and the filtrate was concentrated under reduced pressure to dryness, to afford **37B** (2.3 g).
LCMS m/z =232.3[M+H]⁺

### Step 2: Preparation of 37C

To a reaction flask were added **37B** (0.32 g, 1.38 mmol), tert-butyl 7-bromo-4-chloro-1-oxoisoindoline-2-carboxylate (CAS: 2628351-94-8, 0.48 g, 1.38 mmol), Pd₂(dba)₃ (0.25 g, 0.28 mmol), Xant-Phos (0.16 g, 0.28 mmol), potassium carbonate (0.57 g, 4.14 mmol) and 1,4-dioxane (25 mL), and the mixture was purged three times with nitrogen, and reacted at 100°C under nitrogen for 4 h. The reaction system was cooled to room temperature, and an appropriate amount of silica gel was added. Then, the mixture was concentrated under reduced pressure, and the residue was purified by silica gel column chromatography (eluents: DCM-CH₃OH=100-0 to 90-10) to afford **37C** (0.49 g, yield: 89%).

### Step 3: Preparation of37D

To a reaction flask were added **37C** (0.49 g, 0.99 mmol), 1B-1 (0.26 g, 0.99 mmol), XPHOS-Pd-G2 (0.16 g, 0.2 mmol), X-PHOS (94 mg, 0.2 mmol), potassium phosphate (0.63 g, 2.97 mmol), 1,4-dioxane (20 mL) and water (4 mL), and the mixture was purged three times with nitrogen and reacted at 90°C under nitrogen for 5 h. The reaction system was cooled to room temperature, ethyl acetate and water were added, and the mixture was stirred for layer separation. An appropriate amount of silica gel was added to the organic layer, the resulting mixture was concentrated under reduced pressure, and the residue was purified by silica gel column chromatography (eluents: DCM-CH₃OH=100-0 to 90-10) to afford **37D** (0.24 g, yield: 41%).

### Step 4: Preparation of 37E

To a reaction flask were added **8B** (129 mg, 0.28 mmol) and dichloromethane (2 mL), trifluoroacetic acid (1 mL) was added under stirring, and the mixture was reacted at room temperature for 1 h. The reaction system was concentrated under reduced pressure to dryness, and then the residue was redissolved in 3 mL of dichloromethane, and 0.5 mL of triethylamine was added. The mixture was stirred, and then concentrated under reduced pressure, **37D** (0.12 g, 0.20 mmol), acetic acid (0.024 g, 0.40 mmol), anhydrous sodium sulphate (85 mg, 0.6 mmol) and chloroform (10 mL) were added to the residue, and the resulting mixture was stirred at 60°C overnight. Sodium triacetoxyborohydride (0.13 g, 0.60 mmol) was added in small amounts and multiple portions, and the mixture was reacted for another 5 h. The reaction system was cooled to room temperature, water and dichloromethane were added, and the mixture was stirred for layer separation. The organic layer was washed with saturated sodium bicarbonate aqueous solution, and an appropriate amount of silica gel was added. The resulting mixture was concentrated under reduced pressure, the residue was purified by column chromatography (eluents: DCM-CH₃OH=100-0 to 90-10) to afford **37E** (0.15 g, yield: 79%)

### Step 5: Preparation of compound 37

**37E** (0.15 g) was dissolved in 2 mL of dichloromethane, 1 mL of trifluoroacetic acid was added, and the mixture was stirred at room temperature for 1 h, and then concentrated. The obtained crude product was subjected to **acidic prep-HPLC to afford** the trifluoroacetic acid salt of **compound 37** (100 mg).

LCMS m/z =841.3 [M+H]⁺
¹H NMR (400 MHz, D₂O) δ 8.54 - 8.40 (m, 1H), 8.27 - 8.16 (m, 1H), 8.09 (d, 1H), 8.05 - 7.90 (m, 2H), 7.80 - 7.69 (m, 2H), 7.48 - 7.30 (m, 3H), 6.72 (d, 1H), 4.89 - 4.78 (m, 1H), 4.37 (s, 2H), 4.22 - 4.06 (m, 1H), 3.86 - 3.70 (m, 1H), 3.68 - 3.53 (m, 2H), 3.53 - 3.34 (m, 5H), 3.29 - 3.16 (m, 1H), 3.10 - 2.93 (m, 1H), 2.90 - 2.68 (m, 5H), 2.57 - 2.40 (m, 2H), 2.29 - 2.12 (m, 4H), 2.12 - 1.91 (m, 5H), 1.80 - 1.64 (m, 1H).

### Example 38: Preparation of compound 38

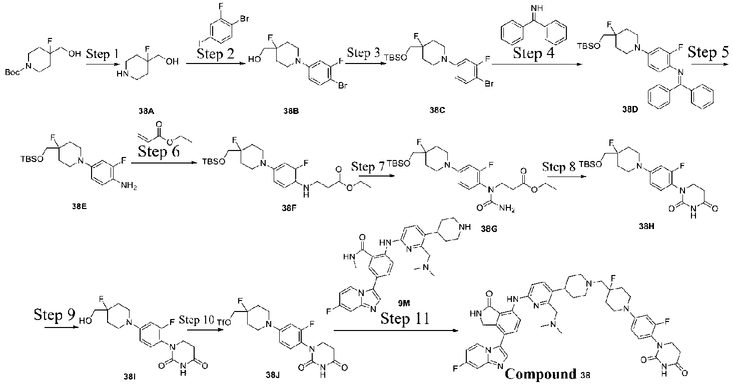

### Step 1: Preparation of 38A

1-Tert-butoxycarbonyl-4-fluoro-4-(hydroxymethyl)piperidine (5.00 g,21.43 mmol) was added to a 250 mL round bottom flask, 4 M hydrogen chloride dioxane solution (60 mL) was added, and the mixture was reacted at room temperature for 2 h. The reaction solution was directly concentrated under reduced pressure to afford the hydrochloride of **38A** (3.63 g).

### Step 2: Preparation of 38B

The hydrochloride of compound **38A** (3.63 g), 1-bromo-2-fluoro-4-iodobenzene (5.85 g, 19.44 mmol), L-proline (0.90 g, 7.78 mmol), cuprous iodide (0.74 g, 3.89 mmol) and potassium carbonate (8.06 g, 58.32 mmol) were added to DMSO (100 mL), and the mixture was purged three times with nitrogen, and reacted at 90°C for 16 h. The reaction solution was cooled to room temperature and diluted with 300 mL of ethyl acetate, the organic phase was washed 3 times with water and once with saturated sodium chloride aqueous solution, dried over anhydrous sodium sulphate, filtered and then concentrated under reduced pressure, and the concentrated residue was purified by silica gel column chromatography (mobile phase: petroleum ether/ethyl acetate =2/1) to afford **38B** (3.00 g, yield: 50%).
LCMS m/z =306.0[M+H]⁺

### Step 3: Preparation of 38C

Compound **38B** (3.00 g, 9.80 mmol) was dissolved in DMF (40 mL), triethylamine (1.98 g, 19.60 mmol) was added, and tert-butyldimethylsilyl chloride (2.22 g, 14.70 mmol) was slowly added. After the addition was completed, the mixture was reacted at room temperature for 4 h. The reaction solution was diluted with 200 mL of ethyl acetate, the organic phase was washed 3 times with water and once with saturated sodium chloride, dried over anhydrous sodium sulphate, filtered and then concentrated under reduced pressure, and the concentrated residue was purified by silica gel column chromatography (mobile phase: petroleum ether/ethyl acetate =5/1) to afford **38C** (3.66 g, yield: 88%).

### Step 4: Preparation of 38D

**38C** (2.96 g,7.04 mmol), benzophenone imine (1.79 g, 9.86 mmol), caesium carbonate (4.59 g, 14.08 mmol), palladium acetate (0.32 g, 1.41 mmol), and XANT PHOS (0.41 g, 0.70 mmol) were added to dioxane solution (50 mL), and the mixture was reacted at 105°C under nitrogen atmosphere for 16 h. The reaction solution was cooled to room temperature, and filtered through celite to remove the solid, and the filter cake was washed with dichloromethane. The organic phase was collected and concentrated under reduced pressure, and the residue was purified by silica gel column chromatography (mobile phase: petroleum ether/ethyl acetate (v/v)=10/1) to afford **38D** (1.57 g, yield: 43%).

### Step 5: Preparation of 38E

**38D**(1.57 g, 3.01 mmol) was added to methanol (50 mL), palladium on carbon (1.46 g, wt%=10%) and ammonium acetate (1.43 g, 18.54 mmol) were added, and the mixture was reacted at room temperature under hydrogen atmosphere (balloon pressure) for 16 h. The reaction solution was filtered through celite, the filtrate was concentrated under reduced pressure, and the residue was purified by silica gel column chromatography (mobile phase: ethyl acetate/petroleum ether (v/v)=1/1) to afford **38E** (0.69 g, yield: 64%).
LCMS m/z = 357.2[M+H]⁺

### Step 6: Preparation of 38F

**38E** (1.04 g, 2.92 mmol), ethyl acrylate (0.88 g, 8.76 mmol) and *N,N-*diisopropylethylamine (1.13 g, 8.76 mmol) were successively added to ethanol (10 mL), and the mixture was heated to 100°C and reacted for 72 h. The reaction system was concentrated under reduced pressure, and the residue was separated and purified by column chromatography (mobile phase: petroleum ether/ethyl acetate (v/v)=6/1) to afford **38F** (1.22 g, yield: 91%).
LCMS m/z =457.2[M+H]⁺

### Step 7: Preparation of 38G

**38F** (1.22 g, 2.66 mmol) and N,N-diisopropylethylamine (1.03 g, 7.98 mmol) were added to tetrahydrofuran (40 mL), and then triphosgene (0.87 g, 2.93 mmol) was slowly added, and the mixture was reacted at room temperature for 1 h. Aqueous ammonia (10 mL) was added, and the resulting mixture was heated to 50°C and reacted for another 2 h. The reaction solution was diluted with 100 mL of ethyl acetate, the organic phase was washed 3 times with water and once with saturated sodium chloride, dried over anhydrous sodium sulphate, and concentrated under reduced pressure, and the residue was separated and purified by column chromatography (mobile phase: dichloromethane/methanol (v/v)=15/1) to afford **38G** (1.24 g, yield: 93%).
LCMS m/z = 500.3 [M+H]⁺

### Step 8: Preparation of 38H

**38G** (1.24 g, 2.47 mmol) was added to acetonitrile (30 mL), and then benzyltrimethylammonium hydroxide (40% solution in methanol, 2.92 mL) was added, and the mixture was heated to 60°C and reacted for 0.5 h. Silica gel was added, the resulting mixture was concentrated under reduced pressure, and the residue was separated and purified by column chromatography (mobile phase: dichloromethane/methanol (v/v)=15/1) to afford **38H** (0.29 g, yield: 26%).
LCMS m/z = 454.3 [M+H]⁺

### Step 9: Preparation of 38I

**38H**(0.27 g, 0.60 mmol) was added to tetrahydrofuran (10 mL), and then TBAF(0.47 g,1.80 mmol) was added, and the mixture was reacted at room temperature for 0.5 h. Silica gel was added, the resulting mixture was concentrated under reduced pressure, and the residue was separated and purified by column chromatography (mobile phase: dichloromethane/methanol (v/v)=10/1) to afford **38I** (0.195 g, yield: 97%).

### Step 10: Preparation of 38J

**38I** (0.20 g, 0.59 mmol) was added to pyridine (5 mL), and then trifluoromethanesulphonic anhydride (0.25 g, 0.89 mmol) was added, and the mixture was reacted at room temperature for 1 h. The reaction solution was diluted with 50 mL of ethyl acetate, the organic phase was washed 3 times with water and once with saturated sodium chloride aqueous solution, dried over anhydrous sodium sulphate, and concentrated under reduced pressure, and the residue was separated and purified by column chromatography (mobile phase: dichloromethane/methanol (v/v)=15/1) to afford **38J** (0.206 g, yield: 74%).

### Step 11: Preparation of compound 38

Compound **38J** (0.057 g, 0.12 mmol) and **9M** (0.058 g, 0.12 mmol) were dissolved in acetonitrile (10 mL), triethylamine (0.036 g, 0.36 mmol) was added at room temperature, and the mixture was heated to 50°C and reacted for 16 h. The reaction solution was diluted with 50 mL of ethyl acetate, the organic phase was washed 3 times with water and once with saturated sodium chloride aqueous solution, dried over anhydrous sodium sulphate, and concentrated under reduced pressure, and the residue was separated and purified by column chromatography (mobile phase: dichloromethane/methanol (v/v)=15/1) to afford **compound 38** (46 mg).

LCMS m/z =821.2[M+H]⁺
¹H NMR (400 MHz, CD₃OD/CDCl₃(v/v)=1/1) δ 8.64 (d, 1H), 8.21 (dd, 1H), 7.73 - 7.60 (m, 3H), 7.30 (dd, 1H), 7.16 (t, 1H), 7.04 (d, 1H), 6.95 - 6.81 (m, 1H), 6.44 - 6.23 (m, 2H), 4.36 (s, 2H), 4.01 (s, 2H), 3.75 (t, 2H), 3.62 - 3.43 (m, 4H), 3.29 - 3.15 (m, 2H), 3.02 - 2.76 (m, 5H), 2.64 (s, 6H), 2.48 - 2.33 (m, 3H), 2.30 - 2.11 (m, 3H), 1.95 - 1.81 (m, 4H).

### Example 39: Preparation of compound 39

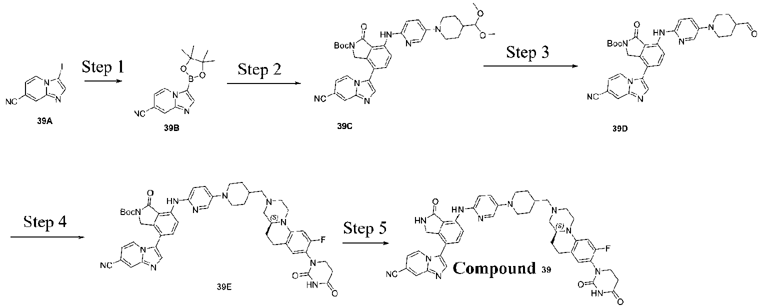

### Step 1: Preparation of 39B

Under nitrogen at -15°C, isopropylmagnesium chloride-lithium chloride (8 mL, 1.3M in THF) was added dropwise to a solution of **39A** (2 g, 7.43 mmol) and isopropyl pinacol borate (2.21 g, 11.89 mmol) in tetrahydrofuran (70 mL). The mixture was stirred at -15°C for 2 h, the reaction was quenched with saturated ammonium chloride aqueous solution (150 mL) and subjected to suction filtration, and the obtained solid was slurried with ethyl acetate to afford compound **39B** (1.6 g, yield: 80%).

### Step 2: Preparation of 39C

Compound **1B** (0.8 g, 1.55 mmol), XPHOS-Pd-G2 (0.12 g, 0.16 mmol), compound **39B** (0.5 g, 1.86 mmol), X-PHOS (0.15 g, 0.31 mmol) and potassium phosphate (0.99 g, 4.65 mmol) were added to a mixed solvent of dioxane (60 mL) and water (20 mL), and the mixture was reacted at 100°C under nitrogen atmosphere for 2 h. The reaction system was cooled to room temperature, and then water (30 mL) was added. The mixture was extracted with ethyl acetate (30 mL×3), the organic phases were combined, dried over anhydrous sodium sulphate, filtered and then concentrated under reduced pressure, and the concentrated residue was purified by silica gel column chromatography (petroleum ether/ethyl acetate (v/v)=2/3) to afford **39C** (0.6 g, yield: 62 %).

LCMS m/z =624.3[M+H]⁺
¹H NMR (400 MHz, CD₃OD) δ 8.51 (d, 1H), 8.45 - 8.37 (m, 1H), 8.22 - 8.14 (m, 1H), 8.04 - 7.95 (m, 2H), 7.69 (d, 1H), 7.44 (dd, 1H), 7.15 (dd, 1H), 6.98 (d, 1H), 4.70 (s, 2H), 4.13 (d, 1H), 3.68 - 3.60 (m, 2H), 3.38 (s, 6H), 2.74 - 2.61 (m, 2H), 1.90 - 1.83 (m, 2H), 1.81 - 1.71 (m, 1H), 1.55 (s, 9H), 1.53-1.43 (m, 2H).

### Step 3: Preparation of compound 39D

Compound **39C** (0.6 g, 3.32 mmol) was dissolved in tetrahydrofuran (20 mL), hydrochloric acid (2N) (120 mL) was added dropwise at room temperature, and the mixture was stirred at room temperature for 2 h. The resulting mixture was quenched with saturated sodium bicarbonate aqueous solution, and extracted with ethyl acetate (110 mL×3). The organic phases were combined, dried over anhydrous sodium sulphate, filtered and then concentrated under reduced pressure, and the concentrated residue was purified by silica gel column chromatography (dichloromethane/methanol (v/v)=100/3) to afford compound **39D** (0.6 g, yield: 90%).
LCMS m/z =578.3[M+H]⁺

### Step 4: Preparation of compound 39E

Compound **39D** (0.27 g, 0.47 mmol) and compound **5G** (0.15 g, 0.47 mmol) were dissolved in dichloromethane solution (10 mL), glacial acetic acid (0.056 g, 0.94 mmol) was added dropwise at room temperature, and then sodium sulphate (0.088 g, 0.62 mmol) and sodium triacetoxyborohydride (0.2 g, 0.94 mmol) were added. After the addition was completed, the mixture was stirred at room temperature for 16 h. The resulting mixture was adjusted to be basic with 1*N* sodium hydroxide aqueous solution, and extracted 3 times with dichloromethane, the organic phases were combined, dried over anhydrous sodium sulphate, and concentrated under reduced pressure, and the residue was purified by silica gel column chromatography (dichloromethane/methanol (V/V)=15/1) to afford compound **39E** (0.22 g, yield: 53%).

### Step 5: Preparation of compound 39

**39E** (0.22 g, 0.12 mmol) was dissolved in dichloromethane (10 mL), trifluoroacetic acid (3 mL) was added dropwise at room temperature, and the mixture was stirred at room temperature for 1 h. The reaction solution was directly concentrated under reduced pressure to remove trifluoroacetic acid, and the crude product was subjected to **acidic prep-HPLC** to afford the trifluoroacetic acid salt of **compound 39** (0.11 g).

LCMS m/z =780.2[M+H]⁺
¹H NMR (400 MHz, D₂O) δ 8.71 (d, 1H), 8.66 (s, 1H), 8.40 (s, 1H), 8.27 - 8.19 (m, 1H), 8.17 - 8.04 (m, 2H), 7.90 (d, 1H), 7.75 (d, 1H), 7.56 (d, 1H), 7.17 (d, 1H), 6.93 (d, 1H), 4.50 (s, 2H), 4.23 - 4.12 (m, 1H), 3.93 - 3.81 (m, 5H), 3.80 - 3.70 (m, 1H), 3.62 - 3.50 (m, 1H), 3.46 - 3.24 (m, 6H), 3.16 - 3.05 (m, 1H), 2.96 (t, 2H), 2.93 - 2.76 (m, 2H), 2.50 - 2.34 (m, 1H), 2.28 - 2.10 (m, 3H), 1.94 - 1.68 (m, 3H).

### Example 40: Preparation of compound 40

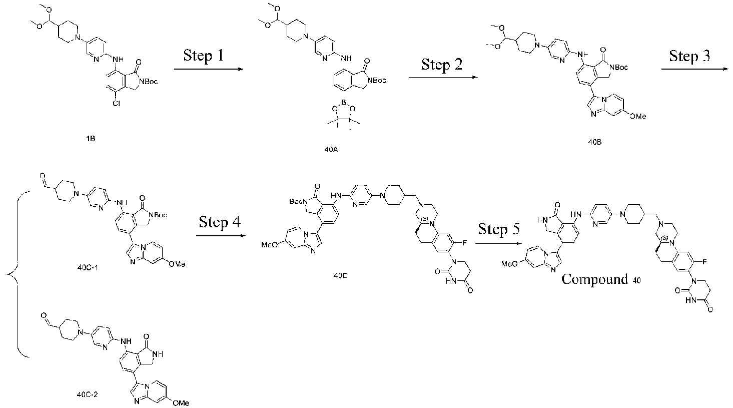

With reference to the synthesis of the aforementioned compound and by **acidic prep-HPLC,** the trifluoroacetic acid salt of **compound 40** (25 mg) was obtained.

LCMS m/z =393.3[(M+2H)/2]⁺
¹H NMR (400 MHz, CD₃OD) δ 8.53 - 8.44 (m, 1H), 8.37 (d, 1H), 8.11 - 8.03 (m, 1H), 7.98 (s, 1H), 7.70 (d, 1H), 7.64 - 7.53 (m, 1H), 7.34 - 7.26 (m, 1H), 7.17 - 6.97 (m, 3H), 6.81 (d, 1H), 4.41 (s, 2H), 4.20 - 4.03 (m, 4H), 3.83 - 3.63 (m, 6H), 3.46 - 3.38 (m, 1H), 3.25 - 3.12 (m, 4H), 3.04 - 2.70 (m, 7H), 2.21 - 2.05 (m, 2H), 2.06 - 1.94 (m, 2H), 1.86 - 1.73 (m, 1H), 1.66 - 1.50 (m, 2H).

### Example 41: Preparation of compound 41

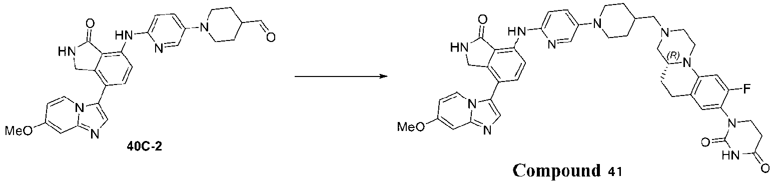

With reference to the synthetic method of example 12 and by **acidic prep-HPLC,** the trifluoroacetic acid salt of **compound 41** (110 mg) was obtained.

LCMS m/z =393.3[(M+2H)/2]⁺
¹H NMR (400 MHz, DMSO-*d₆*) δ 10.37 (s, 1H), 9.92 (s, 1H), 9.86 - 9.61 (m, 1H), 8.82 (s, 1H), 8.58 (d, 1H), 8.48 (d, 1H), 8.23 (s, 1H), 8.05 (d, 1H), 7.74 (d, 1H), 7.51 (dd, 1H), 7.45 - 7.35 (m, 1H), 7.12 (dd, 1H), 7.02 (dd, 2H), 6.91 (d, 1H), 4.40 (s, 2H), 4.17 - 4.08 (m, 1H), 4.03 (s, 3H), 3.76 - 3.53 (m, 6H), 3.47 - 3.33 (m, 1H), 3.26 - 2.99 (m, 4H), 2.96 - 2.58 (m, 7H), 2.15 - 1.79 (m, 4H), 1.74 - 1.57 (m, 1H), 1.51 - 1.30 (m, 2H).

### Example 42: Preparation of compound 42

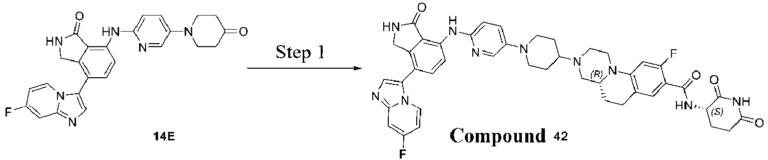

To a reaction flask were added **8B** (0.12 g, 0.26 mmol) and dichloromethane (3 mL), trifluoroacetic acid (2 mL) was added under stirring, and the mixture was reacted at room temperature for 2 h and concentrated under reduced pressure to dryness. The residue was redissolved in 2 mL of dichloromethane, and the resulting mixture was basified with 0.5 mL of triethylamine and then concentrated to dryness. Compound **14E** (0.1 g, 0.22 mmol) was added to the obtained crude, trichloromethane (10 mL) was added, and glacial acetic acid (40 mg, 0.66 mmol) was added dropwise at room temperature. Then, anhydrous sodium sulphate (0.2 g) was added, and the mixture was stirred at 60°C overnight. Sodium triacetoxyborohydride (0.19 g, 0.88 mmol) was slowly added in small amounts and multiple portions, and the resulting mixture was stirred at 60°C for another 24 h. The mixture was adjusted to be basic with 1*N* sodium hydroxide aqueous solution, and extracted 3 times with dichloromethane, the organic phases were combined, dried over anhydrous sodium sulphate, and concentrated under reduced pressure, and the crude product was subjected to **acidic prep-HPLC to afford** the trifluoroacetic acid salt of **compound 42** (25 mg).

LCMS m/z =801.3[M+H]⁺
¹H NMR (400 MHz, D₂O) δ 8.55 - 8.43 (m, 1H), 8.03 (s, 1H), 7.97 - 7.80 (m, 3H), 7.80 - 7.71 (m, 2H), 7.51 - 7.42 (m, 2H), 7.41 - 7.30 (m, 1H), 6.80 - 6.67 (m, 1H), 4.90 - 4.79 (m, 1H), 4.38 (s, 2H), 4.23 - 4.10 (m, 1H), 3.89 - 3.64 (m, 4H), 3.57 - 3.42 (m, 2H), 3.35 - 3.15 (m, 2H), 3.06 - 2.70 (m, 7H), 2.39 - 2.20 (m, 4H), 2.18 - 2.04 (m, 1H), 2.00 - 1.84 (m, 2H), 1.81 - 1.67 (m, 1H).

### Example 43: Preparation of compound 43

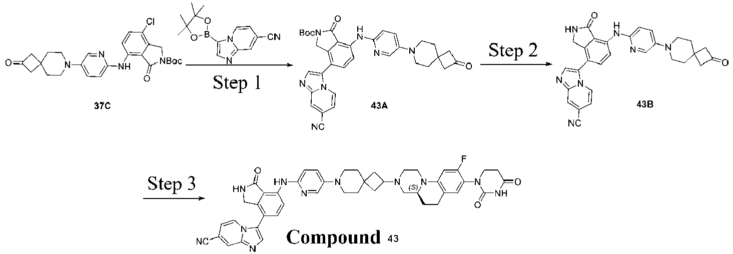

### Step 1: Preparation of 43A

To a reaction flask were added **37C** (400 mg, 0.80 mmol), **39B** (320 mg,1.2 mmol), XPHOS-Pd-G2 (126 mg, 0.16 mmol), X-PHOS (76 mg, 0.16 mmol), potassium phosphate (509 mg, 2.4 mmol), 1,4-dioxane (20 mL) and water (4 mL), and the mixture was purged three times with nitrogen and reacted at 90°C under nitrogen for 3 h. The reaction system was cooled to room temperature, ethyl acetate and water were added, and the mixture was stirred for layer separation. An appropriate amount of silica gel was added to the organic layer, the resulting mixture was concentrated under reduced pressure, and the residue was purified by silica gel column chromatography (eluents: DCM-CH₃OH=100-0 to 90-10) to afford **43A** (380 mg, yield: 78%).

### Step 2: Preparation of 43B

To a reaction flask were added **43A** (380 mg, 0.63 mmol) and dichloromethane (4 mL), trifluoroacetic acid (2 mL) was added under stirring, and the mixture was reacted at room temperature for 1 h. The reaction system was concentrated under reduced pressure to dryness, and then the residue was redissolved in 30 mL of dichloromethane. The mixture was adjusted to be basic with sodium carbonate aqueous solution and stirred for layer separation, and the organic layer was washed once with water, dried over anhydrous sodium sulphate, and concentrated in vacuum under reduced pressure to afford **43B** (250 mg).
LCMS m/z = 504.2[M+H]⁺

### Step 3: Preparation of compound 43

To a reaction flask were added **43B** (90 mg, 0.18 mmol), **5G** (57 mg, 0.18 mmol) and trichloromethane (20 mL), and 0.1 mL of acetic acid was added dropwise. The reaction solution was stirred at 70°C overnight, and refluxed at 80°C for 5 h, sodium triacetoxyborohydride (110 mg, 0.54 mmol) was added, and the mixture was reacted at 80°C for 3 h. Saturated sodium carbonate aqueous solution and dichloromethane were added, and the resulting mixture was stirred for layer separation. The organic layer was dried over anhydrous sodium sulphate, filtered, and concentrated under reduced pressure to dryness, and the residue was subjected to **acidic prep-HPLC to afford** the trifluoroacetic acid salt of **compound 43** (50 mg).

LCMS m/z = 403.8[(M+2H)/2]⁺
¹H NMR (400 MHz, DMSO-*d₆*) δ 10.36 (s, 1H), 10.07 - 9.73 (m, 1H), 8.77 (s, 1H), 8.59 - 8.49 (m, 2H), 8.46 - 8.42 (m, 1H), 8.15 (s, 1H), 8.03 (d, 1H), 7.73 (d, 1H), 7.48 (dd, 1H), 7.22 (dd, 1H), 7.10 - 6.86 (m, 3H), 4.39 (s, 2H), 4.19 - 4.16 (m, 1H), 3.82 - 3.77 (m, 1H), 3.61 (t, 2H), 3.55 - 3.44 (m, 2H), 3.35 - 3.23 (m, 1H), 3.19 - 2.97 (m, 5H), 2.97 - 2.84 (m, 1H), 2.83 - 2.60 (m, 5H), 2.31 - 2.17 (m, 2H), 2.14 - 1.99 (m, 3H), 1.83 - 1.57 (m, 5H).

### Example 44: Preparation of compound 44

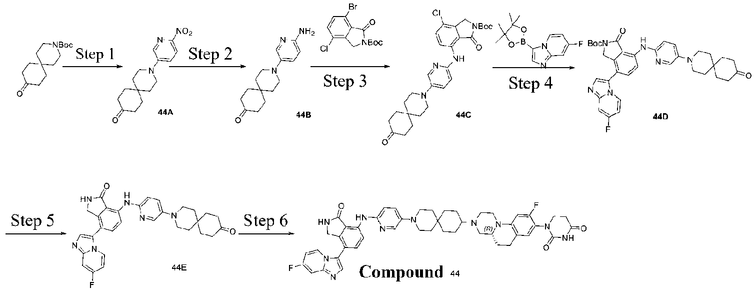

With reference to the synthesis of the aforementioned compound and by **acidic prep-HPLC,** the trifluoroacetic acid salt of **compound 44** (20 mg) was obtained.

LCMS m/z =827.3[M+H]⁺
¹H NMR (400 MHz, DMSO-*d₆*) δ 10.36 (s, 1H), 9.89 (s, 1H), 8.78 (s, 1H), 8.65 - 8.51 (m, 2H), 8.19 (s, 1H), 8.05 (d, 1H), 7.87 (dd, 1H), 7.72 (d, 1H), 7.50 (dd, 1H), 7.41 - 7.28 (m, 1H), 7.07 - 6.97 (m, 2H), 6.90 (d, 1H), 4.37 (s, 2H), 4.20 - 4.08 (m, 1H), 3.62-3.59 (m, 4H), 3.33 - 2.99 (m, 8H), 2.95-2.83 (m, 1H), 2.81 - 2.62 (m, 4H), 2.11 - 1.80 (m, 5H), 1.76 - 1.43 (m, 7H), 1.28 - 1.12 (m, 2H).

### Example 45: Preparation of compound 45

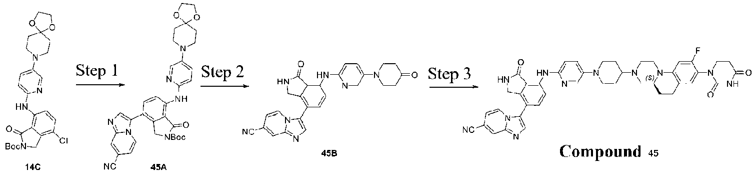

With reference to the synthetic method of step 4 to step 6 of example 14 and by **acidic prep-HPLC,** the trifluoroacetic acid salt of **compound 45** (10 mg) was obtained.

LCMS m/z =766.3[M+H]⁺
¹H NMR (400 MHz, DMSO-*d₆*) δ 10.37 (s, 1H), 9.91 (s, 1H), 8.79 (s, 1H), 8.57 (d, 1H), 8.54 - 8.48 (m, 1H), 8.47 - 8.40 (m, 1H), 8.14 (s, 1H), 8.07 (d, 1H), 7.73 (d, 1H), 7.51 (dd, 1H), 7.22 (dd, 1H), 7.07 - 6.99 (m, 2H), 6.92 (d, 1H), 4.40 (s, 2H), 4.22 - 4.10 (m, 1H), 3.92 - 3.75 (m, 2H), 3.74 - 3.56 (m, 4H), 3.33 - 2.85 (m, 5H), 2.78 - 2.63 (m, 6H), 2.28 - 2.13 (m, 2H), 2.09 - 1.97 (m, 1H), 1.88 - 1.76 (m, 2H), 1.73 - 1.60 (m, 1H).

### Example 46: Preparation of compound 46

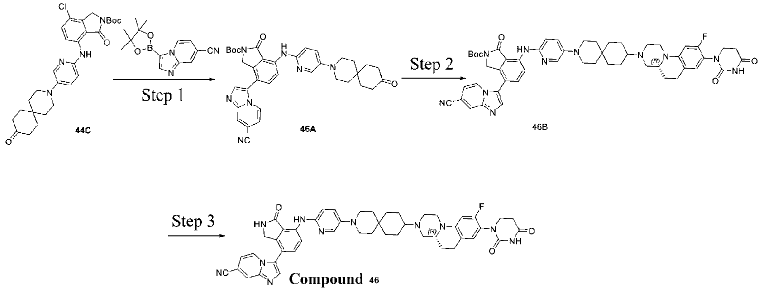

### Step 1: Preparation of 46A

To a reaction flask were added **44C** (600 mg, 1.14 mmol), **39B** (0.46 g, 1.71 mmol), XPHOS-Pd-G2 (90 mg, 0.11 mmol), X-PHOS (54 mg, 0.11 mmol), potassium phosphate (726 mg, 3.42 mmol), 1,4-dioxane (25 mL) and water (5 mL), and the mixture was purged three times with nitrogen and reacted at 90°C under nitrogen for 5 h. The reaction system was cooled to room temperature, ethyl acetate and water were added, and the mixture was stirred for layer separation. An appropriate amount of silica gel was added to the organic layer, the resulting mixture was concentrated under reduced pressure, and the residue was purified by silica gel column chromatography (eluents: DCM-CH₃OH=100-0 to 90-10) to afford **46A** (0.41 g, yield: 57%).
LCMS m/z = 632.3[M+H]⁺

### Step 2: Preparation of 46B

To a reaction flask were added **46A** (130 mg, 0.21 mmol), **6F** (80 mg, 0.25 mmol) and trichloromethane (20 mL), and 0.1 mL of acetic acid was added dropwise. The reaction solution was stirred at 70°C overnight, and refluxed at 80°C for 5 h, sodium triacetoxyborohydride (130 mg, 0.63 mmol) was added, and the mixture was reacted at 80°C for 3 h. Saturated sodium carbonate aqueous solution and dichloromethane were added, and the resulting mixture was stirred for layer separation. The organic layer was dried over anhydrous sodium sulphate, filtered, and concentrated under reduced pressure to dryness, and the residue was purified by silica gel column chromatography (eluents: DCM-CH₃OH=100-0(eluents:90-10) to afford **46B** (100 mg, yield: 52%).
LCMS m/z = 934.3[M+H]⁺

### Step 3: Preparation of compound 46

**46E** (100 mg, 0.11 mmol) was dissolved in 2 mL of dichloromethane, 1 mL of trifluoroacetic acid was added, and the mixture was stirred at room temperature for 1 h. The reaction solution was concentrated under reduced pressure to dryness, and the residue was subjected to **acidic prep-HPLC** to afford the trifluoroacetic acid salt of **compound 46** (20 mg).

LCMS m/z = 834.4[M+H]⁺
¹H NMR (400 MHz, DMSO-*d₆*) δ 10.37 (s, 1H), 9.89 (s, 1H), 8.78 (s, 1H), 8.59 - 8.48 (m, 2H), 8.45 (s, 1H), 8.15 (s, 1H), 8.10 - 7.98 (m, 1H), 7.73 (d, 1H), 7.55 - 7.42 (m, 1H), 7.22 (dd, 1H), 7.06 - 6.85 (m, 3H), 4.40 (s, 2H), 4.21 - 4.06 (m, 1H), 3.70 - 3.57 (m, 4H), 3.34 - 2.98 (m, 8H), 2.95 - 2.58 (m, 5H), 2.09 - 1.81 (m, 5H), 1.76 - 1.37 (m, 7H), 1.29 - 1.10 (m, 2H).

### Example 47: Preparation of compound 47

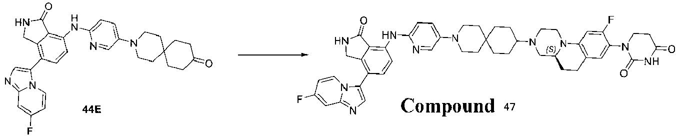

With reference to the synthetic method of step 6 of example 44 and by **acidic prep-HPLC,** the trifluoroacetic acid salt of **compound 47** (30 mg) was obtained.

LCMS m/z =827.5[M+H]⁺
¹H NMR (400 MHz, DMSO-*d₆*) δ 10.37 (s, 1H), 9.92 (s, 1H), 8.81 (s, 1H), 8.70 - 8.52 (m, 2H), 8.25 (s, 1H), 8.10 - 8.03 (m, 1H), 7.99 - 7.88 (m, 1H), 7.73 (d, 1H), 7.60 - 7.49 (m, 1H), 7.44 - 7.34 (m, 1H), 7.08 - 6.85 (m, 3H), 4.38 (s, 2H), 4.22 - 4.04 (m, 1H), 3.71 - 3.53 (m, 4H), 3.37 - 2.98 (m, 8H), 2.97 - 2.62 (m, 5H), 2.15 - 1.79 (m, 5H), 1.78 - 1.40 (m, 7H), 1.31 - 1.07 (m, 2H).

### Example 48: Preparation of compound 48

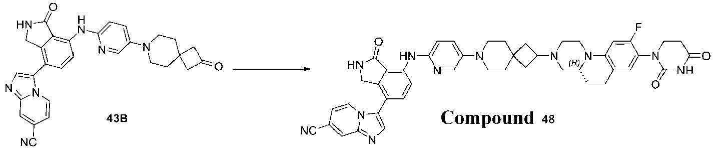

To a reaction flask were added **43B** (250 mg, 0.50 mmol), **6F** (159 mg, 0.50 mmol) and trichloromethane (30 mL), and 0.1 mL of acetic acid was added dropwise. The reaction solution was stirred at 70°C overnight, and refluxed at 80°C for 5 h, sodium triacetoxyborohydride (320 mg, 1.5 mmol) was added, and the mixture was reacted at 80°C for 3 h. Saturated sodium carbonate aqueous solution and dichloromethane were added, and the resulting mixture was stirred for layer separation. The organic layer was dried over anhydrous sodium sulphate, filtered, and concentrated under reduced pressure to dryness, the residue was purified by preparative HPLC (instrument: waters 2767 preparative liquid chromatographic instrument; chromatographic column: XBridge@ Prep C18 (30 mm×150 mm); composition of mobile phase: acetonitrile, 5 mmol/L ammonium bicarbonate aqueous solution), and the prepared solution was lyophilised to afford **compound 48** (50 mg, yield: 15%).

LCMS m/z = 806.5[M+H]⁺
¹H NMR (400 MHz, DMSO-*d₆*) δ 10.33 (s, 1H), 9.85 (s, 1H), 8.76 (s, 1H), 8.59 - 8.49 (m, 2H), 8.46 - 8.39 (m, 1H), 8.13 (s, 1H), 8.00 (d, 1H), 7.72 (d, 1H), 7.43 (dd, 1H), 7.20 (dd, 1H), 7.01 - 6.86 (m, 2H), 6.72 (d, 1H), 4.39 (s, 2H), 3.83 - 3.71 (m, 1H), 3.59 (t, 2H), 3.15 - 3.06 (m, 2H), 3.05 - 2.92 (m, 3H), 2.92 - 2.81 (m, 2H), 2.80 - 2.56 (m, 6H), 2.06 - 1.94 (m, 2H), 1.94 - 1.80 (m, 2H), 1.76 - 1.50 (m, 8H).

### Example 49: Preparation of compound 49

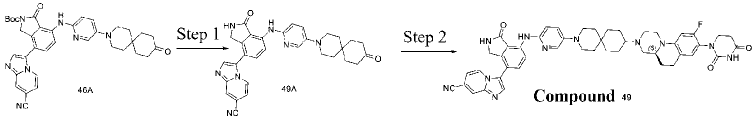

With reference to the synthetic method of step 5 to step 6 of example 44 and by **acidic prep-HPLC,** the trifluoroacetic acid salt of **compound 49** (60 mg) was obtained.

LCMS m/z =417.8[(M+2H)/2]⁺
¹H NMR (400 MHz, DMSO-*d₆*) δ 10.37 (s, 1H), 9.93 (s, 1H), 8.80 (s, 1H), 8.60 - 8.50 (m, 2H), 8.48 - 8.42 (m, 1H), 8.16 (s, 1H), 8.13 - 8.04 (m, 1H), 7.74 (d, 1H), 7.62 - 7.46 (m, 1H), 7.24 - 7.20 (m, 1H), 7.03 - 6.85 (m, 3H), 4.40 (s, 2H), 4.17 - 4.10 (m, 1H), 3.68 - 3.50 (m, 4H), 3.38 - 2.98 (m, 8H), 2.97 - 2.60 (m, 5H), 2.10 - 1.80 (m, 5H), 1.75 - 1.40 (m, 7H), 1.30 - 1.09 (m, 2H).

### Example 50: Preparation of compound 50

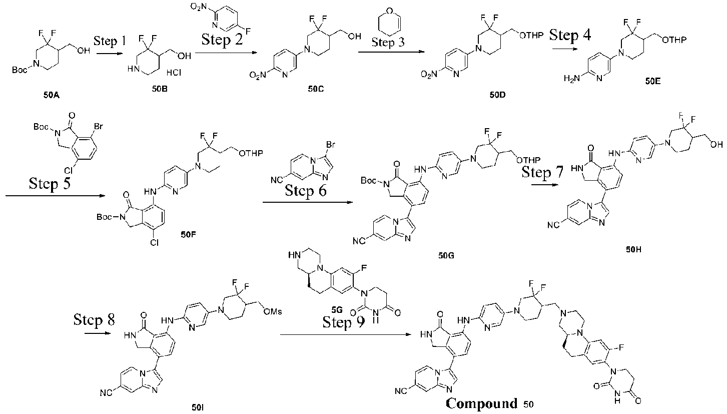

### Step 1: Preparation of 50B

**50A** (5.00 g, 19.90 mmol) was dissolved in hydrogen chloride 1,4-dioxane solution (50 mL, 4N), and the mixture was reacted at room temperature for 2 h. The reaction solution was directly concentrated under reduced pressure to afford **50B** (3.73 g).
LCMS m/z =152.2[M+H]⁺

### Step 2: Preparation of 50C

**50B** (3.73 g) and 5-fluoro-2-nitropyridine (2.82 g, 19.88 mmol) were dissolved in N,N-dimethylformamide (100 mL), sodium bicarbonate (5.01 g, 59.64 mmol) was added at room temperature, and the mixture was reacted at 100°C for 2 h. The reaction solution was cooled to room temperature, diluted with 500 mL of ethyl acetate, and washed 3 times with water and once with saturated sodium chloride aqueous solution, the organic phase was dried over anhydrous sodium sulphate and then concentrated under reduced pressure, and the residue was subjected to flash column chromatography (mobile phase: dichloromethane/methanol (v/v)=20/1) to afford **50C** (2.96 g, yield: 54%).
LCMS m/z =274.0[M+H]⁺

### Step 3: Preparation of 50D

**50C** (2.96 g, 10.83 mmol), 3,4-dihydro-2H-pyran (2.92 g, 34.66 mmol) and pyridinium 4-toluenesulphonate (0.33 g, 1.30 mmol) were added to a 500 mL reaction flask, and then dichloromethane (100 mL) was added, and the mixture was heated to 45°C and reacted for 1 h. The reaction solution was cooled to room temperature, and directly concentrated under reduced pressure, and the residue was subjected to flash column chromatography (mobile phase: petroleum ether/ethyl acetate (v/v)=1/1) to afford **50D** (3.85 g).
LCMS m/z =358.1[M+H]⁺

### Step 4: Preparation of 50E

**50D** (0.50 g, 1.40 mmol) was dissolved in tetrahydrofuran (20 mL), and then zinc powder (0.46 g, 7.00 mmol) and a solution of ammonium chloride (0.37 g, 7.00 mmol) in water (4 mL) were added, and the mixture was reacted at room temperature for 10 min. Dichloromethane was added to the reaction solution, and the resulting mixture was stirred. The organic phase was collected, dried over anhydrous sodium sulphate and then concentrated under reduced pressure, and the residue was subjected to flash column chromatography (mobile phase: dichloromethane/methanol (v/v)=15/1) to afford **50E** (0.43 g, yield: 94%).
LCMS m/z =328.2[M+H]⁺

### Step 5: Preparation of 50F

**50E** (0.43 g, 1.31 mmol) and tert-butyl 7-bromo-4-chloro-1-oxoisoindoline-2-carboxylate (0.45 g, 1.31 mmol) were dissolved in 1,4-dioxane solution (20 mL), and tris(dibenzylideneacetone)dipalladium (0.12 g, 0.13 mmol), Xant-phos (0.15 g, 0.26 mmol) and potassium carbonate (0.54 g, 3.93 mmol) were added. The mixture was reacted at 95°C under nitrogen for 3 h. The reaction solution was cooled to room temperature, diluted with 100 mL of dichloromethane, and subjected to suction filtration through celite, and the filter cake was washed 3 times with dichloromethane. The filtrates were combined, and concentrated under reduced pressure, and the residue was subjected to flash column chromatography (mobile phase: dichloromethane/methanol (v/v)=20/1) to afford **50F** (0.58 g, yield: 74%).
LCMS m/z =593.2[M+H]⁺

### Step 6: Preparation of 50G

**50F** (0.70 g, 1.18 mmol), tetrahydroxydiboron (0.19 g, 2.12 mmol), XPHOS-Pd-G2 (0.093 g, 0.12 mmol), X-PHOS (0.056 g, 0.12 mmol), ethylene glycol (0.22 g, 3.54 mmol) and potassium acetate (0.35 g, 3.54 mmol) were added to a reaction flask, anhydrous ethanol (20 mL) was added, and the mixture was reacted at 80°C under nitrogen for 1 h. The reaction system was cooled to room temperature, 5 mL of water, XPHOS-Pd-G2 (0.046 g, 0.06 mmol), X-PHOS (0.028 g, 0.06 mmol), potassium phosphate (0.75 g, 3.54 mmol) and 3-bromo-7-cyanoimidazolo[1,2-A]pyridine (0.26 g, 1.18 mmol) were added, and the mixture was reacted at 80°C under nitrogen for 2 h. The reaction solution was cooled to room temperature, and water was added to precipitate a yellow solid. The mixture was subjected to suction filtration to afford a solid, the solid was redissolved in dichloromethane, and then the mixture was dried over anhydrous sodium sulphate, and concentrated under reduced pressure. The residue was subjected to flash column chromatography (mobile phase: petroleum ether/ethyl acetate (v/v)=1/2) to afford **50G** (0.49 g, yield: 60%).
LCMS m/z =600.2[M-99]⁺

### Step 7: Preparation of 50H

**50G** (0.49 g, 0.71 mmol) was dissolved in dichloromethane (8 mL), and then trifluoroacetic acid (8 mL) was added, and the mixture was reacted at room temperature for 2 h. The reaction solution was directly concentrated under reduced pressure to remove the solvent, the residue was redissolved in dichloromethane/methanol ((v/v)=10/1), and resulting mixture was adjusted to be basic with potassium carbonate solid, subjected to suction filtration to afford the filtrate. The filtrate was concentrated under reduced pressure, and then the residue was subjected to flash column chromatography (mobile phase: dichloromethane/methanol (v/v)=15/1) to afford compound **50H** (0.23 g, yield: 62%).
LCMS m/z =516.2[M+H]⁺

### Step 8: Preparation of 501

**50H** (0.17 g, 0.33 mmol) was added to dichloromethane (20 mL), and then triethylamine (0.2 g, 0.27 mL, 1.98 mmol) was added. Methanesulphonic anhydride (0.345 g, 1.98 mmol) was added in three portions, and the mixture was reacted at room temperature for 1 h. The reaction solution was diluted with 20 mL of dichloromethane, and the organic phase was washed once with ammonium chloride aqueous solution and once with saturated sodium chloride aqueous solution, dried over anhydrous sodium sulphate, and concentrated under reduced pressure to afford **50I** (0.19 g).

### Step 9: Preparation of compound 50

**501** (0.19 g, 0.32 mmol) and **5G** (0.112 g, 0.35 mmol) were dissolved in N,N-dimethylformamide (3 mL), N,N-diisopropylethylamine (0.124 g, 0.16 mL, 0.96 mmol) was added at room temperature, and the mixture was heated to 80°C and reacted for 16 h. The reaction solution was cooled, 10 mL of ice water was added to precipitate a yellow solid, and the mixture was filtered. The filter cake was dried, and separated and purified by column chromatography (mobile phase: dichloromethane/methanol (v/v)=15/1) to afford **compound 50** (170 mg, yield: 65%).
LCMS m/z =408.8[(M+2H)/2]⁺

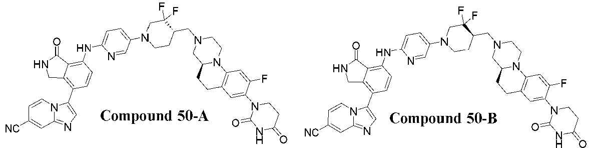

**Compound 50** was subjected to chiral separation and purification.

Preparation conditions: Instrument: CAS-05-Prep-HPLC-C; Column: IA column; Mobile phase: A for DCM; B for IPA+ACN; **Isocratic elution:** 70% B in A; Column temperature: room temperature; Wavelength: 254 nm.

Analysis conditions: **Instrument:** CAS-05-HPLC-AB(SHIMADZU LC-20AD); **Mobile phase:** IA-IPA+ACN(IPAm). Wavelength: 254 nm.

After preparative separation, the components with the same retention time were combined and concentrated under reduced pressure to afford compound **50-1** (67 mg) and compound **50-2**(66 mg).

**Among compound 50-1 and compound 50-2, one has the structure 50-A and the other has the structure 50-B.**

Retention time of **compound 50-1** under analysis conditions: 2.07 min, LCMS m/z =816.5[M+H]⁺
¹H NMR (400 MHz, DMSO-*d₆*) δ 10.32 (s, 1H), 9.79 (s, 1H), 8.75 (s, 1H), 8.57 - 8.37 (m, 3H), 8.12 (s, 1H), 7.81 - 7.65 (m, 2H), 7.19 (dd, 1H), 7.14 (dd, 1H), 7.01 (d, 1H), 6.93 (d, 1H), 6.73 (d, 1H), 4.38 (s, 2H), 3.93 - 3.74 (m, 3H), 3.73-3.63 (m, 1H), 3.59 (t, 2H), 3.19 - 3.08 (m, 1H), 3.07 - 2.90 (m, 3H), 2.87 - 2.70 (m, 3H), 2.70 - 2.56 (m, 3H), 2.48 - 2.37 (m, 2H), 2.15 - 2.03 (m, 1H), 1.97 - 1.77 (m, 3H), 1.69 - 1.58 (m, 2H).

Retention time of **compound 50-2** under analysis conditions: 2.94 min, LCMS m/z =816.5[M+H]⁺
¹H NMR (400 MHz, DMSO-*d₆*) δ 10.32 (s, 1H), 9.79 (s, 1H), 8.74 (s, 1H), 8.55 - 8.39 (m, 3H), 8.12 (s, 1H), 7.81 - 7.64 (m, 2H), 7.19 (dd, 1H), 7.14 (dd, 1H), 7.00 (d, 1H), 6.93 (d, 1H), 6.73 (d, 1H), 4.38 (s, 2H), 3.91 - 3.73 (m, 3H), 3.73-3.63 (m, 1H), 3.60 (t, 2H), 3.17 - 3.07 (m, 1H), 3.06 - 2.88 (m, 3H), 2.87 - 2.70 (m, 3H), 2.70 - 2.54 (m, 3H), 2.48 - 2.36 (m, 2H), 2.15 - 2.04 (m, 1H), 1.96 - 1.75 (m, 3H), 1.73 - 1.54 (m, 2H).

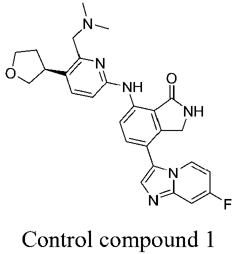

### Biological test examples

### Test example 1: Detection of SLP76 phosphorylation level in Jurkat cells

Jurkat cells from ATCC were placed in a RPMI-1640 complete medium (supplemented with 10% FBS and 1% penicillin-streptomycin solution) and cultured at 37°C and 5% CO₂. The cells in the logarithmic growth phase were collected, the cell density was adjusted to 5 × 10⁵ cells/well with the culture medium, and the cells were added to a 6-well cell culture plate at a volume of 1 mL/well. The compound to be tested was prepared to 3-fold the final concentration, 500 µL of the compounds at different concentrations were added to administration wells, a medium containing 0.3% DMSO was added to control wells, and the mixture was incubated at 37°C and 5% CO₂ for 4 hours. 500 µL of CD3 antibody (BD, Cat# 555329; final concentration: 1 µg/mL) was added, and the mixture was incubated at 37°C and 5% CO₂ for 10 minutes. After the incubation was completed, cells were collected in a 1.5 mL centrifuge tube and washed twice with pre-chilled PBS. Protease inhibitor cocktail, phosphatase inhibitor, and lysis buffer were prepared at a ratio of 1 : 1 : 100. 10 µL of lysis buffer was added to each sample to resuspend the cells, and the mixture was placed on ice and shaken repeatedly for 15 minutes until the cells were completely lysed. Then, the resulting mixture was centrifuged at 12000 rpm/min and 4°C for 15 minutes, the supernatant was collected, and the protein content was determined using the BCA method. The protein sample to be tested was diluted to 2 mg/mL to 0.8 mg/mL, and the levels of phosphorylated SLP76 (p-SLP76) and total SLP76 protein were detected using a fully automated protein expression quantitative analyser (ProteinSimple) (all antibodies were from CST). The software (Compass for SW) of the fully automated protein expression quantitative analyser was used to process the raw data and calculate the peak area A. The expression level R of p-SLP76 relative to total SLP76 protein was calculated as A_{[p-SLP76]}/A_{[SLP76]}. The inhibition rate of p-SLP76 was calculated as [1-(R_{administration well}- R_{negative control})/(R_{positive control}- R_{negative control})]× 100%, where R_{negative} control is the control well containing only 0.3% DMSO medium, and R_{positive control} is the control well with CD3 antibody. IC₅₀ values were calculated using a four-parameter non-linear fitting model in Graphpad 8.3.0 software.

Conclusion: The compounds of the present application, such as the example compounds, have a good inhibitory effect on the phosphorylation of SLP76 in Jurkat cells.

### Test example 2: Detection of HPK1 protein expression level in Jurkat cells

Jurkat cells from ATCC were placed in a RPMI-1640 complete medium (supplemented with 10% FBS and 1% penicillin-streptomycin solution) and cultured at 37°C and 5% CO₂. The cells in the logarithmic growth phase were collected, the cell density was adjusted to 5 × 10⁵ cells/well with the culture medium, and the cells were added to a 6-well cell culture plate at a volume of 1 mL/well. The compound to be tested was prepared to 2-fold the final concentration, 1 mL of the compounds at different concentrations were added to administration wells, a medium containing 0.2% DMSO was added to control wells, and the mixture was incubated at 37°C and 5% CO₂ for 48 hours. The cells were collected into a 1.5 mL centrifuge tube, 25 µL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 0.2 mg/mL, HPK1 was detected using a fully automated protein expression quantitative analyser (ProteinSimple), and the internal reference protein was β-actin (all antibodies were from CST). The software (Compass for SW) of the fully automated protein expression quantitative analyser was used to process the raw data and calculate the peak area and the HPK1 degradation rate relative to the control group. The inhibition rate of HPK1 was calculated as A_{administration well}/A_{control well}× 100%, where A_{administration well} is the relative peak area of the administration group, and A_{control well} is the relative peak area of the vehicle control group. DC₅₀ values were calculated using a four-parameter non-linear fitting model in Graphpad 8.3.0 software.

Conclusion: The compounds of the present application, such as the example compounds, have a degradation effect on HPK1 kinase.

### Test example 3: HPK1 kinase assay (Km concentration ATP)

The kinase HPK1 (Carna, Cat.No 07-410) was prepared into 2× kinase solution using 1× kinase buffer. The substrate Fluorescein-PKC (Invitrogen, Cat. No. PV3506) and ATP (working concentration: 9 µM) (Sigma, Cat. No. 2383-5G) were prepared into 2× substrate solution using 1×kinase buffer. The detection reagent was diluted to 2-fold the final concentration using an antibody diluent. 100 nL of compounds at different concentrations and 5 µL of kinase solution were added to each well of a 384-well plate, and the mixture was incubated at room temperature for 10 minutes. After the incubation was completed, 5 µL of substrate solution was added to each well, and the resulting mixture was reacted at room temperature for 90 minutes. After the reaction was completed, 10 µL of detection reagent was added to each well to terminate the reaction, and the mixture was incubated at room temperature for 60 minutes. Fluorescence readings were detected using an Envision instrument with excitation at 320 nm and emission at 520 nm and 495 nm. IC₅₀ value was calculated using XLFit excel add-in version 5.4.0.8 software. The calculation formula of the inhibition rate was shown in formula 3-1, where max is the Ratio of the DMSO control, min is the Ratio of the negative control without enzyme, and sample is the Ratio of the compound. $Ratio = {RFU}_{520 nm} / {RFU}_{495 nm}$ $Inhibition % = \left(max-sample\right) / \left(max-min\right) \times 100 %$

**Table 3-1 Inhibitory activity of test compounds against HPK1 kinase**

| Compound No. | IC₅₀ (µM) |
|---|---|
| Trifluoroacetic acid salt of compound 1 | A |
| Trifluoroacetic acid salt of compound 2 | A |
| Trifluoroacetic acid salt of compound 3 | A |
| Trifluoroacetic acid salt of compound 4 | A |
| Compound 5 | A |
| Trifluoroacetic acid salt of compound 6 | A |

| | |
|---|---|
| Note: In table 3-1, A < 0.01 µM | |

Conclusion: The compounds of the present invention, such as the example compounds, have an inhibitory effect on HPK1 kinase. Specifically, compounds 1, 2, 3, 4, 5 and 6 have good inhibitory activity against HPK1 kinase.

### Test example 4: Detection of HPK1 protein expression level in Jurkat cells (24 h)

Jurkat cells from ATCC were placed in a RPMI-1640 complete medium (supplemented with 10% FBS and 1% penicillin-streptomycin solution) and cultured at 37°C and 5% CO₂. The cells in the logarithmic growth phase were collected, the cell density was adjusted to 5 × 10⁵ cells/well with the culture medium, and the cells were added to a 6-well cell culture plate at a volume of 1 mL/well. The compound to be tested was prepared to 2-fold the final concentration, 1 mL of the compounds at different concentrations were added to administration wells, a medium containing 0.2% DMSO was added to control wells, and the plate was incubated at 37°C and 5% CO₂ for 24 hours. The cells were collected into a 1.5 mL centrifuge tube, 30 µL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 0.2 mg/mL, HPK1 was detected using a fully automated protein expression quantitative analyser (ProteinSimple), and the internal reference protein was β-actin (all antibodies were from CST). The software (Compass for SW) of the fully automated protein expression quantitative analyser was used to process the raw data and calculate the peak area after internal reference normalisation and the HPK1 degradation rate relative to the control group. The HPK1 degradation rate was calculated according to formula 4-1, where A_{administration well} is the relative peak area of the administration group after internal reference normalisation, and A_{control well} is the relative peak area of the vehicle control group after internal reference normalisation. DC₅₀ values were calculated using a four-parameter non-linear fitting model in Graphpad 8.3.0 software. ${HPK 1}_{degradation} % = 100 - \left({A}_{administration well}/{A}_{control well}\times 100%\right)$

**Table 4-1 Degradation activity of test compounds against HPK1 protein in Jurkat cells (24 h)**

| Compound No. | DC₅₀ (nM) | Compound No. | DC₅₀ (nM) |
|---|---|---|---|
| Trifluoroacetic acid salt of compound 1 | A | Compound 16 | A |
| Trifluoroacetic acid salt of compound 2 | A | Trifluoroacetic acid salt of compound 19 | A |
| Trifluoroacetic acid salt of | A | Trifluoroacetic acid | A |
| compound 3 | | salt of compound 20 | |
| Trifluoroacetic acid salt of compound 4 | A | Trifluoroacetic acid salt of compound 31 | A |
| Compound 5 | A | Trifluoroacetic acid salt of compound 32 | A |
| Trifluoroacetic acid salt of compound 6 | A | Trifluoroacetic acid salt of compound 34 | A |
| Trifluoroacetic acid salt of compound 7 | A | Trifluoroacetic acid salt of compound 35 | A |
| Trifluoroacetic acid salt of compound 8 | A | Trifluoroacetic acid salt of compound 36 | A |
| Trifluoroacetic acid salt of compound 9 | A | Trifluoroacetic acid salt of compound 37 | A |
| Trifluoroacetic acid salt of compound 13 | A | Trifluoroacetic acid salt of compound 39 | A |
| Trifluoroacetic acid salt of compound 14 | A | Trifluoroacetic acid salt of compound 42 | A |
| Trifluoroacetic acid salt of compound 15 | A | | |

| | | | |
|---|---|---|---|
| Note: In table 4-1, A < 20 nM | | | |

Conclusion: The compounds of the present invention, such as the example compounds, have good degradation activity against HPK1 protein in Jurkat cells.

### Test example 5: HPK1 kinase assay (1 mM ATP)

The kinase HPK1 (Carna, Cat.No 07-410) was prepared into 2× kinase solution using 1× kinase buffer. The substrate Fluorescein-PKC (Invitrogen, Cat. No. PV3506) and ATP (working concentration: 1 mM) (Sigma, Cat. No. 2383-5G) were prepared into 2× substrate solution using 1×kinase buffer. The detection reagent was diluted to 2-fold the final concentration using an antibody diluent. 100 nL of compounds at different concentrations and 5 µL of kinase solution were added to each well of a 384-well plate, and the mixture was incubated at room temperature for 10 minutes. After the incubation was completed, 5 µL of substrate solution was added to each well, and the resulting mixture was reacted at room temperature for 90 minutes. After the reaction was completed, 10 µL of detection reagent was added to each well to terminate the reaction, and the mixture was incubated at room temperature for 60 minutes. Fluorescence readings were detected using an Envision instrument with excitation at 320 nm and emission at 520 nm and 495 nm. IC₅₀ value was calculated using XLFit excel add-in version 5.4.0.8 software. The calculation formula of the inhibition rate was shown in formula 5-1, wherein max is the Ratio of the DMSO control, min is the Ratio of the negative control without enzyme, and sample is the Ratio of the compound. $Ratio = {RFU}_{520 nm} / {RFU}_{495 nm}$ $Inhibition % = \left(max-sample\right) / \left(max-min\right) \times 100 %$

**Table 5-1 Inhibitory activity of test compounds against HPK1 kinase**

| Compound No. | IC₅₀ (nM) | Compound No. | IC₅₀ (nM) |
|---|---|---|---|
| Compound 1-1 | A | Trifluoroacetic acid salt of compound 25 | A |
| Compound 1-2 | A | Trifluoroacetic acid salt of compound 28 | A |
| Trifluoroacetic acid salt of compound 7 | A | Trifluoroacetic acid salt of compound 29 | A |
| Trifluoroacetic acid salt of compound 8 | A | Trifluoroacetic acid salt of compound 30 | A |
| Trifluoroacetic acid salt of compound 9 | A | Trifluoroacetic acid salt of compound 31 | A |
| Trifluoroacetic acid salt of compound 10 | A | Trifluoroacetic acid salt of compound 32 | A |
| Trifluoroacetic acid salt of compound 11 | A | Trifluoroacetic acid salt of compound 33 | A |
| Trifluoroacetic acid salt of compound 12 | A | Trifluoroacetic acid salt of compound 34 | A |
| Trifluoroacetic acid salt of compound 13 | A | Trifluoroacetic acid salt of compound 35 | A |
| Trifluoroacetic acid salt of compound 14 | A | Trifluoroacetic acid salt of compound 36 | A |
| Trifluoroacetic acid salt of compound 15 | A | Trifluoroacetic acid salt of compound 37 | A |
| Compound 16 | A | Compound 38 | A |
| Trifluoroacetic acid salt of compound 17 | A | Trifluoroacetic acid salt of compound 39 | A |
| Trifluoroacetic acid salt of compound 18 | A | Trifluoroacetic acid salt of compound 42 | A |
| Trifluoroacetic acid salt of compound 19 | A | Trifluoroacetic acid salt of compound 43 | A |
| Trifluoroacetic acid salt of compound 20 | A | Trifluoroacetic acid salt of compound 44 | A |
| Trifluoroacetic acid salt of compound 21 | A | Trifluoroacetic acid salt of compound 45 | A |
| Trifluoroacetic acid salt of compound 22 | A | Trifluoroacetic acid salt of compound 46 | A |
| Trifluoroacetic acid salt of compound 23 | A | Trifluoroacetic acid salt of compound 47 | A |
| Trifluoroacetic acid salt of compound 24 | A | Compound 48 | A |

| | | | |
|---|---|---|---|
| Note: In table 5-1, A<50 nM | | | |

Conclusion: The compounds of the present invention, such as the example compounds, have good inhibitory activity against HPK1 kinase.

### Test example 6: Pharmacokinetic test in mice

**Experimental objective:** In this experiment, a single dose of test substances was administered to ICR mice intravenously and intragastrically, the concentrations of the test compounds in plasma of mice were measured, and the pharmacokinetic characteristics and bioavailability of the test compounds in mice were evaluated.

**Experimental animals:** Male ICR mice, 20-35 g, purchased from Chengdu Dossy Experimental Animals Co., Ltd. (SCXK (CHUAN) 2020-030).

**Experimental method:** On the day of the experiment, the ICR mice were randomly grouped according to their body weights. The animals were fasted but with water available overnight before the administration, and fed 4 h after the administration.

| Group | Administration information | | | | | | |
|---|---|---|---|---|---|---|---|
| | Test substance | Administra tion dosage * (mg/kg) | Administr ation concentrat ion (mg/mL) | Administrat ion volume (mL/kg) | Collected sample | Mode of administration | Vehicle |
| G1 | | 2.5 | 0.5 | 5 | Plasma | Intravenous administration | 10% DMA+10% Solutol+80% (saline) or 5% DMA + 5% Solutol + 90% Saline or 5% DMA+5% HS-15+90% NS |
| G1-2 | Compound of the present invention | 1 | 0.2 | 5 | | | |
| G2 | | 10 | 1 | 10 | Plasma | Oral administration (intragastric administration) | 5% Solutol+5% TPGS+30% PEG400+60% (20% SBE-b-CD) |
| G2-2 | | 5 | 0.5 | 10 | | | |

| | | | | | | | |
|---|---|---|---|---|---|---|---|
| ^{*}Dosage is calculated based on free base. | | | | | | | |

**Sampling:** Blood was taken from the orbits of the mice at specified time points, and placed in an EDTAK₂ centrifuge tube. Centrifugation was performed at 5000 rpm at 4°C for 10 min, and the plasma was collected.

Time points for plasma collection in G1 and G1-2 groups: 0, 5 min, 15 min, 30 min, 1 h, 2 h, 4 h, 7 h, and 24 h.

Time points for plasma collection in G2 and G2-2 groups: 0, 5 min, 15 min, 30 min, 1 h, 2 h, 4 h, 7 h, and 24 h.

Before analysis and detection, all the samples were stored at -60°C or lower. The samples were analysed quantitatively by LC-MS/MS.

**Table 6-1 PK data of test compound drug in mice**

| Compound No. | Mode of administration: i. g. | Cmax (ng/mL) | AUC (h·ng·mL⁻¹) | T_{1/2} (h) |
|---|---|---|---|---|
| Trifluoroacetic acid salt of compound 1 | 10 mg/kg | 1071 | 14726 | 6.73 |
| Trifluoroacetic acid salt of compound 2 | 10 mg/kg | 1279 | 16792 | 5.79 |
| Trifluoroacetic acid salt of compound 4 | 10 mg/kg | 1139 | 15203 | 7.27 |
| Compound 5 | 10 mg/kg | 1378 | 19970 | 6.45 |
| Trifluoroacetic acid salt of compound 6 | 10 mg/kg | 1519 | 20763 | 5.35 |
| Trifluoroacetic acid salt of compound 8 | 10 mg/kg | 1461 | 18257 | 4.38 |
| Trifluoroacetic acid salt of compound 15 | 10 mg/kg | 1616 | 23618 | 7.64 |
| Trifluoroacetic acid salt of compound 19 | 10 mg/kg | 3699 | 50475 | 6.68 |
| Trifluoroacetic acid salt of compound 20 | 10 mg/kg | 1010 | 12568 | 5.51 |
| Trifluoroacetic acid salt of compound 31 | 5 mg/kg | 553 | 6496 | 3.03 |
| Trifluoroacetic acid salt of compound 32 | 10 mg/kg | 1249 | 15802 | 4.37 |
| Trifluoroacetic acid salt of compound 35 | 10 mg/kg | 2648 | 37077 | 12.1 |
| Trifluoroacetic acid salt of compound 39 | 10 mg/kg | 2072 | 25648 | 3.75 |
| Compound 48 | 10 mg/kg | 2295 | 25894 | 4.90 |
| Control compound 1 | 10 mg/kg | 93.8 | 248 | 2.05 |

| | | | | |
|---|---|---|---|---|
| * Note: i. g. (intragastrically) administration of compounds. | | | | |

**Table 6-2 PK data of test compound drug in mice**

| Compound No. | Mode of administration: IV | T_{1/2} (h) | Clearance (mL·kg⁻¹·min⁻¹) |
|---|---|---|---|
| Compound 48 | 1 mg/kg | 5.30 | 1.67 |
| Control compound 1 | 2.5 mg/kg | 0.890 | 169 |

| | | | |
|---|---|---|---|
| * Note: IV (intravenous) administration of compounds. | | | |

Conclusion: The compounds synthesised using the technique of the present invention, such as the example compounds, have favourable pharmacokinetic properties (such as, better oral absorption, longer half-life, higher Cmax, and lower clearance) in mice, which are better than those of control compound 1.

### Test example 7: Pharmacokinetic test in rats

**Experimental animals:** Male SD rats, 180-200 g, purchased from Chengdu Dossy Experimental Animals Co., Ltd. with the laboratory animal production license number of SCXK (CHUAN) 2020-030.

**Experimental design:** on the day of the experiment, the SD rats were randomly grouped according to their body weights. The animals were fasted but with water available overnight before the administration, and fed 4 h after the administration.

| Group | Administration information | | | | | |
|---|---|---|---|---|---|---|
| | Test compound | Administration dosage (mg/kg) | Administration concentration (mg/mL) | Administration volume (mL/kg) | Collected sample | Mode of administration |
| G1 | Compound of the present invention | 2.5 | 0.5 | 5 | Plasma | Intravenous administration |
| G1-2 | | 1 | 0.2 | 5 | | |
| G2 | | 10 | 1 | 10 | Plasma | Intragastric administration |
| G2-2 | | 5 | 0.5 | 10 | | |

| | | | | | | |
|---|---|---|---|---|---|---|
| Note: Vehicle for intravenous administration: 5% DMA+5% Solutol+90% Saline or 10% DMA+10% Solutol+80% Saline; | | | | | | |

Vehicle for intragastric administration: 5% Solutol+5% TPGS+30% PEG400+60% (20% SBE-β-CD)

Blood was taken from the orbits of the mice at specified time points, and placed in an EDTAK₂ centrifuge tube. Centrifugation was performed at 5000 rpm for 10 min, and the plasma was collected.

Blood sampling time points for the intravenous administration group were: 0, 5 min, 15 min, 30 min, 1 h, 2 h, 4 h, 6 h, 8 h and 24 h; blood sampling time points for the intragastric administration group were: 0, 5 min, 15 min, 30 min, 1 h, 2 h, 4 h, 6 h, 8 h and 24 h. Before analysis and detection, all the samples were stored at - 60°C or lower. The samples were analysed quantitatively by LC-MS/MS.

**Table 7-1 PK data of test compound drug in rats**

| Compound No. | Mode of administration: i. g. | Cmax (ng/mL) | AUC (h·ng·mL⁻¹) | T_{1/2} (h) |
|---|---|---|---|---|
| Trifluoroacetic acid | 10 mg/kg | 721 | 8356 | 6.40 |
| salt of compound 6 | | | | |
| Trifluoroacetic acid salt of compound 19 | 10 mg/kg | 465 | 5859 | 7.17 |
| Trifluoroacetic acid salt of compound 20 | 10 mg/kg | 382 | 5307 | 5.29 |
| Trifluoroacetic acid salt of compound 31 | 5 mg/kg | 464 | 4391 | 4.48 |
| Trifluoroacetic acid salt of compound 39 | 10 mg/kg | 777 | 7263 | 4.55 |
| Control compound 1 | 10 mg/kg | 14.6 | 61.1 | 1.71 |

| | | | | |
|---|---|---|---|---|
| * Note: i. g. (intragastrically) administration of compounds. | | | | |

**Table 7-2 PK data of test compound drug in rats**

| Compound No. | Mode of administration: IV | T_{1/2} (h) | Clearance (mL·kg⁻¹·min⁻¹) |
|---|---|---|---|
| Trifluoroacetic acid salt of compound 6 | 2.5 mg/kg | 6.58 | 9.78 |
| Trifluoroacetic acid salt of compound 19 | 2.5 mg/kg | 7.66 | 11.0 |
| Trifluoroacetic acid salt of compound 20 | 2.5 mg/kg | 4.78 | 13.4 |
| Trifluoroacetic acid salt of compound 31 | 1 mg/kg | 4.88 | 5.38 |
| Trifluoroacetic acid salt of compound 39 | 2.5 mg/kg | 4.21 | 6.58 |
| Control compound 1 | 2.5 mg/kg | 0.986 | 233 |

| | | | |
|---|---|---|---|
| * Note: IV (intravenous) administration of compounds. | | | |

Conclusion: The compounds synthesised using the technique of the present invention, such as the example compounds, have favourable pharmacokinetic properties (such as, better oral absorption, longer half-life, higher Cmax, and lower clearance) in rats, which are better than those of control compound 1.

### 8. Test for hERG potassium ion channel

**Experimental platform:** Electrophysiological manual patch-clamp system

**Cell line:** Chinese hamster ovary (CHO) cell lines stably expressing hERG potassium ion channel

**Experimental method:** In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current (I _{hERG}) was depolarised from -80 mV to +20 mV for 2 s, then repolarised to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula: $Inhibition % = \left[1-\left(I/Io\right)\right] \times 100 %$ where Inhibition% represents the percentage of inhibition of hERG potassium current by the compound, and *I* and *I*o represent the amplitude of hERG potassium current after and before the administration, respectively.

Compound IC₅₀ was calculated using GraphPad Prism 5 software by fitting according to the following equation: $Y = Bottom + \left(Top-Bottom\right) / \left(1+{10}^{∧}\left(\left({LogIC}_{50}-X\right)*HillSlope\right)\right)$ where X represents the Log value of the tested concentration of the test sample, Y represents the inhibition percentage at the corresponding concentration, and Bottom and Top represent the minimum and maximum inhibition percentage, respectively.

Conclusion: The compounds of the present invention, such as the example compounds, have no obvious inhibitory effect on the hERG potassium ion channel.

### 9. IL-2 detection assay in cells

Activating effect of compounds on IL-2 secretion by T cells was evaluated using the ELISA method. Frozen PBMCs were thawed, and pan-T cells were isolated and purified using a T cell sorting kit (Stemcell, 17951). The cells were resuspended in 1640 complete medium and incubated in a 37°C&5%CO₂ incubator overnight. A 96-well plate coated with an anti-human CD3 antibody (Thermo, 16-0037-38) at a final concentration of 5 µg/mL was incubated at 37°C for 2 h. The overnight-cultured cells were collected by centrifugation, the supernatant was discarded, and the cells were resuspended in 1640 complete medium, and seeded in the CD3 antibody-coated 96-well plate at a density of 10⁵cells/well. The compounds at different concentrations were added, and the mixture was incubated in a 37°C&5%CO₂ incubator for 24 hours. After the incubation was completed, the resulting mixture was centrifuged at 1000 rpm for 1 minute to collect the supernatant, and IL-2 was detected according to the ELISA instructions. DMSO without compounds was used as the control group to calculate the activation rate of the compounds, and prism was used to fit the activation curve. Maximum activation fold = maximum activation rate/control group.

**Table 9-1 Results of IL-2 activation by test compounds**

| Compound No. | Maximum activation fold | Activation fold at 100 nM concentration |
|---|---|---|
| Compound 1-1 | -- | >10 |
| Trifluoroacetic acid salt of compound 6 | >10 | >10 |
| Trifluoroacetic acid salt of compound 7 | >10 | >10 |
| Trifluoroacetic acid salt of compound 19 | >10 | >10 |
| Trifluoroacetic acid salt of compound 31 | >10 | >10 |
| Trifluoroacetic acid salt of compound 32 | >10 | -- |
| Trifluoroacetic acid salt of compound 35 | >10 | -- |
| Trifluoroacetic acid salt of compound 39 | >10 | >10 |
| Control compound 1 | <10 | <10 |

Conclusion: Compared with the control compound, the compounds of the present invention, such as the example compounds, have a better activating effect on IL-2.

### 10. Pharmacokinetic test in monkeys

**Experimental animals:** Male cynomolgus monkeys, 3-5 kg, 4-6 monkeys/compound.

**Experimental method:** On the day of the test, 4-6 monkeys/compound were randomly grouped according to their body weights. The animals were fasted but with water available for 14 to 18 h one day before administration, and fed 4 h after administration.

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 2 | | 1 | 1 | 1 | Plasma | Intravenous administration |
| G2 | 2 | | 10 | 2 | 5 | Plasma | Intragastric administration |
| G3 | 3 | Compound | 1 | 1 | 1 | Plasma | Intravenous administration |
| G4 | 3 | | 10 | 2 | 5 | Plasma | Intragastric administration |
| G5 | 3 | | 5 | 1 | 5 | Plasma | Intragastric administration |

| | | | | | | | |
|---|---|---|---|---|---|---|---|
| Note: Solvent for intravenous administration: 5% DMA + 5% Solutol + 90% Saline; Vehicle for intragastric administration: 5% DMSO+5% Solutol+30% PEG400+60% (20% SBE-CD). (DMA: dimethylacetamide; Solutol: polyethylene glycol-15-hydroxystearate; Saline: physiological saline .) | | | | | | | |

Before and after the administration, 1.0 mL of blood was taken from the limb veins and placed in an EDTAK2 centrifuge tube. Centrifugation was performed at 5000 rpm at 4°C for 10 min, and the plasma was collected. Blood sampling time points for the intravenous group and intragastric administration group were: 0, 5 min, 15 min, 30 min, 1 h, 2 h, 4 h, 6 h, 8 h, 10 h, 12 h, and 24 h. Before analysis and detection, all samples were stored at -60°C or lower, and the samples were quantitatively analysed by LC-MS/MS.

Conclusion: The compounds of the present invention, such as the example compounds, have good pharmacokinetic properties in monkeys.

### 11. Pharmacokinetic test in beagle dogs

**Experimental animals:** Male beagle dogs, about 8-10 kg.

**Experimental method:** On the day of the experiment, the beagle dogs were randomly grouped according to their body weights. The animals were fasted but with water available for 14 to 18 h one day before administration, and fed 4 h after administration.

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | | 1 | 1 | 1 | Plasma | Intravenous administration |
| G2 | 3 | Test compound | 5 | 1 | 5 | Plasma | Intragastric administration |
| G3 | 3 | | 3 | 0.6 | 5 | Plasma | Intragastric administration |

| | | | | | | | |
|---|---|---|---|---|---|---|---|
| Note: Solvent for intravenous administration: 5% DMA + 5% Solutol + 90% Saline; Vehicle for intragastric administration: 5% Solutol+5% TPGS+30% PEG400+60% (20% SBE-b-CD). | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and the plasma was collected. Blood sampling time points for groups G1, G2 and G3 (the intravenous group and intragastric administration group) were: 0, 5 min, 15 min, 30 min, 1 h, 2 h, 4 h, 6 h, 8 h, 10 h, 12 h, 24 h, and 48 h. Before analysis and detection, all samples were stored at -60°C or lower, and the samples were quantitatively analysed by LC-MS/MS.

**Conclusion:** The compounds of the present invention, such as the example compounds, have good pharmacokinetic properties in beagle dogs.

## Claims

1. A compound or a stereoisomer, a racemate, a tautomer, a deuterated substance, a solvate, a prodrug, a metabolite, a pharmaceutically acceptable salt or a cocrystal thereof, **characterised in that** the compound is a compound as shown in general formula (I),
B-L-K (I);
L is selected from a bond or -C₁₋₅₀ hydrocarbyl-, wherein the hydrocarbyl has 1 to 20 methylene units optionally replaced by -Ak- or -Cy-;
each -Ak- is independently selected from -(CH₂)_{q}-, -(CH₂)_{q}-O-, -O-(CH₂)_{q}-, - (CH₂)_{q}-S-, -S-(CH₂)_{q}-, -(CH₂)_{q}-NR^{L}-, -NR^{L}-(CH₂)_{q}-, -(CH₂)_{q}-NR^{L}C(=O)-, - NR^{L}(CH₂)_{q}C(=O)-, -(CH₂)_{q}-C(=O)NR^{L}-, -C(=O)-, -C(=O)-(CH₂)_{q}-NR^{L}-, -(C≡C)_{q}-, -CH=CH-, -Si(R^{L})₂-, -Si(OH)(R^{L})-, -Si(OH)₂-, -P(=O)(OR^{L})-, -P(=O)(R^{L})-, -S-, - S(=O)-, -S(=O)₂- or a bond, wherein the CH and -CH₂- are optionally substituted with 1 to 2 R^{z};
each q is independently selected from 0, 1, 2, 3, 4, 5 or 6;
R^{L} is selected from H, C₁₋₄ alkyl, C₃₋₇ carbocyclyl, or 4- to 10-membered heterocyclyl, wherein the alkyl, carbocyclyl or heterocyclyl is optionally substituted with 1 to 4 R^{z};
each -Cy- is independently selected from a bond or one of the following groups optionally substituted with 1 to 4 R^{L2}: 4- to 8-membered mono-heterocyclyl, 4- to 12-membered fused-heterocyclyl, 5- to 13-membered spiro-heterocyclyl, 7- to 12-membered bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl;
B is 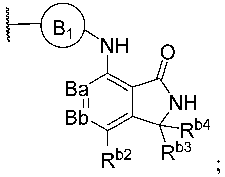
Ba or Bb is selected from N or CR^{b};
R^{b} is selected from H, deuterium, halogen, OH, CN, NH₂, NHC₁₋₄ alkyl, N(C₁₋₄ alkyl)₂, C₁₋₄ alkyl, C₂₋₄ alkenyl, C₂₋₄ alkynyl, C₁₋₄ alkoxy, or -C₀₋₂ alkylene-C₃₋₆ cycloalkyl, wherein the alkyl, alkylene, alkenyl, alkynyl, alkoxy or cycloalkyl is optionally substituted with 1 to 4 R^{z};
B₁ is selected from C₆₋₁₀ carbocyclyl or 5- to 10-membered heterocyclyl, wherein the B₁ is optionally substituted with 1 to 4 R^{b1};
R^{b2} is selected from C₆₋₁₀ carbocyclyl or 5- to 10-membered heterocyclyl, wherein the R^{b2} is optionally substituted with 1 to 4 R^{b2a};
R^{b3} and R^{b4} are each independently selected from H, deuterium, halogen, or C₁₋₄ alkyl, wherein the alkyl is optionally substituted with 1 to 4 halogen;
R^{b1} and R^{b2a} are each independently selected from deuterium, halogen, OH, CN, NH₂, C₁₋₄ alkyl, C₂₋₄ alkenyl, C₂₋₄ alkynyl, C₁₋₄ alkoxy, -C₀₋₂ alkylene-C₃₋₆ cycloalkyl, -C₀₋₂ alkylene-O-C₃₋₆ cycloalkyl, -C₀₋₂ alkylene-4- to 7-membered heterocycloalkyl, -C₀₋₂ alkylene-O-4- to 7-membered heterocycloalkyl, -C₀₋₂ alkylene-NHC₁₋₄ alkyl, or -C₀₋₂ alkylene-N(C₁₋₄ alkyl)₂, wherein the alkyl, alkylene, alkenyl, alkynyl, alkoxy, cycloalkyl or heterocycloalkyl is optionally substituted with 1 to 4 R^{z};
K is 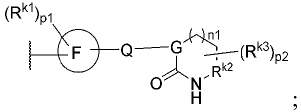 G is selected from N or CH;
each Q is independently selected from a bond, -O-, -S-, -CH₂-, -NR^{q}-, - C(=O)-, -NR^{q}C(=O)-, or -C(=O)NR^{q}-;
a nitrogen-nitrogen bond, a nitrogen-oxygen bond, or a nitrogen-sulphur bond cannot be directly formed between Q and G;
R^{q} is selected from H or C₁₋₄ alkyl;
F is selected from C₃₋₂₀ carbocyclyl, C₆₋₂₀ aryl, 3- to 20-membered heterocyclyl or 5- to 20-membered heteroaryl;
each R^{k1} is independently selected from H, deuterium, halogen, OH, =O, NH₂, CN, COOH, CONH₂, C₁₋₄ alkyl, C₂₋₄ alkenyl, C₂₋₄ alkynyl, C₁₋₄ alkoxy, C₃₋₆ cycloalkyl, or 3- to 6-membered heterocycloalkyl, wherein the alkyl, alkenyl, alkynyl, alkoxy, cycloalkyl or heterocycloalkyl is optionally substituted with 1 to 4 R^{z};
each R^{k2} is independently selected from a bond, -C(=O)-, -S(=O)₂-, -S(=O)- or -C(R^{k3})₂-;
each R^{k3} is independently selected from H, deuterium, halogen, OH, =O, NH₂, CN, COOH, CONH₂, C₁₋₄ alkyl, C₁₋₄ alkoxy, C₃₋₈ cycloalkyl, or 3- to 8-membered heterocyclyl, wherein the alkyl, alkoxy, cycloalkyl or heterocyclyl is optionally substituted with 1 to 4 R^{z};
alternatively, two R^{k3} are directly connected to form C₃₋₈ carbocyclyl or 4- to 8-membered heterocyclyl, wherein the carbocyclyl or heterocyclyl is optionally substituted with 1 to 4 R^{z};
R^{L2} and R^{z} are each independently selected from deuterium, halogen, OH, =O, CF₃, SF₅, CN, NH₂, NO₂, COOH, CONH₂, NHC₁₋₄ alkyl, N(C₁₋₄ alkyl)₂, COOH, CONH₂, C₁₋₄ alkyl, C₂₋₄ alkenyl, C₂₋₄ alkynyl, C₁₋₄ alkoxy, -S-C₁₋₄ alkyl, or -C₀₋₄ alkylene-C₃₋₆ cycloalkyl, wherein the alkyl, alkylene, alkoxy, alkenyl, alkynyl, or cycloalkyl is optionally substituted with 1 to 4 substituents selected from deuterium, F, Cl, Br, I, OH, CN, C₁₋₄ alkyl, or C₁₋₄ alkoxy;
n1 is selected from 0, 1, 2 or 3;
each p1 or p2 is independently selected from 0, 1, 2, 3, 4 or 5.

2. The compound or the stereoisomer, the racemate, the tautomer, the deuterated substance, the solvate, the prodrug, the metabolite, the pharmaceutically acceptable salt or the cocrystal thereof according to claim 1, **characterised in that**
L is -Ak1-Cy1-Ak2-Cy2-Ak3-Cy3-Ak4-Cy4-Ak5-;
Ak1, Ak2, Ak3, Ak4 and Ak5 are each independently selected from -(CH₂)_{q}-, -(CH₂)_{q}-O-, -O-(CH₂)_{q}-, -(CH₂)_{q}-S-, -S-(CH₂)_{q}-, -(CH₂)_{q}-NR^{L}-, -NR^{L}-(CH₂)_{q}-, - (CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 R^{z};
each R^{L} is independently selected from H or C₁₋₄ alkyl;
each Cy1, Cy2, Cy3 or Cy4 is independently selected from a bond or one of the following groups optionally substituted with 1 to 4 R^{L2}: 4- to 7-membered nitrogen-containing mono-heterocyclyl, 4- to 12-membered nitrogen-containing fused-heterocyclyl, 5- to 13-membered nitrogen-containing spiro-heterocyclyl, 7-to 12-membered nitrogen-containing bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl;
F is selected from a C₃₋₇ monocyclic group, a C₄₋₁₀ fused ring group, a C₅₋₁₂ spiro ring group, a C₅₋₁₀ bridged ring group, 4- to 7-membered mono-heterocyclyl, 4- to 10-membered bicyclic fused-heterocyclyl, 8- to 15-membered tricyclic fused-heterocyclyl, 12- to 19-membered tetracyclic fused-heterocyclyl, 5- to 17-membered spiro-heterocyclyl, 5- to 10-membered bridged-heterocyclyl, C₆₋₁₄ aryl, or 5- to 10-membered heteroaryl;
B₁ is selected from phenyl, benzo C₄₋₆ carbocyclyl, benzo 4- to 6-membered heterocyclyl, 5- to 6-membered heteroaryl, or 8- to 10-membered heteroaryl, wherein the B₁ is optionally substituted with 1 to 4 R^{b1};
R^{b2} is selected from phenyl, benzo C₄₋₆ carbocyclyl, benzo 4- to 6-membered heterocyclyl, 5- to 6-membered heteroaryl, or 8- to 10-membered heteroaryl, wherein the R^{b2} is optionally substituted with 1 to 4 R^{b2a}.

3. The compound or the stereoisomer, the racemate, the tautomer, the deuterated substance, the solvate, the prodrug, the metabolite, the pharmaceutically acceptable salt or the cocrystal thereof according to claim 2, **characterised in that**
R^{L} is selected from H, methyl or ethyl;
Cy1, Cy2, Cy3 and Cy4 are each independently selected from a bond or one of the following groups optionally substituted with 1 to 4 R^{L2}: phenyl, pyridyl, pyrimidyl, pyrazinyl, pyridazinyl, pyrazolyl, thiazolyl, oxazolyl, triazolyl, 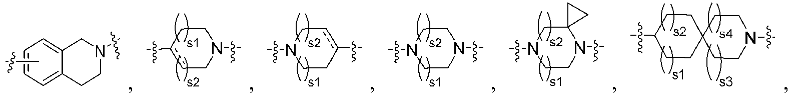 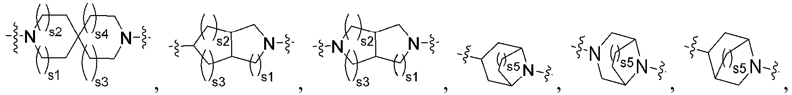 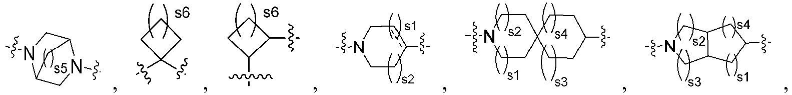 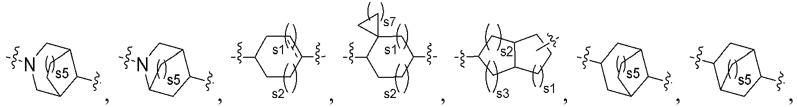 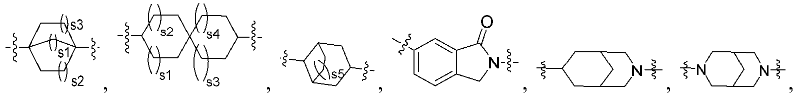 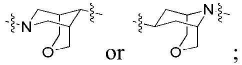
s1, s3 and s5 are each independently selected from 0, 1 or 2;
s2 and s4 are each independently selected from 0 or 1;
s6 is selected from 0, 1, 2 or 3;
s7 is selected from 1, 2 or 3; 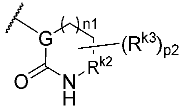 is selected from 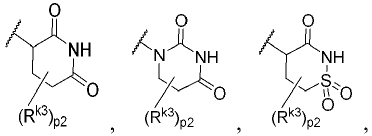 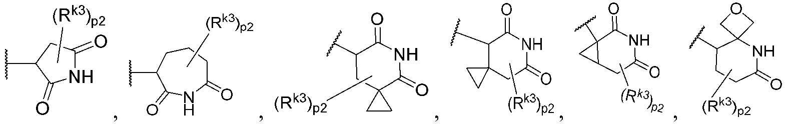 or 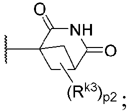
F is selected from cyclobutyl, cyclopentyl, cyclohexyl, bicyclo[1.1.1]pentanyl, 6,7-dihydro-5H-cyclopenta[c]pyridyl, 2,3-dihydro-1H-indenyl, phenyl, naphthyl, anthracenyl, phenanthryl, azetidinyl, pyrrolidyl, piperidyl, morpholinyl, pyridyl, pyrimidyl, pyridazinyl, pyrazinyl, triazinyl, pyrrolyl, pyrazolyl, imidazolyl, triazolyl, oxazolyl, furyl, thienyl, thiazolyl, 2-pyridonyl, 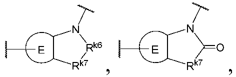 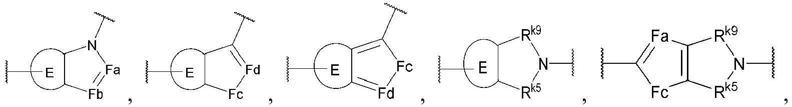 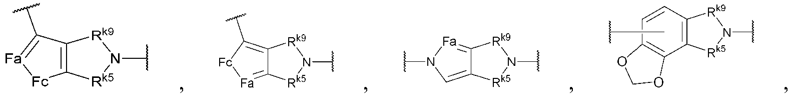 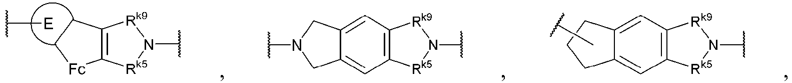 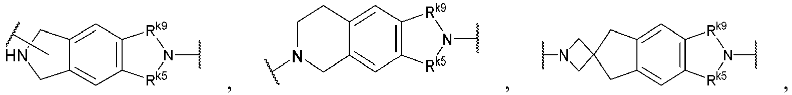 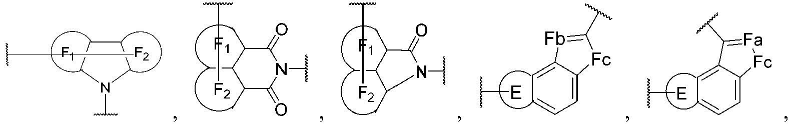 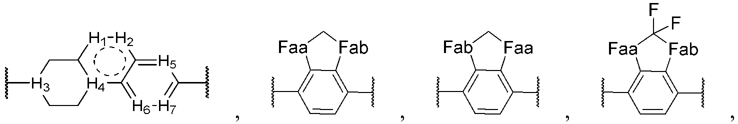 or 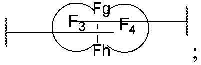 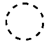 represents that the ring is an aromatic ring or a non-aromatic ring;
Fa is selected from N, CH or CR^{k1};
Fb is selected from N, CH or CR^{k1};
Fc is selected from O, S, NH, N(CH₃) or NR^{k7a};
Fd is selected from N, CH or CR^{k1};
Fg is selected from N or C;
Fh is selected from N or C;
Faa is selected from a bond, O, or CH₂;
Fab is selected from O or CH₂;
H₁ is selected from N, NH, CH, CH₂, CHR^{k1}, NR^{k1}, CR^{k1}, C(=O), or C(R^{k1})₂;
H₂ is selected from a bond, O, N, NH, CH, CH₂, CHR^{k1}, NR^{k1}, CR^{k1} or C(R^{k1})₂;
H₃ is selected from N or CH;
H₄ is selected from C, N or CH;
H₅, H₆ and H₇ are each independently selected from N, C, CH or CR^{k1}, and H₅, H₆ and H₇ contain at most 2 N;
ring E is selected from phenyl or 5- to 6-membered heteroaryl, wherein the ring E is optionally substituted with 1 to 3 R^{k1};
ring F₁, ring F₂, ring F₃ and ring F₄ are each independently selected from phenyl or 5- to 6-membered heteroaryl, wherein the ring F₁, ring F₂, ring F₃ and ring F₄ are optionally substituted with 1 to 2 R^{k1};
Q is selected from a bond, CH₂, NH, N(CH₃), O, S, C(=O), NHC(=O), C(=O)NH, N(CH₃)C(=O), or C(=O)N(CH₃);
R^{k1} and R^{k3} are each independently selected from H, deuterium, F, Cl, Br, I, OH, =O, NH₂, CF₃, CN, COOH, CONH2, methyl, ethyl, isopropyl, methoxy, ethoxy, isopropoxy, or cyclopropyl, wherein the methyl, ethyl, isopropyl, methoxy, ethoxy, isopropoxy or cyclopropyl is optionally substituted with 1 to 4 substituents selected from deuterium, F, Cl, Br, I, OH, or NH₂;
each R^{k5} is independently selected from 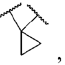 C(CH₃)₂, C(=O), CH₂, CH₂CH₂, S(=O)₂ 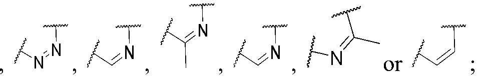
each R^{k6} is independently selected from C(=O), CH, S(=O), S(=O)₂, CH₂ or N;
each R^{k7} is independently selected from 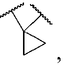 C(CH₃)₂, CH₂, O or NR^{k7a};
R^{k7a} is selected from H, methyl, ethyl, propyl, isopropyl, vinyl, propenyl, allyl, ethynyl, propynyl, propargyl, cyclopropyl, cyclobutyl, cyclopentyl, cyclohexyl, azetidinyl, pyrrolidyl, piperidyl, oxetanyl, tetrahydrofuryl, or tetrahydropyranyl, wherein the methyl, ethyl, propyl, isopropyl, cyclopropyl, cyclobutyl, cyclopentyl, cyclohexyl, azetidinyl, pyrrolidyl, piperidyl, oxetanyl, tetrahydrofuryl or tetrahydropyranyl is optionally substituted with 1 to 4 substituents selected from deuterium, F, Cl, Br, I, OH, CN, CF₃, C₁₋₄ alkyl, C₁₋₄ alkoxy, C₂₋₄ alkenyl, C₂₋₄ alkynyl, or C₃₋₆ cycloalkyl;
each R^{k9} is independently selected from a bond, 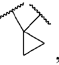 C(CH₃)₂, C(=O), CH₂, CH₂CH₂ or S(=O)₂;
R^{L2} and R^{z} are each independently selected from deuterium, F, Cl, Br, I, OH, =O, CF₃, SF₅, CN, NH₂, NO₂, COOH, CONH₂, N(CH₃)₂, NHCH₃, methyl, ethyl, vinyl, ethynyl, methoxy, ethoxy, methylthio, cyclopropyl, cyclobutyl, cyclopentyl, cyclohexyl, -CH₂-cyclopropyl, -CH₂-cyclobutyl, -CH₂-cyclopentyl, or -CH₂-cyclohexyl, wherein the methyl, ethyl, vinyl, ethynyl, methoxy, ethoxy, methylthio, cyclopropyl, cyclobutyl, cyclopentyl, or cyclohexyl is optionally substituted with 1 to 4 substituents selected from deuterium, F, Cl, Br, I, OH, CN, C₁₋₄ alkyl, or C₁₋₄ alkoxy;
each p2 is independently selected from 0, 1, 2 or 3.

4. The compound or the stereoisomer, the racemate, the tautomer, the deuterated substance, the solvate, the prodrug, the metabolite, the pharmaceutically acceptable salt or the cocrystal thereof according to claim 3, **characterised in that**
each ring E is independently selected from phenyl, pyridyl, pyridazinyl, pyrazinyl, pyrimidyl, pyrrolyl, pyrazolyl, imidazolyl, thiazolyl, furyl, thienyl or oxazolyl, wherein the ring E is optionally substituted with 1 to 3 R^{k1};
ring F₁ and ring F₂ are each independently selected from phenyl, pyridyl, thiazolyl, furyl, thienyl or oxazolyl, wherein the ring F₁ and ring F₂ are optionally substituted with 1 to 2 R^{k1};
ring F₃ and ring F₄ are each independently selected from phenyl, pyridyl, pyridazinyl, pyrazinyl, pyrimidyl, triazinyl, pyrrolyl, pyrazolyl, imidazolyl, thiazolyl, furyl, thienyl or oxazolyl, wherein the ring F₃ and ring F₄ are optionally substituted with 1 to 2 R^{k1};
B₁ is selected from phenyl, thiazolyl, furyl, thienyl, oxazolyl, pyrazolyl, pyridyl, pyrimidyl, pyrazinyl, or pyridazinyl, wherein the B₁ is optionally substituted with 1 to 4 R^{b1};
R^{b2} is selected from 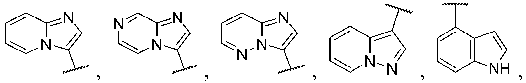 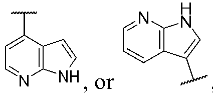 wherein the R^{b2} is optionally substituted with 1 to 4 R^{b2a};
R^{b1} and R^{b2a} are each independently selected from deuterium, F, Cl, Br, I, OH, CN, NH₂, NHCH₃, N(CH₃)₂ or one of the following groups optionally substituted with 1 to 4 R^{z}: methyl, ethyl, propyl, isopropyl, ethynyl, -CH₂-ethynyl, methoxy, ethoxy, cyclopropyl, cyclobutyl, -O-cyclopropyl, -O-cyclobutyl, -CH₂-cyclopropyl, -CH₂-cyclobutyl, oxetanyl, azetidinyl, tetrahydrofuryl, pyrrolidyl, -CH₂NHCH₃, - CH₂N(CH₃)₂, tetrahydropyranyl, piperidyl, -CH₂-azetidinyl, -CH₂-pyrrolidyl, or - CH₂-piperidyl;
Ak1, Ak2, Ak3, Ak4 and Ak5 are each independently selected from a bond, - O-, -S-, -OCH₂-, -CH₂O-, -OCH₂CH₂-, -CH₂CH₂O-, -C≡C-, -C(CH₃)₂-, -CH₂-, - C(CH₃)₂-, -CH₂CH₂-, -CH₂CH₂CH₂-, -N(CH₃)-, -NH-, -CH₂N(CH₃)-, -CH₂NH-, - NHCH₂-, -CH₂CH₂N(CH₃)-, -CH₂CH₂NH-, -NHCH₂CH₂-, -C(=O)-, - C(=O)CH₂NH-, -CH₂C(=O)NH-, -C(=O)NH- or -NHC(=O)-;
Cy1, Cy2, Cy3 and Cy4 are each independently selected from a bond or one of the following optionally substituted groups: 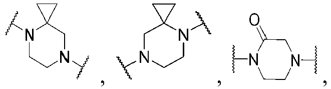 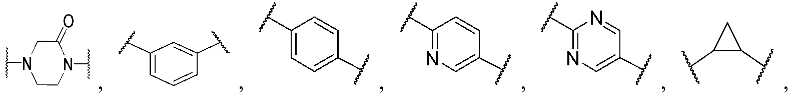 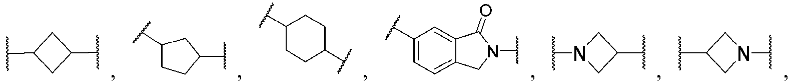 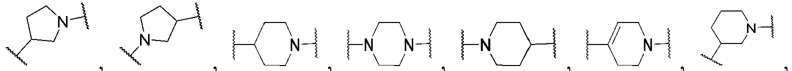 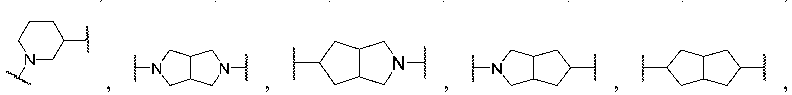 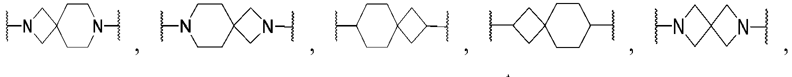 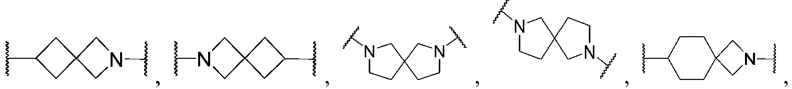 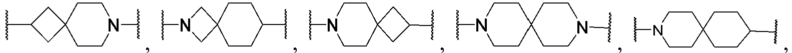 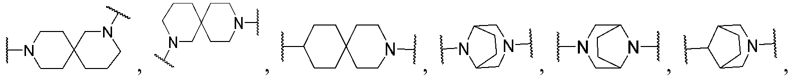 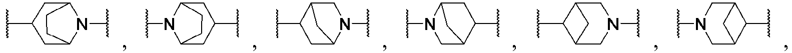 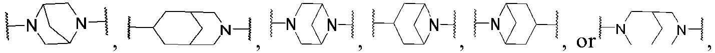 which, when substituted, is substituted with 1 to 4 substituents selected from deuterium, F, CF₃, OH, =O, COOH, CN, NH₂, hydroxymethyl, methyl, methoxy, or cyclopropyl.

5. The compound or the stereoisomer, the racemate, the tautomer, the deuterated substance, the solvate, the prodrug, the metabolite, the pharmaceutically acceptable salt or the cocrystal thereof according to claim 4, **characterised in that**
B is selected from 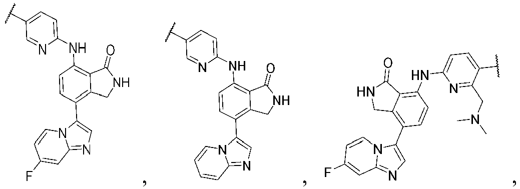 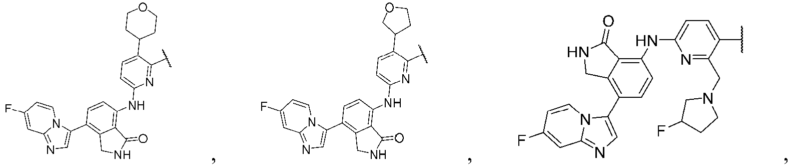 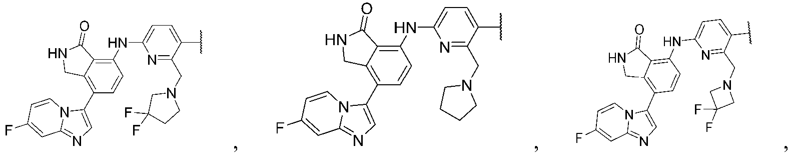 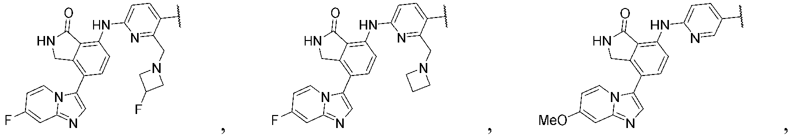 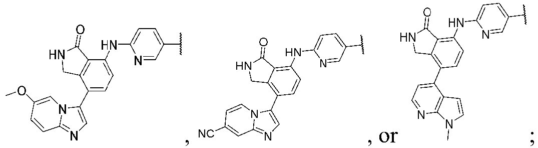
L is selected from a bond, -Ak1-, -NHCH₂-, -Cy1-, -Cy1-CH₂-, -Cy1-C≡C-, - Cy1-Cy2-, -Cy1-CH₂-Cy2-, -CH₂-Cy2-, -Cy1-Cy2-Cy3-, -Cy1-CH₂-Cy2-Cy3-, - Cy1-Cy2-CH₂-Cy3-, -NH-Cy1-, -NH-Cy1-Cy2-, -NH-Cy1-CH₂-Cy2, -Cy1-Ak2-, - Ak1-Cy1-, -Ak1-Cy1-Ak2-, -Ak1-Cy2-Ak2-Cy3-, or -Ak1-Cy2-Cy3-;
Cy1, Cy2 and Cy3 are each independently selected from one of the following optionally substituted groups: 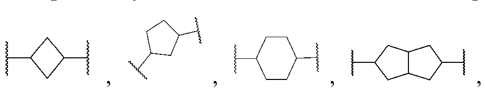 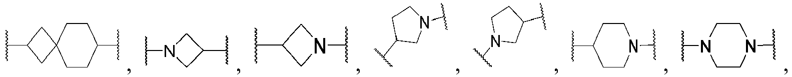 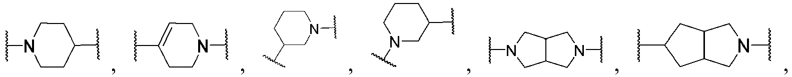 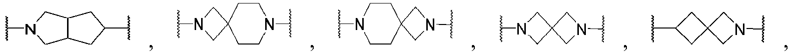 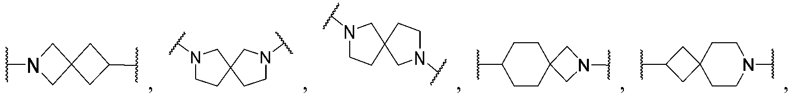 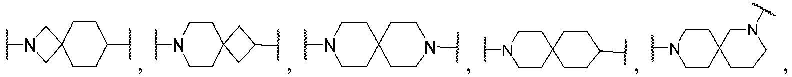 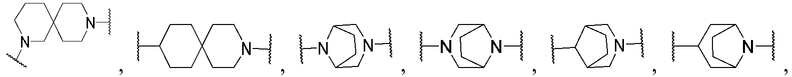 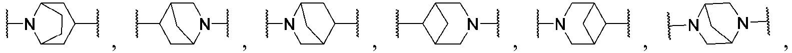 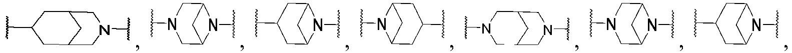 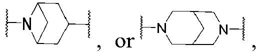 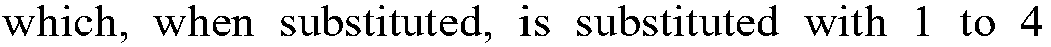 substituents selected from deuterium, F, CF₃, OH, =O, COOH, CN, NH₂, hydroxymethyl, methyl, methoxy, or cyclopropyl.

6. The compound or the stereoisomer, the racemate, the tautomer, the deuterated substance, the solvate, the prodrug, the metabolite, the pharmaceutically acceptable salt or the cocrystal thereof according to claim 1 or 5, **characterised in that**
L is selected from one of the structures shown in Table L-1 or Table L-2;
K is selected from one of the structural fragments shown in Table K-1 or Table K-2.

7. The compound or the stereoisomer, the racemate, the tautomer, the deuterated substance, the solvate, the prodrug, the metabolite, the pharmaceutically acceptable salt or the cocrystal thereof according to claim 1, **characterised in that** the compound as shown in general formula (I) is general formula (Ia), 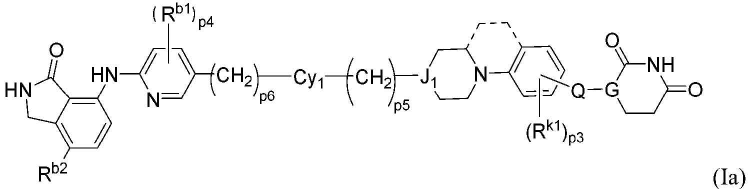
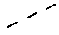 is a single bond 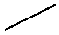 or is absent;
J₁ is selected from N or CH;
Cy1 is selected from one of the following optionally substituted groups: 4- to 7-membered nitrogen-containing mono-heterocyclyl, 4- to 12-membered nitrogen-containing fused-heterocyclyl, 5- to 13-membered nitrogen-containing spiro-heterocyclyl, 7- to 12-membered nitrogen-containing bridged-heterocyclyl, C₃₋₇ monocycloalkyl, C₄₋₇ monocycloalkenyl, C₄₋₁₂ fused cycloalkyl, C₅₋₁₃ spirocycloalkyl, C₅₋₁₂ bridged cycloalkyl, 5- to 10-membered heteroaryl or C₆₋₁₀ aryl, preferably one of the following optionally substituted groups: 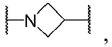 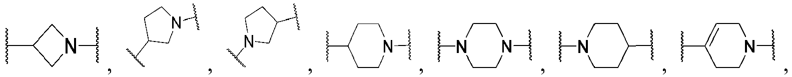 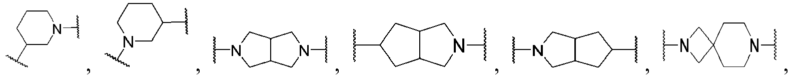 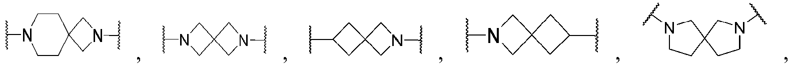 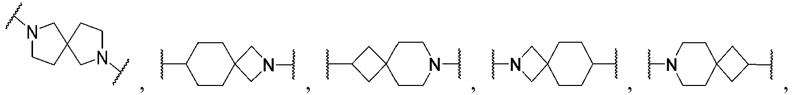 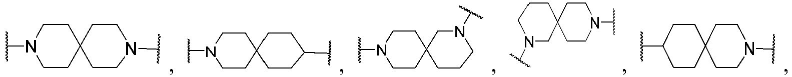 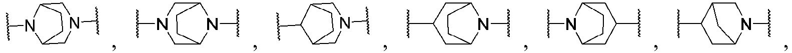 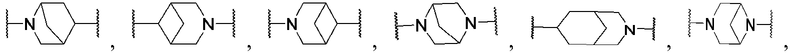 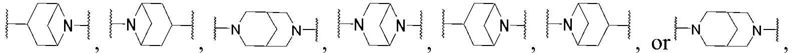 which, when substituted, is substituted with 1 to 4 substituents selected from deuterium, F, CF₃, OH, =O, COOH, CN, NH₂, hydroxymethyl, methyl, methoxy, or cyclopropyl;
G is selected from N or CH;
Q is selected from C(=O)NH or a bond;
R^{b2} is selected from 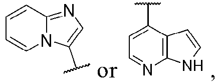 wherein the R^{b2} is optionally substituted with 1 to 4 R^{b2a};
each R^{b2a} is independently selected from deuterium, F, Cl, Br, OH, CN, NH₂, NHCH₃, N(CH₃)₂, methyl, ethyl, methoxy, or CF₃;
each R^{b1} is independently selected from deuterium, F, Cl, Br, I, OH, CN, NH₂, NHCH₃, N(CH₃)₂, methyl, ethyl, -CH₂NHCH₃, -CH₂N(CH₃)₂, tetrahydrofuryl, tetrahydropyranyl, piperidyl, -CH₂-azetidinyl, -CH₂-pyrrolidyl, or -CH₂-piperidyl, wherein the methyl, ethyl, tetrahydrofuryl, tetrahydropyranyl, piperidyl, azetidinyl, pyrrolidyl, or piperidyl is optionally substituted with 1 to 4 substituents selected from deuterium, F, CF₃, OH, =O, COOH, CN, or NH₂;
each R^{k1} is independently selected from H, deuterium, F, Cl, Br, NH₂, CF₃, CN, methyl, or ethyl;
p3 is selected from 0, 1, 2 or 3;
p4 is selected from 0, 1, 2 or 3;
p5 is selected from 0 or 1;
p6 is selected from 0 or 1.

8. The compound or the stereoisomer, the racemate, the tautomer, the deuterated substance, the solvate, the prodrug, the metabolite, the pharmaceutically acceptable salt or the cocrystal thereof according to claim 1, **characterised in that** the compound has a structure selected from one of the structures shown in Table E.

9. A pharmaceutical composition, **characterised by** comprising the compound or the stereoisomer, the racemate, the tautomer, the deuterated substance, the solvate, the prodrug, the metabolite, the pharmaceutically acceptable salt or the cocrystal thereof according to any one of claims 1-8, and a pharmaceutically acceptable carrier, wherein preferably, the pharmaceutical composition comprises 1-1500 mg of the compound or the stereoisomer, the racemate, the tautomer, the deuterated substance, the solvate, the prodrug, the metabolite, the pharmaceutically acceptable salt or the cocrystal thereof according to any one of claims 1-8.

10. Use of the compound or the stereoisomer, the racemate, the tautomer, the deuterated substance, the solvate, the prodrug, the metabolite, the pharmaceutically acceptable salt or the cocrystal thereof according to any one of claims 1-8, or the pharmaceutical composition according to claim 9 in the preparation of a medicament for the treatment of a disease related to HPK1 activity or expression.

11. Use of the compound or the stereoisomer, the racemate, the tautomer, the deuterated substance, the solvate, the prodrug, the metabolite, the pharmaceutically acceptable salt or the cocrystal thereof according to any one of claims 1-8, or the pharmaceutical composition according to claim 9 in the preparation of a medicament for the treatment of a disease related to the inhibition or degradation of HPK1.

12. The use according to claim 11, **characterised in that** the disease is cancer, preferably a solid tumour.

13. A method for treating a disease in a mammal, **characterised by** comprising administering to a subject a therapeutically effective amount of the compound or the stereoisomer, the racemate, the tautomer, the deuterated substance, the solvate, the prodrug, the metabolite, the pharmaceutically acceptable salt or the cocrystal thereof according to any one of claims 1-8, or the pharmaceutical composition according to claim 9, wherein the therapeutically effective amount is preferably 1-1500 mg, and the disease is preferably cancer, more preferably a solid tumour.
